(19) Europäisches Patentamt / European Patent Office / Office européen des brevets

(11) **EP 4 692 088 A1**

(12) **EUROPEAN PATENT APPLICATION**
published in accordance with Art. 153(4) EPC


(43) Date of publication:
**11.02.2026 Bulletin 2026/07**

(21) Application number: **24777897.0**

(22) Date of filing: **22.03.2024**

(51) International Patent Classification (IPC):
***C07D 487/04*** *(2006.01)* ***A61K 31/519*** *(2006.01)*
***A61P 35/00*** *(2006.01)*

(52) Cooperative Patent Classification (CPC):
**A61K 31/4545; A61K 31/496; A61K 31/498; A61K 31/4995; A61K 31/506; A61K 31/519; A61K 31/5377; A61P 35/00; C07D 405/14; C07D 471/04; C07D 487/04; C07D 495/04; C07D 498/04; C07D 513/04; C07D 519/00**

(86) International application number:
**PCT/CN2024/083215**

(87) International publication number:
**WO 2024/199108 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.03.2023 CN 202310301834**
**09.08.2023 CN 202311004828**
**18.08.2023 CN 202311050091**
**14.03.2024 CN 202410295061**



(71) Applicant: **SHANGHAI JEYOU PHARMACEUTICAL CO., LTD.**
**Shanghai 201315 (CN)**

(72) Inventors:
- **BIE, Pingyan**
**Shanghai 201315 (CN)**
- **ZHOU, Mingwei**
**Shanghai 201315 (CN)**
- **PENG, Jianbiao**
**Shanghai 201315 (CN)**

(74) Representative: **Dehns**
**10 Old Bailey**
**London EC4M 7NG (GB)**


(54) **COMPOUND ACTING AS WRN HELICASE INHIBITOR**


(57) Disclosed are a compound as shown in formula (I), and a pharmaceutically acceptable salt thereof, and specifically disclosed is a compound acting as a WRN helicase inhibitor.

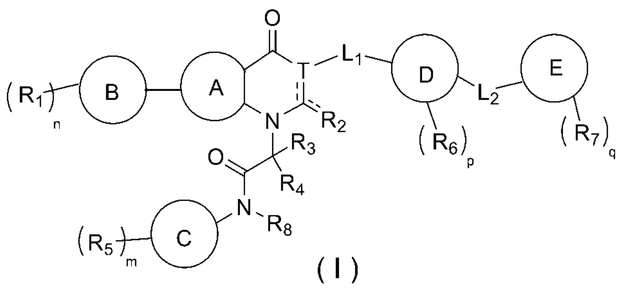

(I)


EP 4 692 088 A1

## Description

The present invention claims the right of the following priorities:

Application No. CN2023103018345, filing date: March 24, 2023;
Application No. CN202311004828X, filing date: August 09, 2023;
Application No. CN2023110500915, filing date: August 18, 2023;
Application No. CN2024102950619, filing date: March 14, 2024.

## TECHNICAL FIELD

The present disclosure relates to a compound of formula (I) and a pharmaceutically acceptable salt thereof. Specifically, the present disclosure relates to a class of compounds as WRN helicase inhibitors.

## BACKGROUND

The entire human genome contains numerous short tandem repeat sequence regions. These repetitive DNA regions are known as "microsatellites" and are prone to slippage and errors during replication, thus highly reliant on the MMR (mismatch repair) system for repair. When the MMR (mismatch repair) system becomes dysfunctional, it leads to dMMR (deficient mismatch repair), resulting in the inability to recognize and repair microsatellite replication errors. This causes MSI (microsatellite instability), which may lead to frameshift mutations, thereby causing abnormalities in tumor-related genes and further inducing the occurrence and development of cancer. In 2017, Immune Checkpoint Inhibitors (ICIs) were approved for the treatment of Microsatellite Instability-High/Deficient Mismatch Repair (MSI-H/dMMR) tumors, making MSI-H/dMMR the first "pan-cancer" tumor biomarker. Microsatellite instability-high (MSI-H) cancer cells rely on WRN (Werner syndrome RecQ helicase) helicase activity. Inhibition of WRN can induce DNA double-strand breaks, activate the DNA damage response, and induce apoptosis and cell cycle arrest. Common treatment approaches for dMMR/MSI-H cancer patients include targeted therapy, chemotherapy, and immunotherapy. The clinical efficacy of targeted therapy and chemotherapy is limited by drug resistance and toxicity, and approximately half of the patients receiving immunotherapy do not respond positively to immune checkpoint inhibitors. 45-60% of MSI-H cancer patients do not respond to immunotherapy, and the primary and secondary resistance issues in targeted therapy, chemotherapy, and immunotherapy urgently need to be resolved. In 2019, the Broad Institute of Harvard and MIT analyzed the Achilles and drive databases to evaluate the dependency of each cell line on different targets. They discovered that the activity of the RecQ DNA helicase WRN is essential both *in vivo* and *in vitro* for dMMR/MSI-H cell lines, whereas MSS cells do not depend on WRN for survival. In MSI-H models, knockout of WRN induces double-strand DNA breaks and selectively promotes apoptosis and cell cycle arrest. This tumor-suppressive mechanism differs from targeted drugs (which inhibit cancer cell-specific oncogenic alterations) and immunotherapies (which inhibit immune evasion and tolerance). In 2021, Dr. Mathew J Garnett's research group at the Wellcome Sanger Institute demonstrated, using PDX models, that WRN inhibitors could serve as second- or third-line monotherapy for dMMR patients. In dMMR tumors, tumor mutability is negatively correlated with response to immune checkpoint blockade, whereas WRN sensitivity is independent of mutation load. Due to the different modes of action, combination therapy of checkpoint inhibitors, chemotherapy, or targeted therapy with WRN inhibitors may suppress cross-resistance and promote tumor eradication. Furthermore, since the loss of DNA repair modulates the structure of neoantigens and increases mutational burden, leading to enhanced immune responses, WRN inhibition may also synergize with immunotherapy. Thus, WRN can serve as a critical target for monotherapy or combination therapy with targeted agents, chemotherapy, or immunotherapy in dMMR/MSI-H tumors.

MSI-H tumors can occur in multiple sites, with the highest incidence observed in endometrial cancer (31%), colon adenocarcinoma (20%), and gastric cancer (19%). Approximately 325,000 new cases of MSI-H tumors are diagnosed annually in the United States, and about 300,000 in China, indicating substantial market value for drug development. In mismatch repair-proficient (MSS) tumors, loss (or reduction) of WRN expression does not affect tumor cell growth. In mismatch repair-deficient (MSI-H) tumors, the concurrent loss (or reduction) of WRN expression can lead to increased accumulation of DNA double-strand breaks in cells, cell cycle arrest at the G1 or G2/M phase, and ultimately tumor cell death. This is the so-called synthetic lethality effect. The development of WRN helicase inhibitors is expected to be one of the effective treatment approaches for MSI-H cancers.

## SUMMARY

In one aspect of the present disclosure, the present disclosure provides a compound of formula (I), an optical isomer thereof, or a pharmaceutically acceptable salt thereof,

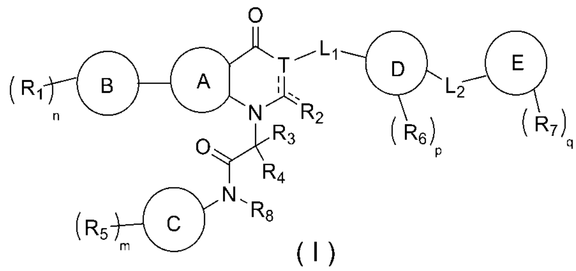

( I )

wherein

ring A is selected from the group consisting of phenyl and 5- to 10-membered heteroaryl, wherein the phenyl and 5- to 10-membered heteroaryl are optionally substituted with 1, 2, or 3 $R_a$;
ring B is selected from the group consisting of $C_{3-20}$ cycloalkyl, 3- to 20-membered heterocycloalkyl, $C_{4-20}$ cycloalkenyl, 4- to 20-membered heterocycloalkenyl, $C_{6-20}$ aryl, and 5-to 20-membered heteroaryl;
ring C is selected from the group consisting of $C_{6-20}$ aryl and 5- to 20-membered heteroaryl;
ring D is selected from the group consisting of $C_{3-20}$ cycloalkyl, 3- to 20-membered heterocycloalkyl, 4- to 20-membered heterocycloalkenyl, $C_{6-20}$ aryl, and 5- to 20-membered heteroaryl;
ring E is selected from the group consisting of $C_{3-20}$ cycloalkyl, 5- to 20-membered heterocycloalkyl, $C_{6-20}$ aryl, and 5- to 20-membered heteroaryl;
$L_1$ is selected from the group consisting of a single bond, $-N(R_{b1})-$, $-N(R_{b1})C(=O)-$, - O-, -S-, $-(CR_{b2}R_{b3})_t-$, -C(=O)-, -C(=S)-, -S(=O)-, $-S(=O)_2-$, and

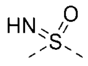 ;

$L_2$ is selected from the group consisting of a single bond, $-N(R_{b1})-$, $-N(R_{b1})C(=O)-$, - O-, -S-, $-(CR_{b2}R_{b3})_t-$, -C(=O)-, -C(=S)-, -S(=O)-, $-S(=O)_2-$, and

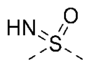 ;

each $R_1$ is independently selected from the group consisting of H, F, Cl, Br, OH, $N(R_{b4})_2$, CN, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;
alternatively, two $R_1$ are connected together to form a $C_{3-20}$ cycloalkyl, 3- to 20-membered heterocycloalkyl, $C_{6-20}$ aryl, or 5- to 20-membered heteroaryl group;

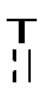 is 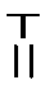 , and 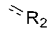 is 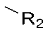;

alternatively, 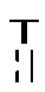 is 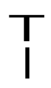 , and 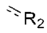 is 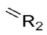;

when 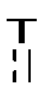 is 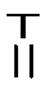 , T is C;

when 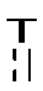 is 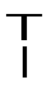 , T is selected from the group consisting of N and CH;

when 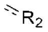 is 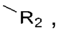 , $R_2$ is selected from the group consisting of H, F, Cl, Br, OH, $N(R_{b4})_2$, CN, $SF_5$, CHO, COOH, $C_{1-20}$ alkyl, $C_{1-20}$ heteroalkyl, $C_{3-20}$ cycloalkyl, $C_{2-20}$ alkenyl, $C_{2-20}$ alkynyl, 3- to 20-membered heterocycloalkyl, $C_{4-20}$ cycloalkenyl, 4- to 20-membered heterocycloalkenyl, $C_{6-20}$ aryl, and 5- to 20-membered heteroaryl, wherein the $C_{1-20}$ alkyl, $C_{1-20}$ heteroalkyl, $C_{3-20}$ cycloalkyl, $C_{2-20}$ alkenyl, $C_{2-20}$ alkynyl, 3- to 20-membered heterocycloalkyl, $C_{4-20}$ cycloalkenyl, 4- to 20-membered heterocycloalkenyl, $C_{6-20}$ aryl, and 5-to 20-membered heteroaryl are optionally substituted with 1, 2, or 3 R;

when 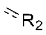 is 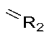, $R_2$ is selected from the group consisting of O and S;
$R_3$ and $R_4$ are each independently selected from the group consisting of H, F, Cl, Br, OH, $N(R_{b4})_2$, CN, $C_{1-20}$ alkyl, $C_{1-20}$ heteroalkyl, $C_{3-20}$ cycloalkyl, and 3- to 20-membered heterocycloalkyl, wherein the $C_{1-20}$ alkyl, $C_{1-20}$ heteroalkyl, $C_{3-20}$ cycloalkyl, and 3- to 20-membered heterocycloalkyl are optionally substituted with 1, 2, or 3 R;
alternatively, $R_3$ and $R_4$ are connected together to form a $C_{3-20}$ cycloalkyl or 3- to 20-membered heterocycloalkyl

group, wherein the $C_{3-20}$ cycloalkyl or 3- to 20-membered heterocycloalkyl is optionally substituted with 1, 2, or 3 R;

each $R_5$ is independently selected from the group consisting of H, F, Cl, Br, OH, $N(R_{b4})_2$, $SF_5$, CN, CHO, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;

alternatively, two $R_5$ are connected together to form a $C_{3-20}$ cycloalkyl, 3- to 20-membered heterocycloalkyl, $C_{4-20}$ cycloalkenyl, 4- to 20-membered heterocycloalkenyl, $C_{6-20}$ aryl, or 5- to 20-membered heteroaryl group, wherein the $C_{3-20}$ cycloalkyl, 3- to 20-membered heterocycloalkyl, $C_{4-20}$ cycloalkenyl, 4- to 20-membered heterocycloalkenyl, $C_{6-20}$ aryl, and 5-to 20-membered heteroaryl are optionally substituted with 1, 2, or 3 R;

each $R_6$ is independently selected from the group consisting of H, F, Cl, Br, OH, $N(R_{b4})_2$, CN, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;

alternatively, two $R_6$ are connected together to form a $C_{3-20}$ cycloalkyl, 3- to 20-membered heterocycloalkyl, $C_{6-20}$ aryl, or 5- to 20-membered heteroaryl group, wherein the $C_{3-20}$ cycloalkyl, 3- to 20-membered heterocycloalkyl, $C_{6-20}$ aryl, and 5- to 20-membered heteroaryl are optionally substituted with 1, 2, or 3 R;

each $R_7$ is independently selected from the group consisting of H, F, Cl, Br, OH, $N(R_{b4})_2$, CN, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;

$R_8$ is selected from the group consisting of H, $C_{1-20}$ alkyl, $C_{1-20}$ heteroalkyl, $C_{3-20}$ cycloalkyl, and 3- to 20-membered heterocycloalkyl, wherein the $C_{1-20}$ alkyl, $C_{1-20}$ heteroalkyl, $C_{3-20}$ cycloalkyl, and 3- to 20-membered heterocycloalkyl are optionally substituted with 1, 2, or 3 R;

each $R_a$ is independently selected from the group consisting of H, F, Cl, Br, OH, $NH_2$, CN, $C_{1-20}$ alkyl, $C_{1-20}$ heteroalkyl, $C_{3-20}$ cycloalkyl, and 3- to 20-membered heterocycloalkyl, wherein the $C_{1-20}$ alkyl, $C_{1-20}$ heteroalkyl, $C_{3-20}$ cycloalkyl, and 3- to 20-membered heterocycloalkyl are optionally substituted with 1, 2, or 3 R;

$R_{b1}$ is selected from the group consisting of H, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;

$R_{b2}$ and $R_{b3}$ are each independently selected from the group consisting of H, F, Cl, Br, OH, $NH_2$, CN, $C_{1-20}$ alkyl, $C_{1-20}$ heteroalkyl, $C_{3-20}$ cycloalkyl, and 3- to 20-membered heterocycloalkyl, wherein the $C_{1-20}$ alkyl, $C_{1-20}$ heteroalkyl, $C_{3-20}$ cycloalkyl, and 3- to 20-membered heterocycloalkyl are optionally substituted with 1, 2, or 3 R;

alternatively, $R_{b2}$ and $R_{b3}$ are connected together to form a $C_{3-20}$ cycloalkyl or 3- to 20-membered heterocycloalkyl group;

each $R_{b4}$ is independently selected from the group consisting of H, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;

m, n, p, q, and t are each independently selected from the group consisting of 0, 1, 2, and 3;

each R is independently selected from the group consisting of H, F, Cl, Br, I, OH, $NH_2$, CN, $SF_5$, CHO, COOH,

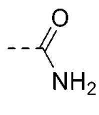

, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R';

R' is selected from the group consisting of H, F, Cl, Br, I, OH, $NH_2$, $CH_3$, $CF_3$, $C_2H_5$, CN, $SF_5$, CHO, COOH, and

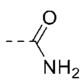;

the $C_{1-6}$ heteroalkyl, $C_{1-20}$ heteroalkyl, 3- to 20-membered heterocycloalkyl, 4- to 20-membered heterocycloalkenyl, 5- to 20-membered heteroaryl, or 5- to 10-membered heteroaryl contains 1, 2, or 3 heteroatoms or heteroatom groups independently selected from the group consisting of -O-, -NH-, -S-, -C(=O)-, -C(=O)O-, -S(=O)-, $-S(=O)_2-$, and N.

In some embodiments of the present disclosure, ring A is selected from the group consisting of phenyl and 5- to 6-membered heteroaryl, wherein the phenyl and 5- to 6-membered heteroaryl are optionally substituted with 1, 2, or 3 $R_a$;

ring B is selected from the group consisting of $C_{3-6}$ cycloalkyl, 5- to 6-membered heterocycloalkyl, $C_{5-6}$ cycloalkenyl, 5- to 6-membered heterocycloalkenyl, $C_{6-10}$ aryl, and 5- to 10-membered heteroaryl;
ring C is selected from the group consisting of $C_{6-10}$ aryl and 5- to 10-membered heteroaryl;
ring D is selected from the group consisting of $C_{3-6}$ cycloalkyl, 5- to 6-membered heterocycloalkyl, 5- to 6-membered heterocycloalkenyl, $C_{6-10}$ aryl, and 5- to 10-membered heteroaryl;
ring E is selected from the group consisting of $C_{3-6}$ cycloalkyl, 5- to 6-membered heterocycloalkyl, $C_{6-10}$ aryl, and 5- to 10-membered heteroaryl;
$L_1$ is selected from the group consisting of a single bond, $-N(R_{b1})-$, -O-, -S-, $-C(R_{b2})_2-$, -C(=O)-, -C(=S)-, -S(=O)-, $-S(=O)_2-$, and

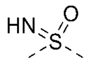;

$L_2$ is selected from the group consisting of a single bond, $-N(R_{b1})-$, -O-, -S-, $-C(R_{b2})_2-$, -C(=O)-, -C(=S)-, -S(=O)-, $-S(=O)_2-$, and

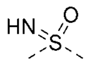;

each $R_a$ is independently selected from the group consisting of H, F, Cl, Br, OH, $NH_2$, CN, $C_{1-6}$ alkyl, $C_{1-6}$ heteroalkyl, $C_{3-6}$ cycloalkyl, and 3- to 6-membered heterocycloalkyl, wherein the $C_{1-6}$ alkyl, $C_{1-6}$ heteroalkyl, $C_{3-6}$ cycloalkyl, and 3- to 6-membered heterocycloalkyl are optionally substituted with 1, 2, or 3 R;
$R_{b1}$ is selected from the group consisting of H, $C_{1-6}$ alkyl, $C_{1-6}$ heteroalkyl, $C_{3-6}$ cycloalkyl, and 3- to 6-membered heterocycloalkyl, wherein the $C_{1-6}$ alkyl, $C_{1-6}$ heteroalkyl, $C_{3-6}$ cycloalkyl, and 3- to 6-membered heterocycloalkyl are optionally substituted with 1, 2, or 3 R;
each $R_{b2}$ is independently selected from the group consisting of H, F, Cl, Br, OH, $NH_2$, CN, $C_{1-6}$ alkyl, $C_{1-6}$ heteroalkyl, $C_{3-6}$ cycloalkyl, and 3- to 6-membered heterocycloalkyl, wherein the $C_{1-6}$ alkyl, $C_{1-6}$ heteroalkyl, $C_{3-6}$ cycloalkyl, and 3- to 6-membered heterocycloalkyl are optionally substituted with 1, 2, or 3 R;
each $R_1$ is independently selected from the group consisting of H, F, Cl, Br, OH, $NH_2$, CN, $C_{1-6}$ alkyl, $C_{1-6}$ heteroalkyl, $C_{3-6}$ cycloalkyl, and 3- to 6-membered heterocycloalkyl, wherein the $C_{1-6}$ alkyl, $C_{1-6}$ heteroalkyl, $C_{3-6}$ cycloalkyl, and 3- to 6-membered heterocycloalkyl are optionally substituted with 1, 2, or 3 R;
alternatively, two $R_1$ are connected together to form a $C_{4-6}$ cycloalkyl or 4- to 6-membered heterocycloalkyl group;

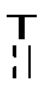 is 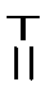, and 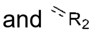 is 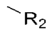;

alternatively, 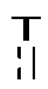 is 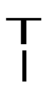, and 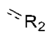 is 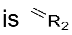;

when 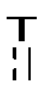 is 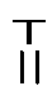, T is C;

when 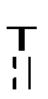 is 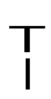, T is selected from the group consisting of N and CH;

when 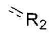 is 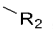, $R_2$ is selected from the group consisting of H, F, Cl, Br, OH, $NH_2$, CN, $C_{1-6}$ alkyl, $C_{1-6}$ heteroalkyl, $C_{3-6}$ cycloalkyl, and 3- to 6-membered heterocycloalkyl, wherein the $C_{1-6}$ alkyl, $C_{1-6}$ heteroalkyl, $C_{3-6}$ cycloalkyl, and 3- to 6-membered heterocycloalkyl are optionally substituted with 1, 2, or 3 R;

when 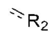 is 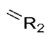, $R_2$ is selected from the group consisting of O and S;
$R_3$ and $R_4$ are each independently selected from the group consisting of H, F, Cl, Br, OH, $NH_2$, CN, $C_{1-6}$ alkyl, $C_{1-6}$ heteroalkyl, $C_{3-6}$ cycloalkyl, and 3- to 6-membered heterocycloalkyl, wherein the $C_{1-6}$ alkyl, $C_{1-6}$ heteroalkyl, $C_{3-6}$ cycloalkyl, and 3- to 6-membered heterocycloalkyl are optionally substituted with 1, 2, or 3 R;
alternatively, $R_3$ and $R_4$ are connected together to form a $C_{3-6}$ cycloalkyl or 3- to 6-membered heterocycloalkyl group, wherein the $C_{3-6}$ cycloalkyl or 3- to 6-membered heterocycloalkyl is optionally substituted with 1, 2, or 3 R;
each $R_5$ is independently selected from the group consisting of H, F, Cl, Br, OH, $NH_2$, $SF_5$, CN, HC(=O)-, $C_{1-6}$ alkyl, $C_{1-6}$ heteroalkyl, $C_{3-6}$ cycloalkyl, and 3- to 6-membered heterocycloalkyl, wherein the $C_{1-6}$ alkyl, $C_{1-6}$ heteroalkyl, $C_{3-6}$ cycloalkyl, and 3- to 6-membered heterocycloalkyl are optionally substituted with 1, 2, or 3 R;
alternatively, two $R_5$ are connected together to form a $C_{4-6}$ cycloalkyl or 4- to 6-membered heterocycloalkyl group, wherein the $C_{4-6}$ cycloalkyl and 4- to 6-membered heterocycloalkyl are optionally substituted with 1, 2, or 3 R;
each $R_6$ is independently selected from the group consisting of H, F, Cl, Br, OH, $NH_2$, CN, $C_{1-6}$ alkyl, $C_{1-6}$ heteroalkyl,

$C_{3-6}$ cycloalkyl, and 3- to 6-membered heterocycloalkyl, wherein the $C_{1-6}$ alkyl, $C_{1-6}$ heteroalkyl, $C_{3-6}$ cycloalkyl, and 3- to 6-membered heterocycloalkyl are optionally substituted with 1, 2, or 3 R;

alternatively, two $R_6$ are connected together to form a $C_{4-6}$ cycloalkyl or 4- to 6-membered heterocycloalkyl group, wherein the $C_{4-6}$ cycloalkyl and 4- to 6-membered heterocycloalkyl are optionally substituted with 1, 2, or 3 R;

each $R_7$ is independently selected from the group consisting of H, F, Cl, Br, OH, $NH_2$, CN, $C_{1-6}$ alkyl, $C_{1-6}$ heteroalkyl, $C_{3-6}$ cycloalkyl, and 3- to 6-membered heterocycloalkyl, wherein the $C_{1-6}$ alkyl, $C_{1-6}$ heteroalkyl, $C_{3-6}$ cycloalkyl, and 3- to 6-membered heterocycloalkyl are optionally substituted with 1, 2, or 3 R;

$R_8$ is selected from the group consisting of H, $C_{1-6}$ alkyl, $C_{3-6}$ cycloalkyl, and 3- to 6-membered heterocycloalkyl, wherein the $C_{1-6}$ alkyl, $C_{1-6}$ heteroalkyl, $C_{3-6}$ cycloalkyl, and 3- to 6-membered heterocycloalkyl are optionally substituted with 1, 2, or 3 R;

m, n, p, and q are each independently selected from the group consisting of 0, 1, 2, and 3;

each R is independently selected from the group consisting of H, F, Cl, Br, I, OH, $NH_2$, CN, COOH,

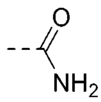,

$C_{1-6}$ alkyl, $C_{1-6}$ alkoxy, $C_{1-6}$ alkylthio, and $C_{1-6}$ alkylamino, wherein the $C_{1-6}$ alkyl, $C_{1-6}$ alkoxy, $C_{1-6}$ alkylthio, or $C_{1-6}$ alkylamino is optionally substituted with 1, 2, or 3 R';

R' is selected from the group consisting of F, Cl, Br, I, OH, $NH_2$, and $CH_3$;

the $C_{1-6}$ heteroalkyl, 5- to 6-membered heterocycloalkyl, 5- to 6-membered heterocycloalkenyl, 5- to 6-membered heteroaryl, or 5- to 10-membered heteroaryl contains 1, 2, or 3 heteroatoms or heteroatom groups independently selected from the group consisting of -O-, -NH-, -S-, -C(=O)-, -C(=O)O-, -S(=O)-, $-S(=O)_2-$, and N.

In some embodiments of the present disclosure, each R is independently selected from the group consisting of H, F, Cl, Br, I, OH, $NH_2$, CN, $SF_5$, CHO, COOH,

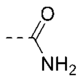,

$C_{1-6}$ alkyl, $C_{1-6}$ alkoxy, $C_{1-6}$ alkylthio, $C_{1-6}$ alkylamino, -$C_{1-6}$ alkyl-$C_{1-6}$ alkoxy, -$C_{1-6}$ alkyl-$C_{1-6}$ alkylthio, - $C_{1-6}$ alkyl-$C_{1-6}$ alkylamino, $C_{1-6}$ alkyl-OH, $C_{1-6}$ alkyl-$NH_2$, -C(=O)-$C_{1-6}$ alkyl, $C_{1-6}$ alkyl-C(=O)-$C_{1-6}$ alkyl, -NH-C(=O)-$C_{1-6}$ alkyl, $C_{1-6}$ alkyl-NH-C(=O)-$C_{1-6}$ alkyl, -NH-$S(=O)_2$-$C_{1-6}$ alkyl, $C_{1-6}$ alkyl-NH-$S(=O)_2$-$C_{1-6}$ alkyl, $C_{3-6}$ cycloalkyl, phenyl, naphthyl, pyridyl, pyrimidinyl, pyridazinyl, pyrazinyl, thienyl, pyrrolyl, pyrazolyl, imidazolyl, triazolyl, piperidinyl, piperazinyl, pyranyl, furanyl, thiazolyl, oxazolyl, and thiopyranyl,

wherein the $C_{1-6}$ alkyl, $C_{1-6}$ alkoxy, $C_{1-6}$ alkylthio, $C_{1-6}$ alkylamino, -$C_{1-6}$ alkyl-$C_{1-6}$ alkoxy, -$C_{1-6}$ alkyl-$C_{1-6}$ alkylthio, -$C_{1-6}$ alkyl-$C_{1-6}$ alkylamino, $C_{1-6}$ alkyl-OH, $C_{1-6}$ alkyl-$NH_2$, - C(=O)-$C_{1-6}$ alkyl, $C_{1-6}$ alkyl-C(=O)-$C_{1-6}$ alkyl, -NH-C(=O)-$C_{1-6}$ alkyl, $C_{1-6}$ alkyl-NH-C(=O)-$C_{1-6}$ alkyl, -NH-$S(=O)_2$-$C_{1-6}$ alkyl, $C_{1-6}$ alkyl-NH-$S(=O)_2$-$C_{1-6}$ alkyl, $C_{3-6}$ cycloalkyl, phenyl, naphthyl, pyridyl, pyrimidinyl, pyridazinyl, pyrazinyl, thienyl, pyrrolyl, pyrazolyl, imidazolyl, triazolyl, piperidinyl, piperazinyl, pyranyl, furanyl, thiazolyl, oxazolyl, and thiopyranyl are optionally substituted with 1, 2, or 3 R', and other variables are as defined in the present disclosure.

In some embodiments of the present disclosure, each R is independently selected from the group consisting of H, F, Cl, Br, I, OH, $NH_2$, CN, $SF_5$, CHO, COOH,

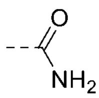,

$CH_3$, $CF_3$, $CHF_2$, $CH_2F$, $CF_2Cl$, $CF_2Br$, $CF_2I$,

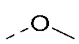, 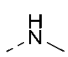, and 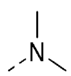,

and other variables are as defined in the present disclosure.

In some embodiments of the present disclosure, R is selected from the group consisting of H, F, Cl, Br, OH, $NH_2$,

COOH,

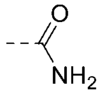,

Me, $CF_3$, $CHF_2$, $CH_2F$,

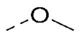, 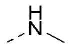,

and

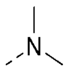,

and other variables are as defined in the present disclosure.

In some embodiments of the present disclosure, ring A is selected from the group consisting of phenyl, pyridyl, pyridazinyl, pyrimidinyl, thienyl, thiazolyl, oxazolyl, isoxazolyl, 1H-imidazolyl, 1H-pyrazolyl, and 1H-pyrrolyl, wherein the phenyl, pyridyl, pyridazinyl, pyrimidinyl, thienyl, thiazolyl, oxazolyl, isoxazolyl, 1H-imidazolyl, 1H-pyrazolyl, and 1H-pyrrolyl are optionally substituted with 1, 2, or 3 $R_a$, and other variables are as defined in the present disclosure.

In some embodiments of the present disclosure, ring A is selected from the group consisting of

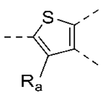, 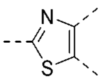, 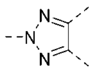, 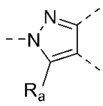, 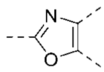, 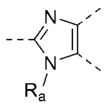, 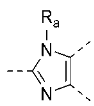, 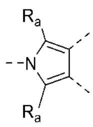,

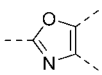, 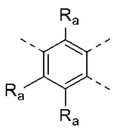, 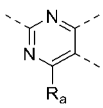, 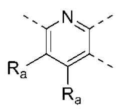, 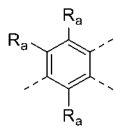, and 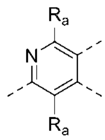,

and other variables are as defined in the present disclosure.

In some embodiments of the present disclosure, each $R_a$ is independently selected from the group consisting of H, F, Cl, Br, OH, $NH_2$, CN, Me,

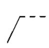, 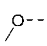, 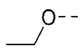, 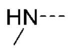, 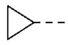,

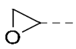, 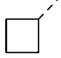, and 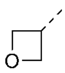,

wherein the Me,

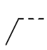, 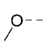, 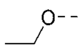, 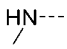, 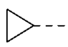, 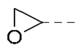, 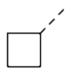,

and

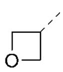

are optionally substituted with 1, 2, or 3 R, and other variables are as defined in the present disclosure.

In some embodiments of the present disclosure, each $R_a$ is independently selected from the group consisting of H, F, Cl, Br, OH, $NH_2$, CN, Me, $CF_3$,

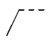, 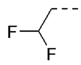, 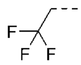, 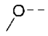,

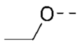, 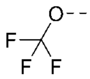, 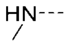, 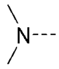, 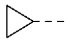, 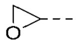, 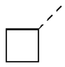, and 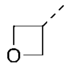,

and other variables are as defined in the present disclosure.

In some embodiments of the present disclosure, ring A is selected from the group consisting of

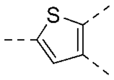, 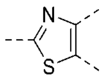, 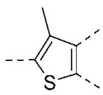, 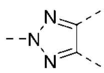, 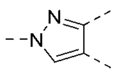, 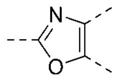, 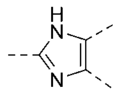,

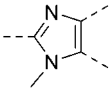, 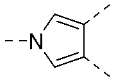, 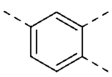, 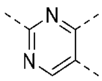, 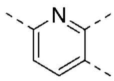, 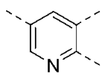, and 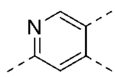,

and other variables are as defined in the present disclosure.

In some embodiments of the present disclosure, when 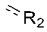 is 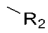, $R_2$ is selected from the group consisting of H, F, Cl, Br, OH, $NH_2$, CN, $SF_5$, CHO, COOH, $C_{1-6}$ alkyl, $C_{1-6}$ alkoxy, $C_{1-6}$ alkylthio, $C_{1-6}$ alkylamino, $C_{2-6}$ alkenyl, $C_{2-6}$ alkynyl, $C_{3-6}$ cycloalkyl, 3- to 6-membered heterocycloalkyl, $C_{4-6}$ cycloalkenyl, 4- to 6-membered heterocycloalkenyl, $C_{6-10}$ aryl, and 5- to 10-membered heteroaryl, wherein the $C_{1-6}$ alkyl, $C_{1-6}$ alkoxy, $C_{1-6}$ alkylthio, $C_{1-6}$ alkylamino, $C_{2-6}$ alkenyl, $C_{2-6}$ alkynyl, $C_{3-6}$ cycloalkyl, 3- to 6-membered heterocycloalkyl, $C_{4-6}$ cycloalkenyl, 4- to 6-membered heterocycloalkenyl, $C_{6-10}$ aryl, and 5- to 10-membered heteroaryl are optionally substituted with 1, 2, or 3 R, and other variables are as defined in the present disclosure.

In some embodiments of the present disclosure, when 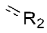 is 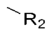, $R_2$ is selected from the group consisting of H, F, Cl, Br, OH, $NH_2$, CN, Me,

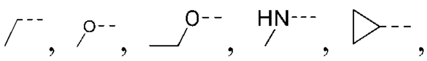,

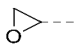, 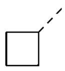, and 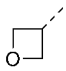,

wherein the Me,

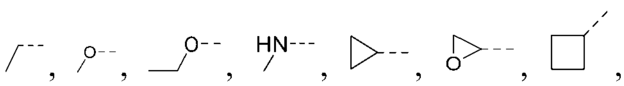,

and

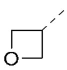

are optionally substituted with 1, 2, or 3 R, and other variables are as defined in the present disclosure.

In some embodiments of the present disclosure, $R_2$ is selected from the group consisting of H, F, Cl, Br, OH, $NH_2$, CN, Me, $CF_3$,

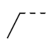, 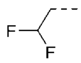, 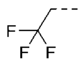, 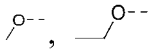, 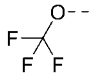,

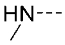, 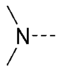, 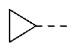, 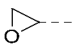, 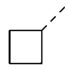, and 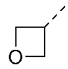,

and other variables are as defined in the present disclosure.

In some embodiments of the present disclosure, the structural moiety

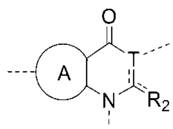

is selected from the group consisting of

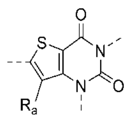, 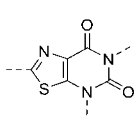, 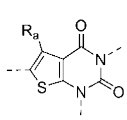, 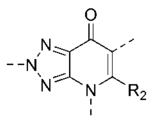,

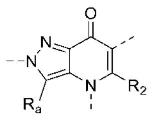, 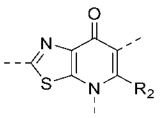, 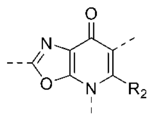, 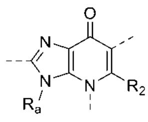, 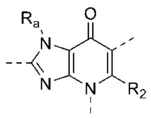, 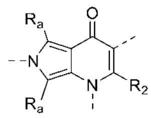, 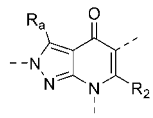,

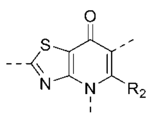, 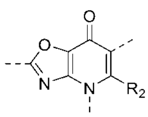, 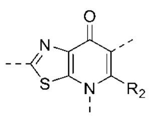, 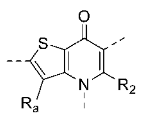, 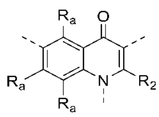, 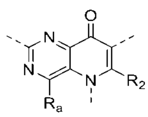, 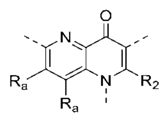,

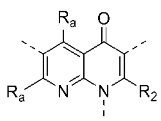, 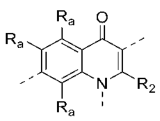, 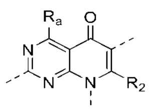, 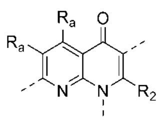, and 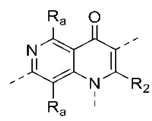,

and other variables are as defined in the present disclosure.

In some embodiments of the present disclosure, the structural moiety

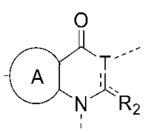

is selected from the group consisting of

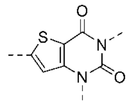, 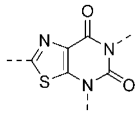, 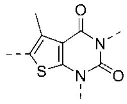, 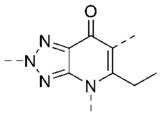,

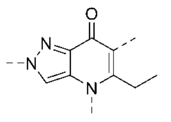, 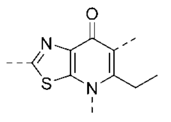, 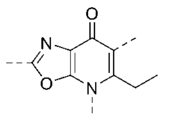, 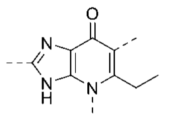, 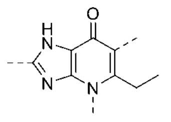, 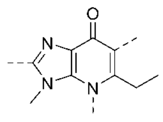,

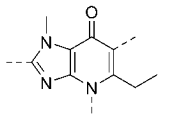, 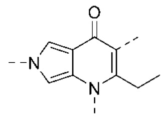, 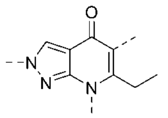, 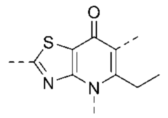, 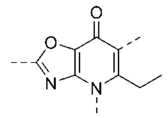, 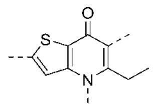,

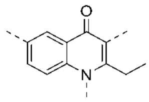, 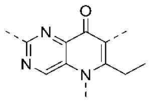, 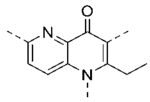, 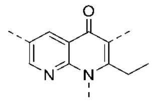, 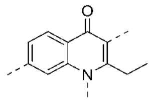, 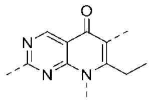, 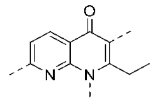,

and

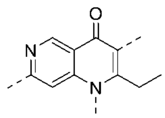,

and other variables are as defined in the present disclosure.

In some embodiments of the present disclosure, each $R_1$ is independently selected from the group consisting of H, F, Cl, Br, OH, $NH_2$, CN, $C_{1-6}$ alkyl, $C_{1-6}$ alkoxy, $C_{1-6}$ alkylthio, $C_{1-6}$ alkylamino, -$C_{1-6}$ alkyl-$C_{1-6}$ alkoxy, -$C_{1-6}$ alkyl-$C_{1-6}$ alkylthio, -$C_{1-6}$ alkyl-$C_{1-6}$ alkylamino, $C_{1-6}$ alkyl-OH, $C_{1-6}$ alkyl-$NH_2$, $C_{1-6}$ alkyl-C(=O)-, $C_{1-6}$ alkyl-C(=O)-$C_{1-6}$ alkyl, $C_{1-6}$ alkyl-O-C(=O)-, $C_{1-6}$ alkyl-O-C(=O)-$C_{1-6}$ alkyl, -NH-C(=O)-$C_{1-6}$ alkyl, $C_{1-6}$ alkyl-NH-C(=O)-$C_{1-6}$ alkyl, $C_{1-6}$ alkyl-$S(=O)_2$-, $C_{1-6}$ alkyl-$S(=O)_2$-$C_{1-6}$ alkyl, -NH-$S(=O)_2$-$C_{1-6}$ alkyl, $C_{1-6}$ alkyl-NH-$S(=O)_2$-$C_{1-6}$ alkyl, $C_{3-6}$ cycloalkyl, $C_{3-6}$ cycloalkyl-$S(=O)_2$-, and 3- to 6-membered heterocycloalkyl,

wherein the $C_{1-6}$ alkyl, $C_{1-6}$ alkoxy, $C_{1-6}$ alkylthio, $C_{1-6}$ alkylamino, -$C_{1-6}$ alkyl-$C_{1-6}$ alkoxy, -$C_{1-6}$ alkyl-$C_{1-6}$ alkylthio, -$C_{1-6}$ alkyl-$C_{1-6}$ alkylamino, $C_{1-6}$ alkyl-OH, $C_{1-6}$ alkyl-$NH_2$, $C_{1-6}$ alkyl-C(=O)-, $C_{1-6}$ alkyl-C(=O)-$C_{1-6}$ alkyl, $C_{1-6}$ alkyl-O-C(=O)-, $C_{1-6}$ alkyl-O-C(=O)-$C_{1-6}$ alkyl, -NH-C(=O)-$C_{1-6}$ alkyl, $C_{1-6}$ alkyl-NH-C(=O)-$C_{1-6}$ alkyl, $C_{1-6}$ alkyl-$S(=O)_2$-, $C_{1-6}$ alkyl-$S(=O)_2$-$C_{1-6}$ alkyl, -NH-$S(=O)_2$-$C_{1-6}$ alkyl, $C_{1-6}$ alkyl-NH-$S(=O)_2$-$C_{1-6}$ alkyl, $C_{3-6}$ cycloalkyl, $C_{3-6}$ cycloalkyl-$S(=O)_2$-, and 3- to 6-membered heterocycloalkyl are optionally substituted with 1, 2 or 3 R, and other variables are as defined in the present disclosure.

In some embodiments of the present disclosure, each $R_1$ is independently selected from the group consisting of H, F, Cl, Br, OH, $NH_2$, CN, $C_{1-3}$ alkyl, $C_{1-3}$ alkoxy, $C_{1-3}$ alkylthio, $C_{1-3}$ alkylamino, $C_{1-3}$ alkyl-C(=O)-, $C_{1-3}$ alkyl-O-C(=O)-, $C_{3-6}$ cycloalkyl, 3- to 6-membered heterocycloalkyl, and $C_{3-6}$ cycloalkyl-$S(=O)_2$-, wherein the $C_{1-3}$ alkyl, $C_{1-3}$ alkoxy, $C_{1-3}$ alkylthio, $C_{1-3}$ alkylamino, $C_{1-3}$ alkyl-C(=O)-, $C_{1-3}$ alkyl-O-C(=O)-, $C_{3-6}$ cycloalkyl, 3- to 6-membered heterocycloalkyl, and $C_{3-6}$ cycloalkyl-$S(=O)_2$- are optionally substituted with 1, 2, or 3 R, and other variables are as defined in the present disclosure.

In some embodiments of the present disclosure, each $R_1$ is independently selected from the group consisting of H, F, Cl, Br, OH, $NH_2$, CN, Me,

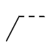, 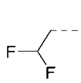, 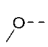, 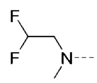,

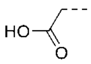, 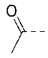, 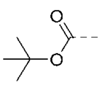, 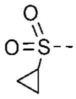, 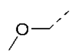, and 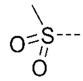,

and other variables are as defined in the present disclosure.

In some embodiments of the present disclosure, each $R_1$ is independently selected from the group consisting of H, F, Cl, Br, OH, $NH_2$, CN, Me,

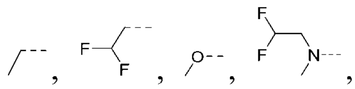

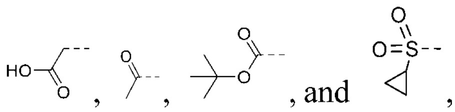

and other variables are as defined in the present disclosure.

In some embodiments of the present disclosure, ring B is selected from the group consisting of cyclohexyl, 3,6-dihydro-2H-pyranyl, 3,4-dihydro-2H-pyranyl, morpholinyl, cyclohexenyl, piperidinyl, 2,3-dihydro-1,4-dioxinyl, 6-oxa-3-azabicyclo[3.1.1]heptanyl, 1,2,3,4-tetrahydropyridinyl, 1,2,3,6-tetrahydropyridinyl, tetrahydro-2H-pyranyl, 5,6-dihydro-2H-pyran-2-keto, phenyl, pyridyl, pyrrolidinyl, 2-oxa-6-azaspiro[3.3]heptanyl, 1,1-dioxo-3,6-dihydro-2H-thiopyranyl, oxepinyl, azetidinyl, 2-oxa-7-azaspiro[4.4]nonanyl, and hexahydro-1H-furo[3,4-c]pyrrolyl, and other variables are as defined in the present disclosure.

In some embodiments of the present disclosure, ring B is selected from the group consisting of cyclohexyl, 3,6-dihydro-2H-pyranyl, 3,4-dihydro-2H-pyranyl, morpholinyl, cyclohexenyl, piperidinyl, 2,3-dihydro-1,4-dioxinyl, 6-oxa-3-azabicyclo[3.1.1]heptanyl, 1,2,3,4-tetrahydropyridinyl, 1,2,3,6-tetrahydropyridinyl, tetrahydro-2H-pyranyl, 5,6-dihydro-2H-pyran-2-keto, phenyl, pyridyl, and pyrrolidinyl, and other variables are as defined in the present disclosure.

In some embodiments of the present disclosure, the structural moiety

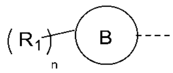

is selected from the group consisting of

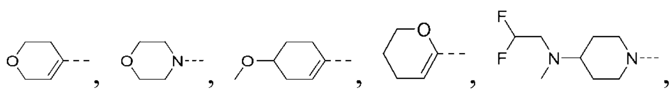

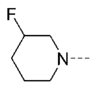, 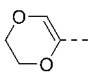, 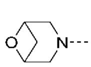, 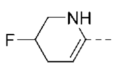, 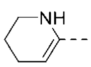, 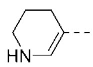, 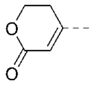, 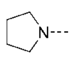, 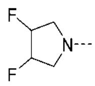, 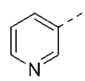,

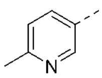, 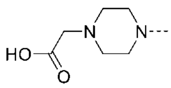, 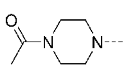, 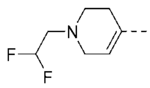, 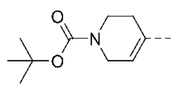, 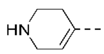, 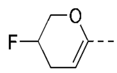, 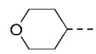,

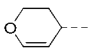, 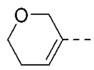, 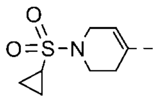, 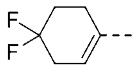, 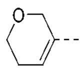, 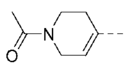, 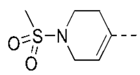, 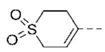,

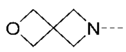, 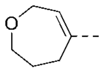, 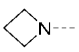, 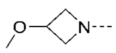, 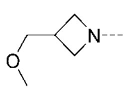, 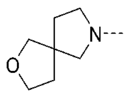, and 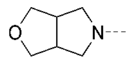,

and other variables are as defined in the present disclosure.

In some embodiments of the present disclosure, the structural moiety

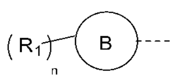

is selected from the group consisting of

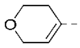, 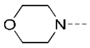, 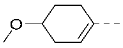, 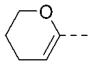, 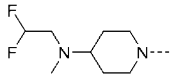,

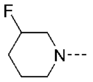, 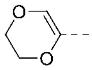, 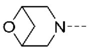, 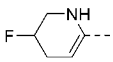, 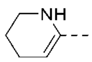, 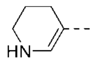, 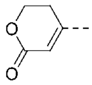, 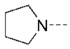, 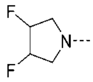, 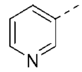,

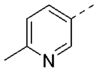, 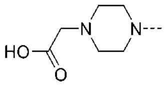, 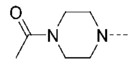, 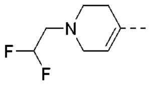, 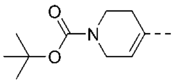, 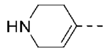, 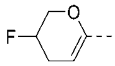, 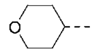,

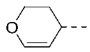, 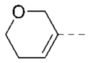, and 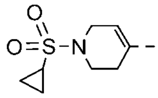,

and other variables are as defined in the present disclosure.

In some embodiments of the present disclosure, each $R_5$ is independently selected from the group consisting of H, F, Cl, Br, OH, $NH_2$, CN, $SF_5$, CHO, $C_{1-6}$ alkyl, $C_{1-6}$ alkoxy, $C_{1-6}$ alkylthio, $C_{1-6}$ alkylamino, $C_{3-6}$ cycloalkyl, and $C_{3-6}$ heterocycloalkyl, wherein the $C_{1-6}$ alkyl, $C_{1-6}$ alkoxy, $C_{1-6}$ alkylthio, $C_{1-6}$ alkylamino, $C_{3-6}$ cycloalkyl, and $C_{3-6}$ heterocycloalkyl are optionally substituted with 1, 2, or 3 R;

alternatively, two $R_5$ are connected together to form a $C_{3-6}$ cycloalkyl, 3- to 6-membered heterocycloalkyl, $C_{4-10}$ cycloalkenyl, 4- to 10-membered heterocycloalkenyl, $C_{6-10}$ aryl, or 5- to 10-membered heteroaryl group, wherein the $C_{3-6}$ cycloalkyl, 3- to 6-membered heterocycloalkyl, $C_{4-10}$ cycloalkenyl, 4- to 10-membered heterocycloalkenyl, $C_{6-10}$ aryl, and 5-to 10-membered heteroaryl are optionally substituted with 1, 2, or 3 R, and other variables are as defined in the present disclosure.

In some embodiments of the present disclosure, each $R_5$ is independently selected from the group consisting of H, F, Cl, Br, OH, $NH_2$, CN, $SF_5$, CHO, $C_{1-6}$ alkyl, $C_{1-6}$ alkoxy, $C_{1-6}$ alkylthio, $C_{1-6}$ alkylamino, $C_{3-6}$ cycloalkyl, and $C_{3-6}$ heterocycloalkyl, wherein the $C_{1-6}$ alkyl, $C_{1-6}$ alkoxy, $C_{1-6}$ alkylthio, $C_{1-6}$ alkylamino, $C_{3-6}$ cycloalkyl, and $C_{3-6}$ heterocycloalkyl are optionally substituted with 1, 2, or 3 R;

alternatively, two $R_5$ are connected together to form a $C_{3-6}$ cycloalkyl, 3- to 6-membered heterocycloalkyl, $C_{4-10}$ cycloalkenyl, 4- to 10-membered heterocycloalkenyl, $C_{6-10}$ aryl, or 5- to 10-membered heteroaryl group, wherein the $C_{3-6}$ cycloalkyl, 3- to 6-membered heterocycloalkyl, $C_{4-10}$ cycloalkenyl, 4- to 10-membered heterocycloalkenyl, and $C_{6-10}$ aryl are optionally substituted with 1, 2, or 3 R, and other variables are as defined in the present disclosure.

In some embodiments of the present disclosure, each $R_5$ is independently selected from the group consisting of H, F, Cl, Br, OH, $NH_2$, CN, $SF_5$, Me, CHO,

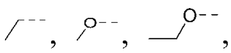

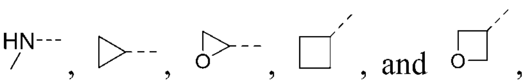

wherein the Me,

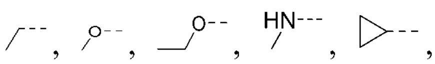

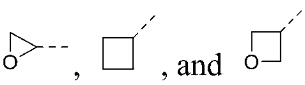

are optionally substituted with 1, 2, or 3 R;

alternatively, two $R_5$ are connected together to form

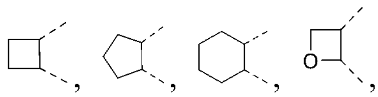

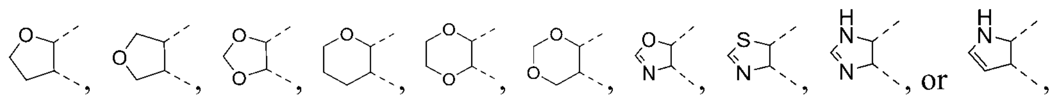

or

wherein the

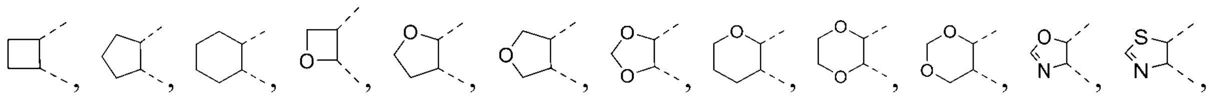

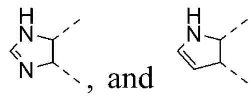

and

are optionally substituted with 1, 2, or 3 R, and other variables are as defined in the present disclosure.

In some embodiments of the present disclosure, each $R_5$ is independently selected from the group consisting of H, F, Cl, Br, OH, $NH_2$, CN, $SF_5$, Me, HC(=O)-,

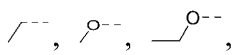

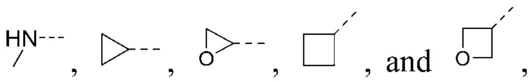

and

wherein the Me,

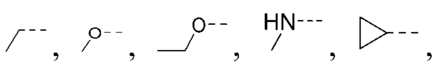

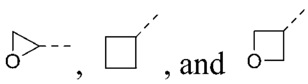

and

are optionally substituted with 1, 2, or 3 R, and other variables are as defined in the present disclosure.

In some embodiments of the present disclosure, each $R_5$ is independently selected from the group consisting of H, F, Cl, Br, OH, $NH_2$, $SF_5$, CN, Me, $CF_3$, CHO,

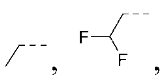

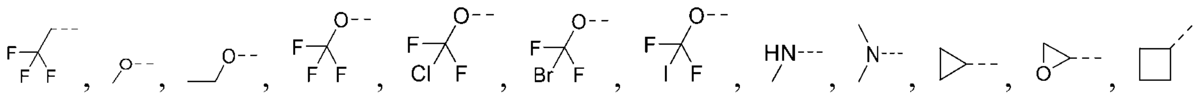

and

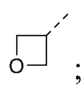

;

alternatively, two $R_5$ are connected together to form

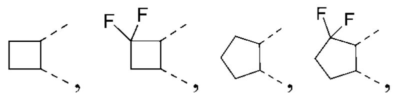

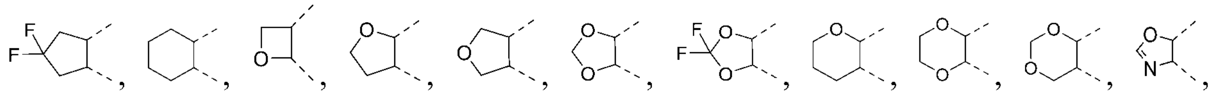

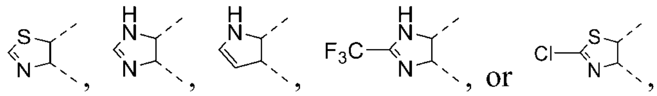

or

and other variables are as defined in the present disclosure.

In some embodiments of the present disclosure, each $R_5$ is independently selected from the group consisting of H, F, Cl, Br, OH, $NH_2$, $SF_5$, CN, Me, $CF_3$, HC(=O)-,

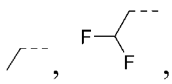

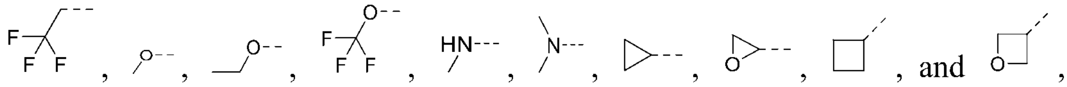

and

and other variables are as defined in the present disclosure.

In some embodiments of the present disclosure, ring C is selected from the group consisting of phenyl, pyridyl, pyrimidinyl, pyridazinyl, pyrazinyl, and thienyl, and other variables are as defined in the present disclosure.

In some embodiments of the present disclosure, the structural moiety

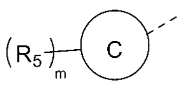

is selected from the group consisting of

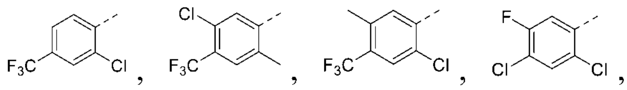

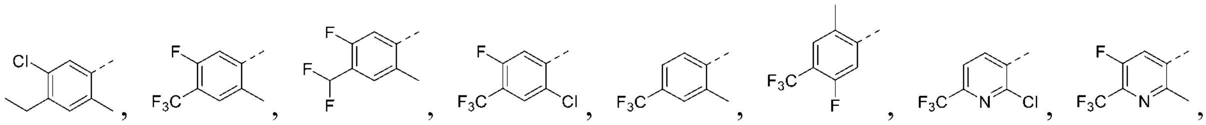

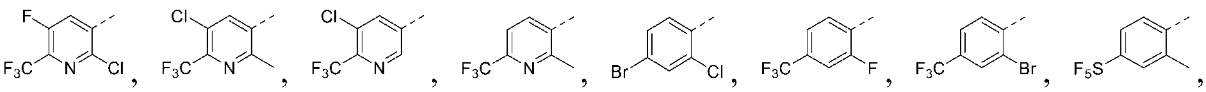

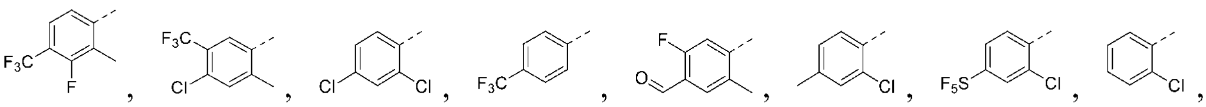

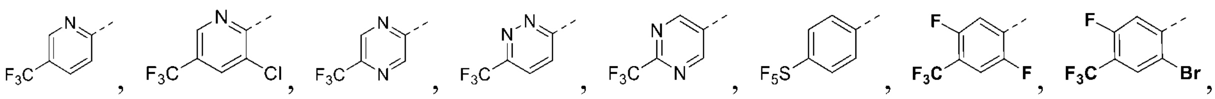

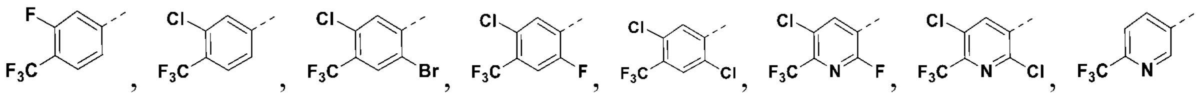
, , , , , , , ,

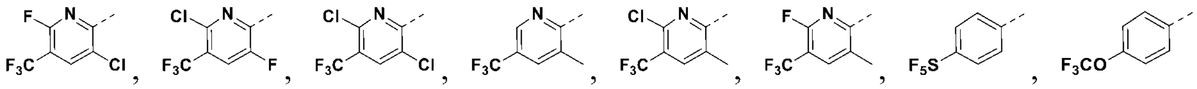
, , , , , , , ,

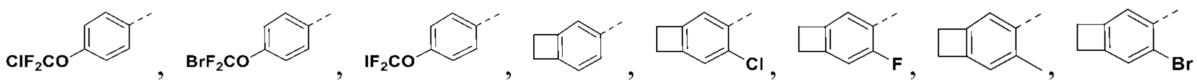
, , , , , , , ,

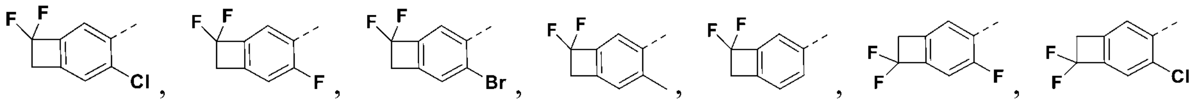
, , , , , , ,

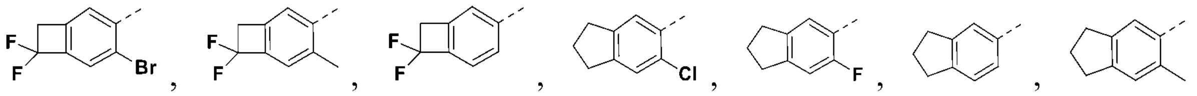
, , , , , , ,

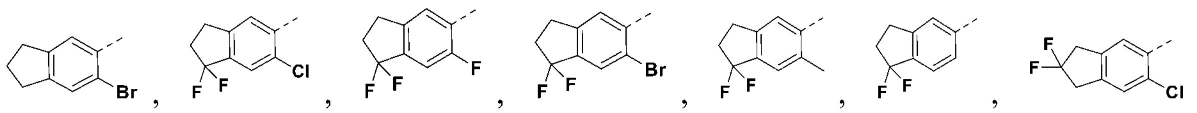
, , , , , , ,

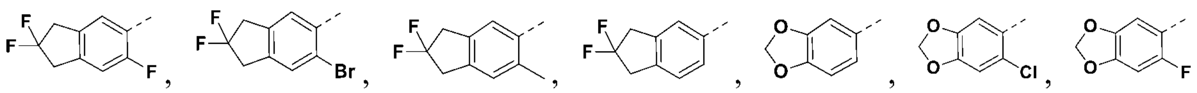
, , , , , , ,

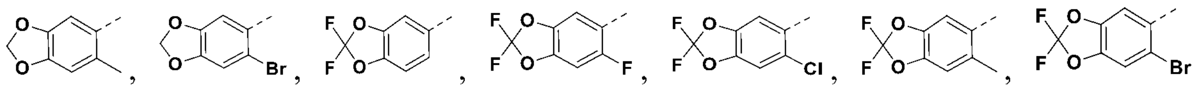
, , , , , , ,

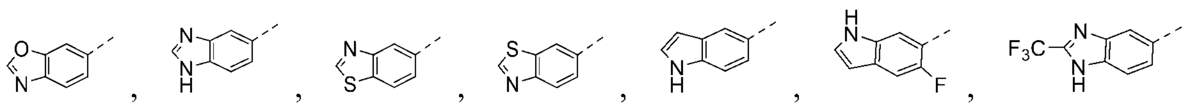
, , , , , , ,

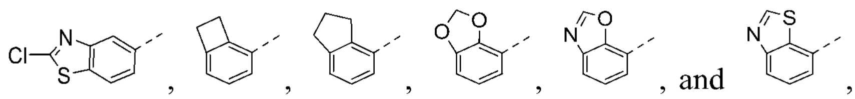
, , , , , and ,

and other variables are as defined in the present disclosure.

In some embodiments of the present disclosure, the structural moiety

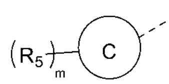

is selected from the group consisting of

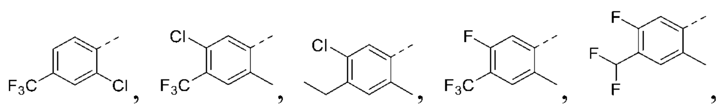
, , , , ,

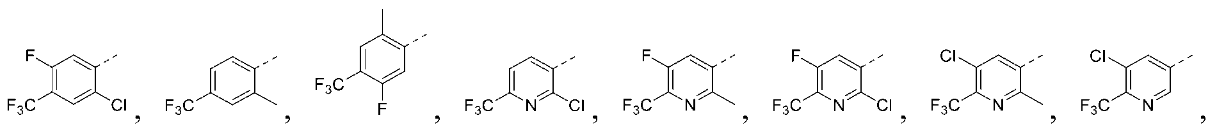

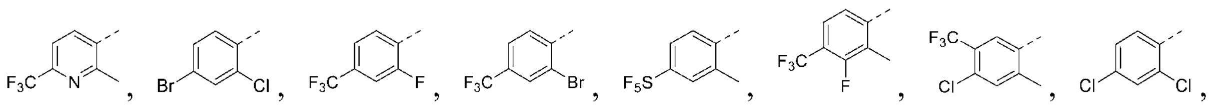

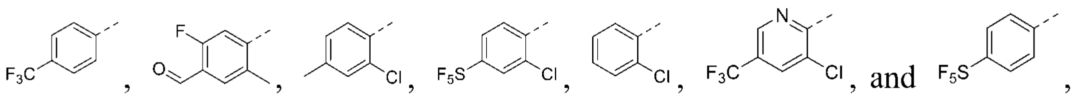
and

and other variables are as defined in the present disclosure.

In some embodiments of the present disclosure, each $R_6$ is independently selected from the group consisting of H, F, Cl, Br, OH, $NH_2$, CN, Me,

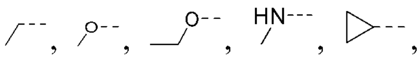

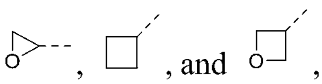
and

wherein the Me,

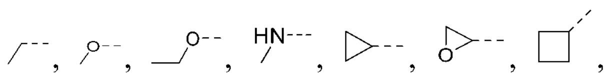

and

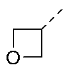

are optionally substituted with 1, 2, or 3 R, and other variables are as defined in the present disclosure.

In some embodiments of the present disclosure, each $R_6$ is independently selected from the group consisting of H, F, Cl, Br, OH, $NH_2$, CN, Me, $CF_3$,

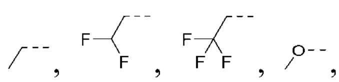

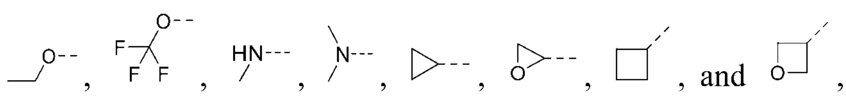
and

and other variables are as defined in the present disclosure.

In some embodiments of the present disclosure, ring D is selected from the group consisting of

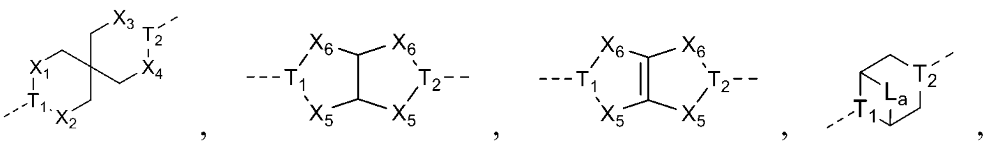

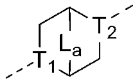, 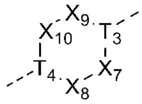, and 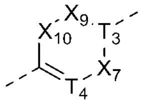;

$T_1$, $T_2$, $T_3$, and $T_4$ are each independently selected from the group consisting of N and CH;
$X_1$, $X_2$, $X_3$, and $X_4$ are each independently selected from the group consisting of a single bond and $CH_2$;
$X_5$ and $X_6$ are each independently selected from the group consisting of a single bond, $CH_2$, and $CH_2CH_2$, and $X_5$ and $X_6$ are not simultaneously a single bond;
$X_7$, $X_8$, $X_9$, and $X_{10}$ are each independently selected from the group consisting of a single bond, NH, O, S, $CH_2$, and 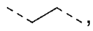, and no more than three of $X_7$, $X_8$, $X_9$, and $X_{10}$ are single bonds simultaneously;
$L_a$ is selected from the group consisting of $C_{1-6}$ alkyl, $C_{2-6}$ alkenyl, and $C_{1-6}$ heteroalkyl, wherein the $C_{1-6}$ alkyl and $C_{1-6}$ heteroalkyl are optionally substituted with 1, 2, or 3 R, and other variables are as defined in the present disclosure.

In some embodiments of the present disclosure, ring D is selected from the group consisting of

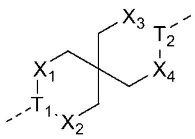, 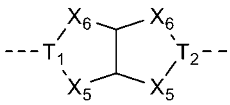, 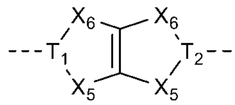, 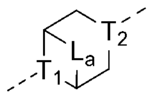,

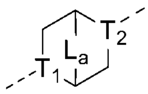, and 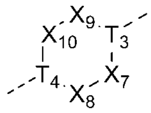,

and other variables are as defined in the present disclosure.

In some embodiments of the present disclosure, ring D is selected from the group consisting of

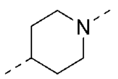, 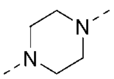, 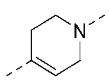, 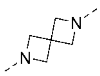, 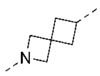, 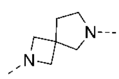, 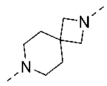,

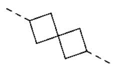, 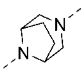, 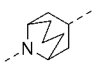, 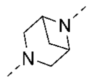, 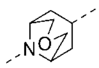, 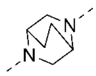, 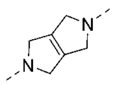, 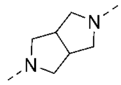, 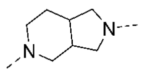,

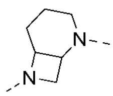, 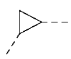, 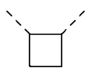, 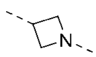, 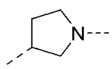, and 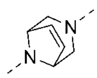,

and other variables are as defined in the present disclosure.

In some embodiments of the present disclosure, ring D is selected from the group consisting of piperidinyl, piperazinyl, and 1,2,3,6-tetrahydropyridinyl, and other variables are as defined in the present disclosure.

In some embodiments of the present disclosure, the structural moiety

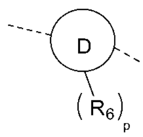

is selected from the group consisting of

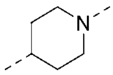, 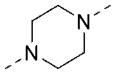, 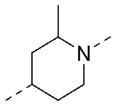, 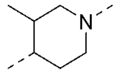, 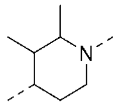,

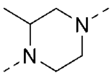, 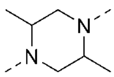, 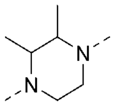, 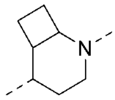, 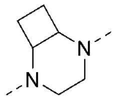, 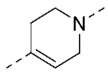, 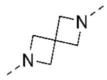, 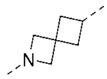,

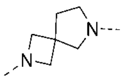, 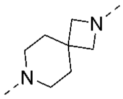, 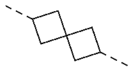, 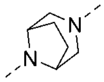, 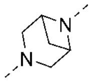, 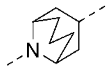, 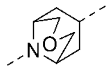, 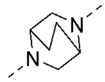,

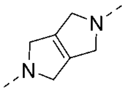, 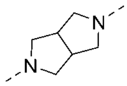, 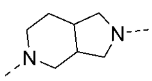, 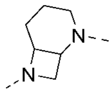, 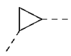, 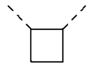, 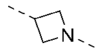, 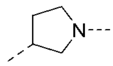,

and

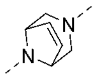,

and other variables are as defined in the present disclosure.

In some embodiments of the present disclosure, the structural moiety

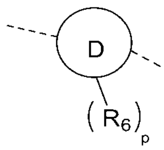

is selected from the group consisting of

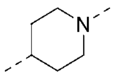, 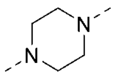, 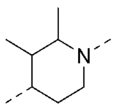, 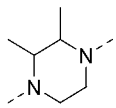, 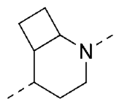,

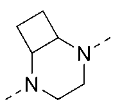, and 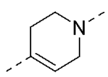,

and other variables are as defined in the present disclosure.

In some embodiments of the present disclosure, each $R_7$ is independently selected from the group consisting of H, F, Cl, Br, OH, $NH_2$, CN, Me,

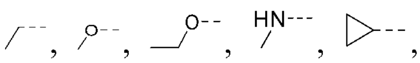,

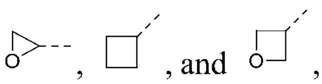 and,

wherein the Me,

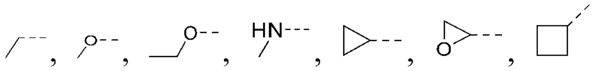, , , , , , ,

and

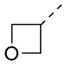

are optionally substituted with 1, 2, or 3 R, and other variables are as defined in the present disclosure.

In some embodiments of the present disclosure, each $R_7$ is independently selected from the group consisting of H, F, Cl, Br, OH, $NH_2$, CN, Me, $CF_3$,

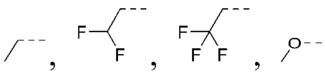, , , ,

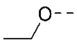, 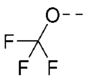, 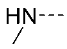, 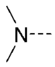, 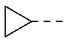, 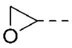, 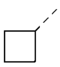, and 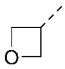,

and other variables are as defined in the present disclosure.

In some embodiments of the present disclosure, ring E is selected from the group consisting of phenyl, pyridyl, pyrimidinyl, pyridazinyl, pyrazinyl, and thienyl, and other variables are as defined in the present disclosure.

In some embodiments of the present disclosure, the structural moiety

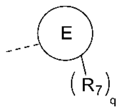

is selected from the group consisting of

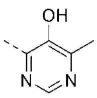, 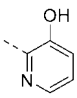, 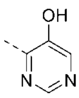, 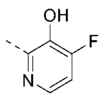, 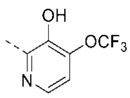, 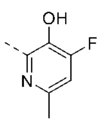,

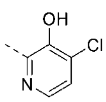, 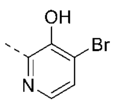, 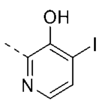, 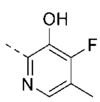, 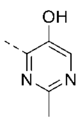, 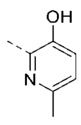, 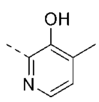, 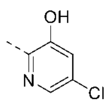, 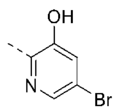,

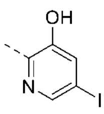, 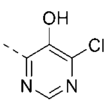, 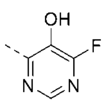, 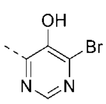, 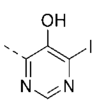, 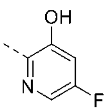, 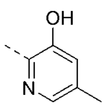, 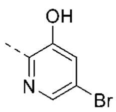, 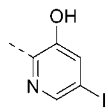,

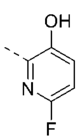, 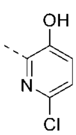, 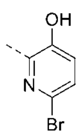, and 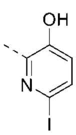,

and other variables are as defined in the present disclosure.

In some embodiments of the present disclosure, the structural moiety

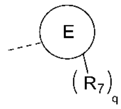

is selected from the group consisting of

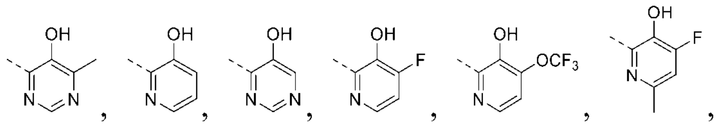

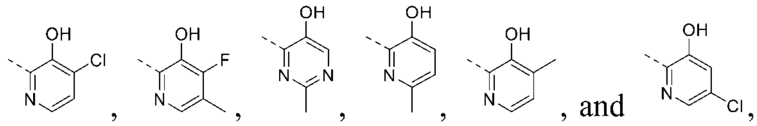
, and

and other variables are as defined in the present disclosure.

In some embodiments of the present disclosure, $R_3$ and $R_4$ are each independently selected from the group consisting of H, F, Cl, Br, OH, $NH_2$, CN, Me,

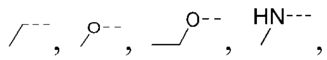

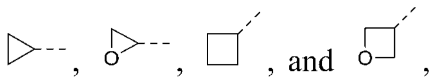
, and

wherein the Me,

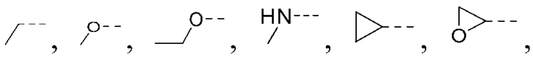

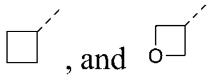
, and

are optionally substituted with 1, 2, or 3 R, and other variables are as defined in the present disclosure.

In some embodiments of the present disclosure, $R_3$ and $R_4$ are each independently selected from the group consisting of H, F, Cl, Br, OH, $NH_2$, CN, Me, $CF_3$,

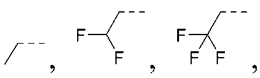

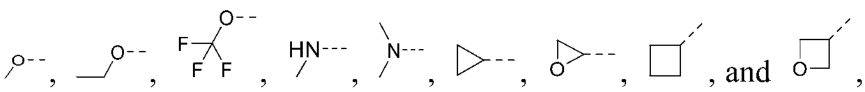
, and

and other variables are as defined in the present disclosure.

In some embodiments of the present disclosure, $R_8$ is selected from the group consisting of H, Me,

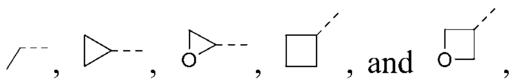
, and

wherein the Me,

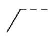, 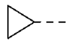,

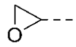, 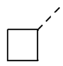, and 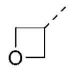

are optionally substituted with 1, 2, or 3 Rs, and other variables are as defined in the present disclosure.

In some embodiments of the present disclosure, $R_8$ is selected from the group consisting of H, Me, $CF_3$,

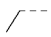, 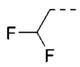, 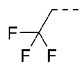, 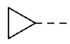, 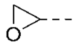, 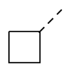, and 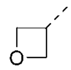,

and other variables are as defined in the present disclosure.

The present disclosure also provides a compound of the following formula, an optical isomer thereof, or a pharmaceutically acceptable salt thereof, selected from the group consisting of:

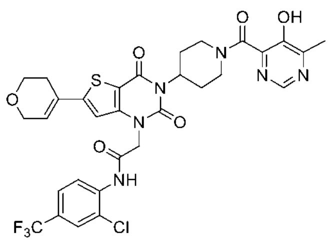 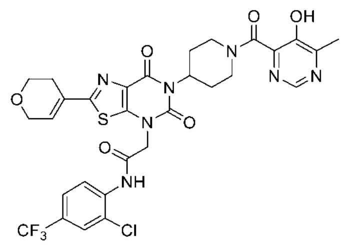 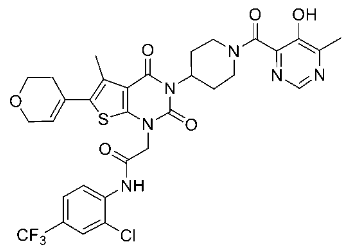

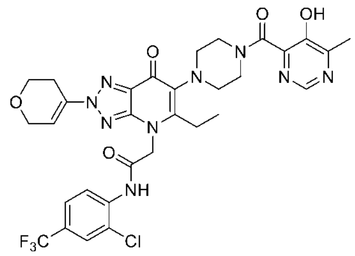 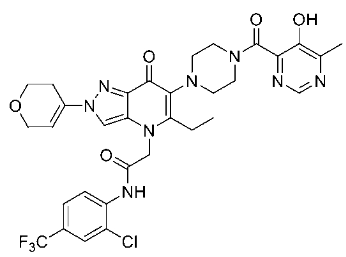 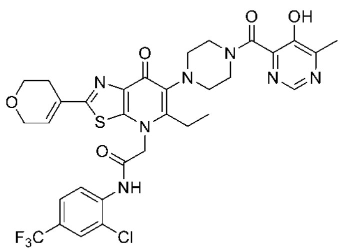

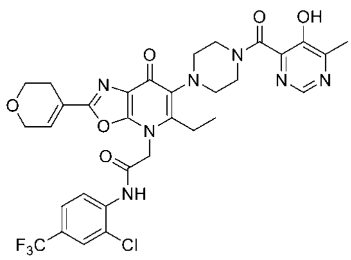 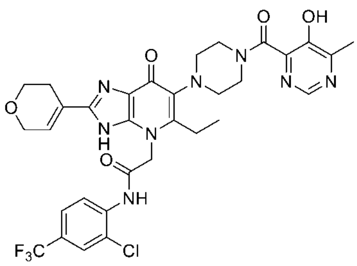 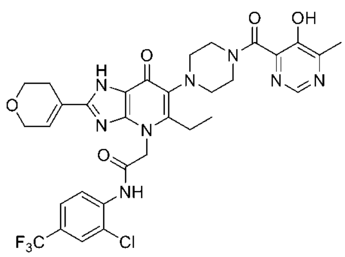

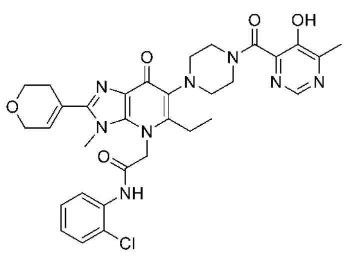 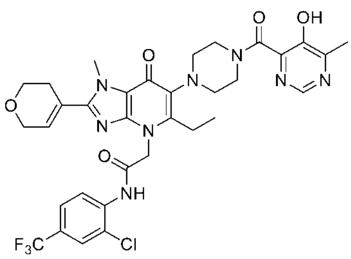 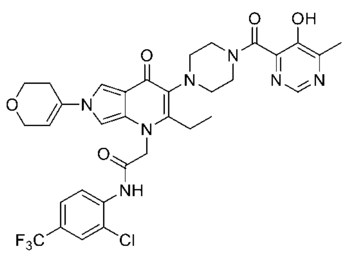

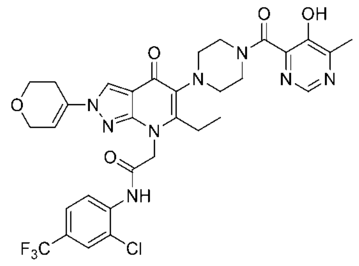

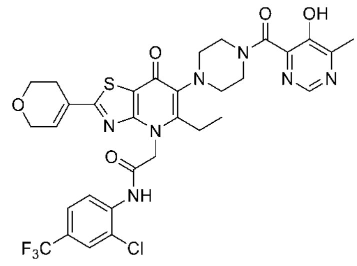

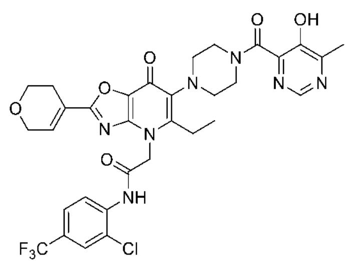

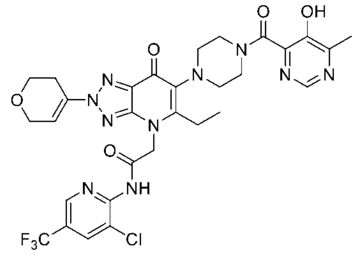

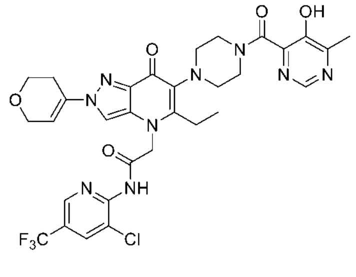

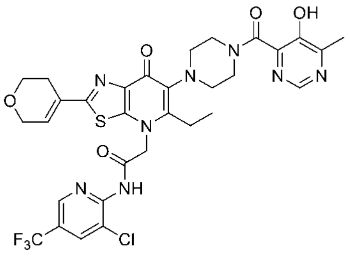

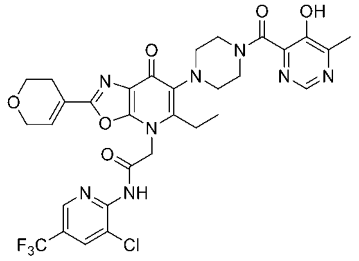

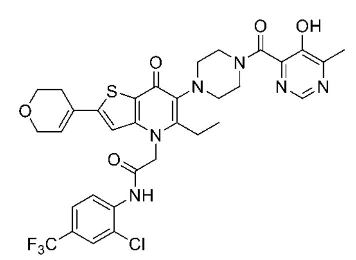

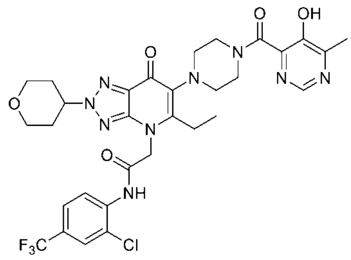

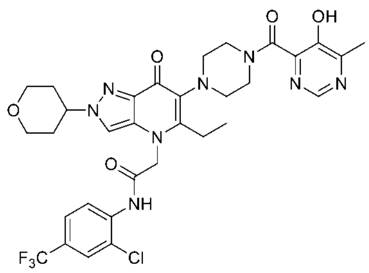

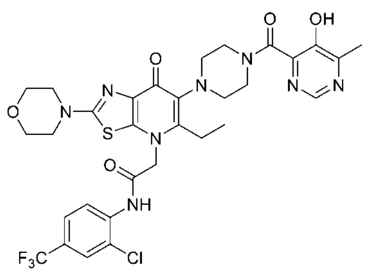

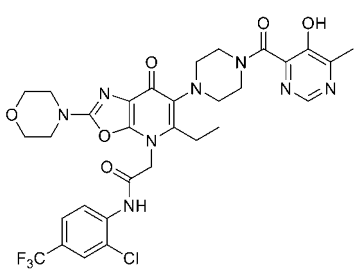

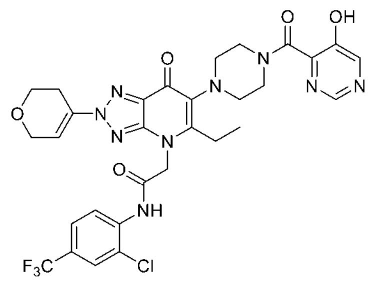

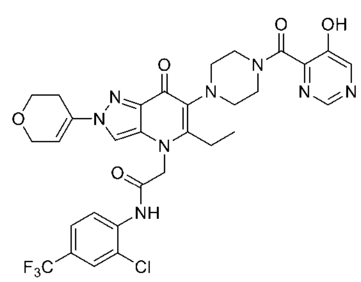

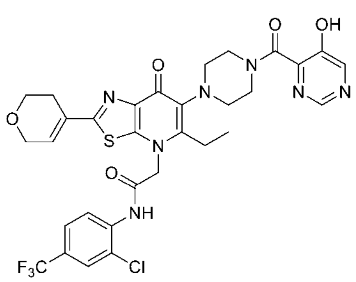

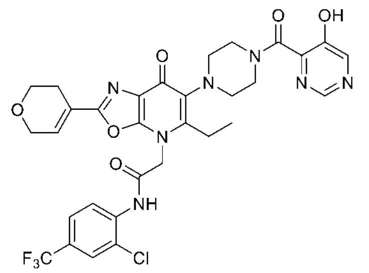

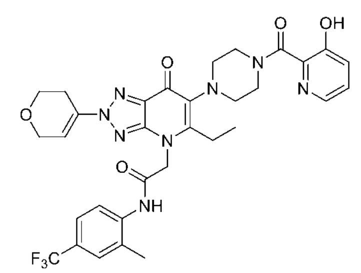

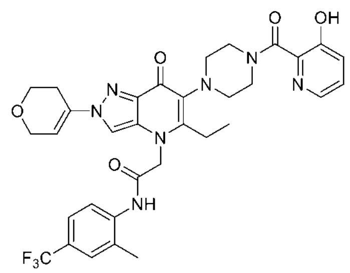

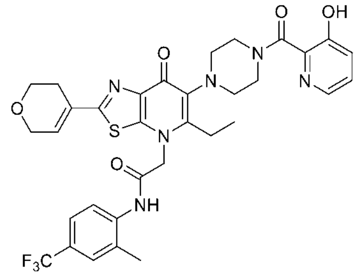
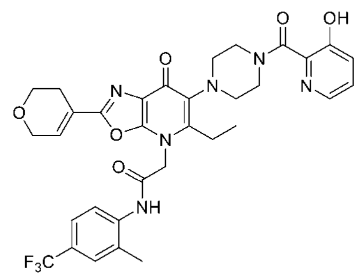
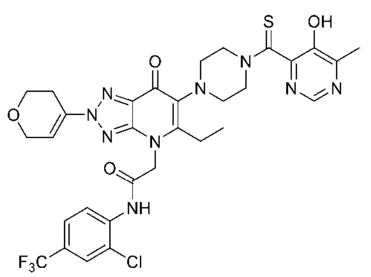
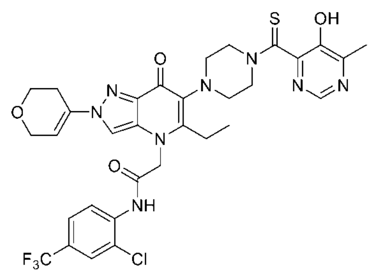
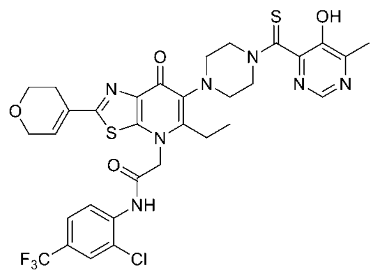
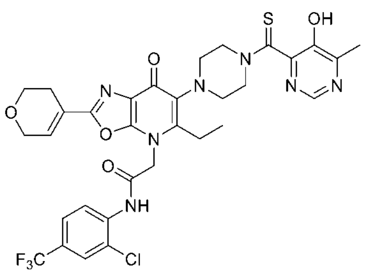
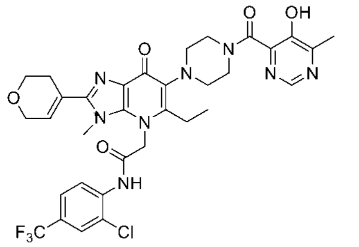
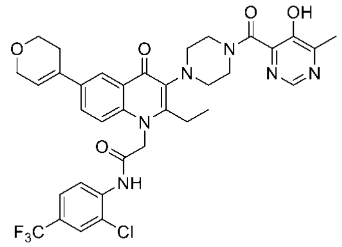
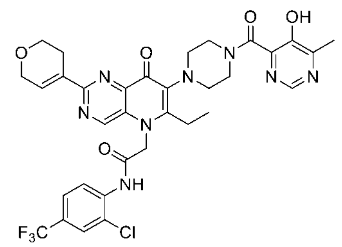
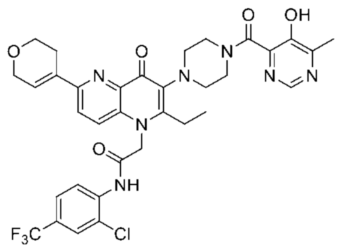
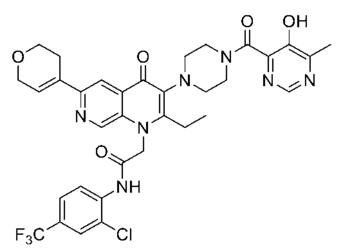
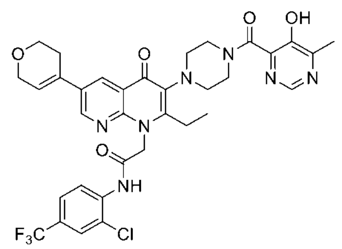
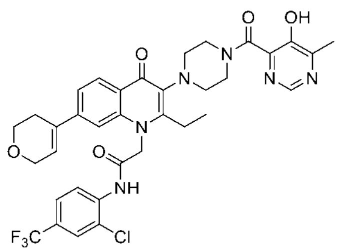
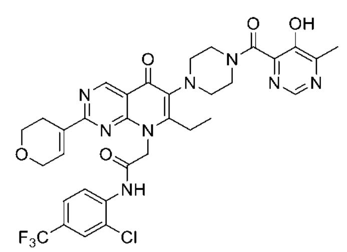
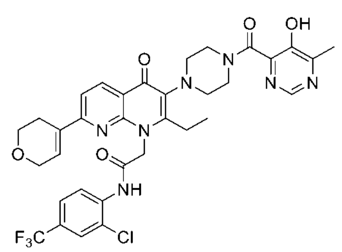
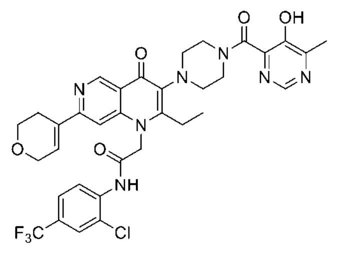
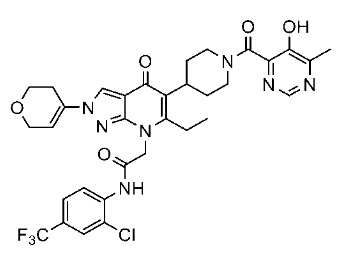
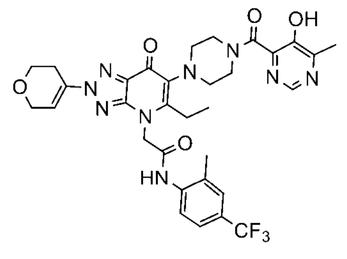

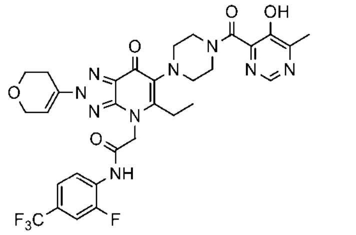
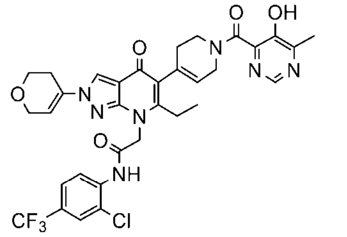
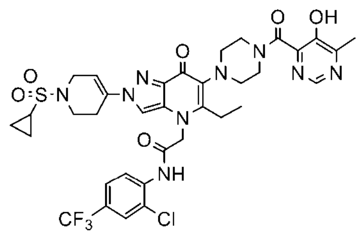
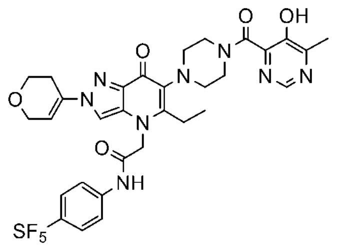
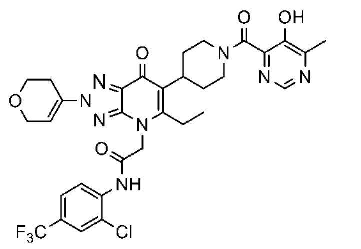
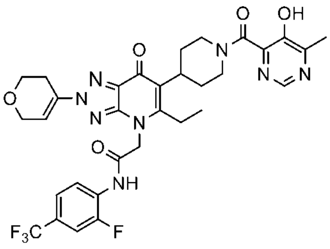
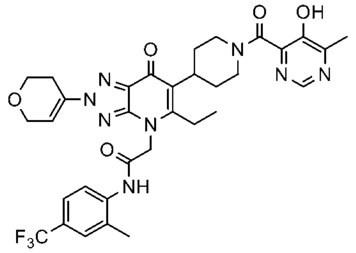
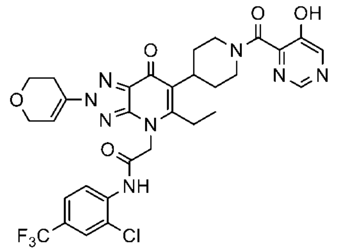
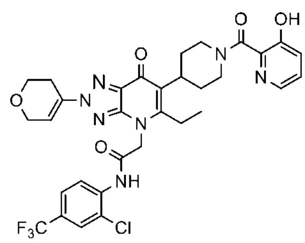
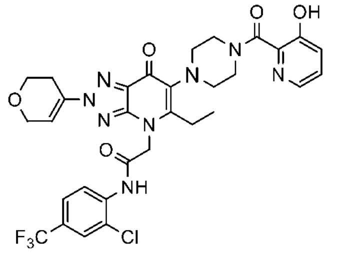
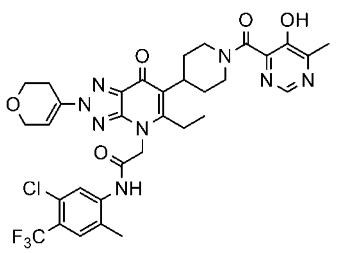
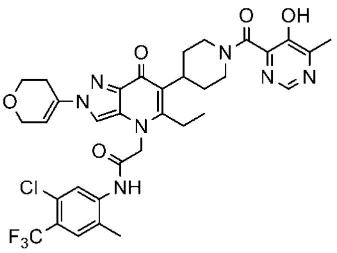
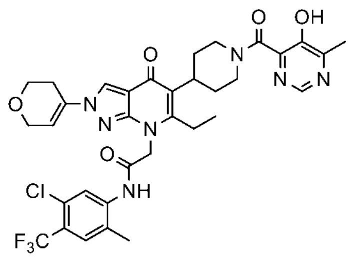
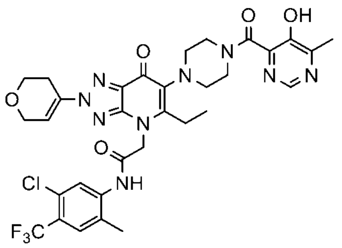
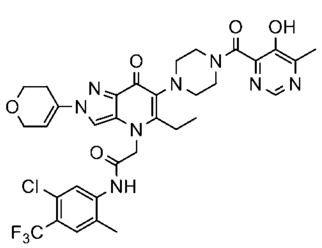
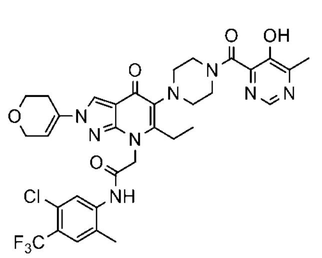
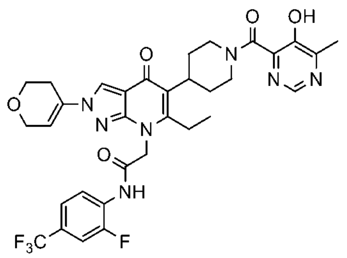
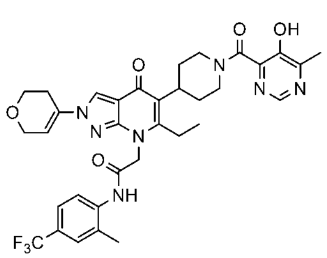

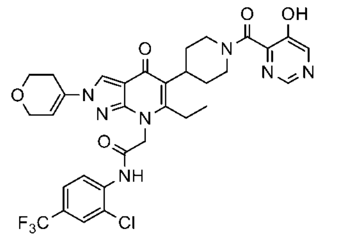
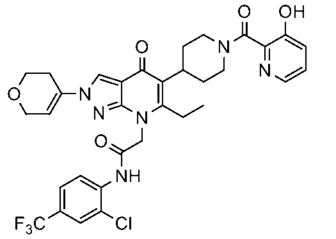
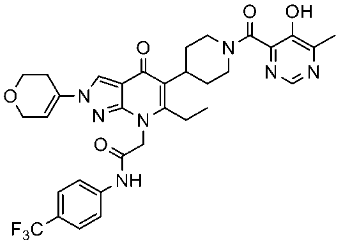

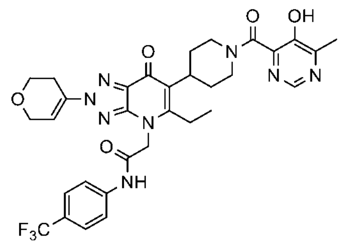
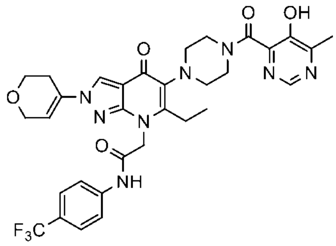
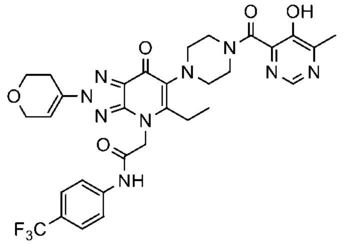

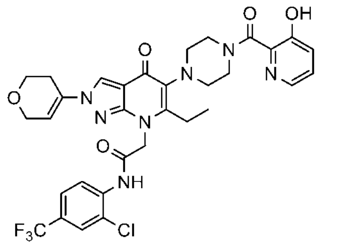
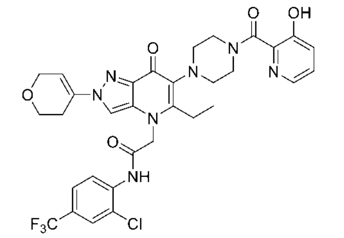
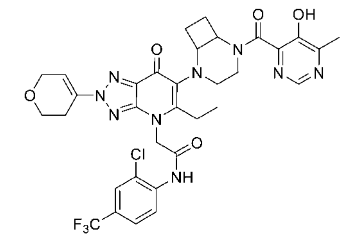

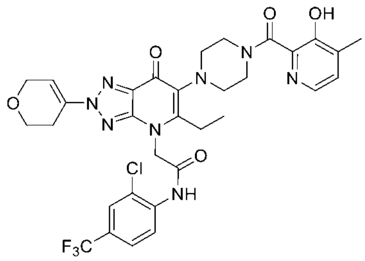
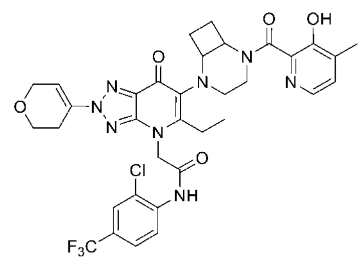
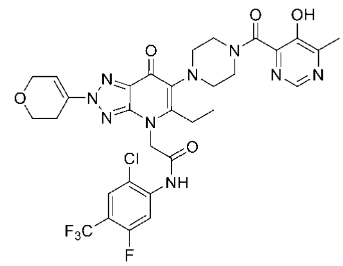

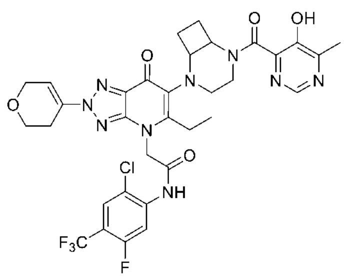
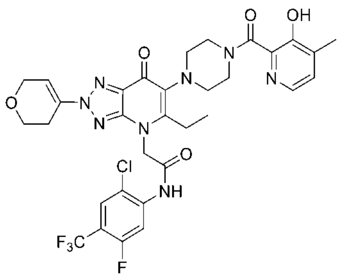
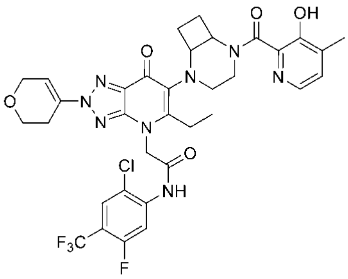

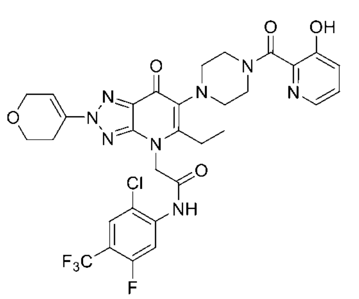
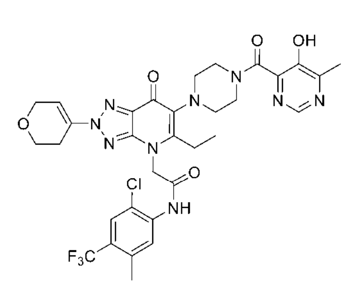
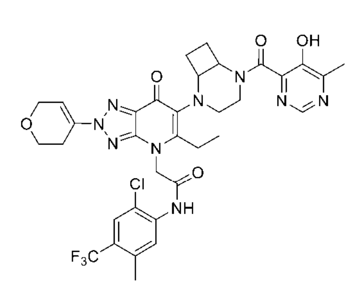

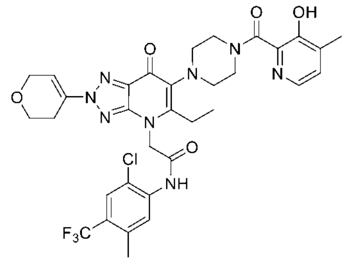
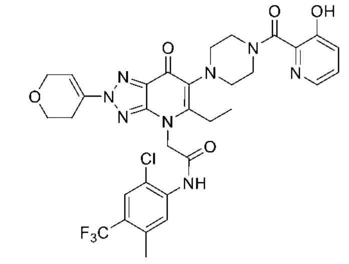
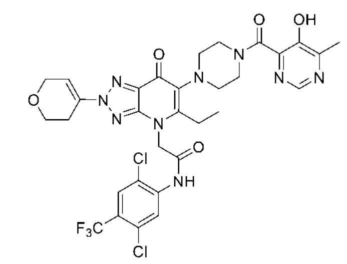
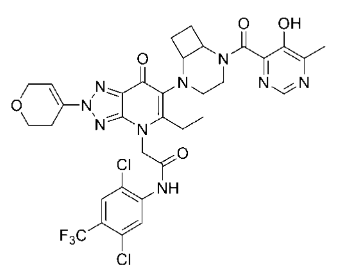
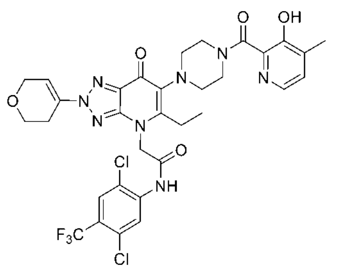
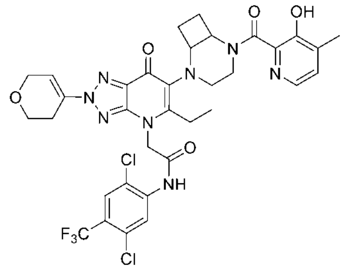
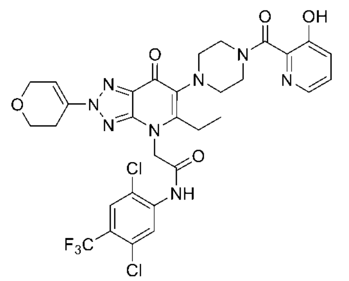
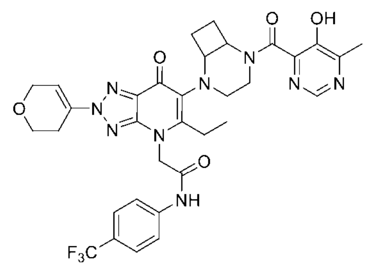
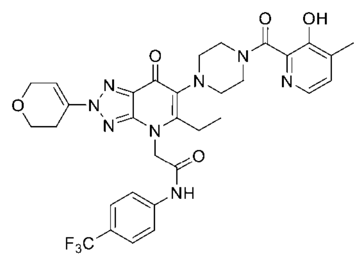
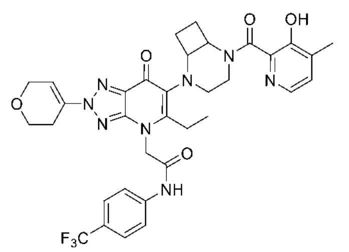
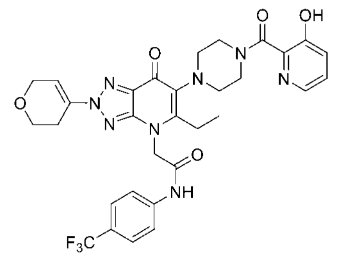
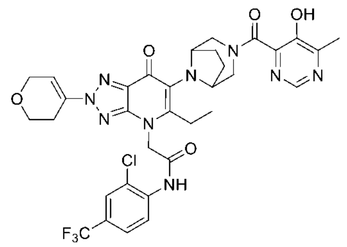
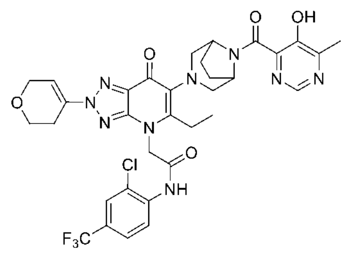
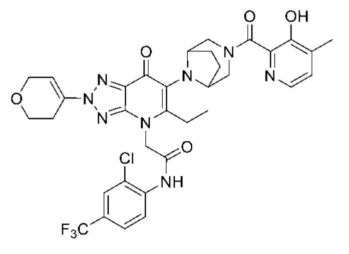
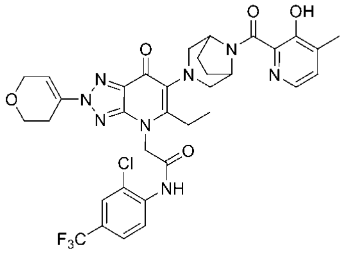
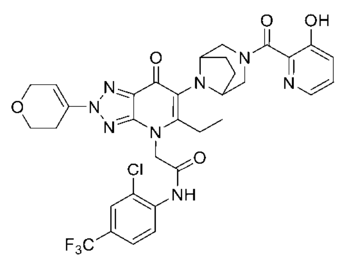
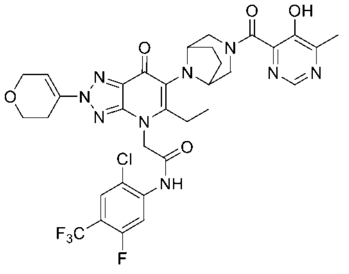
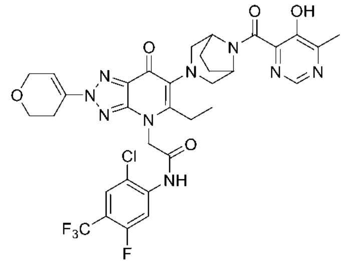

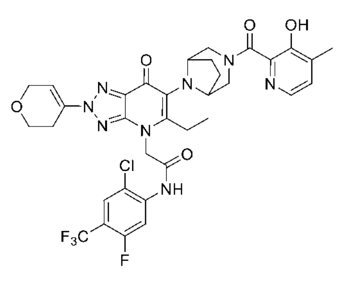

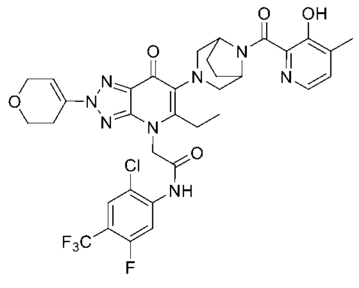

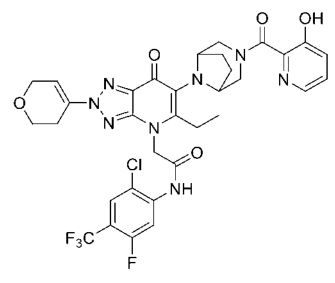

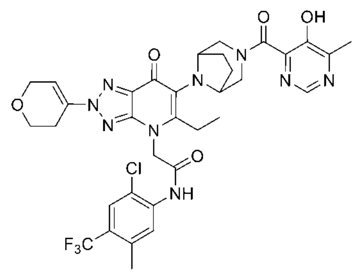

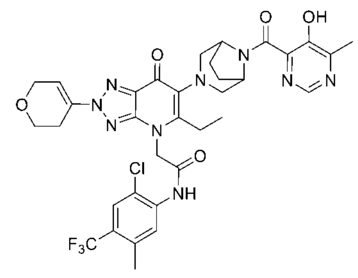

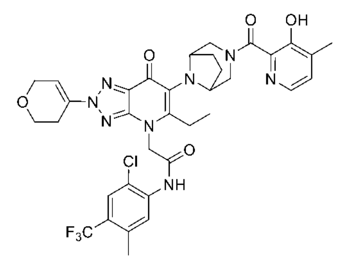

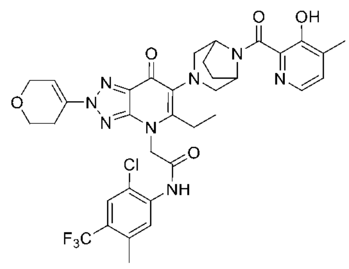

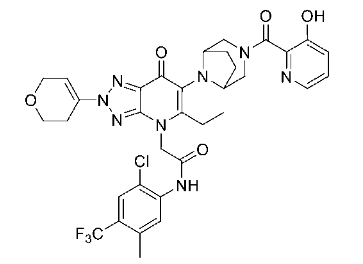

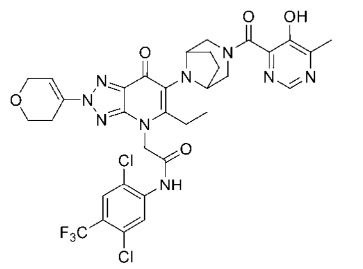

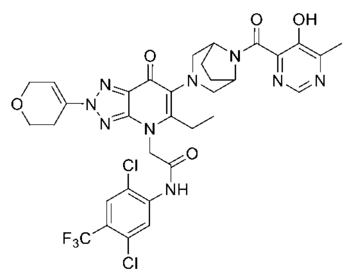

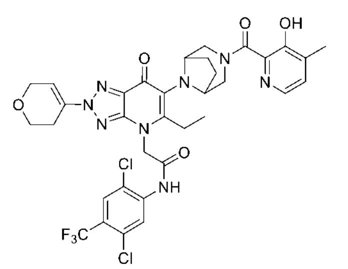

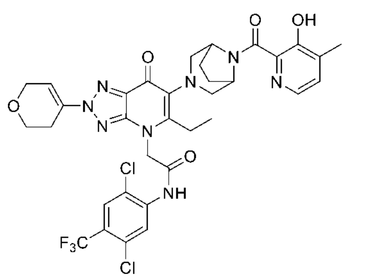

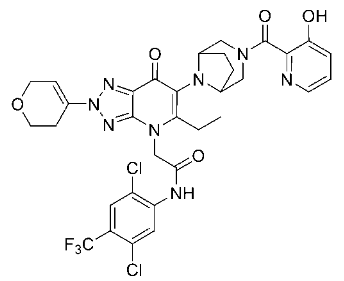

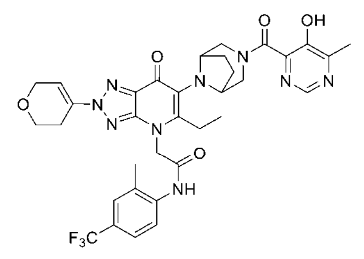

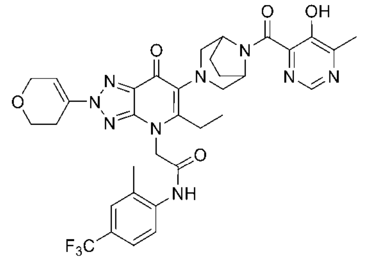

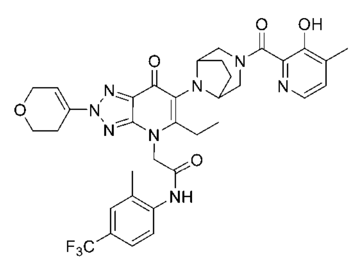

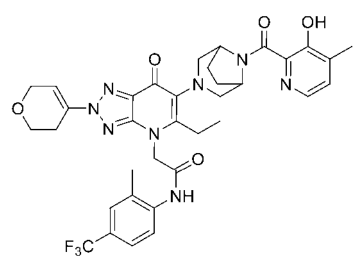

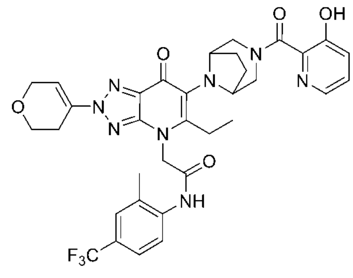

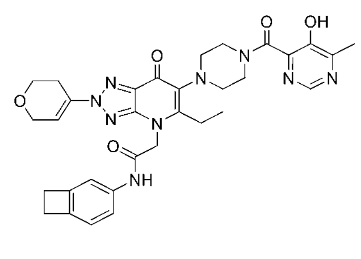

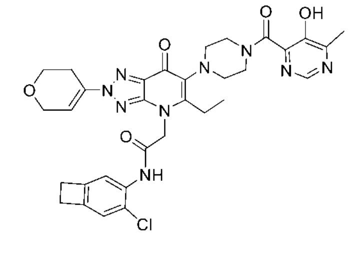

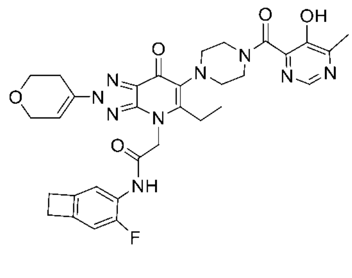

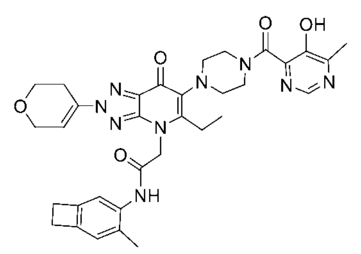

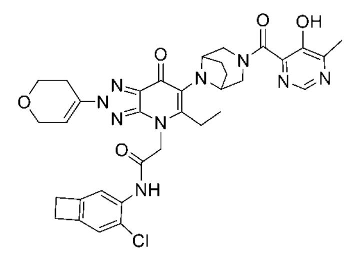

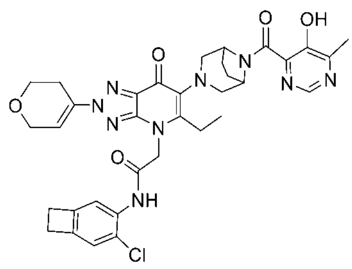

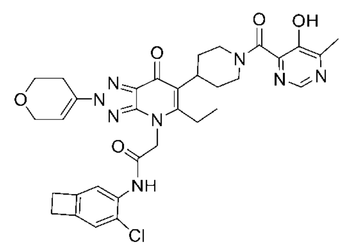

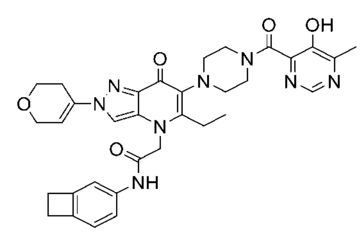

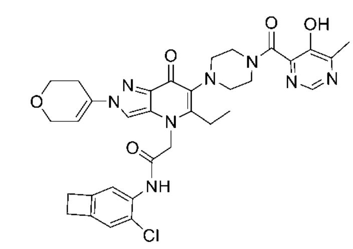

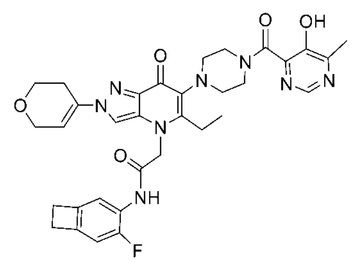

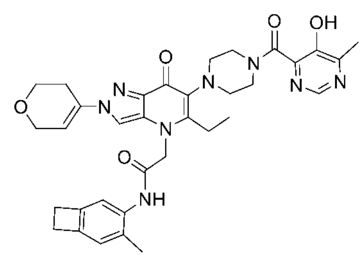

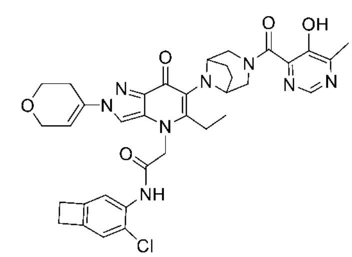

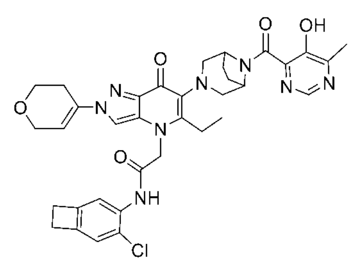

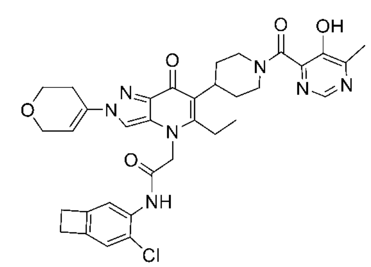

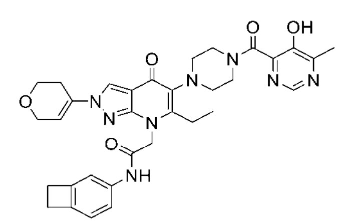

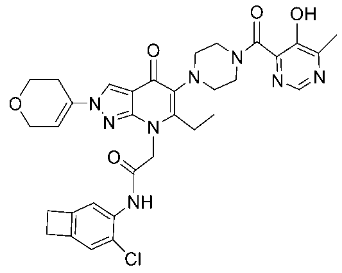
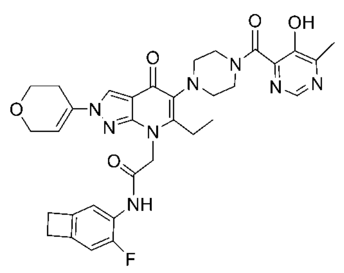
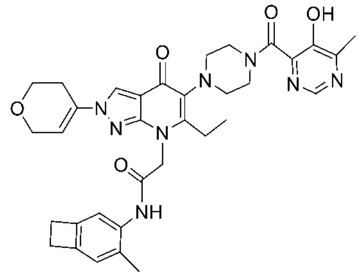
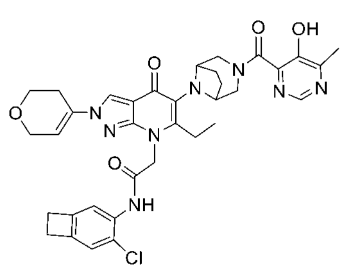
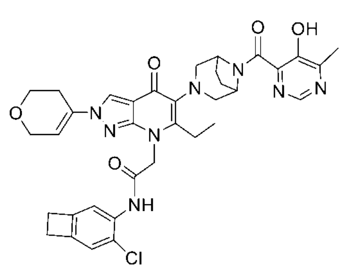
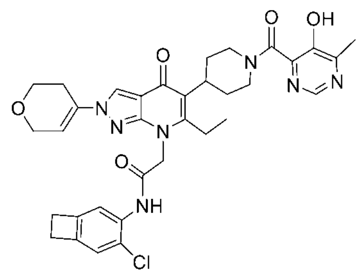
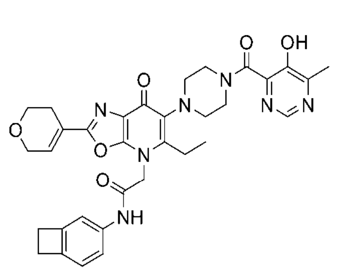
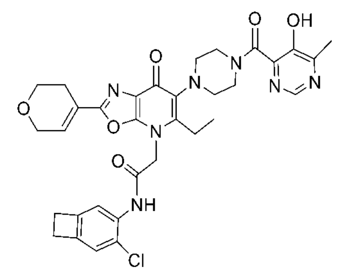
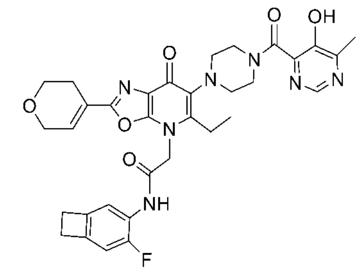
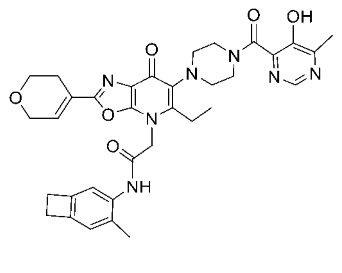
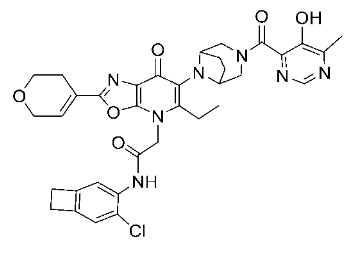
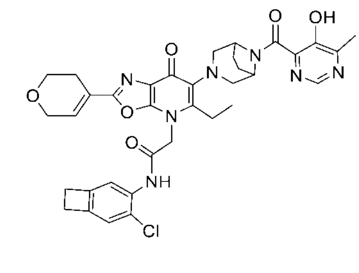
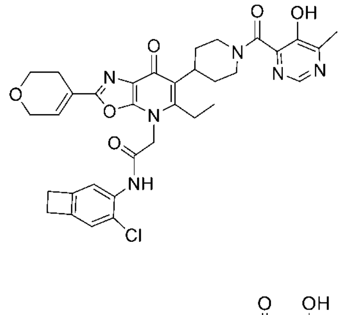
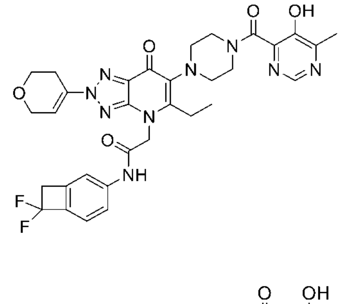
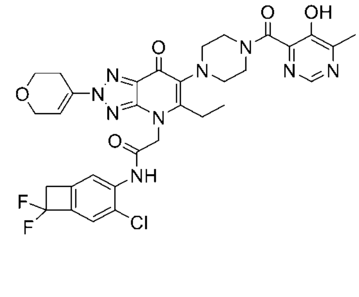
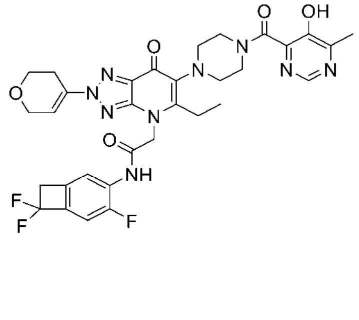
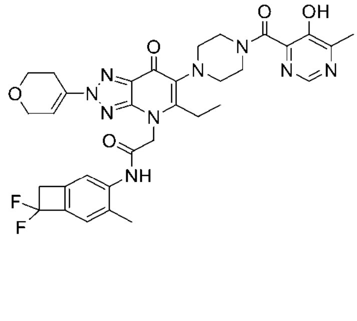
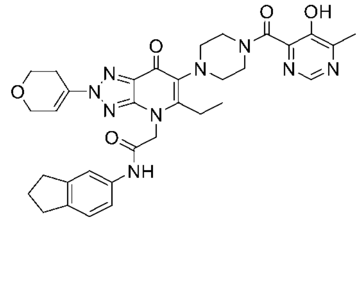

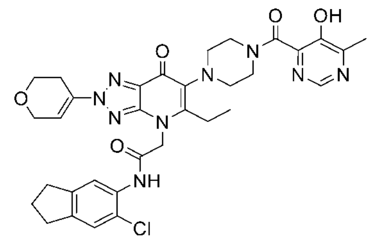
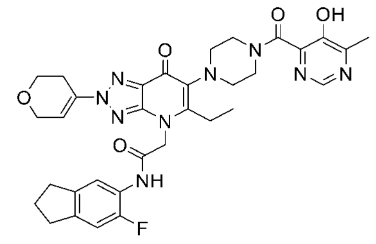
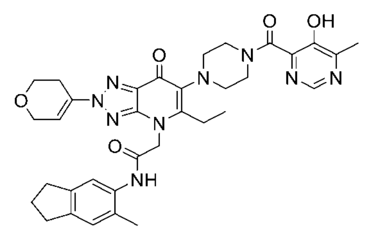
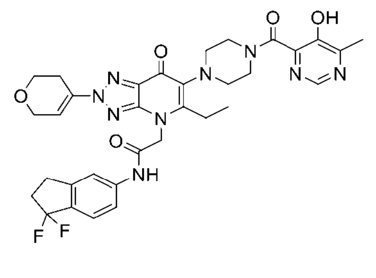
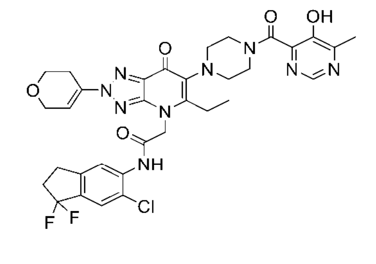
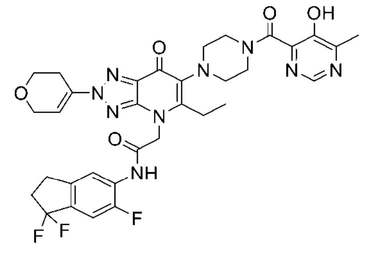
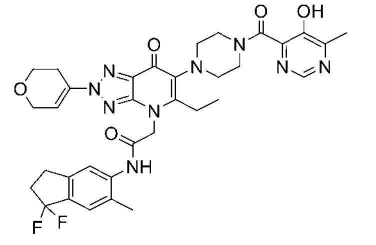
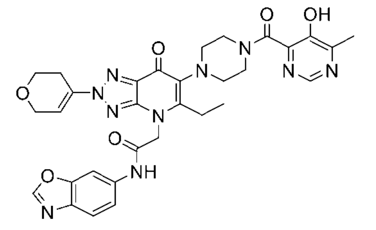
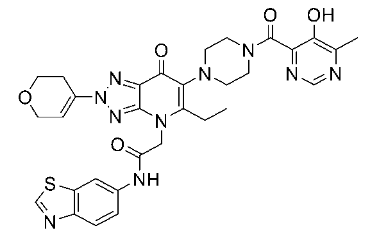
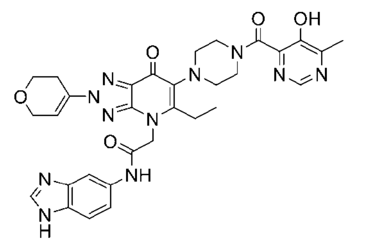
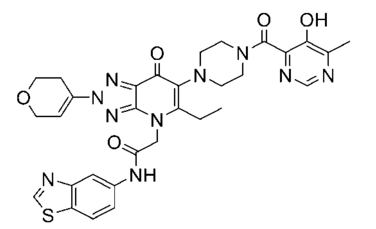
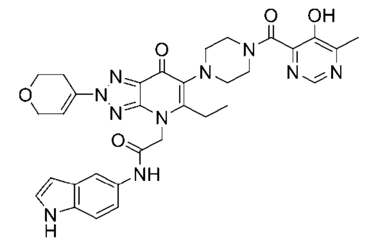
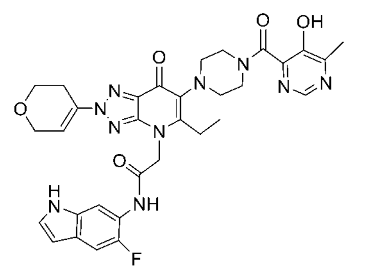
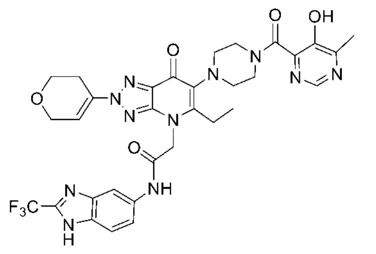
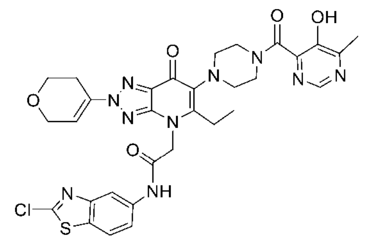
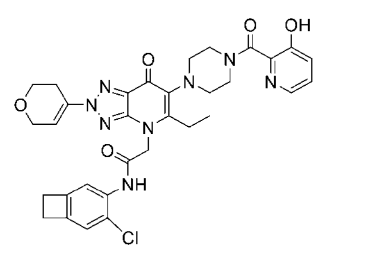
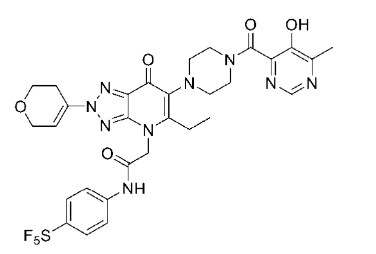
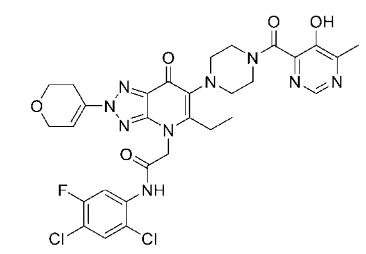

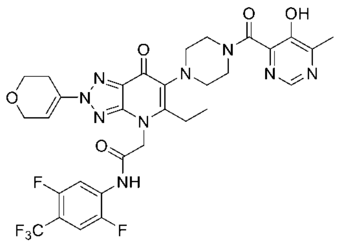.

In another aspect of the present disclosure, the present disclosure further provides a pharmaceutical composition. In some embodiments of the present disclosure, the pharmaceutical composition comprises the aforementioned compound, the optical isomer thereof, or the pharmaceutically acceptable salt thereof.

In some embodiments of the present disclosure, the pharmaceutical composition further comprises a pharmaceutically acceptable excipient.

In yet another aspect of the present disclosure, the present disclosure further provides a use of the compound, the optical isomer thereof, or the pharmaceutically acceptable salt thereof, or the pharmaceutical composition in the manufacture of a medicament for treating a disease associated with a tumor.

An objective of the present disclosure is to provide a compound as a WRN inhibitor, or a stereoisomer, deuterated derivative, solvate, prodrug, metabolite, pharmaceutically acceptable salt or cocrystal thereof, and intermediates and preparation methods therefor, as well as the use thereof in the manufacture of a medicament for treating a disease associated with a microsatellite instability-high tumor.

In some embodiments of the present disclosure, the disease associated with the tumor is one or more of diseases associated with solid tumors.

The compounds of the present disclosure may be used alone or in combination with other chemotherapeutic drugs, targeted therapeutic drugs, or immunotherapeutic drugs for the treatment of various tumors, particularly microsatellite instability-high (MSI) malignant tumors, or mismatch repair deficient (dMMR) malignant tumors, or malignant tumors detected with a high number of $(TA)_n$ repeat sequences, including but not limited to colorectal cancer, gastric cancer, endometrial cancer, ovarian cancer, and the like.

## Definition and description

Unless otherwise specified, the following terms and phrases when used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, other problems, or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by bringing the neutral form of the compound into contact with a sufficient amount of acid in a solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, trifluoroacetic acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by a conventional chemical method. Generally, such salt can be prepared by reacting the free acid or base form of the compound with a stoichiometric amount of an appropriate base or acid in water or an organic

solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (R)- and (S)-enantiomers, diastereomers isomers, (D)-isomers, (L)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are comprised within the scope of the present disclosure.

Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) comprises interconversion through proton migration, such as keto-enol isomerization and imine-enamine isomerization. Valence tautomer comprises some recombination of bonding electrons for mutual transformation. A specific example of keto-enol tautomerization is the tautomerism between two tautomers of pentane-2,4-dione and 4-hydroxypent-3-en-2-one.

The compound of the present disclosure may contain an unnatural proportion of atomic isotope at one or more than one atom that constitute the compound. For example, the compound can be radiolabeled with a radioactive isotope, such as tritium ($^3H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs and the like. All isotopic variations of the compound of the present disclosure, whether radioactive or not, are encompassed within the scope of the present disclosure.

"Optional" or "optionally" means that the subsequent event or condition may occur but not requisite, and the description includes the instance in which the event or condition occurs and the instance in which the event or condition does not occur.

The term "substituted with..." means one or more hydrogen atoms on a specific atom are substituted with the substituent, including deuterium and hydrogen variables, as long as the valence of the specific atom is normal and the substituted compound is stable. The term "optionally substituted with..." means an atom may or may not be substituted, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 1, 2, or 3 R, the group can be optionally substituted with up to three R, wherein the definition of R at each occurrence is independent. Moreover, a combination of the substituent and/or the variant thereof is allowed only when the combination results in a stable compound. For example,

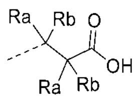

can be selected from the group consisting of

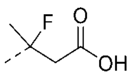, 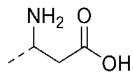, 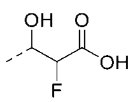, and 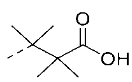,

etc.

When one of the variables is a single bond, it means that the two groups linked by the single bond are linked directly. For example, when $L_2$ in

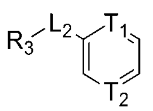

represents a single bond, the structure is actually

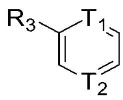.

A hyphen ("-") being not between two letters or symbols indicates the linkage site of a substituent. For example, $C_{1-6}$ alkylcarbonyl- refers to a $C_{1-6}$ alkyl linked to the rest of the molecule through a carbonyl group. However, when the linkage site of a substituent is obvious to those skilled in the art, for example, a halogen substituent, "-" can be omitted.

Unless otherwise specified, when the valence bond of a group has a dashed line "⸝⸍", such as in

" 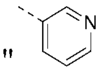 ",

the dashed line indicates the linkage site of the group to the rest of the molecule.

When the listed substituents do not indicate via which atom it is linked to the substituted group, this substituent can be bonded via any atom, for example, pyridyl, as a substituent, can link to the substituted group via any carbon atom on the pyridine ring.

When the listed linking group does not indicate the direction for linking, the direction for linking is arbitrary, for example, the linking group L contained in

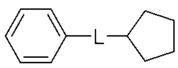

is

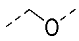,

then

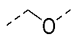

can link phenyl and cyclopentyl to form

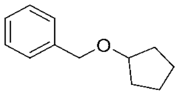

in the direction same as left-to-right reading order, and can link phenyl and cyclopentyl to form

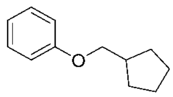

in the direction contrary to left-to-right reading order. A combination of the linking groups, substituents and/or variables thereof is allowed only when such combination can result in a stable compound.

Unless otherwise specified, the number of atoms in a ring refers to the count of atoms constituting the ring itself in compounds formed by atomic bonding into cyclic structures (such as monocyclic compounds, fused ring compounds, spiro compounds, bridged ring compounds, cross-linked compounds, carbocyclic compounds, and heterocyclic compounds). The number of atoms in a ring is usually defined as the number of ring members, for example, "4-to 6-membered ring" refers to a "ring" in which 4 to 6 atoms are arranged around. When a ring is substituted with a substituent, the atoms contained in the substituent are not included in the ring-forming atoms. Unless otherwise specified, benzene is a 6-membered ring, naphthalene is a 10-membered ring, and thiophene is a 5-membered ring.

Unless otherwise defined, the term "alkyl" refers to a saturated hydrocarbon group comprising primary (normal) carbon atoms, or secondary carbon atoms, or tertiary carbon atoms, or quaternary carbon atoms, or combinations thereof, which may represent a linear and/or branched alkyl group, and which can be monovalent (such as methyl), divalent (such as methylene), or multivalent (such as methine). Unless otherwise specifically indicated in the specification, the alkyl may

be optionally substituted.

Unless otherwise specified, the term "$C_{1-20}$ alkyl" refers to a linear or branched saturated hydrocarbon group consisting of 1 to 20 carbon atoms. The $C_{1-20}$ alkyl includes $C_{1-19}$, $C_{1-15}$, $C_{1-10}$, $C_{1-5}$, $C_{1-4}$, $C_{2-20}$, $C_{2-12}$, $C_{2-6}$ alkyl, etc.; it can be monovalent (such as methyl), divalent (such as methylene), or multivalent (such as methine). Examples of $C_{1-20}$ alkyl include, but are not limited to, methyl, ethyl, n-propyl, sec-butyl, n-pentyl, n-hexyl, 1-methylhexyl, n-nonyl, n-decyl, adamantyl, n-undecyl, n-dodecyl, 2-ethyldodecyl, 2-butyldodecyl, n-eicosyl, methylene, 1,2-ethylene, 1,3-propylene, 1,4-butylene, 1,5-pentylene, 1,6-hexylene, 1,7-heptylene, 1,8-octylene, 1,9-nonylene, 1,10-decylene, 1,12-dodecylene, 1,14-tetradecylene, 1,16-hexadecylene, 1,18-octadecylene, 1,20-eicosylene, etc.

Unless otherwise specified, the term "$C_{1-6}$ alkyl" refers to a linear or branched saturated hydrocarbon group consisting of 1 to 6 carbon atoms. The $C_{1-6}$ alkyl includes $C_{1-5}$ alkyl, $C_{1-4}$ alkyl, $C_{2-6}$ alkyl, etc.; it can be monovalent (such as methyl), divalent (such as methylene), or multivalent (such as methine). Examples of $C_{1-6}$ alkyl include, but are not limited to, methyl ("Me"), ethyl ("Et"), propyl such as n-propyl ("n-Pr") or isopropyl ("i-Pr"), butyl such as n-butyl ("n-Bu"), isobutyl ("i-Bu"), sec-butyl ("s-Bu"), or *tert*-butyl ("t-Bu"), pentyl, hexyl, methylene, 1,2-ethylene, 1,3-propylene, 1,4-butylene, 1,5-pentylene, 1,6-hexylene, etc.

Unless otherwise specified, the term "$C_{1-3}$ alkyl" refers to a linear or branched saturated hydrocarbon group consisting of 1 to 3 carbon atoms. The $C_{1-3}$ alkyl includes $C_{1-2}$ alkyl, $C_{2-3}$ alkyl, etc.; it can be monovalent (such as methyl), divalent (such as methylene), or multivalent (such as methine). Examples of $C_{1-3}$ alkyl include, but are not limited to, methyl (Me), ethyl (Et), propyl (including n-propyl and isopropyl), methylene, 1,2-ethylene, 1,3-propylene, etc.

Unless otherwise specified, the term "alkenyl" refers to a hydrocarbon group containing at least one unsaturated site, i.e., a carbon-carbon $sp^2$ double bond, which may represent a linear and/or branched alkenyl group, wherein branched means one or more alkyl groups such as methyl, ethyl, or propyl attached to a linear alkenyl chain. It can be monovalent, divalent, or multivalent. Unless otherwise specifically stated in the specification, the alkenyl may be optionally substituted.

Unless otherwise specified, "$C_{2-20}$ alkenyl" refers to a linear or branched hydrocarbon group consisting of 2 to 20 carbon atoms containing at least one carbon-carbon double bond, and the carbon-carbon double bond may be located at any position of the group. The $C_{2-20}$ alkenyl includes $C_{2-19}$, $C_{2-15}$, $C_{2-10}$, $C_{2-5}$, $C_{2-4}$, $C_{3-20}$, $C_{4-12}$, $C_{5-6}$ alkenyl, etc; it can be monovalent, divalent, or multivalent. Examples of $C_{2-20}$ alkenyl include, but are not limited to, vinyl, propenyl, n-butenyl, 3-methylbut-2-enyl, pentenyl, hexenyl, butadienyl, pentadienyl, hexadienyl, octenyl, decenyl, n-undecenyl, vinylene, propenylene, sec-butenylene, 2-methylbutenylene, etc.

Unless otherwise specified, "$C_{2-6}$ alkenyl" refers to a linear or branched hydrocarbon group consisting of 2 to 6 carbon atoms containing at least one carbon-carbon double bond, and the carbon-carbon double bond may be located at any position of the group. The $C_{2-6}$ alkenyl includes $C_{2-4}$ alkenyl, $C_{2-3}$ alkenyl, $C_4$ alkenyl, $C_3$ alkenyl, $C_2$ alkenyl, etc; it can be monovalent, divalent, or multivalent. Examples of $C_{2-6}$ alkenyl include, but are not limited to, vinyl, propenyl, butenyl, pentenyl, hexenyl, butadienyl, pentadienyl, hexadienyl, vinylene, propenylene, sec-butenylene, etc.

Unless otherwise specified, "$C_{2-3}$ alkenyl" refers to a linear or branched hydrocarbon group consisting of 2 to 3 carbon atoms containing at least one carbon-carbon double bond, and the carbon-carbon double bond may be located at any position of the group. The $C_{2-3}$ alkenyl includes $C_3$ and $C_2$ alkenyl; the $C_{2-3}$ alkenyl can be monovalent, divalent, or multivalent. Examples of $C_{2-3}$ alkenyl include, but are not limited to, vinyl, propenyl, vinylene, propenylene, etc.

Unless otherwise specified, the term "alkynyl" refers to a hydrocarbon group containing at least one unsaturated site, i.e., a carbon-carbon sp triple bond, which may represent a linear and/or branched alkynyl group, wherein branched means one or more alkyl groups such as methyl, ethyl, or propyl attached to a linear alkynyl chain. It can be monovalent, divalent, or multivalent. Unless otherwise specifically stated in the specification, the alkynyl may be optionally substituted.

Unless otherwise specified, the term "$C_{2-20}$ alkynyl" refers to a linear or branched hydrocarbon group consisting of 2 to 20 carbon atoms containing at least one carbon-carbon triple bond, and the carbon-carbon triple bond may be located at any position of the group. The $C_{2-20}$ alkynyl includes $C_{2-19}$, $C_{2-15}$, $C_{2-10}$, $C_{2-5}$, $C_{2-4}$, $C_{3-20}$, $C_{4-12}$, $C_{5-6}$ alkynyl, etc.; it can be monovalent, divalent, or multivalent. Examples of $C_{2-20}$ alkynyl include, but are not limited to, ethynyl, propynyl, ethynylene, propynylene, pentynyl, pentynylene, 1-butynyl, butadiynyl, cyclopropylethynyl, 3-methyl-2-pentynylene, etc.

Unless otherwise specified, the term "$C_{2-6}$ alkynyl" refers to a linear or branched hydrocarbon group consisting of 2 to 6 carbon atoms containing at least one carbon-carbon triple bond, and the carbon-carbon triple bond may be located at any position of the group. It can be monovalent, divalent, or multivalent. The $C_{2-6}$ alkynyl includes $C_{2-5}$, $C_{2-4}$, $C_{2-3}$, $C_2$, $C_{2-6}$, $C_6$, and $C_5$alkynyl, etc. Examples of $C_{2-6}$ alkynyl include, but are not limited to, ethynyl, propynyl, ethynylene, propynylene, pentynyl, pentynylene, etc.

Unless otherwise specified, "$C_{2-3}$ alkynyl" refers to a linear or branched hydrocarbon group consisting of 2 to 3 carbon atoms containing at least one carbon-carbon triple bond, and the carbon-carbon triple bond may be located at any position of the group. It can be monovalent, divalent, or multivalent. The $C_{2-3}$ alkynyl includes $C_3$ and $C_2$ alkynyl. Examples of $C_{2-3}$ alkynyl include, but are not limited to, ethynyl, propynyl, ethynylene, propynylene, etc.

Unless otherwise specified, the term "heteroalkyl" by itself or in combination with another term refers to a stable linear or branched alkyl radical or combination thereof, consisting of a specified number of carbon atoms and at least one

heteroatom or heteroatom group, wherein "alkyl" in "alkyl radical" is as defined above in the present disclosure. In some embodiments, the heteroatom is selected from the group consisting of B, O, N, and S, wherein the nitrogen and sulfur atoms are optionally oxidized, and the nitrogen heteroatom is optionally quaternized. In other embodiments, the heteroatom group is selected from the group consisting of -C(=O)O-, -C(=O)-, -C(=S)-, -S(=O), $-S(=O)_2-$, -C(=O)N(H)-, -N(H)-, - C(=NH)-, $-S(=O)_2N(H)-$, and -S(=O)N(H)-. In some embodiments, the heteroalkyl is $C_{1-20}$ heteroalkyl; in some embodiments, the heteroalkyl is $C_{1-6}$ heteroalkyl; in other embodiments, the heteroalkyl is $C_{1-3}$ heteroalkyl. The heteroatom or heteroatom group may be located at any internal position of the heteroalkyl, including the position where the alkyl is connected to the rest of the molecule. Examples of heteroalkyl include, but are not limited to, $-OCH_3$, $- OCH_2CH_3$, $-OCH_2CH_2CH_3$, $-OCH_2(CH_3)_2$, $-CH_2-CH_2-O-CH_3$, $-NHCH_3$, $-N(CH_3)_2$, $- NHCH_2CH_3$, $-N(CH_3)(CH_2CH_3)$, $-CH_2-CH_2-NH-CH_3$, $-CH_2-CH_2-N(CH_3)-CH_3$, $-SCH_3$, $- SCH_2CH_3$, $-SCH_2CH_2CH_3$, $-SCH_2(CH_3)_2$, $-CH_2-S-CH_2-CH_3$, $-CH_2-CH_2$, $-S(=O)-CH_3$, $-CH_2-CH_2-S(=O)_2-CH_3$, etc.; up to two heteroatoms may be consecutive, such as $-CH_2-NH-OCH_3$. Unless otherwise specifically stated in the specification, the heteroalkyl may be optionally substituted. Unless otherwise specified, the term "alkoxy" refers to an alkyl group that is connected to the rest of the molecule through an oxygen atom, wherein "alkyl" in the "alkyl group" is as defined above in the present disclosure. Unless otherwise specifically stated in the specification, the alkoxy may be optionally substituted.

Unless otherwise specified, the term "$C_{1-20}$ alkoxy" refers to an alkyl group containing 1 to 20 carbon atoms that is connected to the rest of the molecule through an oxygen atom. The $C_{1-20}$ alkoxy includes $C_{1-19}$, $C_{1-10}$, $C_{1-5}$, $C_{2-20}$, $C_{2-8}$, $C_6$, $C_5$, $C_4$ alkoxy, etc. Examples of $C_{1-20}$ alkoxy include, but are not limited to, methoxy, ethoxy, propoxy (including n-propoxy and isopropoxy), butoxy (including n-butoxy, isobutoxy, s-butoxy, and t-butoxy), pentyloxy (including n-pentyloxy, isopentyloxy, and neopentyloxy), hexyloxy, n-hexyloxy, 1-methylhexyloxy, n-nonyloxy, n-decyloxy, n-undecyloxy, n-dodecyloxy, 2-ethyldodecyloxy, n-icosyloxy, methyleneoxy, ethyleneoxy, propyleneoxy, butyleneoxy, pentyleneoxy, heptyleneoxy, dodecyloxy, etc.

Unless otherwise specified, the term "$C_{1-6}$ alkoxy" refers to an alkyl group containing 1 to 6 carbon atoms that is connected to the rest of the molecule through an oxygen atom. The $C_{1-6}$ alkoxy includes $C_{1-4}$, $C_{1-3}$, $C_{1-2}$, $C_{2-6}$, $C_{2-4}$, $C_6$, $C_5$, $C_4$, $C_3$ alkoxy, etc. Examples of $C_{1-6}$ alkoxy include, but are not limited to, methoxy, ethoxy, propoxy (including n-propoxy and isopropoxy), butoxy (including n-butoxy, isobutoxy, s-butoxy, and t-butoxy), pentyloxy (including n-pentyloxy, isopentyloxy, and neopentyloxy), hexyloxy, methyleneoxy, ethyleneoxy, propyleneoxy, butyleneoxy, pentyleneoxy, etc.

Unless otherwise specified, the term "$C_{1-4}$ alkoxy" refers to an alkyl group containing 1 to 4 carbon atoms that is connected to the rest of the molecule through an oxygen atom. The $C_{1-4}$ alkoxy includes $C_{1-3}$, $C_{1-2}$, $C_{2-4}$, $C_4$, $C_3$ alkoxy, etc. Examples of $C_{1-6}$ alkoxy include, but are not limited to, methoxy, ethoxy, propoxy (including n-propoxy and isopropoxy), butoxy (including n-butoxy, isobutoxy, s-butoxy, and t-butoxy), methyleneoxy, ethyleneoxy, propyleneoxy, butyleneoxy, etc.

Unless otherwise specified, the term "$C_{1-3}$ alkoxy" refers to an alkyl group containing 1 to 3 carbon atoms that is connected to the rest of the molecule through an oxygen atom. The $C_{1-3}$ alkoxy includes $C_{1-2}$, $C_{2-3}$, $C_3$, $C_2$ alkoxy, *etc.* Examples of $C_{1-3}$ alkoxy include, but are not limited to, methoxy, ethoxy, propoxy (including n-propoxy and isopropoxy), methyleneoxy, ethyleneoxy, propyleneoxy, etc.

Unless otherwise specified, the term "amino" may be monovalent $--NH_2$, divalent

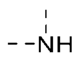,

, or multivalent

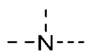.

Unless otherwise specified, the term "alkylamino" refers to an alkyl group connected to the rest of the molecule through an amino group as defined above, wherein "alkyl" in the "alkyl group" is as defined above in the present disclosure. Unless otherwise specifically stated in the specification, the alkylamino may be optionally substituted.

Unless otherwise specified, the term "$C_{1-20}$ alkylamino" refers to an alkyl group containing 1 to 20 carbon atoms that is connected to the rest of the molecule through an amino group. The $C_{1-20}$ alkylamino includes $C_{1-19}$, $C_{1-14}$, $C_{1-12}$, $C_{2-6}$, $C_{2-4}$, $C_{15}$, $C_{10}$, $C_8$, $C_5$, $C_{20}$ alkylamino, etc. Examples of $C_{1-20}$ alkylamino include, but are not limited to, $-NHCH_3$, $-N(CH_3)_2$, $-NHCH_2CH_3$, $-N(CH_3)CH_2CH_3$, $-N(CH_2CH_3)(CH_2CH_3)$, $-NHCH_2CH_2CH_3$, $- NHCH_2(CH_3)_2$, $-NHCH_2CH_2CH_2CH_3$, $-NHCH_2CH_2CH_2CH_2CH_3$, $- NHCH_2CH_2CH_2CH_2CH_2CH_3$, $-N(CH_2CH_2CH_3)(CH_2CH_2CH_2CH_3)$, etc.

Unless otherwise specified, the term "$C_{1-6}$ alkylamino" refers to an alkyl group containing 1 to 6 carbon atoms that is connected to the rest of the molecule through an amino group. The $C_{1-6}$ alkylamino includes $C_{1-4}$, $C_{1-3}$, $C_{1-2}$, $C_{2-6}$, $C_{2-4}$,

$C_6$, $C_5$, $C_4$, $C_3$, $C_2$ alkylamino, etc. Examples of $C_{1-6}$ alkylamino include, but are not limited to, $-NHCH_3$, $-N(CH_3)_2$, $-NHCH_2CH_3$, $-N(CH_3)CH_2CH_3$, $-N(CH_2CH_3)(CH_2CH_3)$, $-NHCH_2CH_2CH_3$, $-NHCH_2(CH_3)_2$, $-NHCH_2CH_2CH_2CH_3$, etc.

Unless otherwise specified, the term "$C_{1-4}$ alkylamino" refers to an alkyl group containing 1 to 4 carbon atoms that is connected to the rest of the molecule through an amino group. The $C_{1-4}$ alkylamino includes $C_{1-3}$, $C_{1-2}$, $C_{2-4}$, $C_4$, $C_3$, $C_2$ alkylamino, etc. Examples of $C_{1-4}$ alkylamino include, but are not limited to, $-NHCH_3$, $-N(CH_3)_2$, $-NHCH_2CH_3$, $-N(CH_3)CH_2CH_3$, $-N(CH_2CH_3)(CH_2CH_3)$, $-NHCH_2CH_2CH_3$, $-NHCH_2(CH_3)_2$, $-NHCH_2CH_2CH_2CH_3$, etc.

Unless otherwise specified, the term "$C_{1-3}$ alkylamino" refers to an alkyl group containing 1 to 3 carbon atoms that is connected to the rest of the molecule through an amino group. The $C_{1-3}$ alkylamino includes $C_{1-2}$, $C_3$, $C_2$ alkylamino, etc. Examples of $C_{1-3}$ alkylamino include, but are not limited to, $-NHCH_3$, $-N(CH_3)_2$, $-NHCH_2CH_3$, $-N(CH_3)CH_2CH_3$, $-NHCH_2CH_2CH_3$, $-NHCH_2(CH_3)_2$, etc.

Unless otherwise specified, the term "alkylthio" refers to an alkyl group that is connected to the rest of the molecule through a sulfur atom, wherein "alkyl" in the "alkyl group" is as defined above in the present disclosure. Unless otherwise specifically stated in the specification, the alkylthio may be optionally substituted.

Unless otherwise specified, the term "$C_{1-20}$ alkylthio" refers to an alkyl group containing 1 to 20 carbon atoms that is connected to the rest of the molecule through a sulfur atom. The $C_{1-20}$ alkylthio includes $C_{1-19}$, $C_{1-14}$, $C_{1-12}$, $C_{2-6}$, $C_{2-4}$, $C_{15}$, $C_{10}$, $C_8$, $C_5$, $C_{20}$ alkylthio, etc. Examples of $C_{1-20}$ alkylthio include, but are not limited to, $-SCH_3$, $-SCH_2CH_3$, $-SCH_2CH_2CH_3$, $-SCH_2(CH_3)_2$, $-SCH_2CH_2CH_2CH_3$, $-SCH_2CH_2(CH_3)_2$, $-SCH_2CH_2CH_2CH_2CH_3$, $-SCH_2CH_2CH_2CH_2CH_2CH_3$, $-SCH_2(CH_2CH_2CH_3)(CH_2CH_2CH_2CH_3)$, etc.

Unless otherwise specified, the term "$C_{1-6}$ alkylthio" refers to an alkyl group containing 1 to 6 carbon atoms that is connected to the rest of the molecule through a sulfur atom. The $C_{1-6}$ alkylthio includes $C_{1-4}$ alkylthio, $C_{1-3}$ alkylthio, $C_{1-2}$ alkylthio, $C_{2-6}$ alkylthio, $C_{2-4}$ alkylthio, $C_6$ alkylthio, $C_5$ alkylthio, $C_4$ alkylthio, $C_3$ alkylthio, $C_2$ alkylthio, etc. Examples of $C_{1-6}$ alkylthio include, but are not limited to, $-SCH_3$, $-SCH_2CH_3$, $-SCH_2CH_2CH_3$, $-SCH_2(CH_3)_2$, etc.

Unless otherwise specified, the term "$C_{1-4}$ alkylthio" refers to an alkyl group containing 1 to 4 carbon atoms that is connected to the rest of the molecule through a sulfur atom. The $C_{1-4}$ alkylthio includes $C_{1-3}$, $C_{1-2}$, $C_{2-4}$, $C_4$, $C_3$, $C_2$ alkylthio, etc. Examples of $C_{1-4}$ alkylthio include, but are not limited to, $-SCH_3$, $-SCH_2CH_3$, $-SCH_2CH_2CH_3$, $-SCH_2(CH_3)_2$, etc.

Unless otherwise specified, the term "$C_{1-3}$ alkylthio" refers to an alkyl group containing 1 to 3 carbon atoms that is connected to the rest of the molecule through a sulfur atom. The $C_{1-3}$ alkylthio includes $C_{1-3}$, $C_{1-2}$, $C_3$ alkylthio, etc. Examples of $C_{1-3}$ alkylthio include, but are not limited to, $-SCH_3$, $-SCH_2CH_3$, $-SCH_2CH_2CH_3$, $-SCH_2(CH_3)_2$, etc.

Unless otherwise specified, the term "cycloalkyl" refers to a stable non-aromatic monocyclic or polycyclic saturated hydrocarbon group consisting of carbon and hydrogen atoms, which may include fused, spiro, and/or bridged ring systems. Monocyclic cycloalkyl groups include, but are not limited to, cyclopropyl, cyclobutyl, cyclopentyl, cyclohexyl, cycloheptyl, and cyclooctyl. Polycyclic cycloalkyl groups include, but are not limited to, adamantyl, norbornyl, decahydronaphthyl, 7,7-dimethyl-bicyclo[2.2.1]heptyl, etc. "$C_{4-6}$ cycloalkyl" refers to a cycloalkyl group having 4 to 6 ring carbon atoms. Similarly, "$C_{3-4}$ cycloalkyl" refers to a cycloalkyl group having 3-4 ring carbon atoms. Unless otherwise specifically stated in the specification, the cycloalkyl may be optionally substituted.

Unless otherwise specified, "$C_{3-20}$ cycloalkyl" refers to a saturated monocyclic or polycyclic hydrocarbon group having 3-20 ring carbon atoms, for example having 3-15 ring carbon atoms, for example 3-6 ring carbon atoms; it can be monovalent, divalent, or multivalent. Examples of $C_{3-20}$ cycloalkyl include, but are not limited to, cyclopropyl, cyclobutyl, cyclopentyl, cyclohexyl, cycloheptyl, cyclooctyl, etc.

Unless otherwise specified, "$C_{3-6}$ cycloalkyl" refers to a saturated monocyclic or bicyclic hydrocarbon group having 3-6 ring carbon atoms, for example having 3-5 ring carbon atoms, for example 3-4 ring carbon atoms; it can be monovalent, divalent, or multivalent. Examples of $C_{3-6}$ cycloalkyl include, but are not limited to, cyclopropyl, cyclobutyl, cyclopentyl, cyclohexyl, etc.

Unless otherwise specified, "$C_{4-6}$ cycloalkyl" refers to a saturated cyclic hydrocarbon group consisting of 4 to 6 carbon atoms in monocyclic and bicyclic systems, and the $C_{4-6}$ cycloalkyl includes $C_{4-5}$ cycloalkyl, $C_{5-6}$ cycloalkyl, $C_4$ cycloalkyl, $C_5$ cycloalkyl, $C_6$ cycloalkyl, etc; it can be monovalent, divalent, or multivalent. Examples of $C_{4-6}$ cycloalkyl include, but are not limited to, cyclobutyl, cyclopentyl, cyclohexyl, etc.

Unless otherwise specified, the term "heterocycloalkyl" refers to a non-aromatic saturated cyclic group existing as a monocyclic ring, fused ring, spiro ring, and/or bridged ring, wherein at least one ring atom is a heteroatom or heteroatom group and the rest are carbon atoms; in some embodiments, each occurrence of a heteroatom is independently selected from the group consisting of B, O, N, and S, wherein nitrogen and sulfur atoms are optionally oxidized (i.e., NO and $S(O)_p$, where p is 1 or 2), and nitrogen heteroatoms are optionally quaternized; in other embodiments, each occurrence of a heteroatom group is independently selected from the group consisting of -C(=O)O-, -C(=O)-, -C(=S)-, -S(=O)-, $-S(=O)_2-$, -C(=O)N(H)-, -N(H)-, -C(=NH)-, $-S(=O)_2N(H)-$, and -S(=O)N(H)-. The heteroatom or heteroatom group may be located at any internal position of the heterocycloalkyl, including the position where the heterocycloalkyl is connected to the rest of the molecule. In some embodiments, the heterocycloalkyl is 3- to 20-membered heterocycloalkyl; in some embodiments, the heterocycloalkyl is 3- to 10-membered heterocycloalkyl; in other embodiments, the heterocycloalkyl is 3- to 6-membered heterocycloalkyl. Unless otherwise specifically stated in the specification, the heterocycloalkyl may be optionally substituted. Unless otherwise specified, the term "3- to 6-membered heterocycloalkyl" by itself or in combination with other terms refers to a saturated cyclic group consisting of 3 to 6 ring atoms, wherein 1, 2, 3, or 4 ring atoms are heteroatoms independently selected from the group consisting of B, O, S, and N, or heteroatom groups as described above, and the rest are carbon atoms, wherein nitrogen atoms are optionally quaternized, and nitrogen and sulfur heteroatoms can be optionally oxidized (i.e., NO and $S(O)_p$, p is 1 or 2). It includes monocyclic and bicyclic systems, wherein the bicyclic system includes a spiro ring, a fused ring, and a bridged ring. Furthermore, with respect to the "3- to 6-membered heterocycloalkyl," the heteroatom or heteroatom group may be located at any internal position of the heterocycloalkyl, including the position where the heterocycloalkyl is connected to the rest of the molecule. The 3- to 6-membered heterocycloalkyl includes 5- to 6-membered, 4-membered, 5-membered, 6-membered heterocycloalkyl, etc. Examples of 3- to 6-membered heterocycloalkyl include, but are not limited to, azetidinyl, oxetanyl, thietanyl, pyrrolidinyl, pyrazolidinyl, imidazolidinyl, tetrahydrothiophenyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxolyl, dithianyl, isoxazolidinyl, isothiazolidinyl, 1,2-oxazinyl, 1,2-thiazinyl, hexahydropyridazinyl, homopiperazinyl, homopiperidinyl, etc.

Unless otherwise specified, the term "cycloalkenyl" in the present disclosure refers to a stable non-aromatic monocyclic or polycyclic hydrocarbon group consisting of carbon and hydrogen atoms, having one or more carbon-carbon $sp^2$ double bonds, which may include fused, spiro, and/or bridged ring systems. Monocyclic cycloalkenyl includes, but is not limited to, cyclopentenyl, cyclohexenyl, cycloheptenyl, cyclooctenyl, etc. Polycyclic cycloalkenyl includes, but is not limited to, bicyclo[2.2.1]hept-2-enyl, etc. Unless otherwise specifically stated in the specification, the cycloalkenyl may be optionally substituted. "$C_{3-7}$ cycloalkenyl" includes $C_3$, $C_4$, $C_5$, $C_6$, and $C_7$ cycloalkeny. Examples of cycloalkenyl include, but are not limited to, cyclobutenyl, cyclopentenyl, and cyclohexenyl.

Unless otherwise specified, the term "heterocycloalkenyl" in the present disclosure refers to a cyclic alkenyl group containing several heteroatoms or heteroatom groups. In some embodiments, each occurrence of a heteroatom is independently selected from the group consisting of B, O, N, and S, wherein nitrogen and sulfur atoms are optionally oxidized (i.e., NO and $S(O)_p$, where p is 1 or 2), and nitrogen heteroatoms are optionally quaternized. In other embodiments, each occurrence of a heteroatom group is independently selected from the group consisting of -C(=O)O-, -C(=O)-, -C(=S)-, -S(=O)-, $-S(=O)_2-$, -C(=O)N(H)-, -N(H)-, - C(=NH)-, $-S(=O)_2N(H)-$, and -S(=O)N(H)-. The term "5- to 6-membered heterocycloalkenyl" by itself or in combination with other terms refers to an unsaturated cyclic group consisting of 5 to 6 ring atoms, respectively, wherein 1, 2, 3, or 4 ring atoms are heteroatoms independently selected from the group consisting of B, O, S, and N, or heteroatom groups as described above, and the rest are carbon atoms, wherein the nitrogen atoms are optionally quaternized, and the nitrogen and sulfur heteroatoms are optionally oxidized (i.e., NO and $S(O)_p$, where p is 1 or 2). Examples of heterocycloalkenyl include, but are not limited to,

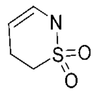,

etc. Unless otherwise specifically stated in the specification, the heterocycloalkenyl may be optionally substituted.

Unless otherwise specified, when a substituent attached to ring A can be connected to ring A to form a ring, it means that the substituent can be connected to any site of ring A to form a new ring with ring A, including a fused ring, a spiro ring, or a bridged ring; wherein ring A may be selected from the group consisting of cycloalkyl, heterocycloalkyl, cycloalkenyl, heterocycloalkenyl, aryl, heteroaryl, etc. as described above. For example, when R in

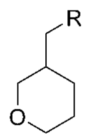

can be connected to

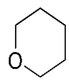

form a 6-membered ring, examples of which include but are not limited to

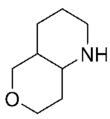, 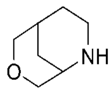, 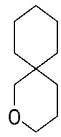,

etc.

Unless otherwise specified, $C_{n-n+m}$ or $C_n$-$C_{n+m}$ includes any specific instance of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$, and $C_{12}$, also includes any range from n to n+m, for example, $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, $C_{9-12}$, etc.; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, also includes any range from n to n+m, for example, 3- to 12-membered ring includes 3-to 6-membered ring, 3- to 9-membered ring, 5- to 6-membered ring, 5- to 7-membered ring, 6-to 7-membered ring, 6- to 8-membered ring, 6- to 10-membered ring, etc.

Unless otherwise specified, the term "aryl" refers to a hydrocarbon ring system group containing at least one aromatic ring. In the present disclosure, the aryl may be a monocyclic, bicyclic, tricyclic, or tetracyclic ring system, which may include fused, spiro, and/or bridged ring systems. The aryl includes, but is not limited to, benzene, naphthalene, anthracene, fluoranthene, phenanthrene, triphenylene, perylene, tetracene, pyrene, benzopyrene, acenaphthene, fluorene, and derivative groups thereof. Unless otherwise specifically stated in the specification, the aryl may optionally be substituted.

Unless otherwise specified, the term "heteroaryl" refers to a heteroaromatic system containing 1 to 4 heteroatoms and 5 to 20 ring atoms, wherein the heteroatoms are selected from the group consisting of oxygen, sulfur, and nitrogen. The heteroaryl is preferably 5- to 10-membered, containing 1 to 3 heteroatoms; more preferably 5- or 6-membered, containing 1 to 3 heteroatoms; non-limiting examples include pyrazolyl, imidazolyl, furanyl, thienyl, thiazolyl, oxazolyl, pyrrolyl, triazolyl, tetrazolyl, pyridyl, pyrimidinyl, thiadiazolyl, pyrazinyl, etc. The heteroaryl can be connected to the rest of the molecule through a heteroatom or a carbon atom. The heteroaryl ring may be fused to an aryl, heterocyclyl, or cycloalkyl ring, wherein the ring attached to the parent structure is a heteroaryl ring, and non-limiting examples thereof include:

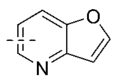 and 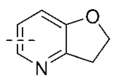.

Non-limiting examples of heteroaryl further include triazine, pyridine, pyrimidine, imidazole, furan, thiophene, benzofuran, benzothiophene, indole, carbazole, pyrroloimidazole, pyrrolopyrrole, thienopyrrole, thienothiophene, furanopyrrole, furanofuran, thienofuran, benzoisoxazole, benzoisothiazole, benzimidazole, quinoline, isoquinoline, naphthyridine, quinoxaline, phenanthridine, perimidine, quinazoline, quinazolinone, dibenzothiophene, dibenzofuran, carbazole, and derivatives thereof. Unless otherwise specifically stated in the specification, the heteroaryl may be optionally substituted or unsubstituted. When substituted, the substituent is preferably one or more groups independently selected from the group consisting of alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, and heterocycloalkylthio.

The term "substituted" as used in the present disclosure means that in any of the above groups (i.e., alkyl, alkenyl, alkynyl, heteroalkyl, alkoxy, alkylamino, alkylthio, cycloalkyl, heterocycloalkyl, cycloalkenyl, heterocycloalkenyl, aryl, heteroaryl), at least one hydrogen atom is replaced by a bond to a non-hydrogen atom, including but not limited to halogen atoms (such as F, Cl, Br, I), groups containing oxygen atoms (such as hydroxyl, alkoxy, ester), groups containing sulfur atoms (such as a thiol group, thioalkyl group, sulfone group, sulfonyl group, or sulfinyl group), groups containing nitrogen atoms (such as amino, amido, alkylamino, dialkylamino, arylamino, aryl-alkyl-amino, diarylamino, N-oxide group, imido, enamino), groups containing silicon atoms (such as trialkylsilyl, dialkylarylsilyl, alkyldiarylsilyl, triarylsilyl), and other heteroatoms in various other groups.

The term "substituted" as used in the present disclosure also means that in any of the above groups (i.e., alkyl, alkenyl, alkynyl, heteroalkyl, alkoxy, alkylamino, alkylthio, cycloalkyl, heterocycloalkyl, cycloalkenyl, heterocycloalkenyl, aryl, heteroaryl), one or more hydrogen atoms are replaced by a higher-order bond (such as a double bond or triple bond) to a heteroatom, for example, oxygen in carbonyl, carboxyl, and ester groups, and nitrogen in imine, oxime, hydrazone, and nitrile groups. For example, "substituted" means that one or more hydrogen atoms in any of the above groups are replaced by $-NR_gR_h$, $-NR_gC(=O)R_h$, $-NR_gC(=O)NR_gR_h$, $-NR_gC(=O)OR_h$, $-NR_gSO_2R_h$, $-OC(=O)NR_gR_h$, $-OR_g$, $-SR_g$, $-SOR_g$, $-SO_2R_g$, $-OSO_2R_g$, $-SO_2OR_g$, $=NSO_2R_g$, and $-SO_2NR_gR_h$. The "substituted" may also mean that one or more hydrogen atoms in any of the above groups are replaced by $-C(=O)R_g$, $-C(=O)OR_g$, $-C(=O)NR_gR_h$, $-CH_2SO_2R_g$, and

$-CH_2SO_2NR_gR_h$. The $R_g$ and $R_h$ are the same or different and are independently selected from the group consisting of hydrogen, alkyl, alkenyl, alkynyl, alkoxy, alkylamino, thioalkyl, aryl, aralkyl, cycloalkyl, cycloalkenyl, cycloalkynyl, cycloalkyl-alkyl, haloalkyl, haloalkenyl, haloalkynyl, heterocyclyl, N-heterocyclyl, heterocycloalkyl-alkyl, heteroaryl, N-heteroaryl, and heteroaryl-alkyl. The "substituted" may also mean that one or more hydrogen atoms in any of the above groups are replaced by amino, cyano, hydroxyl, imino, nitro, oxo, thio, halogen, alkyl, alkenyl, alkynyl, alkoxy, alkylamino, thioalkyl, aryl, aralkyl, cycloalkyl, cycloalkenyl, cycloalkynyl, cycloalkyl-alkyl, haloalkyl, haloalkenyl, haloalkynyl, heterocyclyl, N-heterocyclyl, heterocycloalkyl-alkyl, heteroaryl, N-heteroaryl, and heteroaryl-alkyl. In addition, each of the above substituents may also be optionally substituted with one or more of the above substituents.

It will be understood by those skilled in the art that some compounds of formula (I) can contain one or more than one chiral center. Therefore, the compound has two or more stereoisomers. Therefore, the compounds of the present disclosure can be present in the form of individual stereoisomer (e.g. enantiomers, diastereomers) and mixtures thereof in arbitrary proportions, such as racemates, and under the proper condition, they can be present in the form of the tautomers and geometric isomers thereof.

As used herein, the term "stereoisomer" refers to compounds that have the same chemical constitution, but differ in the arrangement of the atoms or groups in space. Stereoisomer includes enantiomer, diastereomer, conformer, etc.

As used herein, the term "enantiomer" refers to two stereoisomers of a compound that are non-superimposable mirror images of each other.

As used herein, the term "diastereomer" refers to a stereoisomer in which a molecule has two or more chiral centers and whose molecules are not mirror images of each other. Diastereomers have different physical properties such as melting points, boiling points, spectral properties, or biological activities. Diastereomeric mixtures can be separated by high resolution analytical methods such as electrophoresis and chromatography (such as HPLC separation).

Many organic compounds are present in optically active forms, i.e., they have the ability to rotate the plane of plane polarized light. When optically active compounds are described, the prefixes D and L or R and S are used to denote the absolute configuration of the molecule with regard to its chiral centers. The prefixes d and l or (+) and (-) are used to denote the symbols of the compound's rotationally planar polarized light, wherein (-) or 1 indicates that the compound is levorotatory. Compounds with a prefix of (+) or d are dextrorotatory. For a given chemical structure, these stereoisomers are identical except that they are mirror images of each other. Specific stereoisomers can also be referred to as enantiomers, and mixtures of such isomers are often referred to as enantiomeric mixtures. A mixture of enantiomers in a ratio of 50 to 50 is known as a racemic mixture or racemate, which can be present in a chemical reaction or process without stereoselectivity or stereospecificity. The terms "racemic mixture" and "racemate" refer to an equimolar mixture of two enantiomers that is not optically active.

Racemic mixture can be used in its own form or after it is resolved into individual isomers. Resolution may yield stereochemically pure compounds or a mixture enriched in one or more isomers. Methods for separating isomers are well known and include physical methods such as chromatography using chiral adsorbents. Individual isomers in a chiral form can be prepared from chiral precursors. Alternatively, a diastereomer salt can be formed with a chiral acid (such as a single enantiomer of 10-camphorsulfonic acid, camphoric acid, $\alpha$-bromocamphoric acid, tartaric acid, diacetyltartaric acid, malic acid, pyrrolidone-5-carboxylic acid), and then the mixture was chemically separated to obtain a single isomer. The salt is then graded and crystallized, and one or both of the split bases are freed. This process can be optionally repeated to obtain one or two isomers that essentially do not contain the other isomer, i.e., the desired stereoisomer with an optical purity of at least 91%, 92%, 93%, 94%, 95%, 96%, 97%, 98%, 99%, or 99.5% by weight. Alternatively, the racemate can be covalently linked to a chiral compound (auxiliary) to obtain diastereomers, as is well known to those skilled in the art.

The term "tautomer" or "tautomeric form" as used herein refers to structural isomers of different energies that are interconvertible via a low energy barrier. For example, proton tautomers (also called prototropic tautomers) include interconversion through proton migration, such as keto-enol isomerization and imine-enamine isomerization. Valence tautomers include interconversions by recombination of some bonding electrons.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The technical and scientific terms used herein that are not specifically defined have the meanings commonly understood by those skilled in the art to which the present disclosure belongs.

DETAILED DESCRIPTION OF THE PREFERRED EMBODIMENT

The present disclosure The present disclosure is described in detail by the examples below, but it does not mean that there are any adverse restrictions on the present disclosure. The present disclosure has been described in detail herein, and its specific embodiments have also been disclosed; for one skilled in the art, it is obvious to make various modifications and improvements to the embodiments of the present disclosure without departing from the spirit and scope

of the present disclosure.

Example 1: Synthesis of compound 1

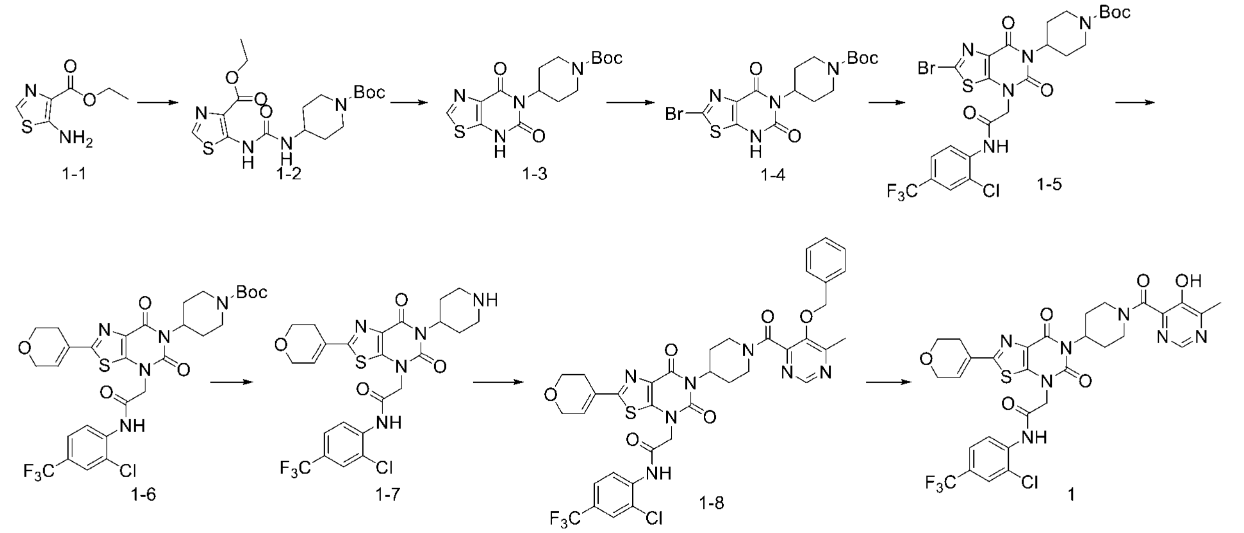

1-1, 1-2, 1-3, 1-4, 1-5, 1-6, 1-7, 1-8, 1

**Step 1: Preparation of compound 1-2**

Compound 1-1 (1.00 g, 5.81 mmol) and triphosgene (1.72 g, 5.81 mmol) were dissolved in ultra-dry tetrahydrofuran (50 mL). Under an ice-water bath and a nitrogen atmosphere, triethylamine (2.35 g, 23.2 mmol) was added dropwise to the reaction system. After the dropwise addition was completed, the reaction system was stirred under an ice-water bath for 1 h. Then, 4-amino-1-*tert*-butoxycarbonylpiperidine (2.02 g, 10.1 mmol) was added. The mixture was naturally warmed to room temperature and stirred for 16 h. LCMS showed the reaction was complete. A saturated aqueous ammonium chloride solution (100 mL) was added to quench the reaction, and the mixture was extracted with ethyl acetate (70 mL $\times$ 3). The organic phases were combined and concentrated under reduced pressure. The resulting residue was purified by reversed-phase column chromatography (water/acetonitrile = 3/2) to obtain compound **1-2** (1.94 g, yield: 84.0%). LC-MS (ESI) $[M+H]^+$ 399.1.

**Step 2: Preparation of compound 1-3**

Under a nitrogen atmosphere, potassium tert-butoxide (1.35 g, 12.0 mmol) was added to a solution of compound **1-2** (800 mg, 2.01 mmol) in ultra-dry tetrahydrofuran (10 mL). The reaction system was stirred at 60°C for 2 h. LCMS showed the reaction was complete. The reaction mixture was concentrated under reduced pressure, and the resulting residue was purified by column chromatography (dichloromethane/methanol = 10/1) to obtain compound **1-3** (338 mg, yield: 47.8%). LC-MS (ESI) $[M+H]^+$ 297.0.

**Step 3: Preparation of compound 1-4**

Under a nitrogen atmosphere, *N*-bromosuccinimide (132 mg, 0.743 mmol) was added to a solution of compound **1-3** (238 mg, 0.675 mmol) in acetonitrile (10 mL). The reaction system was stirred at room temperature for 3 h. LCMS showed the reaction was complete. The reaction mixture was concentrated under reduced pressure, and the resulting residue was purified by reversed-phase column chromatography (water/acetonitrile = 1/1) to obtain compound **1-4** (212 mg, yield: 72.9%). LC-MS (ESI) $[M+H]^+$ 374.9/376.9.

**Step 4: Preparation of compound 1-5**

Under an ice-water bath and a nitrogen atmosphere, *N*-[2-chloro-4-(trifluoromethyl)phenyl]-2-iodoacetamide (202 mg, 0.556 mmol) and *N,N*-diisopropylethylamine (120 mg, 0.927 mmol) were sequentially added to a solution of compound **1-4** (200 mg, 0.464 mmol) in *N,N*-dimethylformamide (5 mL). The reaction system was stirred under an ice-water bath for 2 h. LCMS showed the reaction was complete. Water (20 mL) was added to quench the reaction, and the

mixture was extracted with ethyl acetate (50 mL × 3). The combined organic phases were washed with saturated brine (40 mL × 2), dried over anhydrous sodium sulfate, filtered, and concentrated under reduced pressure. The resulting residue was purified by column chromatography (petroleum ether/ethyl acetate = 2/1) to obtain compound **1-5** (203 mg, yield: 65.7%). LC-MS (ESI) $[M+H-56]^+$ 609.9/611.9.

### Step 5: Preparation of compound 1-6

Compound **1-5** (467 mg, 0.700 mmol), 4-(4,4,5,5-tetramethyl-1,3,2-dioxaborolan-2-yl)-3,6-dihydro-2H-pyran (177 mg, 0.840 mmol), [1,1'-bis(diphenylphosphino)ferrocene]dichloropalladium (14.0 mg, 0.070 mmol), and sodium carbonate (223 mg, 2.10 mmol) were dissolved in 1,4-dioxane/water (10/1 mL). The reaction system was stirred at 80°C for 4 h. LCMS showed the reaction was complete. The reaction mixture was diluted with water (30 mL) and extracted with ethyl acetate (50 mL × 3). The combined organic phases were washed with saturated brine (30 mL), dried over anhydrous sodium sulfate, filtered, and concentrated under reduced pressure. The resulting residue was purified by column chromatography (petroleum ether/ethyl acetate = 1/1) to obtain compound **1-6** (303 mg, yield: 70.5%). LC-MS (ESI) $[M+H]^+$ 670.2.

### Step 6: Preparation of compound 1-7

At room temperature, trifluoroacetic acid (2 mL) was added to a solution of compound **1-6** (118 mg, 176 μmol) in dichloromethane (10 mL). The reaction system was stirred at room temperature for 2 h. LCMS showed the reaction was complete. The reaction mixture was concentrated under reduced pressure to obtain the crude product of compound **1-7** (120 mg). The crude product was directly used in the next step. LC-MS (ESI) $[M+H]^+$ 570.4.

### Step 7: Preparation of compound 1-8

At room temperature, 2-(7-azabenzotriazol-1-yl)-*N,N,N',N'*-tetramethyluronium hexafluorophosphate (79.5 mg, 211 μmol) was added to a solution of the crude product of compound **1-7** (120 mg), 5-benzyloxy-6-methylpyrimidine-4-carboxylic acid (51.5 mg, 211 μmol), and *N,N*-diisopropylethylamine (68.1 mg, 527 μmol) in dichloromethane (10 mL). The reaction system was stirred at room temperature for 4 h. LCMS showed the reaction was complete. The reaction mixture was concentrated under reduced pressure, and the resulting residue was purified by column chromatography (dichloromethane/methanol = 20/1) to obtain compound **1-8** (120 mg, total yield over two steps: 85.6%). LC-MS (ESI) $[M+H]^+$ 796.2.

### Step 8: Preparation of compound 1

Under an ice-water bath and a nitrogen atmosphere, a solution of boron trichloride in dichloromethane (1.00 mol/L, 251 μL, 251 μmol) was added dropwise to a solution of compound **1-8** (100 mg, 126 μmol) in dichloromethane (5 mL). The reaction system was stirred at room temperature for 3 h. LCMS showed the reaction was complete. Water (10 mL) was added to quench the reaction, and the mixture was extracted with dichloromethane (30 mL × 3). The combined organic phases were washed with saturated brine (20 mL), dried over anhydrous sodium sulfate, filtered, and concentrated under reduced pressure. The resulting residue was purified by preparative HPLC to obtain compound **1** (36.0 mg, yield: 40.6%). LC-MS (ESI) $[M+H]^+$ 706.2.

$^1$H NMR (400 MHz, DMSO-$d_6$) δ 10.36 (s, 1H), 10.18 (s, 1H), 8.56 (s, 1H), 8.06 (d, *J* = 8.5 Hz, 1H), 7.98 (d, *J* = 2.1 Hz, 1H), 7.73 (dd, *J* = 9.0, 2.1 Hz, 1H), 6.64 (dd, *J* = 3.1, 1.6 Hz, 1H), 5.09 (s, 1H), 4.96 (d, *J* = 5.1 Hz, 2H), 4.63 (d, *J* = 12.8 Hz, 1H), 4.25 (d, *J* = 2.9 Hz, 2H), 3.81 (t, *J* = 5.4 Hz, 2H), 3.56 (d, *J* = 13.2 Hz, 1H), 3.16 (t, *J* = 12.9 Hz, 1H), 2.89 (s, 1H), 2.55 (s, 4H), 2.43 (s, 3H), 1.73 (d, *J* = 12.0 Hz, 1H), 1.56 (d, *J* = 11.9 Hz, 1H).

### Example 2: Synthesis of compound 2

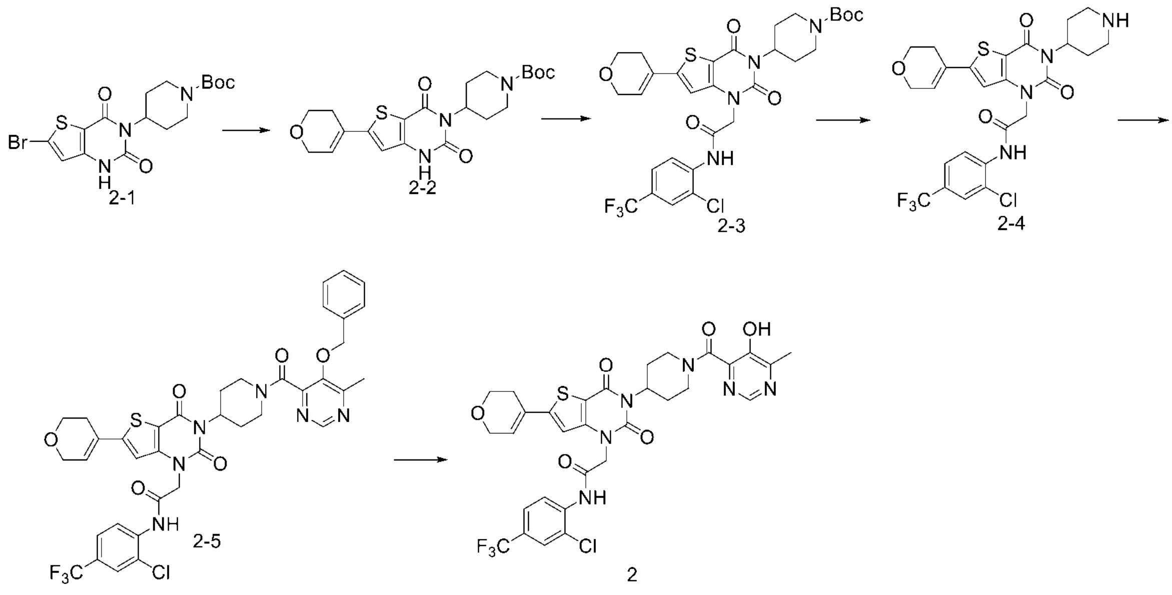

2-1 2-2 2-3 2-4 2-5 2

**Step 1: Preparation of compound 2-2**

Compound 2-1 (800 mg, 1.86 mmol), 4-(4,4,5,5-tetramethyl-1,3,2-dioxaborolan-2-yl)-3,6-dihydro-2H-pyran (469 mg, 2.23 mmol), [1,1'-bis(diphenylphosphino)ferrocene]dichloropalladium (136 mg, 186 μmol), and cesium carbonate (1.82 g, 5.58 mmol) were dissolved in 1,4-dioxane/water (30/5 mL). The reaction system was stirred at 100°C for 12 h. LCMS showed the reaction was complete. The reaction system was cooled to room temperature and concentrated under reduced pressure. The resulting residue was purified by column chromatography (dichloromethane/methanol = 20/1) to obtain compound **2-2** (750 mg, yield: 93.1%). LC-MS (ESI) $[M+H]^+$ 378.0.

**Step 2: Preparation of compound 2-3**

Under a nitrogen atmosphere, *N*-[2-chloro-4-(trifluoromethyl)phenyl]-2-iodoacetamide (629 mg, 1.73 mmol) and *N,N*-diisopropylethylamine (448 mg, 3.46 mmol) were sequentially added to a solution of compound **2-2** (750 mg, 1.73 mmol) in *N,N*-dimethylformamide (5 mL). The reaction system was stirred at 40°C for 4 h. LCMS showed the reaction was complete. Water (20 mL) was added to quench the reaction, and the mixture was extracted with ethyl acetate (50 mL × 3). The combined organic phases were washed with saturated brine (40 mL × 2), dried over anhydrous sodium sulfate, filtered, and concentrated under reduced pressure. The resulting residue was purified by column chromatography (petroleum ether/ethyl acetate = 1/1) to obtain compound **2-3** (770 mg, yield: 66.5%). LC-MS (ESI) $[M+H-56]^+$ 612.8.

**Step 3: Preparation of compound 2-4**

At room temperature, trifluoroacetic acid (2 mL) was added to a solution of compound **2-3** (400 mg, 598 μmol) in dichloromethane (10 mL). The reaction system was stirred at room temperature for 2 h. LCMS showed the reaction was complete. The reaction mixture was concentrated under reduced pressure to obtain the crude product of compound **2-4** (340 mg). The crude product was directly used in the next step. LC-MS (ESI) $[M+H]^+$ 569.0.

**Step 4: Preparation of compound 2-5**

At room temperature, 2-(7-azabenzotriazol-1-yl)-*N,N,N',N'*-tetramethyluronium hexafluorophosphate (232 mg, 717 μmol) was added to a solution of the crude product of compound **2-4** (340 mg), 5-benzyloxy-6-methylpyrimidine-4-carboxylic acid (175 mg, 717 μmol), and *N,N*-diisopropylethylamine (312 μL, 1.79 mmol) in dichloromethane (10 mL). The reaction system was stirred at room temperature for 2 h. LCMS showed the reaction was complete. The reaction mixture was concentrated under reduced pressure, and the resulting residue was purified by column chromatography (dichloromethane/methanol = 20/1) to obtain compound **2-5** (450 mg, total yield over two steps: 94.7%). LC-MS (ESI) $[M+H]^+$ 795.2.

**Step 5: Preparation of compound 2**

Under an ice-water bath and a nitrogen atmosphere, a solution of boron trichloride in dichloromethane (1.00 mol/L, 880 μL, 880 μmol) was added dropwise to a solution of compound **2-5** (350 mg, 440 μmol) in dichloromethane (10 mL). The reaction system was stirred at room temperature for 3 h. LCMS showed the reaction was complete. Water (10 mL) was added to quench the reaction, and the mixture was extracted with dichloromethane (30 mL × 3). The combined organic phases were washed with saturated brine (20 mL), dried over anhydrous sodium sulfate, filtered, and concentrated under reduced pressure. The resulting residue was purified by preparative HPLC to obtain compound **2** (85.0 mg, yield: 27.4%). LC-MS (ESI) $[M+H]^+$ 705.0.

$^1H$ NMR (400 MHz, DMSO-$d_6$) δ 10.20 (d, *J* = 17.6 Hz, 2H), 8.56 (s, 1H), 8.10 (d, *J* = 8.6 Hz, 1H), 7.97 (d, *J* = 2.1 Hz, 1H), 7.72 (dd, *J* = 8.7, 2.2 Hz, 1H), 7.46 (s, 1H), 6.52 (s, 1H), 5.04 (d, *J* = 17.4 Hz, 3H), 4.62 (d, *J* = 12.8 Hz, 1H), 4.24 (d, *J* = 3.2 Hz, 2H), 3.81 (t, *J* = 5.4 Hz, 2H), 3.55 (d, *J* = 13.2 Hz, 1H), 3.13 (t, *J* =12.9 Hz, 1H), 2.86 (t, *J* = 12.9 Hz, 1H), 2.62 - 2.53 (m, 2H), 2.48 - 2.44 (m, 2H), 2.43 (s, 3H), 1.72 (d, J=11.9 Hz, 1H), 1.55 (d, *J* = 12.1 Hz, 1H).

**Example 3: Synthesis of compound 3**

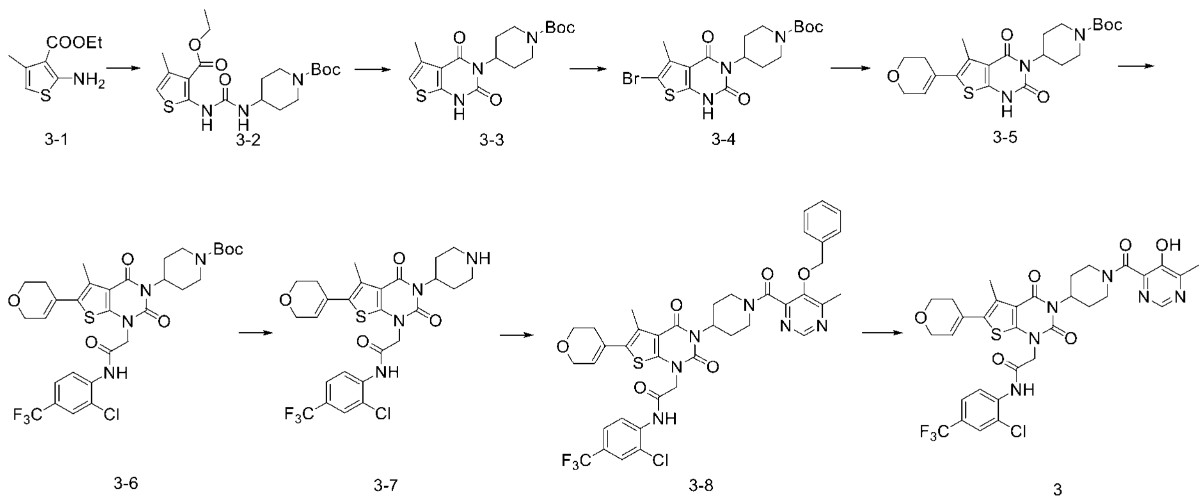

3-1 3-2 3-3 3-4 3-5

3-6 3-7 3-8 3

**Step 1: Preparation of compound 3-2**

Compound **3-1** (2.50 g, 13.5 mmol) and triphosgene (1.40 g, 4.72 mmol) were dissolved in dichloromethane (25 mL). Under an ice-water bath and a nitrogen atmosphere, triethylamine (4.10 g, 40.5 mmol) was added dropwise to the reaction system. After the dropwise addition was completed, the reaction system was stirred under an ice-water bath for 1 h. Then, 4-amino-1-*tert*-butoxycarbonylpiperidine (2.52 g, 12.58 mmol) was added. The mixture was naturally warmed to room temperature and reacted for 4 h. LCMS showed the reaction was complete. Water (100 mL) was added to quench the reaction, and the mixture was extracted with dichloromethane (70 mL × 3). The organic phases were combined and concentrated under reduced pressure. The resulting residue was purified by column chromatography (petroleum ether/ethyl acetate = 3/1) to obtain compound 3-2 (2.60 g, yield: 51.7%). LC-MS (ESI) $[M+H-56]^+$ 356.2.

**Step 2: Preparation of compound 3-3**

Under a nitrogen atmosphere, potassium *tert*-butoxide (2.13 g, 18.9 mmol) was added to a solution of compound **3-2** (2.60 g, 6.32 mmol) in ultra-dry tetrahydrofuran (30 mL). The reaction system was stirred at 60°C for 6 h. LCMS showed the reaction was complete. Water (50 mL) was added to quench the reaction, and the mixture was extracted with ethyl acetate (70 mL × 3). The combined organic phases were washed with saturated brine (40 mL), dried over anhydrous sodium sulfate, filtered, and concentrated under reduced pressure. The resulting residue was purified by column chromatography (petroleum ether/ethyl acetate = 1/1) to obtain compound **3-3** (1.10 g, yield: 47.6%). LC-MS (ESI) $[M+H-56]^+$ 310.4.

**Step 3: Preparation of compound 3-4**

Under an ice-water bath and a nitrogen atmosphere, N-bromosuccinimide (487 mg, 2.74 mmol) was added to a solution of compound **3-3** (1.00 g, 2.74 mmol) in dichloromethane (20 mL). The reaction system was stirred under an ice-water bath for 1 h. LCMS showed the reaction was complete. Water (50 mL) was added to quench the reaction, and the mixture was extracted with ethyl acetate (70 mL × 3). The combined organic phases were washed with saturated brine (40 mL), dried over anhydrous sodium sulfate, filtered, and concentrated under reduced pressure. The resulting residue was purified by column chromatography (petroleum ether/ethyl acetate = 1/1) to obtain compound **3-4** (950 mg, yield: 78.1%). LC-MS (ESI) $[M+H-56]^+$ 388.0/390.0.

**Step 4: Preparation of compound 3-5**

Compound **3-4** (900 mg, 2.03 mmol), 4-(4,4,5,5-tetramethyl-1,3,2-dioxaborolan-2-yl)-3,6-dihydro-2H-pyran (511 mg, 2.43 mmol), [1,1'-bis(diphenylphosphino)ferrocene]dichloropalladium (148 mg, 202 μmol), and cesium carbonate (1.98 g, 6.08 mmol) were dissolved in 1,4-dioxane/water (10/3 mL). The reaction system was stirred at 100°C for 12 h. LCMS showed the reaction was complete. The reaction system was cooled to room temperature, quenched with water (50 mL), and extracted with ethyl acetate (70 mL × 3). The combined organic phases were washed with saturated brine (40 mL), dried over anhydrous sodium sulfate, filtered, and concentrated under reduced pressure. The resulting residue was purified by column chromatography (petroleum ether/ethyl acetate = 1/1) to obtain compound **3-5** (300 mg, yield: 33.1%). LC-MS (ESI) $[M+H-56]^+$ 392.2.

**Step 5: Preparation of compound 3-6**

Under an ice-water bath and a nitrogen atmosphere, *N*-[2-chloro-4-(trifluoromethyl)phenyl]-2-iodoacetamide (211 mg, 581 μmol) and *N,N*-diisopropylethylamine (150 mg, 1.16 mmol) were sequentially added to a solution of compound **3-5** (260 mg, 581 μmol) in N,N-dimethylformamide (5 mL). The reaction system was stirred at 40°C for 3 h. LCMS showed the reaction was complete. Water (20 mL) was added to quench the reaction, and the mixture was extracted with ethyl acetate (50 mL × 3). The combined organic phases were washed with saturated brine (40 mL), dried over anhydrous sodium sulfate, filtered, and concentrated under reduced pressure. The resulting residue was purified by column chromatography (petroleum ether/ethyl acetate = 1/1) to obtain compound **3-6** (250 mg, yield: 63.0%). LC-MS (ESI) $[M+H-56]^+$ 627.2.

**Step 6: Preparation of compound 3-7**

At room temperature, trifluoroacetic acid (2 mL) was added to a solution of compound **3-6** (250 mg, 366 μmol) in dichloromethane (10 mL). The reaction system was stirred at room temperature for 2 h. LCMS showed the reaction was complete. The reaction mixture was concentrated under reduced pressure to obtain the crude product of compound 3-7 (250 mg). The crude product was directly used in the next step. LC-MS (ESI) $[M+H]^+$ 583.2.

**Step 7: Preparation of compound 3-8**

At room temperature, 2-(7-azabenzotriazol-1-yl)-*N,N,N',N'*-tetramethyluronium hexafluorophosphate (162 mg, 429 μmol) was added to a solution of the crude product of compound **3-7** (250 mg), 5-benzyloxy-6-methylpyrimidine-4-carboxylic acid (105 mg, 429 μmol), and N,N-diisopropylethylamine (55.4 mg, 429 μmol) in dichloromethane (10 mL). The reaction system was stirred at room temperature for 12 h. LCMS showed the reaction was complete. The reaction mixture was concentrated under reduced pressure, and the resulting residue was purified by column chromatography (dichloromethane/methanol = 20/1) to obtain compound **3-8** (200 mg, total yield over two steps: 67.6%). LC-MS (ESI) $[M+H]^+$ 809.4.

**Step 8: Preparation of compound 3**

Under an ice-water bath and a nitrogen atmosphere, a solution of boron trichloride in dichloromethane (1.00 mol/L, 247 μL, 247 μmol) was added dropwise to a solution of compound **3-8** (100 mg, 123 μmol) in dichloromethane (10 mL). The reaction system was stirred at room temperature for 2 h. LCMS showed the reaction was complete. Water (20 mL) was added to quench the reaction, and the mixture was extracted with dichloromethane (30 mL × 3). The combined organic phases were washed with saturated brine (20 mL), dried over anhydrous sodium sulfate, filtered, and concentrated under reduced pressure. The resulting residue was purified by preparative HPLC to obtain compound **3** (13.6 mg, yield: 15.3%). LC-MS (ESI) $[M+H]^+$ 719.2.

$^1H$ NMR (400 MHz, DMSO-$d_6$) δ 10.33 (s, 1H), 10.18 (s, 1H), 8.56 (s, 1H), 8.06 (d, *J* = 8.5 Hz, 1H), 7.97 (d, *J* = 2.1 Hz, 1H), 7.73 (dd, *J* = 8.8, 2.1 Hz, 1H), 6.04 - 5.92 (m, 1H), 5.06 (s, 1H), 4.88 (d, *J* = 3.6 Hz, 2H), 4.63 (d, *J* = 12.9 Hz, 1H), 4.21 (m, 2H), 3.78 (t, *J* = 5.4 Hz, 2H), 3.56 (d, *J* = 13.3 Hz, 1H), 3.13 (t, *J* = 12.9 Hz, 1H), 2.86 (t, *J* = 12.7 Hz, 1H), 2.59 (d, *J* = 13.6 Hz, 1H), 2.45 (s, 3H), 2.43 (s, 3H), 2.36 - 2.30 (m, 3H), 1.70 (d, *J* = 12.0 Hz, 1H), 1.53 (d, *J* = 12.0 Hz, 1H).

**Example 4: Synthesis of compound 4**

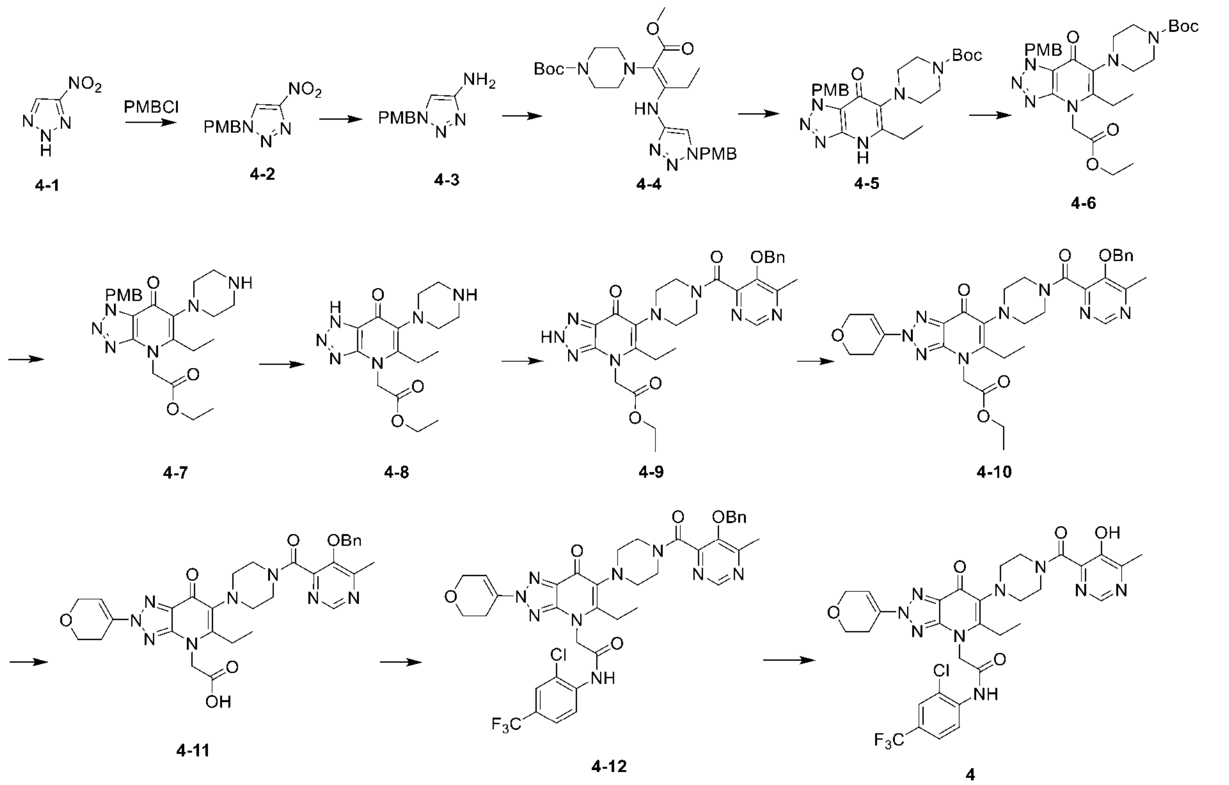


4-1 4-2 4-3 4-4 4-5 4-6

4-7 4-8 4-9 4-10

4-11 4-12 4

**Step 1: Preparation of compound 4-2**

Starting material 4-1 (3.80 g, 33.31 mmol) and PMBCl (7.85 g, 49.97 mmol) were dissolved in DMF (40.0 mL), and potassium carbonate (6.90 g, 49.97 mmol) was added at room temperature. The reaction system was stirred at 60°C for 5 h and then cooled. Water (50 mL) was added to quench the reaction, followed by the addition of a mixed solvent of petroleum ether and ethyl acetate (40 mL, 5:1). After stirring for 5 min, the mixture was filtered. The filter cake was washed with a mixed solvent of petroleum ether and ethyl acetate (5:1) to obtain compound 4-2 (3.90 g) with a yield of 50%.

**Step 2: Preparation of compound 4-3**

Compound 4-2 (3.40 g, 14.52 mmol) was dissolved in a mixed solvent of methanol and water (40 mL, 3:1). Iron powder (2.44 g, 43.55 mmol) and ammonium chloride (3.92 g, 72.58 mmol) were then added. The reaction system was stirred at 70°C for 2 h and then cooled. The suspension was filtered through diatomite, and the filtrate was concentrated to dryness under vacuum. The residue was dissolved in ethyl acetate, washed with saturated brine, separated, and dried. The solvent was removed using a rotary evaporator to obtain compound 4-3 (2.55 g) with a yield of 86.0%. LC-MS(ESI) $[M+H]^+$: 205.2.

**Step 3: Preparation of compound 4-4**

Compound 4-3 (2 g, 9.79 mmol), *tert*-butyl 4-(1-methoxy-1,3-dioxolan-2-yl)piperazine-1-carboxylate (3.5 g, 11.13 mmol), and TsOH (168.44 mg, 979.30 μmol) were dissolved in EtOH (35 mL). After the addition was completed, the system was heated to 100°C and stirred for 12 h. TLC showed the disappearance of the starting material. The reaction system was directly quenched with water, and the product was extracted with ethyl acetate. The organic phase was

washed with an aqueous sodium carbonate solution, separated, dried, and then concentrated to dryness under vacuum to obtain compound 4-4 (4.91 g). The residue was directly used in the next step. The crude product yield was 100%. LC-MS(ESI)[M+H]$^+$: 501.2.

**Step 4: Preparation of compound 4-5**

Compound 4-4 (4 g, 7.99 mmol) was dissolved in diphenyl ether:dichloromethane (5 mL, 4:1), and the solution was slowly added dropwise to diphenyl ether (43 mL) that had been preheated to an internal temperature of 220°C. After the addition was completed, the system was stirred at 220°C for 4.5 min. After the system was cooled to room temperature, the reaction was quenched directly with cyclohexane. The solid was filtered and washed with cyclohexane to obtain compound 4-5 (1.1 g) with a yield of 29.4%. LC-MS (ESI) [M+H]$^+$: 469.2.

**Step 5: Preparation of compound 4-6**

Compound 4-5 (1 g, 2.13 mmol) was dissolved in DMF (7 mL), and ethyl bromoacetate (712.84 mg, 4.27 mmol) and cesium carbonate (2.10 g, 6.40 mmol) were added thereto. After the addition was completed, the system was stirred at room temperature for 2 h. The reaction system was directly quenched with water, and the product was extracted with ethyl acetate. The organic phase was separated, dried, and concentrated to dryness under vacuum. The residue was purified by silica gel column chromatography (dichloromethane:methanol = 10:1) to obtain compound 4-6 (0.27 g) with a yield of 22.9%. LC-MS (ESI) [M+H]$^+$: 555.2.

**Step 6: Preparation of compound 4-7**

Compound 4-6 (170 mg, 306.51 μmol) was dissolved in DCM (2 mL), and TFA (1 mL) was added thereto at room temperature. After the addition was completed, the system was stirred at room temperature for 0.5 h. The disappearance of the starting material was detected by TLC. The system was directly concentrated to dryness under vacuum. The residue was directly used in the next step, and the crude product yield was 100%. LC-MS(ESI)[M+H]$^+$:455.2.

**Step 7: Preparation of compound 4-8**

Compound 4-7 (150 mg, 330.02 μmol) was dissolved in TFA (2 mL), and TfOH (0.05 mL) was added thereto. After the addition was completed, the system was stirred at room temperature for 10 min. The disappearance of the starting material was detected by TLC. The system was directly concentrated to dryness under vacuum. The residue was directly used in the next step, and the crude product yield was 100%. LC-MS(ESI)[M+H]$^+$: 335.2.

**Step 8: Preparation of compound 4-9**

Compound 4-8 (200 mg, 598.13 μmol) and 5-(benzyloxy)-6-methylpyrimidine-4-carboxylic acid (146.09 mg, 598.13 μmol) were dissolved in DMF (3.96 mL), and DIEA (773.05 mg, 5.98 mmol, 1.04 mL) and HATU (293.36 mg, 777.58 μmol) were added thereto. After the addition was completed, the system was stirred at room temperature for 2 h. The reaction system was directly quenched with water, and the by-products were extracted with ethyl acetate. The aqueous phase was washed with citric acid and extracted with ethyl acetate, then the organic phase was separated, dried, and concentrated to dryness under vacuum. The residue was purified by preparative HPLC to obtain compound 4-9 (110 mg) with a yield of 32.83%. LC-MS (ESI) [M+H]$^+$: 561.2.

**Step 9: Preparation of compound 4-10**

Compound 4-9 (16 mg, 28.54 μmol), (3,6-dihydro-2H-pyran-4-yl)boronic acid (12.78 mg, 99.89 μmol), and $PhenCuPPh_3Br$ (6.70 mg, 11.42 μmol) were dissolved in DMSO (1 mL). After the addition was completed, the system was heated to 90°C under air balloon protection and stirred for 3 h. The reaction system was directly quenched with water, and the product was extracted with ethyl acetate. The organic phase was separated, dried, and concentrated to dryness under vacuum. The residue was purified by medium-pressure preparative column (DCM:MeOH = 10:1) to obtain compound 4-10 (11 mg) with a yield of 59.88%. LC-MS (ESI) [M+H]$^+$: 643.2.

**Step 10: Preparation of compound 4-11**

Compound 4-10 (17 mg, 26.45 μmol) was dissolved in a mixed solvent of water (1.5 mL), MeOH (1.5 mL), and THF (1.5 mL), then NaOH (10.58 mg, 264.51 μmol) was added thereto. After the addition was completed, the system was

stirred at room temperature for 0.25 h. The pH was adjusted to 4.0 with 3N hydrochloric acid, and ethyl acetate was added to extract the product. The organic phase was separated, dried, and subjected to rotary evaporation to obtain the crude product 4-11 (16 mg) with a crude product yield of 100%. LC-MS (ESI) $[M+H]^+$: 615.2.

**Step 11: Preparation of compound 4-12**

Compound 4-11 (16 mg, 26.05 μmol), 2-chloro-4-trifluoromethylaniline (15.20 mg, 78.17 μmol), and pyridine (20.37 mg, 261 μmol) were dissolved in DCM (0.5 mL), then phosphorus oxychloride (20.29 mg, 130 μmol) was added thereto. After the addition was completed, the system was stirred at room temperature for 0.5 h. TLC showed a product spot. The reaction system was directly quenched with water, and the product was extracted with ethyl acetate. The organic phase was separated, dried, and concentrated to dryness under vacuum. The residue was purified by medium-pressure preparative column (dichloromethane:methanol = 10:1) to obtain compound 4-12 (16 mg) with a yield of 77.88%. LC-MS (ESI) $[M+H]^+$: 792.2.

**Step 12: Preparation of compound 4**

Compound 4-12 (18 mg, 22.75 μmol) was dissolved in DCM (2 mL), then boron trichloride (180.00 μL, 1N) was added thereto at room temperature. After the addition was completed, the system was stirred at room temperature for 0.25 h. The reaction system was directly quenched with methanol, and the residue was purified by preparative HPLC to obtain the crude product of compound 4 (10.63 mg, containing isomers). The crude product 4 was further purified by SFC (column: ChiralPak AD, 250 × 30 mm I.D., 10 μm; mobile phase [A: carbon dioxide, B: isopropanol (containing 0.1% ammonia water)]; composition B%: 40%, flow rate: 150 mL/min, column temperature: 38°C, wavelength: 220 nm, cycle time: about 5 min) to obtain compound 4 (4.11 mg), with a yield of 38.66% (retention time of the target product: 0.788 min; analytical method: column: ChiralPak AD, 50 × 4.6 mm I.D., 3 μm; mobile phase [A: carbon dioxide, B: isopropanol (containing 0.05% DEA)]; 40% B, flow rate: 3 mL/min, column temperature: 35°C). LC-MS (ESI) [M+H]+: 702.2.

1HNMR (400 MHz, Methanol-d4) δ 8.31 (s, 1H), 8.04 (d, J = 8.8 Hz, 1H), 7.74-7.68 (m, 1H), 7.55-7.47 (m, 1H), 6.79 - 6.73 (m, 1H), 5.29 (s, 2H), 4.30-4.22 (m, 2H), 3.94-3.85 (m, 2H), 3.84-3.72 (m, 2H), 3.42 - 3.28 (m, 1H), 3.10 - 2.95 (m, 3H), 2.81 (s, 3H), 2.71-2.60 (m, 1H), 2.38 (s, 3H), 2.09 (t, J = 7.6 Hz, 1H), 1.99-1.87 (m, 1H), 0.80 (t, J = 6.4 Hz, 3H).

**Example 5: Synthesis of compound 5**

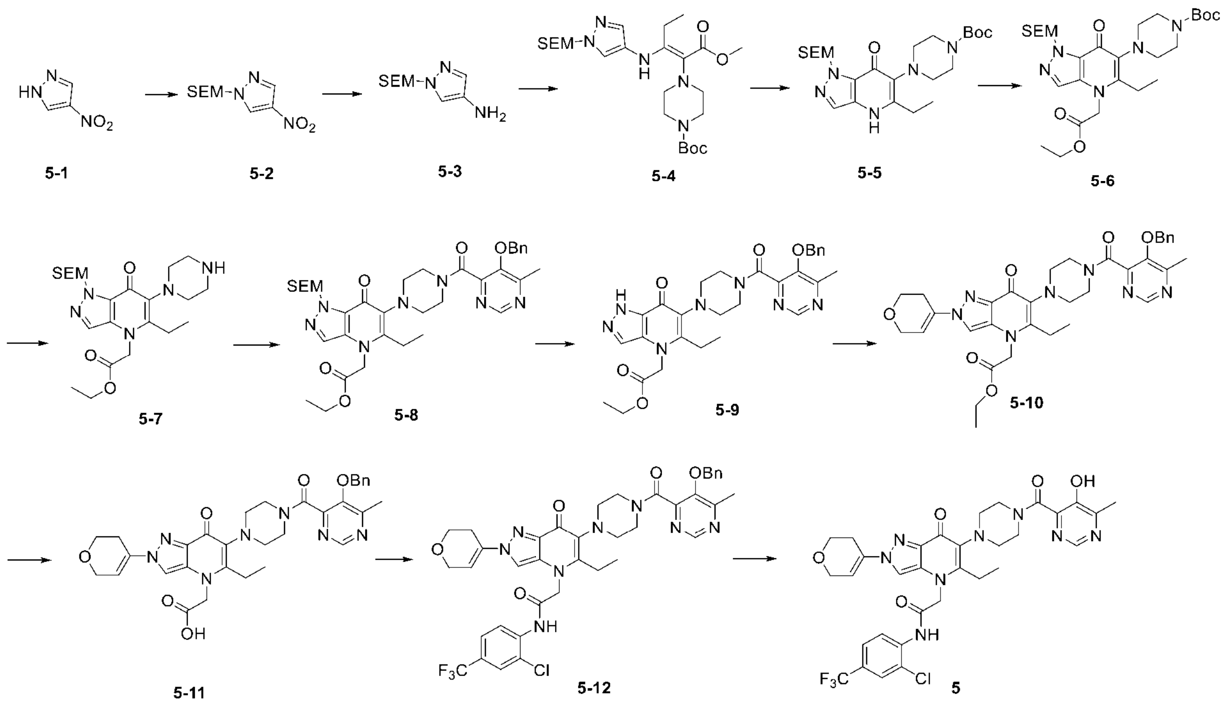

5-1, 5-2, 5-3, 5-4, 5-5, 5-6, 5-7, 5-8, 5-9, 5-10, 5-11, 5-12, 5

**Step 1: Synthesis of compound 5-2**

A solution of compound 5-1 (1.0 g, 8.84 mmol) in THF (30 mL) was added dropwise to a solution of NaH (1.06 g, 26.54 mmol, 60% purity) in THF (30 mL) at 0°C, stirred at 0°C for 0.5 h, followed by dropwise addition of 2-(trimethylsilyl) ethoxymethyl chloride (1.77 g, 10.62 mmol, 1.88 mL). The mixture was reacted at 0°C for an additional 1.5 h. The mixture was slowly added to a saturated ammonium chloride solution (50 mL) and extracted with ethyl acetate (50 mL × 2). The combined organic phases were washed with saturated brine, dried over anhydrous sodium sulfate, and concentrated to obtain compound 5-2 (2.1 g, 8.63 mmol) with a yield of 97.58%.

**Step 2: Synthesis of compound 5-3**

Compound 5-2 (1.0 g, 4.11 mmol) was dissolved in MeOH (10 mL), and Pd/C (200 mg, 10% purity) was added. The mixture was reacted under a hydrogen atmosphere at 25°C for 4 h until the starting material was completely consumed. The mixture was filtered and then concentrated by rotary evaporation under reduced pressure to obtain compound 5-3 (870 mg, 4.08 mmol) with a yield of 99.2%. LC-MS (ESI) $[M+H]^+$: 214.2.

**Step 3: Synthesis of compound 5-4**

Compound 5-3 (1.0 g, 4.69 mmol) and compound *tert*-butyl 4-(1-methoxy-1,3-dioxolan-2-yl)piperazine-1-carboxylate (1.47 g, 4.69 mmol) were dissolved in toluene (10 mL). *p*-Toluenesulfonic acid (80.71 mg, 468.71 μmol) was added, and the mixture was reacted at 120°C for 16 h until the starting material was completely consumed. The mixture was poured into a saturated sodium bicarbonate solution (30 mL) and extracted with ethyl acetate (30 mL × 2). The organic phases were combined, washed with saturated brine, dried over anhydrous sodium sulfate, and concentrated to obtain compound 5-4 (1.28 g) with a yield of 53.58%. LC-MS (ESI) $[M+H]^+$ :510.4.

**Step 4: Synthesis of compound 5-5**

Diphenyl ether (8 mL) was heated to 280°C, and compound 5-4 (680 mg, 1.33 mmol) was added under reflux. The reaction was carried out for 5 min until the product was formed. The mixture was cooled to room temperature, then poured into cyclohexane, stirred, and then filtered. The filtrate was concentrated, and then both the filtrate and the filter cake were separately subjected to column chromatography (PE/EA = 10/1) to obtain compound 5-5 (100 mg) with a yield of 15.69%. LC-MS (ESI) $[M+H]^+$ :478.4.

**Step 5: Synthesis of compound 5-6**

Compound 5-5 (160 mg, 334.96 μmol) was dissolved in ACN (3 mL), followed by the addition of cesium carbonate (327.41 mg, 1.00 mmol) and ethyl bromoacetate (83.91 mg, 502.44 μmol). The mixture was reacted at 25°C for 1 h until the reaction was complete. The reaction mixture was poured into water (10 mL) and extracted with ethyl acetate (10 mL × 2). The combined organic phases were washed with saturated brine, dried over anhydrous sodium sulfate, and purified by silica gel column chromatography (PE/EA = 1/1) to obtain compound 5-6 (83 mg) with a yield of 43.9%. LC-MS (ESI) $[M+H]^+$ :564.4.

**Step 6: Synthesis of compound 5-7**

Compound 5-6 (30 mg, 53.21 μmol) was dissolved in MeOH (3 mL), followed by the addition of HCl (4 M, 4.00 mmol, 1 mL). The mixture was reacted at 25°C for 4 h until the starting material was completely consumed. The mixture was poured into a saturated sodium bicarbonate solution (10 mL) and extracted with ethyl acetate (10 mL × 2). The organic phases were combined, washed with saturated brine, dried over anhydrous sodium sulfate, and concentrated to obtain compound 5-7 (24 mg) with a yield of 97.3%. LC-MS (ESI) $[M+H]^+$: 464.3.

**Step 7: Synthesis of compound 5-8**

Compound 5-7 (177.00 mg, 724.69 μmol) was dissolved in DMF (5 mL), followed by the addition of HATU (455.67 mg, 1.21 mmol), DIEA (234.15 mg, 1.81 mmol, 315.57 μL), and compound 5-(benzyloxy)-6-methylpyrimidine-4-carboxylic acid (280 mg, 603.91 μmol). The resulting mixture was reacted at 25°C for 2 h until the reaction was complete. The reaction mixture was poured into water (10 mL) and extracted with ethyl acetate (10 mL × 2). The organic phases were combined, washed with saturated brine, dried over anhydrous sodium sulfate, and concentrated to obtain compound 5-8 (360 mg) with a yield of 86.4%. LC-MS (ESI) $[M+H]^+$: 690.2.

**Step 8: Synthesis of compound 5-9**

Compound 5-8 (350 mg, 507.34 μmol) was dissolved in a solution of HCl (4 M, 24.00 mmol, 6 mL) in dioxane (6 mL). The reaction was carried out at 25°C for 4 h until the starting material was completely consumed. The reaction mixture was poured into a saturated sodium bicarbonate solution (15 mL) and extracted with ethyl acetate (15 mL × 2). The organic phases were combined, washed with saturated brine, dried over anhydrous sodium sulfate, and purified by silica gel column chromatography (DCM/MeOH = 10/1) to obtain compound 5-9 (200 mg) with a yield of 70.4%. LC-MS (ESI) $[M+H]^+$: 690.2.

**Step 9: Synthesis of compound 5-10**

Compound 5-9 (300 mg, 536.08 μmol) was dissolved in DMSO (1 mL), and compound (3,6-dihydro-2H-pyran-4-yl)boronic acid(102.87 mg, 804.12 μmol) and PhenCuPPh (30.45 mg, 53.61 μmol) were added. The resulting reaction system was stirred under air until the reaction was complete. The reaction mixture was quenched with water and extracted with ethyl acetate (5 mL × 2). The organic phases were combined, washed with saturated brine, dried over anhydrous sodium sulfate, and concentrated to obtain a crude product. The crude product was purified by silica gel column chromatography (MeOH/DCM = 3%) to obtain compound 5-10 (135 mg, 204.06 μmol) with a yield of 38.1%. LC-MS (ESI) $[M+H]^+$: 642.2.

**Step 10: Synthesis of compound 5-11**

Compound 5-10 (130.00 mg, 202.58 μmol) was dissolved in MeOH (0.5 mL), THF (1.5 mL), and water (0.5 mL). LiOH (16.98 mg, 709.04 μmol) was added. The resulting mixture was reacted at 25°C for 1 h until the starting material was completely consumed. The mixture was adjusted to pH < 4 with hydrochloric acid and extracted with ethyl acetate (10 mL × 2). The organic phases were combined, washed with saturated brine, dried over anhydrous sodium sulfate, and concentrated to obtain compound 5-11 (120 mg, 195.55 μmol) with a yield of 96.53%. LC-MS (ESI) $[M+H]^+$: 612.2.

**Step 11: Synthesis of compound 5-12**

Compound 5-11 (110.00 mg, 179.25 μmol), 2-chloro-4-trifluoromethylaniline (52.58 mg, 268.88 μmol), and pyridine (70.89 mg, 896.26 μmol, 72.20 μL) were dissolved in DCM (1.93 mL). Phosphorus oxychloride (41.23 mg, 268.88 μmol) was added. The resulting reaction mixture was stirred at 25°C for 1 h until the starting material was completely consumed. Water (5 mL) was added to quench the reaction, and the product was extracted with ethyl acetate. The organic phase was separated, dried over anhydrous sodium sulfate, and concentrated under vacuum to obtain a crude product. The crude product was separated by silica gel column chromatography (DCM/MeOH=10/1) to obtain compound 5-12 (130 mg, 164.30 μmol) with a yield of 91.66%. LC-MS (ESI) $[M+H]^+$: 791.2.

**Step 12: Synthesis of compound 5**

Compound 5-12 (60 mg, 75.83 μmol) was dissolved in DCM (2 mL), and boron trichloride (88.85 mg, 758.33 μmol) was added. The mixture was reacted at 25°C for 0.5 h until the starting material was completely consumed. The mixture was separated by preparative HPLC to obtain compound 5 (5 mg, 7.13 μmol) with a yield of 9.40%. LC-MS (ESI) $[M+H]^+$: 701.2.

$^1$H NMR (400 MHz, DMSO-$d_6$) δ 10.24 (s, 1H), 10.20 (s, 1H), 8.65 (s, 1H), 8.58 (s, 1H), 8.15 (d, *J* = 8.5 Hz, 1H), 7.98 (d, *J* = 2.0 Hz, 1H), 7.72 (d, *J* = 8.6 Hz, 1H), 6.41 (d, *J* = 2.4 Hz, 1H), 5.16 (s, 2H), 4.49 (d, *J* = 12.2 Hz, 1H), 4.28 (d, *J* = 3.0 Hz, 2H), 3.89 (t, *J* = 5.5 Hz, 2H), 3.73 (q, *J* = 13.1 Hz, 2H), 3.46 (d, *J* = 12.5 Hz, 1H), 3.19 (t, *J* = 12.3 Hz, 1H), 2.93 (t, *J* = 12.2 Hz, 3H), 2.71 (s, 3H), 2.44 (s, 3H), 2.00 (q, *J* = 6.9, 6.3 Hz, 1H), 1.17 (t, *J* = 7.4 Hz, 3H).

**Example 6: Synthesis of compound 6**

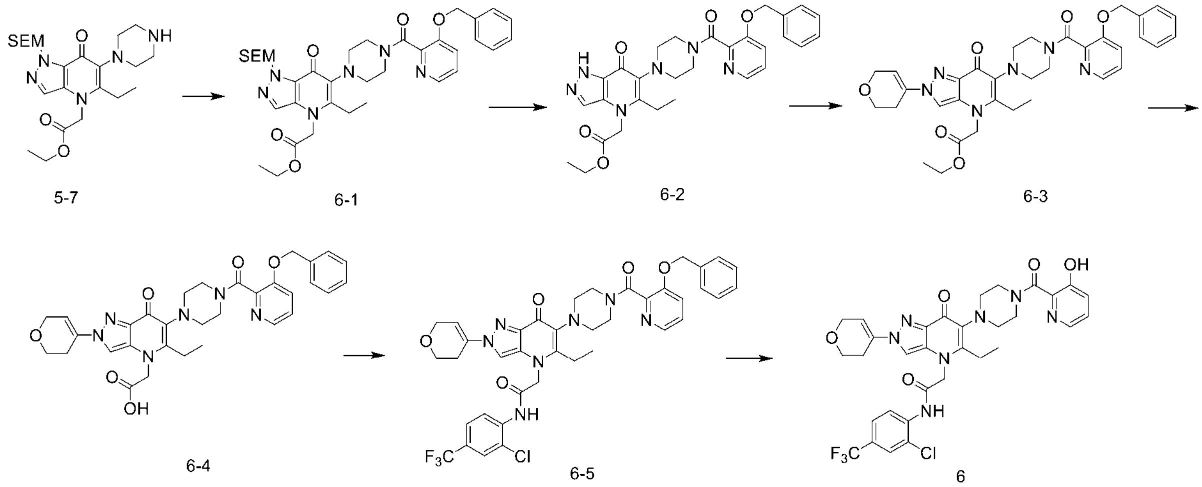

5-7 6-1 6-2 6-3

6-4 6-5 6

**Step 1: Preparation of compound 6-1**

Intermediate 5-7 (160 mg, 345.83 μmol) and 3-(benzyloxy)picolinic acid (87.20 mg, 380.41 μmol) were dissolved in *N,N*-dimethylformamide (1 mL). 2-(7-Azabenzotriazol-1-yl)-*N,N,N',N'*-tetramethyluronium hexafluorophosphate (157.79 mg, 414.99 μmol) and *N,N*-diisopropylethylamine (134.09 mg, 1.04 mmol, 180.71 μL) were sequentially added. After the addition was completed, the resulting reaction system was stirred at 25°C for 1 h. LC-MS showed completion of the reaction. The reaction mixture was directly concentrated under reduced pressure, and the resulting residue was purified by silica gel column (DCM/MeOH = 5%) to obtain compound 6-1 (210 mg) with a yield of 90.11%. LC-MS (ESI) $[M+H]^+$: 675.2.

**Step 2: Preparation of compound 6-2**

Compound 6-1 (210.31 mg, 311.63 μmol) was dissolved in a solution of HCl in dioxane (4 M, 4.00 mmol, 1 mL). The resulting reaction system was stirred at 25°C for 1 h. LC-MS showed completion of the reaction. The reaction mixture was cooled to 25°C, and the pH was adjusted to neutral with saturated sodium bicarbonate. The mixture was extracted with ethyl acetate (5 mL × 2). The organic phases were combined, washed with saturated brine, dried over anhydrous sodium sulfate, and concentrated under reduced pressure to obtain a crude product 6-2 (150 mg) with a yield of 75.13%. LC-MS (ESI) $[M+H]^+$: 545.2.

**Step 3: Preparation of compound 6-3**

Compound 6-2 (67 mg, 123.03 μmol) was dissolved in dimethyl sulfoxide (0.5 mL). 3,6-Dihydro-2H-pyran-4-boronic acid (23.61 mg, 184.54 μmol) and $PhenCuPPhBr_2$ (3.49 mg, 6.15 μmol) were added. The resulting reaction mixture was stirred under air for approximately 12 h until the starting material was completely consumed. The reaction mixture was quenched with water (5 mL) and extracted with ethyl acetate (5 mL × 2). The organic phases were combined, washed with saturated brine, dried over anhydrous sodium sulfate, and concentrated under reduced pressure. The resulting crude product was purified by silica gel column (MeOH/DCM = 3%) to obtain compound 6-3 (43 mg) with a yield of 52.98%. LC-MS (ESI) $[M+H]^+$: 627.2.

**Step 4: Preparation of compound 6-4**

Compound **6-3** (41 mg, 62.15 μmol) was dissolved in methanol (0.5 mL) and tetrahydrofuran (0.5 mL). A solution of lithium hydroxide (4.47 mg, 186.45 μmol) in water (0.2 mL) was added. After the addition was completed, the resulting reaction system was stirred at room temperature for 5 h until the starting material was completely consumed. The reaction mixture was concentrated under reduced pressure to remove the organic solvent. The remaining aqueous phase was adjusted to neutral pH with 1 M dilute hydrochloric acid and extracted with ethyl acetate (5 mL × 2). The organic phases were combined, washed with saturated brine, dried over anhydrous sodium sulfate, and concentrated under reduced pressure to obtain a crude product 6-4 (35 mg), which was directly used in the next step. The crude product yield was 83.72%. LC-MS (ESI) $[M+H]^+$: 599.2.

**Step 5: Preparation of compound 6-5**

Intermediate 6-4 (25 mg, 37.17 μmol), 3-chloro-4-aminobenzotrifluoride (10.90 mg, 55.75 μmol), and pyridine (14.70 mg, 185.84 μmol, 14.97 μL) were dissolved in dichloromethane (0.5 mL). Phosphorus oxychloride (17.10 mg, 111.50 μmol) was added. After the addition was completed, the resulting reaction system was stirred at 25°C for 2 h until the reaction was complete. After the reaction mixture was concentrated under reduced pressure, the resulting residue was purified by silica gel column (DCM/MeOH = 50:1) to obtain compound 6-5 (23 mg) with a yield of 70.64%. LC-MS (ESI) $[M+H]^+$: 776.4.

**Step 6: Preparation of compound 6**

Intermediate 6-5 (25 mg, 28.54 μmol) was dissolved in dichloromethane (0.5 mL), and boron trichloride (16.72 mg, 142.68 μmol) was added. After the addition was completed, the resulting reaction system was stirred at 25°C for 1 h. LC-MS showed completion of the reaction The reaction mixture was concentrated under reduced pressure, and the resulting residue was purified by preparative HPLC to obtain compound 6 (2.16 mg) with a yield of 11.03%. LC-MS (ESI) $[M+H]^+$: 686.2.

$^1H$ NMR (400 MHz, DMSO-$d_6$) δ 10.18 (s, 1H), 8.63 (s, 1H), 8.15 (d, *J* = 8.8 Hz, 1H), 8.07 - 8.04 (m, 1H), 7.97 (d, *J* = 1.9 Hz, 1H), 7.72 (d, *J* = 9.0 Hz, 1H), 7.28 (d, *J* = 3.8 Hz, 2H), 6.41 (s, 1H), 5.16 (s, 2H), 4.52 (d, *J* = 12.3 Hz, 1H), 4.28 (d, *J* = 2.9 Hz, 2H), 3.89 (t, *J* = 5.4 Hz, 2H), 3.73 (t, *J* = 14.6 Hz, 2H), 3.18 (q, *J* = 12.7 Hz, 3H), 2.91 (q, *J* = 12.0, 9.3 Hz, 3H), 2.71 (tt, *J* = 6.6, 2.9 Hz, 4H), 1.17 (t, *J* = 7.4 Hz, 3H).

**Example 7: Synthesis of compound 7**

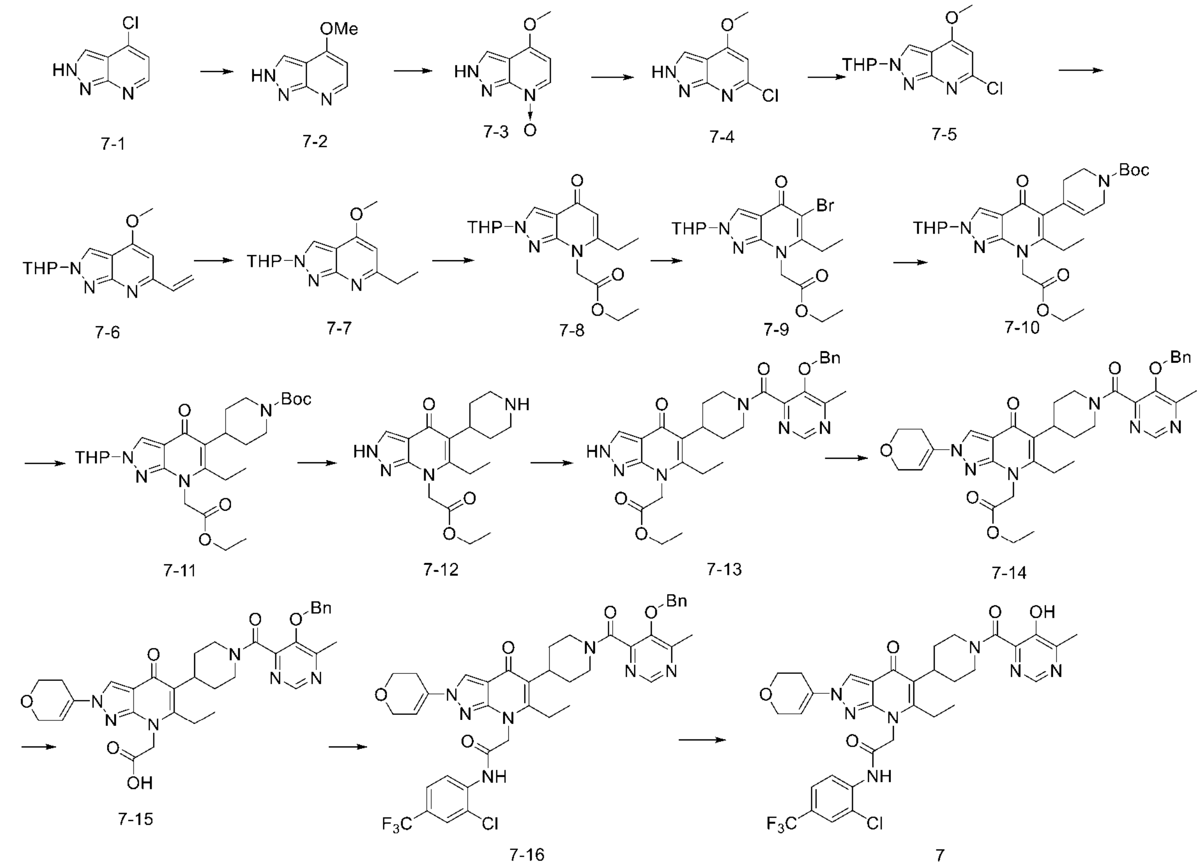

7-1 7-2 7-3 7-4 7-5

7-6 7-7 7-8 7-9 7-10

7-11 7-12 7-13 7-14

7-15 7-16 7

**Step 1: Preparation of compound 7-2**

At room temperature, compound 7-1 (23 g, 150 mmol) was dissolved in dimethyl sulfoxide (200 mL), and sodium methoxide (135 g, 750 mmol) was added. Under a nitrogen atmosphere, the reaction system was stirred at 120°C for 16 h.

Water was added, and the mixture was extracted multiple times with ethyl acetate. The organic phases were combined, washed with saturated brine, dried over anhydrous sodium sulfate, and concentrated under reduced pressure. The resulting residue was slurried with pure ethyl acetate to obtain the target compound 7-2 (11.9 g) with a yield of 43.28%. LC-MS (ESI) $[M+H]^+$: 150.1.

**Step 2: Preparation of compound 7-3**

At room temperature, intermediate 7-2 (11.9 g, 80 mmol) was dissolved in *N*-methylpyrrolidone (240 mL), followed by the addition of *m*-chloroperoxybenzoic acid (32 g, 184 mmol). The reaction was carried out at room temperature for 16 h until the reaction was complete. Methyl *tert*-butyl ether solution (60 mL) was added, and after stirring for 10 min, the liquid was filtered off. The filter cake was rinsed with ethyl acetate and collected. The filter cake was dissolved in methyl *tert*-butyl ether solution (200 mL), stirred for 30 min, and then the liquid was filtered off. The solid obtained after filtration was dried to obtain compound 7-3 (11.9 g) with a yield of 100%. LC-MS (ESI) $[M+H]^+$: 166.1.

**Step 3: Preparation of compound 7-4**

At room temperature, intermediate 7-3 (10.9 g, 66 mmol) was dissolved in phosphorus oxychloride (100 mL), and the reaction was carried out at 50°C for 16 h until the reaction was complete. The solvent was subjected to rotary evaporation until dryness. Under an ice bath, the solution was adjusted to alkaline with sodium hydroxide, and extracted multiple times with ethyl acetate and water. The organic phases were combined, washed with saturated brine, dried over anhydrous sodium sulfate, and concentrated under reduced pressure. The resulting residue was purified by column chromatography (silica, petroleum ether/ethyl acetate = 2/1) to obtain compound **7-4** (11.0 g) with a yield of 90.78%. LC-MS (ESI) $[M+H]^+$: 183.9.

**Step 4: Preparation of compound 7-5**

At room temperature, intermediate 7-4 (11 g, 60 mmol) was dissolved in tetrahydrofuran (110 mL), followed by the addition of 3,4-dihydro-2H-pyran (7.6 g, 90 mmol) and *p*-toluenesulfonic acid monohydrate (1.1 g, 6 mmol). The resulting reaction mixture was reacted at room temperature for 16 h until the reaction was complete, diluted with water (100 mL), and extracted with ethyl acetate (200 mL × 3). The organic phases were combined, washed with saturated brine, dried over anhydrous sodium sulfate, and concentrated under reduced pressure. The resulting residue was purified by column chromatography (silica, petroleum ether/ethyl acetate = 2/1) to obtain compound 7-5 (14.8 g) with a yield of 92.27%. LC-MS(ESI) $[M+H]^+$:267.9.

**Step 5: Preparation of compound 7-6**

At room temperature, intermediate 7-5 (14 g, 52 mmol) and vinylboronic acid pinacol ester (12 g, 78 mmol) were dissolved in a mixed solution of 1,4-dioxane (70 mL) and water (35 mL), followed by the addition of methanesulfonato(2-dicyclohexylphosphino-2',6'-diisopropoxy-1,1'-biphenyl)(2-amino-1,1'-biphenyl-2-yl)palladium(II) (1.3 g, 1.56 mmol) and cesium fluoride (20 g, 130 mmol). The system was purged with nitrogen, and the reaction mixture was stirred and reacted at 110°C for 16 h. LC-MS showed completion of the reaction. The reaction mixture was diluted with water (50 mL) and extracted with ethyl acetate (200 mL × 2). The organic phases were combined, washed with saturated brine, dried over anhydrous sodium sulfate, and concentrated under reduced pressure. The residue was purified by silica gel column chromatography (silica, petroleum ether/ethyl acetate = 5/1) to obtain compound 7-6 (13.2 g) with a yield of 98%. LC-MS (ESI) $[M+H]^+$: 260.0.

**Step 6: Preparation of compound 7-7**

At room temperature, intermediate 7-6 (13.2 g, 51 mmol) was dissolved in methanol (130 mL), and 10% palladium on carbon catalyst (5 g) was added. The reaction was stirred under a hydrogen atmosphere at room temperature for 16 h until the reaction was complete. The reaction mixture was concentrated under reduced pressure, and the resulting residue was purified by silica gel column chromatography (silica, petroleum ether/ethyl acetate = 2/1) to obtain compound 7-7 (10.9 g) with a yield of 81.9%. LC-MS(ESI) $[M+H]^+$: 262.1.

**Step 7: Preparation of compound 7-8**

At room temperature, compound 7-7 (3 g, 11 mmol) was dissolved in acetonitrile (30 mL), and ethyl bromoacetate (19 g, 15 mmol) was added. The reaction was carried out at 70°C under a nitrogen atmosphere for 16 h until the reaction

was complete. The solvent was removed by rotary evaporation under reduced pressure. The residue was purified by reversed-phase column chromatography (A: formic acid/water, B: acetonitrile) to obtain the target compound 7-8 (1.2 g) with a yield of 31.20%. LC-MS(ESI) $[M+H]^+$: 333.9.

**Step 8: Preparation of compound 7-9**

At room temperature, intermediate 7-8 (1.2 g, 3.6 mmol) was dissolved in *N,N*-dimethylformamide (10 mL), followed by the addition of N-bromosuccinimide (0.6 g, 3.6 mmol). The reaction was carried out at room temperature for 16 h until the reaction was complete. The reaction mixture was diluted with water (50 mL) and extracted with ethyl acetate (100 mL × 3). The organic phases were combined, washed with saturated brine, dried over anhydrous sodium sulfate, and concentrated under reduced pressure. The resulting residue was purified by silica gel column chromatography (dichloromethane / (methanol:dichloromethane = 1:1) = 10/1) to obtain the target compound 7-9 (0.4 g) with a yield of 31.56%. LC-MS(ESI) $[M+H]^+$: 411.8.

**Step 9: Preparation of compound 7-10**

At room temperature, intermediate 7-9 (0.4 g, 1.1 mmol) and *N*-Boc-1,2,5,6-tetrahydropyridine-4-boronic acid pinacol ester (0.7 g, 2.2 mmol) were dissolved in a mixed solvent of 1,4-dioxane (20 mL) and water (2 mL), followed by the addition of chloro(2-dicyclohexylphosphino-2',4',6'-triisopropyl-1,1'-biphenyl)[2-(2'-amino-1,1'-biphenyl)]palladium(II) (0.089 g, 0.1 mmol), potassium phosphate (0.7 g, 3.4 mmol), and 2-dicyclohexylphosphino-2',4',6'-triisopropylbiphenyl (0.1 g, 0.3 mmol). After purging with nitrogen, the reaction mixture was stirred and reacted at 100°C for 16 h until the reaction was complete. The reaction mixture was diluted with water (20 mL) and extracted with ethyl acetate (100 mL × 3). The organic phases were combined, washed with saturated brine, dried over anhydrous sodium sulfate, and concentrated under reduced pressure. The resulting residue was purified by silica gel column chromatography (silica, dichloromethane/methanol = 10/1) to obtain the target compound 7-10 (0.3 g) with a yield of 57.8%. LC-MS (ESI) $[M+H]^+$: 515.0.

**Step 10: Preparation of compound 7-11**

At room temperature, compound 7-10 (0.3 g, 0.6 mmol) was dissolved in a mixture of tetrahydrofuran (2 mL) and ethanol (2 mL). Raney nickel catalyst (0.5 g) was added, and the system was purged with hydrogen. The reaction was carried out at room temperature for 16 h until the reaction was complete. After filtration, the filtrate was concentrated under reduced pressure. The resulting residue was purified by C18 column chromatography (A: formic acid/water, B: acetonitrile) to obtain compound 7-11 (0.15 g) with a yield of 49.80%. LC-MS(ESI) $[M+H]^+$: 417.9.

**Step 11: Preparation of compound 7-12**

At room temperature, intermediate 7-11 (0.15 g, 0.29 mmol) was dissolved in tetrahydrofuran (6 mL), followed by the addition of a solution of hydrochloride in dioxane (2 mL, 1 mol/L). The reaction was carried out at room temperature for 16 h until the reaction was complete. The reaction mixture was concentrated to dryness under reduced pressure to obtain compound 7-12 (0.15 g) with a yield of 100%. LC-MS(ESI) $[M+H]^+$: 333.1.

**Step 12: Preparation of compound 7-13**

At room temperature, intermediate 7-12 (0.13 g, 0.25 mmol) was dissolved in *N,N*-dimethylformamide (1.5 mL), followed by the addition of 5-(benzyloxy)-6-methylpyrimidine-4-carboxylic acid (0.067 g, 0.27 mmol), 2-(7-azabenzotriazol-1-yl)-N,N,N',N'-tetramethyluronium hexafluorophosphate (0.14 g, 0.37 mmol), and N,N-diisopropylethylamine (0.16 g, 1.26 mmol). The reaction was carried out at room temperature for 16 h until the starting material was completely consumed. The reaction mixture was diluted with water (10 mL) and extracted with ethyl acetate (20 mL × 2). The organic phases were combined, washed with saturated brine, dried over anhydrous sodium sulfate, and concentrated under reduced pressure. The resulting residue was purified by silica gel column chromatography (dichloromethane/methanol = 10/1) to obtain compound 7-13 (0.10 g) with a yield of 47.14%. LC-MS(ESI) $[M+H]^+$:559.2.

**Step 13: Preparation of compound 7-14**

At room temperature, intermediate 7-13 (0.083 g, 0.15 mmol) was dissolved in dichloromethane (5 mL), followed by the addition of 3,6-dihydro-2H-pyran-4-boronic acid (0.019 g, 0.15 mmol), copper(II) acetate monohydrate (0.045 g, 0.22 mmol), and pyridine (0.047 g, 0.59 mmol). The reaction was carried out at room temperature for 16 h until the reaction was complete. The reaction mixture was diluted with water (10 mL) and extracted with dichloromethane (20 mL × 2). The

organic phases were combined, washed with saturated brine, dried over anhydrous sodium sulfate, and concentrated under reduced pressure. The resulting residue was purified by C18 column chromatography (A: formic acid/water, B: acetonitrile) to obtain compound 7-14 (0.076 g) with a yield of 79.83%. LC-MS (ESI) $[M+H]^+$: 641.1.

**Step 14: Preparation of compound 7-15**

At room temperature, intermediate 7-14 (0.076 g, 0.12 mmol) was dissolved in a mixture of tetrahydrofuran (5 mL) and water (1 mL), followed by the addition of lithium hydroxide (0.02 g, 0.47 mmol), and the reaction was carried out at room temperature for 16 h until the reaction was complete. The solvent was removed by rotary evaporation under reduced pressure. Water (10 mL) was added for dilution, and the pH of the mixture was adjusted to <6 with dilute hydrochloric acid. The mixture was extracted with ethyl acetate (20 mL × 2). The organic phases were combined, washed with saturated brine, dried over anhydrous sodium sulfate, and concentrated under reduced pressure. The residue was lyophilized under vacuum to obtain compound 7-15 (0.076 g) with a yield of 100%. LC-MS (ESI) $[M+H]^+$: 613.3.

**Step 15: Preparation of compound 7-16**

At room temperature, intermediate 7-15 (0.056 g, 0.09 mmol), *N,N,N',N'*-tetramethylchloroformamidinium hexafluorophosphate (0.030 g, 0.108 mmol), 1-methylimidazole (0.011 g, 0.135 mmol), and 3-chloro-4-aminobenzotrifluoride (0.017 g, 0.09 mmol) were dissolved in acetonitrile (3 mL). After the reaction was carried out at room temperature for 1 h, LC-MS showed completion of the reaction. The reaction mixture was diluted with water (10 mL) and extracted with ethyl acetate (20 mL × 2). The organic phases were combined, washed with saturated brine, dried over anhydrous sodium sulfate, and concentrated under reduced pressure. The resulting residue was purified by C18 column chromatography (A: formic acid/water, B: acetonitrile) to obtain compound 7-16 (0.092 g) with a yield of 100%. LC-MS (ESI) $[M+H]^+$: 789.9.

**Step 16: Preparation of compound 7**

At room temperature, compound 7-16 (0.09 g, 0.11 mmol) was dissolved in dichloromethane (5 mL), and boron trichloride (0.1 mL) was added. The reaction system was purged with nitrogen and the reaction was carried out at room temperature for 16 h until the reaction was complete. The reaction mixture was quenched with methanol (5 mL) and concentrated under reduced pressure to remove the solvent. The residue was purified by preparative HPLC to obtain compound 7 (24.75 mg) with a yield of 30.1%. LC-MS (ESI) $[M+H]^+$: 700.2.

$^1$HNMR (400 MHz, MeOH-$d_4$) δ 8.60-8.54 (m,1H), 8.42-8.33(m,1H), 8.21-8.15(m,1H), 7.82-7.76 (m, 1H),7.66-7.58(m,1H), 6.59-6.48(m,1H), 5.38-5.31(m,2H), 4.85-4.78(m, 1H), 4.36-4.31(m,2H), 4.20-4.09(m,1H), 4.00-3.94(m, 2H), 3.31-3.22(m,1H), 3.11-2.79 (m, 6H), 2.77-2.72(m,2H), 2.55 -2.50(m, 3H), 1.74-1.50(m,2H), 1.37-1.31(m,3H).

**Example 8: Synthesis of compound 8**

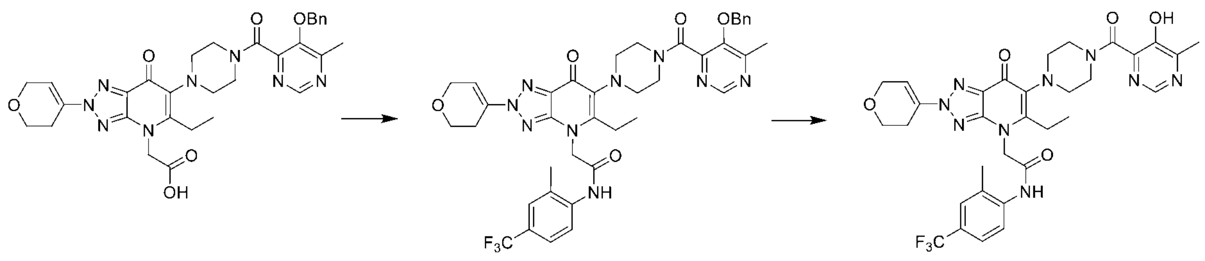

4-11 8-1 8

**Step 1: Preparation of compound 8-1**

Compound 4-11 (21 mg, 34.17 μmol), 2-methyl-4-trifluoromethylaniline (17.95 mg, 102.50 μmol), and pyridine (27.03 mg, 341.66 μmol, 27.52 μL) were dissolved in DCM (1.5 mL). Phosphorus oxychloride (26.19 mg, 170.83 μmol) was added at room temperature. After the addition was completed, the system was stirred at room temperature for 1 h until the reaction was complete. The reaction mixture was quenched with water (5 mL), and the product was extracted with ethyl acetate (10 mL × 2). The organic phase was dried over anhydrous sodium sulfate and concentrated under reduced pressure. The resulting residue was purified by silica gel column chromatography (DCM:MeOH = 19:1) to obtain

compound 8-1 (26 mg) with a yield of 65.0%. LC-MS(ESI)[M+H]$^+$: 772.3.

**Step 2: Preparation of compound 8**

Compound 8-1 (20 mg, 25.91 μmol) was dissolved in DCM (2 mL), and a solution of boron trichloride in hexane (0.2 mL, 1 M) was added at room temperature. The mixture was stirred at room temperature for 1 h until the reaction was complete. The reaction mixture was quenched with methanol (5 mL), and the solvent was removed by rotary evaporation under reduced pressure. The resulting residue was purified by preparative HPLC to obtain the crude product of compound 8 (12 mg, containing isomers). The crude product was further purified by preparative SFC (column: ChiralPak AD, 250 × 30 mm I.D., 10 μm; mobile phase: A: $CO_2$; B: isopropanol (0.1% $NH_3H_2O$); composition: B 40%; flow rate: 150 mL/min; back pressure: 100 bar; column temperature: 38°C; wavelength: 220 nm; cycle time: about 5 min) to obtain compound 8 (4.33 mg, yield: 24.5%). The analytical method for the target product was as follows: column: ChiralPak AD, 50 × 4.6 mm I.D., 3 μm; mobile phase: A: $CO_2$; B: isopropanol (0.05% DEA); composition: B 40%; flow rate: 3 mL/min; back pressure: 100 bar; column temperature: 35°C; wavelength: 220 nm; LC-MS (ESI) [M+H]$^+$: 682.3.

$^1$H NMR (400 MHz, MeOD-*d*$_4$) δ 8.41 (s, 1H), 7.65 (d, *J* = 8.4 Hz, 1H), 7.51 (s, 1H), 7.42 (d, *J* = 8.6 Hz, 1H), 6.81 (s, 1H), 5.28 (s, 2H), 4.33 (s, 2H), 4.00-3.74 (m, 4H), 3.15 - 3.02 (m, 4H), 2.86 (s, 3H), 2.76-2.65 (m, 1H), 2.44 (s, 3H), 2.33 (s, 3H), 2.19-2.09 (m, 1H), 2.03-1.93(m, 1H), 0.84 (t, *J* = 6.8 Hz, 3H).

**Example 9: Synthesis of compound 9**

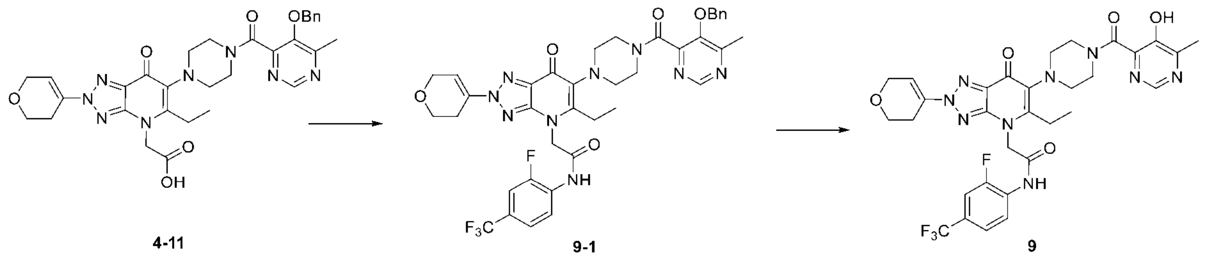

4-11 9-1 9

**Step 1: Preparation of compound 9-1**

Compound 4-11 (30 mg, 48.81 μmol), 2-fluoro-4-trifluoromethylaniline (34.97 mg, 195.23 μmol), and pyridine (38.61 mg, 488.08 μmol, 39.32 μL) were dissolved in dichloromethane (1 mL). Phosphorus oxychloride (37.42 mg, 244.04 μmol) was added at room temperature, and the mixture was stirred at room temperature for 1 h until the reaction was complete. The reaction mixture was poured into water (5 mL) to quench the reaction, extracted with ethyl acetate (10 mL × 2), and the organic phase was separated, dried over anhydrous sodium sulfate, and concentrated to dryness under vacuum. The residue was purified by preparative silica gel column chromatography (DCM:MeOH = 19:1) to obtain compound 9-1 (27.7 mg) with a yield of 73.28%. LC-MS (ESI) [M+H]$^+$: 776.2.

**Step 2: Preparation of compound 9**

Compound 9-1 (30 mg, 38.67 μmol) was dissolved in DCM (3 mL). A solution of boron trichloride in n-hexane (0.3 mL, 1 M) was added at room temperature, and the mixture was stirred at room temperature for 0.6 h until the reaction was complete. Methanol (5 mL) was then added to quench the reaction, and the solvent was evaporated to dryness. The residue was purified by preparative HPLC to obtain the crude product of compound 9 (15 mg, containing isomers). The crude product was further purified by SFC (column: ChiralPak AD, 250 × 30 mm I.D., 10 μm; mobile phase [A: carbon dioxide, B: ethanol (containing 0.1% ammonia water)]; composition B%: 30%, flow rate: 150 mL/min, column temperature: 38°C, wavelength: 220 nm, cycle time: about 9 min) to obtain compound 9 (5.08 mg) with a yield of 33.8%. (The retention time of the product peak was 0.637 min. SFC analysis conditions: column: ChiralPak AD, 50 × 4.6 mm I.D., 3 μm; mobile phase [A: carbon dioxide, B: ethanol (containing 0.05% DEA)]; 5-40% B, flow rate: 3 mL/min, column temperature: 35°C). LC-MS (ESI) [M+H]$^+$: 686.2.

$^1$HNMR(400MHz, Methanol-*d*$_4$) δ 8.42-8.20(m, 2H), 7.57(d, *J* =12.0Hz,1H), 7.47(d, J=8.4 Hz,1H), 6.88-6.79(m, 1H), 5.34(s,2H), 4.36(s, 2H),4.09-3.81(m, 4H), 3.60-3.40(m, 1H), 3.22-3.06 (m, 3H), 2.89(s,3H), 2.84-2.68(m,1H), 2.47 (s,3H), 2.24-2.14(m,1H), 2.10-1.98(m,1H),1.33-1.28 (m,3H).

**Example 10: Synthesis of compound 10**

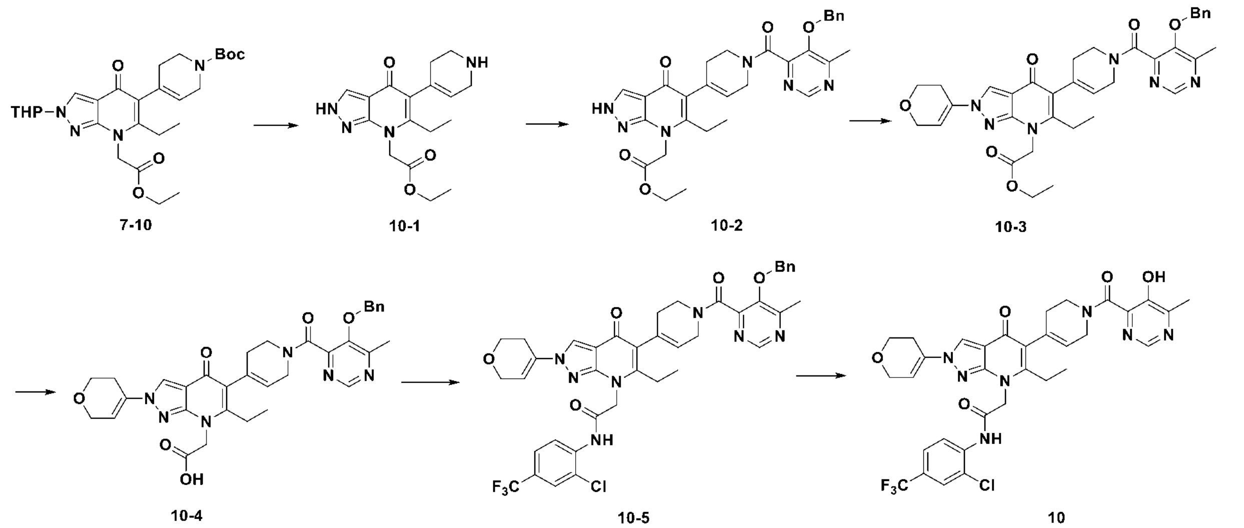

7-10 10-1 10-2 10-3

10-4 10-5 10

**Step 1: Preparation of compound 10-1**

At room temperature, intermediate 7-10 (200 mg, 0.39 mmol) was dissolved in tetrahydrofuran (6 mL), followed by the addition of a solution of hydrochloride in dioxane (2 mL, 1 M). After the reaction was carried out at room temperature for 2 h, LC-MS showed completion of the reaction. The solvent was removed by rotary evaporation under reduced pressure to obtain compound 10-1 (200 mg) with a yield of 100%. LC-MS(ESI) $[M+H]^+$: 331.1.

**Step 2: Preparation of compound 10-2**

At room temperature, intermediate 10-1 (200 mg, 0.39 mmol) was dissolved in *N,N*-dimethylformamide (1.5 mL), followed by the addition of 5-(benzyloxy)-6-methylpyrimidine-4-carboxylic acid (95.2 mg, 0.39 mmol), 2-(7-azabenzotriazol-1-yl)-*N,N,N',N'*-tetramethyluronium hexafluorophosphate (222.2 mg, 0.585 mmol), and *N,N*-diisopropylethylamine (251.6 mg, 1.95 mmol). After the reaction was carried out at room temperature for 1 h, LC-MS showed completion of the reaction. The reaction mixture was diluted with water (10 mL) and extracted with ethyl acetate (10 mL × 2). The organic phases were combined, washed with saturated brine, dried over anhydrous sodium sulfate, and concentrated under reduced pressure. The residue was purified by silica gel column chromatography (silica, dichloromethane/methanol = 10/1) to obtain compound 10-2 (129 mg) with a yield of 59.4%. LC-MS(ESI) $[M+H]^+$: 557.1.

**Step 3: Preparation of compound 10-3**

At room temperature, intermediate 10-2 (69 mg, 0.124 mmol) was dissolved in dichloromethane (5 mL), followed by the addition of 3,6-dihydro-2H-pyran-4-boronic acid (23.8 mg, 0.186 mmol), copper(II) acetate monohydrate (49.9 mg, 0.25 mmol), and triethylamine (37.6 mg, 0.372 mmol). After the reaction was carried out at room temperature for 16 h, LC-MS showed completion of the reaction. The reaction mixture was diluted with water (10 mL) and extracted with dichloromethane (20 mL × 2). The organic phases were combined, washed with saturated brine, dried over anhydrous sodium sulfate, and concentrated under reduced pressure. The residue was purified by C18 column chromatography (acetonitrile/water = 45%, formic acid system) to obtain compound 10-3 (50 mg) with a yield of 63.1%. LC-MS(ESI) $[M+H]^+$: 639.1.

**Step 4: Preparation of compound 10-4**

At room temperature, intermediate 10-3 (50 mg, 0.078 mmol) was dissolved in a mixed solvent of tetrahydrofuran (5 mL) and water (1 mL), followed by addition of lithium hydroxide monohydrate (13.1 mg, 0.31 mmol). After the reaction was carried out at room temperature for 0.5 h, LC-MS showed completion of the reaction. The pH of the mixture was adjusted to 3 with dilute hydrochloric acid (1 M), and the mixture was extracted with ethyl acetate (10 mL × 2). The organic phases were combined, washed with saturated brine, dried over anhydrous sodium sulfate, concentrated under reduced

pressure, and lyophilized to obtain compound 10-4 (46 mg) with a yield of 96.6%. LC-MS(ESI) $[M+H]^+$: 611.2.

**Step 5: Preparation of compound 10-5**

At room temperature, intermediate 10-4 (40 mg, 0.0655 mmol) and 3-chloro-4-aminobenzotrifluoride (38.4 mg, 0.196 mmol) were dissolved in pyridine (1 mL), followed by the dropwise addition of 4 drops of phosphorus oxychloride. The reaction was carried out at room temperature for 0.5 h until the reaction was complete. The reaction mixture was diluted with water (5 mL) and extracted with ethyl acetate (10 mL × 2). The organic phases were combined, washed with saturated brine, dried over anhydrous sodium sulfate, and concentrated under reduced pressure. The residue was purified by C18 column chromatography (acetonitrile/water = 68%, formic acid system) to obtain the target compound 10-5 (20 mg) with a yield of 38.7%. LC-MS(ESI) $[M+H]^+$: 788.2.

**Step 6: Preparation of compound 10**

At room temperature, compound 10-5 (20 mg, 0.025 mmol) was dissolved in dichloromethane (5 mL). A solution of boron trichloride (0.1 mL, 1 M) in dichloromethane was added, and the system was purged with nitrogen. The reaction was carried out at room temperature for 2 h, and LC-MS showed completion of the reaction. Methanol (2 mL) was added to quench the reaction. The solvent was removed by rotary evaporation under reduced pressure. The residue was purified by preparative HPLC to obtain compound 10 (5.02 mg) with a yield of 28.8%. LC-MS(ESI) $[M+H]^+$: 698.2.

$^1H$ NMR (400 MHz, DMSO-$d_6$) δ 10.28 (s, 2H), 8.61 (d, *J* = 5.8 Hz, 1H), 8.53 (s, 1H), 8.07 (d, *J* = 8.6 Hz, 1H), 7.95 (s, 1H), 7.71 (d, *J* = 8.8 Hz, 1H), 6.46 (s, 1H), 5.52 (d, *J* = 66.2 Hz, 1H), 5.31 - 5.08 (m, 2H), 4.40 - 3.94 (m, 4H), 3.85 (t, *J* = 5.4 Hz, 2H), 3.72 - 3.39 (m, 1H), 2.82 - 2.60 (m, 3H), 2.45 - 2.35 (m, 5H), 2.16 - 1.94 (m, 2H), 1.17 (t, *J* = 7.5 Hz, 3H).

**Example 11: Synthesis of compound 11**

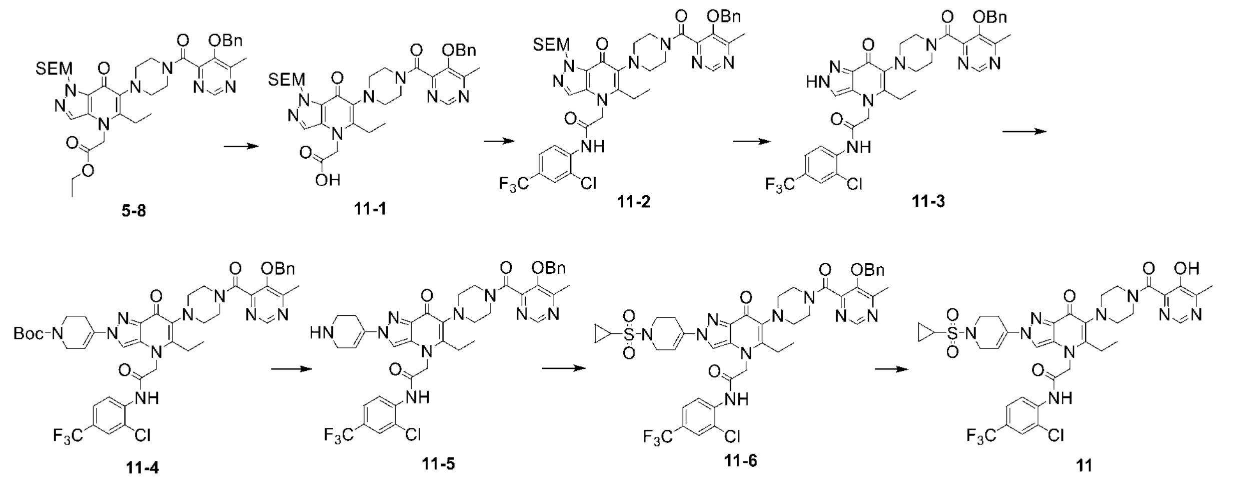

5-8 11-1 11-2 11-3

11-4 11-5 11-6 11

**Step 1: Preparation of compound 11-1**

Compound 5-8 (700 mg, 1.01 mmol) was dissolved in a mixed solvent of MeOH (3 mL), THF (9 mL), and water (3 mL), followed by addition of LiOH (85.05 mg, 3.55 mmol). The resulting mixture was reacted at 25°C for about 1 h until the reaction was complete. The mixture was adjusted to pH<4 with dilute hydrochloric acid and extracted with ethyl acetate (20 mL × 2). The organic phases were combined, washed with saturated brine (60 mL), dried over anhydrous sodium sulfate, and concentrated under reduced pressure to obtain compound 11-1 (650 mg) with a yield of 96.8%. LC-MS (ESI) $[M+H]^+$: 662.4.

**Step 2: Preparation of compound 11-2**

Compound 11-1 (350 mg, 528.84 μmol), 2-chloro-4-trifluoromethylaniline (155.14 mg, 793.27 μmol), and pyridine (209.16 mg, 2.64 mmol, 213.01 μL) were dissolved in DCM (5 mL). Phosphorus oxychloride (121.63 mg, 793.27 μmol, 73.94 μL) was added. After the addition was completed, the resulting reaction mixture was stirred at 25°C for

1 h until the starting material was completely consumed. The solvent was removed by rotary evaporation under reduced pressure. The resulting residue was separated by silica gel column chromatography (DCM/MeOH = 10/1) to obtain compound 11-2 (310 mg) with a yield of 69.8%. LC-MS(ESI) $[M+H]^+$: 839.2.

**Step 3: Synthesis of compound 11-3**

Compound 11-2 (310 mg, 369.32 μmol) was dissolved in HCl methanol solution (4 M, 40.00 mmol, 10 mL), and the mixture was reacted at 25°C for 1 h until the starting material was completely consumed. The reaction mixture was slowly added to a saturated sodium bicarbonate solution (40 mL) and extracted with ethyl acetate (40 mL × 2). The organic phases were combined, washed with saturated brine (40 mL), dried over anhydrous sodium sulfate, and concentrated under reduced pressure. The resulting residue was separated by silica gel column chromatography (DCM/MeOH = 10/1) to obtain compound 11-3 (183 mg) with a yield of 69.9%. LC-MS (ESI) $[M+H]^+$:709.2.

**Step 4: Synthesis of compound 11-4**

Compound 11-3 (183 mg, 258.07 μmol) was dissolved in DMSO (4 mL), followed by the addition of (1-(*tert*-butoxycarbonyl)-1,2,3,6-tetrahydropyridin-4-yl)boronic acid (117.20 mg, 516.14 μmol) and $PhenCuPPhBr_2$ (87.95 mg, 154.84 μmol). The resulting reaction mixture was reacted at 90°C under an air atmosphere for 16 h until the starting material was completely consumed. The solvent was removed by rotary evaporation under reduced pressure, and the resulting residue was separated by silica gel column chromatography (DCM/MeOH = 10/1) to obtain compound 11-4 (200 mg) with a yield of 87.04%. LC-MS(ESI) $[M+H]^+$:890.4.

**Step 12: Synthesis of compound 11-5**

Compound 11-4 (200 mg, 146.01 μmol) was dissolved in DCM (3 mL), and TFA (1.49 g, 13.06 mmol, 1 mL) was added. The resulting mixture was reacted at 25°C for approximately 2 h until the starting material was completely consumed. The reaction mixture was slowly poured into a saturated sodium bicarbonate solution (20 mL) and extracted with ethyl acetate (20 mL × 2). The resulting organic phase was washed with saturated brine, dried over anhydrous sodium sulfate, and concentrated under reduced pressure. The resulting residue was separated by silica gel column chromatography (DCM/MeOH = 5/1) to obtain compound 11-5 (110 mg) with a yield of 95.34%. LC-MS (ESI) $[M+H]^+$: 790.2.

**Step 13: Synthesis of compound 11-6**

Compound 11-5 (100 mg, 126.55 μmol) was dissolved in DCM (4 mL), and TEA (76.83 mg, 759.27 μmol, 105.90 μL) was added. Cyclopropylsulfonyl chloride (88.95 mg, 632.73 μmol) was slowly added dropwise at 25°C. The reaction was carried out at room temperature for about 2 h until the starting material was completely consumed. The mixture was poured into a saturated sodium bicarbonate solution (10 mL) and extracted with ethyl acetate (10 mL × 2). The organic phase was washed with saturated brine (20 mL), dried over anhydrous sodium sulfate, and concentrated under reduced pressure. The resulting residue was purified by silica gel column chromatography (DCM/MeOH = 10/1) to obtain compound 11-6 (100 mg) with a yield of 88.4%. LC-MS (ESI) $[M+H]^+$: 894.2.

**Step 14: Synthesis of compound 11**

Compound 11-6 (100 mg, 111.81 μmol) was dissolved in DCM (4 mL), and a solution of boron trichloride (1 M, 1 mL) in dichloromethane was added. The resulting mixture was reacted at 25°C for 1 h until the starting material was completely consumed. The solvent was removed by rotary evaporation under reduced pressure. The residue was separated by preparative HPLC to obtain compound 11 (5.05 mg) with a yield of 5.62%. LC-MS (ESI) $[M+H]^+$: 804.2.

$^1H$ NMR (400 MHz, DMSO-d6) δ 10.19 (s, 1H), 8.65 (s, 1H), 8.56 (s, 1H), 8.16 (s, 1H), 7.97 (s, 1H), 7.72 (s, 1H), 6.41 (s, 1H), 5.16 (s, 2H), 4.49 (d, *J* = 12.5 Hz, 1H), 4.02 (s, 2H), 3.73 (d, *J* = 12.0 Hz, 2H), 3.55 (s, 2H), 3.46 (d, *J* = 6.5 Hz, 3H), 3.19 (s, 4H), 2.98 - 2.81 (m, 5H), 2.71 (d, *J* = 10.8 Hz, 2H), 2.44 (s, 3H), 1.17 (s, 3H).

**Example 12: Synthesis of compound 12**

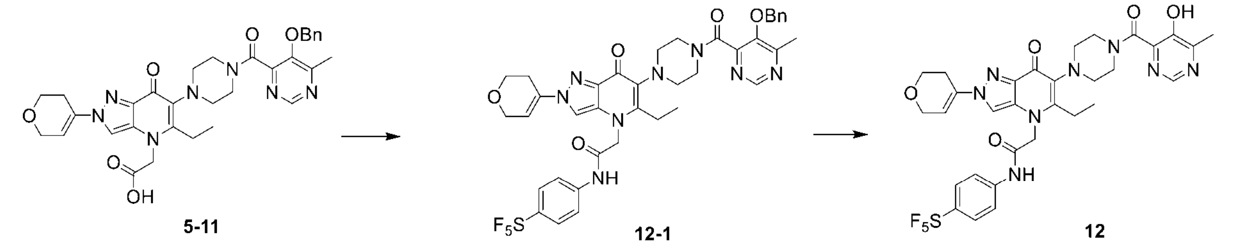

5-11 12-1 12

**Step 1: Synthesis of compound 12-1**

Compound 5-11 (100 mg, 162.96 μmol), 4-pentafluorosulfanylaniline (42.86 mg, 195.55 μmol), and pyridine (64.45 mg, 814.78 μmol, 65.64 μL) were dissolved in dichloromethane (3 mL). Phosphorus oxychloride (37.48 mg, 244.43 μmol) was added. After the addition was completed, the resulting reaction system was stirred at 25°C for 1 h until the reaction was complete. The reaction mixture was concentrated under reduced pressure, and the resulting residue was separated by silica gel column chromatography (DCM/MeOH = 10/1) to obtain compound 12-1 (116 mg, 142.36 μmol) with a yield of 87.36%. LC-MS (ESI) $[M+H]^+$: 815.2.

**Step 2: Synthesis of compound 12**

Compound 12-1 (106 mg, 130.09 μmol) was dissolved in DCM (3 mL). A solution of boron trichloride (1 M, 2 mL) in dichloromethane was added. The reaction was carried out at 25°C for about 1 h until the reaction was complete. The reaction mixture was concentrated under reduced pressure, and the resulting residue was purified by preparative HPLC to obtain compound 12 (4.5 mg, 6.21 μmol) with a yield of 4.77%. LCMS (ESI) $[M+H]^+$:725.2.

$^1H$ NMR (400 MHz, DMSO-$d_6$) δ 10.90 (s, 1H), 8.63 (s, 1H), 8.55 (s, 1H), 7.88 (d, *J* = 9.3 Hz, 2H), 7.78 (d, *J* = 9.0 Hz, 2H), 6.41 (s, 1H), 4.99 (s, 2H), 4.49 (d, *J* = 11.3 Hz, 1H), 4.27 (s, 2H), 3.89 (s, 2H), 3.75 (d, *J* = 30.6 Hz, 2H), 3.51 (s, 1H), 3.19 (s, 2H), 2.90 (s, 3H), 2.71 (s, 3H), 2.43 (s, 3H), 1.15 (s, 3H).

**Example 13: Synthesis of compound 13**

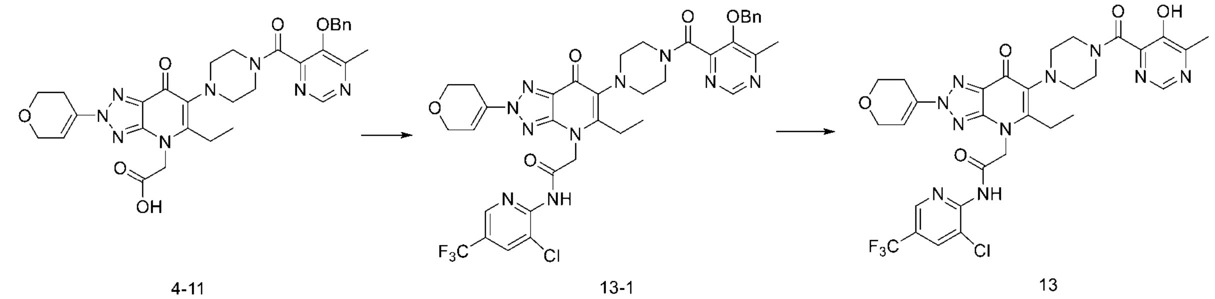

4-11 13-1 13

**Step 1: Synthesis of compound 13-1**

Under a nitrogen atmosphere, compound 4-11 (100 mg, 162.7 μmol), 3-chloro-5-(trifluoromethyl)pyridin-2-amine (64 mg, 325.4 μmol), and pyridine (64.4 mg, 813.5 μmol) were dissolved in dichloromethane (5 mL). Phosphorus oxychloride (49.9 mg, 325.4 μmol) was added, and the resulting mixture was stirred at 25°C for 0.5 h until the reaction was complete. The reaction mixture was concentrated under reduced pressure, and the resulting residue was separated by silica gel column chromatography (DCM/MeOH = 10/1) to obtain compound 13-1 (40 mg) with a yield of 31%. LC-MS (ESI) $[M+H]^+$: 793.2.

**Step 2: Synthesis of compound 13**

Under a nitrogen atmosphere, compound 13-1 (16 mg, 20.2 μmol) was added to trifluoroacetic acid (1 mL), and the mixture was heated to 90°C and reacted for 0.5 h until the reaction was complete. The reaction mixture was concentrated under reduced pressure, and then the resulting residue was purified by preparative HPLC to obtain compound 13 (3.0 mg) with a yield of 21%. LC-MS (ESI) $[M+H]^+$:703.2.

$^1$H NMR (400 MHz, Methanol-d4) δ 8.70 (s, 1H), 8.56 (s, 1H), 8.35 (s, 1H), 6.89 (s, 1H), 5.54 (s, 2H), 4.39 (s, 2H), 4.12 (s, 1H), 4.00 (s, 2H), 3.92 (s, 2H), 3.50 (s, 3H), 3.15 (s, 2H), 2.93 (s, 3H), 2.74 (s, 1H), 2.54 (s, 3H), 1.34 (s, 3H).

**Example 14: Synthesis of compound 14**

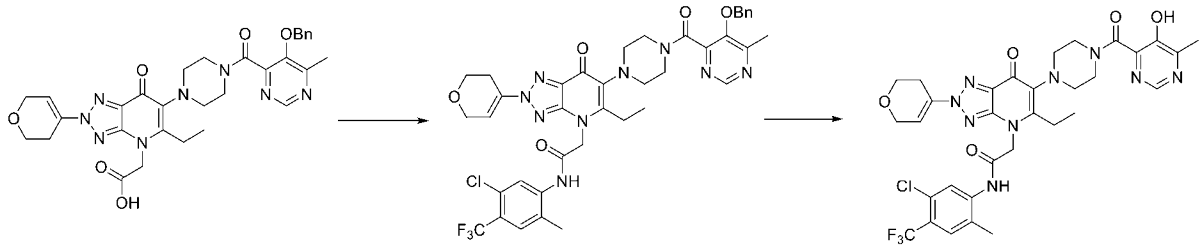

4-11 14-1 14

**Step 1: Synthesis of compound 14-1**

Under a nitrogen atmosphere, compound 4-11 (100 mg, 162.7 μmol), 5-chloro-2-methyl-4-(trifluoromethyl) aniline (37.1 mg, 177.2 μmol), and pyridine (63.7 mg, 805.3 μmol) were dissolved in dichloromethane (6 mL). A solution of phosphorus oxychloride (27.2 mg, 177.2 μmol) in dichloromethane (0.2 mL) was added, and the resulting mixture was stirred at 25°C for 0.5 h until the reaction was complete. The reaction mixture was poured into water and extracted with dichloromethane (2 × 5 mL). The organic phase was dried over anhydrous sodium sulfate and subjected to rotary evaporation under reduced pressure. The residue was purified by silica gel column chromatography (dichloromethane:methanol = 93:7) to obtain compound 14-1 (110 mg) with a yield of 84%. LC-MS (ESI) [M+H]$^+$: 806.4.

**Step 2: Synthesis of compound 14**

Under a nitrogen atmosphere, compound 14-1 (150 mg, 184.2 μmol) was added to trifluoroacetic acid (5 mL). The reaction was carried out at 90°C for 0.5 h until the reaction was complete. The reaction mixture was concentrated under reduced pressure, and the resulting residue was purified by preparative HPLC to obtain compound 14 (99.7 mg) with a yield of 76%. LC-MS(ESI) [M+H]$^+$:716.3.

$^1$H NMR (400 MHz, DMSO-$d_6$) δ 10.24 (s, 1H), 10.11 (s, 1H), 8.58 (s, 1H), 7.99 (s, 1H), 7.76 (s, 1H), 6.79 - 6.72 (m, 1H), 5.27 (s, 2H), 4.51 (d, *J* = 12.4 Hz, 1H), 4.33 (q, *J* = 2.9 Hz, 2H), 3.91 (t, *J* = 5.5 Hz, 2H), 3.70 (d, *J* = 11.4 Hz, 2H), 3.49 (d, *J* = 12.6 Hz, 1H), 3.23 (s, 1H), 3.08 - 2.91 (m, 3H), 2.78 (d, *J* = 23.3 Hz, 3H), 2.59 (d, *J* = 11.4 Hz, 1H), 2.45 (s, 3H), 2.37 (s, 3H), 1.20 (t, *J* = 7.4 Hz, 3H).

**Example 15: Synthesis of compound 15**

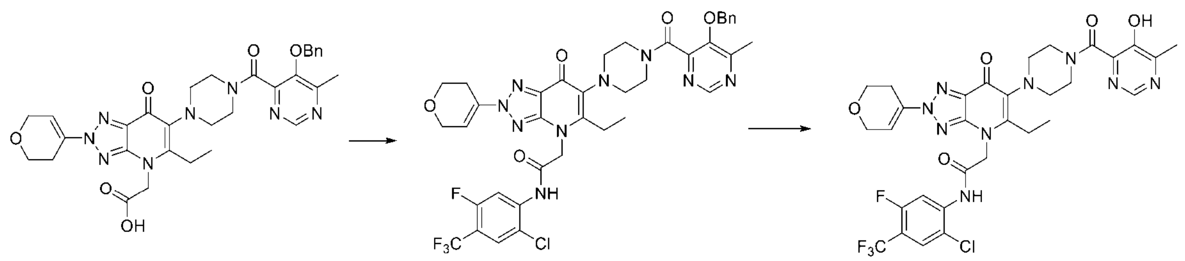

4-11 15-1 15

**Step 1: Synthesis of compound 15-1**

Under a nitrogen atmosphere, compound 4-11 (100 mg, 162.7 μmol), 2-chloro-5-fluoro-4-(trifluoromethyl)aniline (37.8 mg, 177.2 μmol), and pyridine (63.7 mg, 805.3 μmol) were dissolved in dichloromethane (6 mL). Phosphorus oxychloride (27.2 mg, 177.2 μmol) was added, and the resulting mixture was stirred at 25°C for 0.5 h until the reaction was

complete. The reaction mixture was poured into water and extracted with dichloromethane (2 × 5 mL). The organic phase was dried over anhydrous sodium sulfate and subjected to rotary evaporation under reduced pressure. The resulting residue was separated by silica gel column chromatography (dichloromethane:methanol = 93:7) to obtain compound 15-1 (120 mg) with a yield of 87%. LC-MS(ESI) $[M+H]^+$: 810.3.

**Step 2: Synthesis of compound 15**

Under a nitrogen atmosphere, compound 15-1 (100 mg, 117.3 μmol) was added to trifluoroacetic acid (5 mL). The mixture was heated to 90°C and reacted for 0.5 h until the reaction was complete. The reaction mixture was concentrated under reduced pressure, and the resulting residue was purified by preparative HPLC to obtain compound 15 (70.2 mg) with a yield of 83%. LC-MS(ESI) $[M+H]^+$=720.2.

$^1$H NMR (400 MHz, DMSO-$d_6$) δ 10.47 (s, 1H), 10.24 (s, 1H), 8.58 (s, 1H), 8.12 (d, *J* = 12.8 Hz, 1H), 8.02 (d, *J* = 7.3 Hz, 1H), 6.74 (t, *J* = 1.5 Hz, 1H), 5.35 (s, 2H), 4.51 (d, *J* = 12.5 Hz, 1H), 4.31 (d, *J* = 3.0 Hz, 2H), 3.90 (t, *J* = 5.5 Hz, 2H), 3.77 - 3.62 (m, 2H), 3.49 (d, *J* = 12.6 Hz, 1H), 3.22 (t, *J* = 12.0 Hz, 1H), 2.97 (dd, *J* = 11.3, 5.0 Hz, 3H), 2.77 (d, *J* = 22.1 Hz, 3H), 2.58 (d, *J* = 11.5 Hz, 1H), 2.44 (s, 3H), 1.18 (t, *J* = 7.4 Hz, 3H).

**Example 16: Synthesis of compound 16**

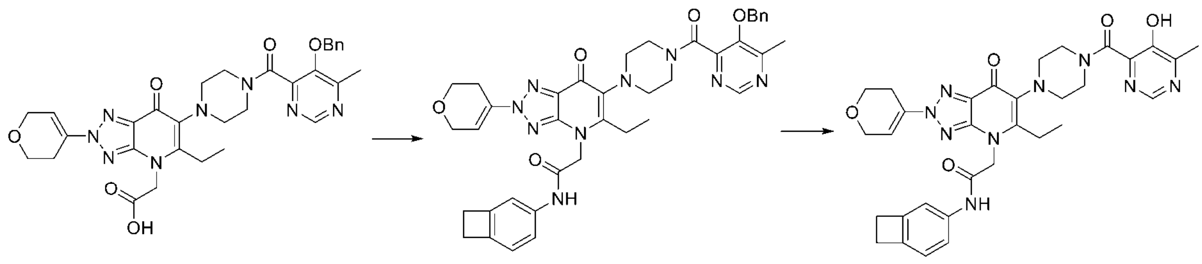

4-11 16-1 16

**Step 1: Synthesis of compound 16-1**

Under a nitrogen atmosphere, compound 4-11 (100 mg, 162.7 μmol), bicyclo[4.2.0]octa-1(6),2,4-trien-3-amine (28.7 mg, 241.1 μmol) (synthesized with reference to WO2023284837), and pyridine (63.6 mg, 803.7 μmol, 64.75 μL) were dissolved in dichloromethane (5 mL), followed by the addition of phosphorus oxychloride (29.6 mg, 192.89 μmol). The resulting mixture was stirred at 25°C for 1 h until the reaction was complete. The reaction mixture was poured into saturated aqueous sodium bicarbonate solution (3 mL) and extracted with dichloromethane (2 × 5 mL). The organic phase was dried over anhydrous sodium sulfate and concentrated under reduced pressure. The residue was separated by silica gel column chromatography (dichloromethane:methanol = 15:1) to obtain compound 16-1 (75 mg) with a yield of 61%. LC-MS (ESI) $[M+H]^+$: 716.4.

**Step 2: Synthesis of compound 16**

Under a nitrogen atmosphere, compound 16-1 (75 mg, 98.2 μmol) was added to trifluoroacetic acid (1.5 mL). The mixture was heated to 90°C and reacted for 0.5 h until the reaction was complete. The reaction mixture was concentrated under reduced pressure, and the resulting residue was purified by preparative HPLC to obtain compound 16 (29.5 mg) with a yield of 48%. LC-MS(ESI) $[M+H]^+$: 626.4.

$^1$H NMR (400 MHz, DMSO-$d_6$) δ 10.36 (s, 1H), 10.23 (s, 1H), 8.57 (s, 1H), 7.37 (s, 1H), 7.28 (dd, *J* = 8.1, 1.5 Hz, 1H), 7.02 (d, *J* = 8.0 Hz, 1H), 6.73 (s, 1H), 5.10 (s, 2H), 4.50 (d, *J* = 12.5 Hz, 1H), 4.31 (d, *J* = 3.0 Hz, 2H), 3.89 (t, *J* = 5.5 Hz, 2H), 3.74-3.65 (m, 2H), 3.48 (d, J = 12.8 Hz, 1H), 3.23 (d, *J* = 12.0 Hz, 1H), 3.08 (s, 4H), 2.97 (d, *J* = 8.8 Hz, 3H), 2.76 (d, *J* = 21.6 Hz, 3H), 2.57 (d, *J* = 11.5 Hz, 1H), 2.44 (s, 3H), 1.17 (t, *J* = 7.4 Hz, 3H).

**Example 17: Synthesis of compound 17**

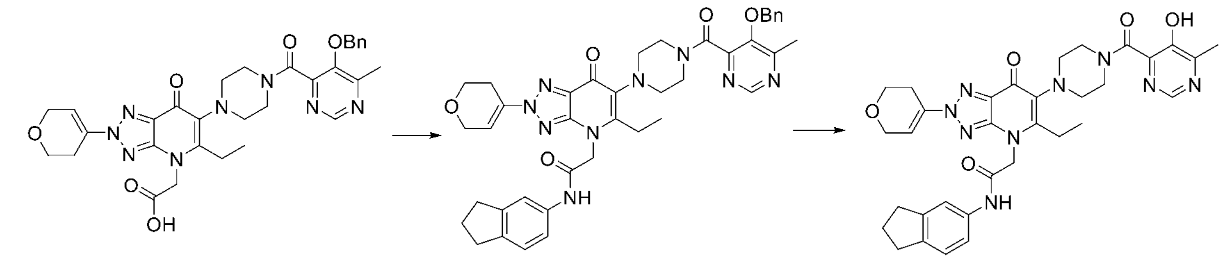

4-11 17-1 17

**Step 1: Synthesis of compound 17-1**

Under a nitrogen atmosphere, compound 4-11 (120 mg, 195.2 μmol), 2,3-dihydro-1H-inden-5-amine (38.5 mg, 289.3 μmol), and pyridine (76.3 mg, 964.5 μmol) were dissolved in dichloromethane (5 mL). Phosphorus oxychloride (35.5 mg, 231.5 μmol) was added, and the resulting mixture was stirred at 25°C for 1 h until the reaction was complete. The reaction mixture was poured into saturated aqueous sodium bicarbonate solution (3 mL) to quench, and extracted with dichloromethane (2 × 5 mL). The organic phase was dried over anhydrous sodium sulfate and subjected to rotary evaporation under reduced pressure. The resulting residue was separated by silica gel column chromatography (dichloromethane:methanol = 15:1) to obtain compound 17-1 (120 mg) with a yield of 81%. LC-MS (ESI) $[M+H]^+$: 730.2.

**Step 2: Synthesis of compound 17**

Under a nitrogen atmosphere, compound 17-1 (120 mg, 155.9 μmol) was added to trifluoroacetic acid (1.0 mL). The reaction was stirred at 90°C for 1 h until the reaction was complete. The reaction mixture was concentrated under reduced pressure, and the resulting residue was purified by preparative HPLC to obtain compound 17 (67.6 mg) with a yield of 68%. LC-MS(ESI) $[M+H]^+$:640.4.

$^1$H NMR (400 MHz, DMSO-$d_6$) δ 10.38 (s, 1H), 8.53 (s, 1H), 7.49 (d, *J* = 1.6 Hz, 1H), 7.25 (dd, *J* = 8.1, 2.0 Hz, 1H), 7.15 (d, *J* = 8.1 Hz, 1H), 6.75 - 6.71 (m, 1H), 5.10 (s, 2H), 4.50 (d, *J* = 12.8 Hz, 1H), 4.31 (q, *J* = 2.9 Hz, 2H), 3.89 (t, *J* = 5.5 Hz, 2H), 3.76 - 3.65 (m, 2H), 3.50 (d, *J* = 12.8 Hz, 1H), 3.22 (t, *J* = 12.3 Hz, 2H), 2.97 (d, *J* = 8.9 Hz, 3H), 2.80 (q, *J* = 7.0 Hz, 7H), 2.57 (d, *J* = 10.9 Hz, 1H), 2.43 (s, 3H), 2.02 - 1.95 (m, 2H), 1.17 (t, *J* = 7.4 Hz, 3H).

**Example 18: Synthesis of compound 18**

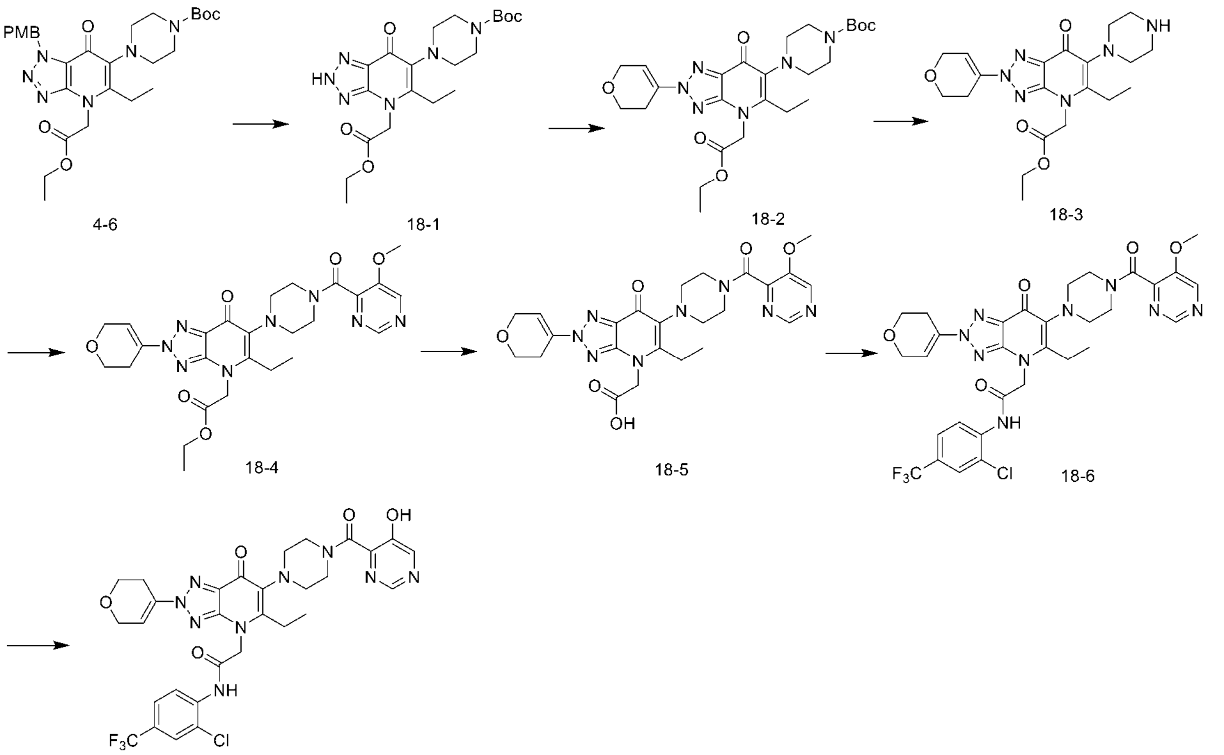

4-6 18-1 18-2 18-3

18-4 18-5 18-6

18

**Step 1: Synthesis of compound 18-1**

Compound 4-6 (9 g, 15.4 mmol) was added to ethanol (400 mL), followed by the addition of palladium on carbon (5%, 1.6 g) and palladium hydroxide on carbon (5%, 2.2 g). The reaction was heated under reflux under a hydrogen atmosphere for 16 h until the reaction was complete. The reaction mixture was filtered and concentrated under reduced pressure. The resulting crude product was purified by slurrying with a mixed solvent (petroleum ether:ethyl acetate = 5:1) to obtain compound 18-1 (6 g) with a yield of 85%. LC-MS (ESI) $[M+H]^+$: 435.4.

**Step 2: Synthesis of compound 18-2**

Compound 18-1 (6.75 g, 14.43 mmol), (3,6-dihydro-2H-pyran-4-yl)boronic acid (3.69 g, 28.86 mmol), and pyridine (11.41 g, 144.28 mmol, 11.62 mL) were dissolved in dichloromethane (140 mL). After ultrasonication to achieve a clear solution, copper(II) acetate (7.86 g, 43.28 mmol) was added. The reaction was carried out at room temperature under an oxygen atmosphere for 2 h until the reaction was complete. The reaction mixture was filtered through diatomite, and the filtrate was sequentially washed with water (3 × 30 mL) and saturated brine (50 mL), dried over anhydrous sodium sulfate, and concentrated under reduced pressure. The resulting crude product was purified by silica gel column (dichloromethane:methanol = 20:1) to obtain compound 18-2 (7.43 g) with a yield of 92%. LC-MS (ESI) $[M+H]^+$: 517.3.

**Step 3: Synthesis of compound 18-3**

Under a nitrogen atmosphere, compound 18-2 (7.43 g, 13.31 mmol) was dissolved in a solution of hydrochloride in dioxane (4 mol/L, 75 mL). The reaction was stirred at 25°C for 0.5 h until the reaction was complete. The reaction mixture was concentrated under reduced pressure to remove the solvent. Water (100 mL) was added, and the pH was adjusted to 8 with saturated sodium carbonate solution. The mixture was extracted with dichloromethane (3 × 50 mL). The organic phases were combined, washed with saturated brine (50 mL), dried over anhydrous sodium sulfate, and concentrated to dryness under reduced pressure to obtain compound 18-3 (5.4 g) with a yield of 93%. LC-MS (ESI) $[M+H]^+$: 417.4.

**Step 4: Synthesis of compound 18-4**

Under a nitrogen atmosphere, compound 18-3 (1.72 g, 4.13 mmol) was dissolved in anhydrous N,N-dimethylformamide (20 mL). 5-Methoxypyrimidine-4-carboxylic acid (0.76 g, 4.96 mmol), 2-(7-azabenzotriazol-1-yl)-*N,N,N',N'*-tetramethyluronium hexafluorophosphate (3.14 g, 8.26 mmol), and *N,N*-diisopropylethylamine (0.53 g, 4.13 mmol) were

added. The reaction was stirred at 25°C for 16 h until the reaction was complete. The reaction mixture was diluted with water (40 mL) and extracted with ethyl acetate (30 mL × 3). The organic phases were combined, washed with saturated brine (50 mL), dried over anhydrous sodium sulfate, and concentrated under reduced pressure. The resulting crude product was purified by silica gel column chromatography (methanol:dichloromethane = 1:9) to obtain compound 18-4 (2.0 g) with a yield of 88%. LC-MS (ESI) $[M+H]^+$: 553.4.

**Step 5: Synthesis of compound 18-5**

Under a nitrogen atmosphere, compound 18-4 (2.5 g, 4.5 mmol) was dissolved in tetrahydrofuran/water (16 mL/4 mL), and lithium hydroxide monohydrate (0.76 g, 18.1 mmol) was added. The reaction system was stirred at 25°C for 1 h until the reaction was complete. Dilute hydrochloric acid (2 mol/L) was added dropwise to adjust the pH to 2. Water (20 mL) was added, and the mixture was extracted with ethyl acetate (20 mL × 3). The organic phases were combined, washed with saturated brine (50 mL), dried over anhydrous sodium sulfate, and concentrated to dryness under reduced pressure to obtain compound 18-5 (2.0 g) with a yield of 84%. LC-MS (ESI) $[M+H]^+$: 525.2.

**Step 6: Synthesis of compound 18-6**

Under a nitrogen atmosphere, compound 18-5 (0.2 g, 0.38 mmol) was dissolved in dichloromethane (10 mL), and 2-chloro-4-trifluoromethylaniline (0.089 g, 0.42 mmol) and pyridine (0.30 g, 3.8 mmol) were added. A solution of phosphorus oxychloride (0.07 g, 0.46 mmol) in dichloromethane (3 mL) was then added. The reaction was carried out at 25°C for 15 min until the reaction was complete. The reaction mixture was diluted with water (10 mL) and extracted with dichloromethane (10 mL × 3). The organic phases were combined, washed with saturated brine (10 mL), dried over anhydrous sodium sulfate, and concentrated under reduced pressure. The resulting crude product was purified by silica gel column chromatography (methanol/dichloromethane = 5%) to obtain compound 18-6 (0.1 g) with a yield of 37%. LC-MS (ESI) $[M+H]^+$: 702.2.

**Step 7: Synthesis of compound 18**

Under a nitrogen atmosphere, compound 18-6 (0.15 g, 0.21 mmol) was dissolved in anhydrous *N,N*-dimethylformamide (4 mL), and aluminum trichloride (0.28 g, 2.1 mmol) was added. The reaction was carried out in a microwave reactor at 150°C for 1 h. The reaction mixture was diluted with water (10 mL) and extracted with ethyl acetate (20 mL × 3). The resulting organic phase was washed with saturated brine (20 mL), dried over anhydrous sodium sulfate, and concentrated under reduced pressure. The resulting crude product was purified by preparative HPLC to obtain compound 18 (20 mg) with a yield of 14%. LC-MS (ESI) $[M+H]^+$: 688.4.

$^1$H NMR (400 MHz, MeOD-d4) δ 8.65 (s, 1H), 8.41 (s, 1H), 8.16 (d, *J* = 8.5 Hz, 1H), 7.83 (d, *J* = 1.6 Hz, 1H), 7.62 (dd, *J* = 8.6, 1.5 Hz, 1H), 6.88 (s, 1H), 5.44 - 5.36 (m, 2H), 4.69 (d, *J* = 12.6 Hz, 1H), 4.39 (d, *J* = 2.8 Hz, 2H), 4.01 - 3.95 (m, 2H), 3.95 - 3.83 (m, 2H), 3.64 - 3.54 (m, 1H), 3.51 - 3.39 (m, 1H), 3.19 - 3.10 (m, 3H), 2.97 - 2.85 (m, 3H), 2.75 (d, *J* = 11.3 Hz, 1H), 1.33 (t, *J* = 5.0 Hz, 3H).

**Example 19: Synthesis of compound 19**

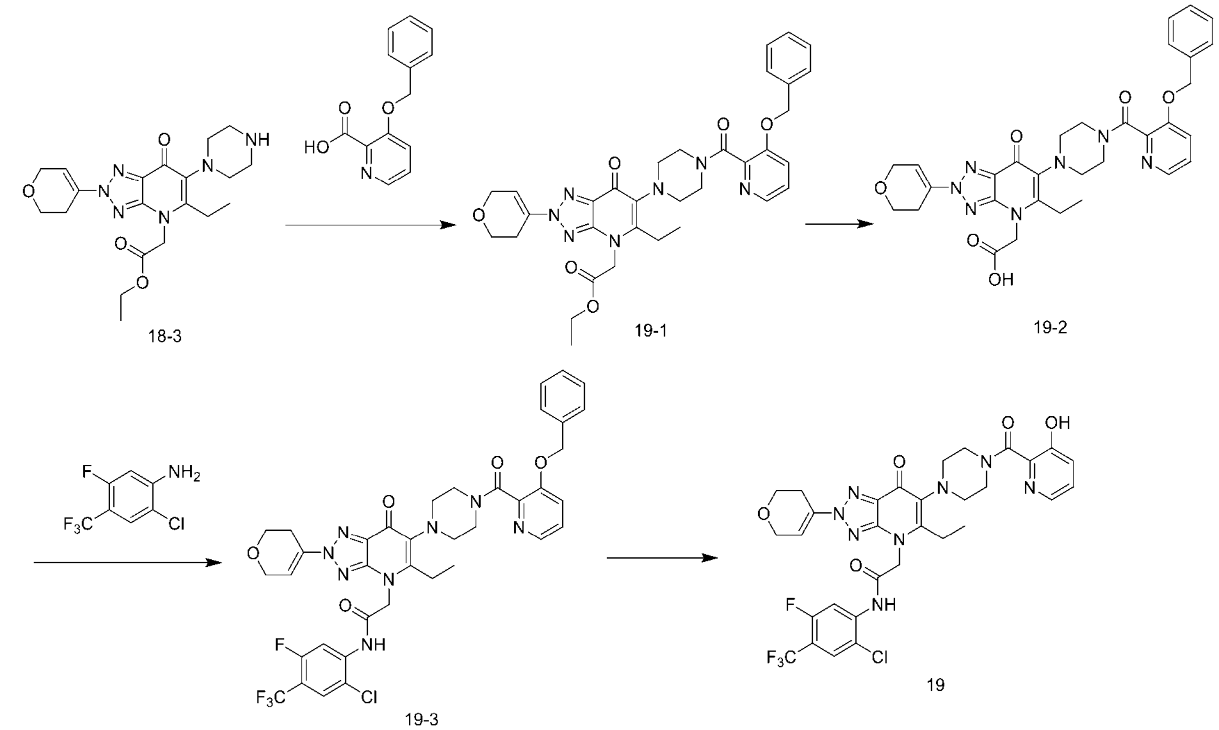

18-3 19-1 19-2 19-3 19

**Step 1: Synthesis of compound 19-1**

Under a nitrogen atmosphere, compound 18-3 (0.26 g, 0.62 mmol) was dissolved in *N,N*-dimethylformamide (3 mL), followed by the addition of 3-(benzyloxy)picolinic acid (0.17 g, 0.74 mmol), 2-(7-azabenzotriazol-1-yl)-*N,N,N',N'*-tetramethyluronium hexafluorophosphate (0.28 g, 0.74 mmol), and *N,N*-diisopropylethylamine (0.4 g, 3.1 mmol). The reaction was stirred at 25°C for 1 h until the reaction was complete. The reaction mixture was diluted with water (6 mL) and extracted with ethyl acetate (5 mL × 3). The organic phases were combined, washed with saturated brine (5 mL), dried over anhydrous sodium sulfate, and concentrated under reduced pressure. The resulting crude product was purified by silica gel column chromatography (methanol/dichloromethane = 6%) to obtain compound 19-1 (0.35 g) with a yield of 89%. LC-MS (ESI) $[M+H]^+$: 628.3.

**Step 2: Synthesis of compound 19-2**

Under a nitrogen atmosphere, compound 19-1 (0.28 g, 0.45 mmol) was dissolved in tetrahydrofuran/water (5 mL/1 mL), and lithium hydroxide monohydrate (57 mg, 1.35 mmol) was added. The reaction was stirred at 25°C for 1 h until the reaction was complete. The reaction mixture was adjusted to pH = 2 with dilute hydrochloric acid (2 mol/L), diluted with water (10 mL), and extracted with ethyl acetate (10 mL × 3). The organic phases were combined, washed with saturated brine (20 mL), dried over anhydrous sodium sulfate, and concentrated under reduced pressure to obtain compound 19-2 (0.24 g) with a yield of 90%. LC-MS (ESI) $[M+H]^+$: 600.3.

**Step 3: Synthesis of compound 19-3**

Under a nitrogen atmosphere, compound 19-2 (100 mg, 0.17 mmol) was dissolved in dichloromethane (5 mL), followed by the addition of intermediate 2-chloro-5-fluoro-4-(trifluoromethyl)aniline (44 mg, 0.20 mmol) and pyridine (130 mg, 1.70 mmol). A solution of phosphorus oxychloride (31 mg, 0.20 mmol) in dichloromethane (3 mL) was then added. The reaction was stirred at room temperature for 15 min until the reaction was complete. The reaction mixture was diluted with water (6 mL) and extracted with dichloromethane (5 mL × 3). The organic phases were combined, washed with saturated brine (5 mL), dried over anhydrous sodium sulfate, and concentrated under reduced pressure. The residue was purified by silica gel column chromatography (methanol/dichloromethane = 5%) to obtain compound 19-3 (66 mg) with a yield of 50%. LC-MS (ESI) $[M+H]^+$: 795.3.

### Step 4: Synthesis of compound 19

Under a nitrogen atmosphere, compound 19-3 (56 mg, 0.70 mmol) was dissolved in trifluoroacetic acid (3 mL), heated to 90°C, and stirred for 3 h until the reaction was complete. The reaction mixture was concentrated to dryness under reduced pressure. The residue was diluted with water (6 mL) and extracted with dichloromethane (5 mL × 3). The organic phases were combined, sequentially washed with water (5 mL × 6) and saturated sodium chloride solution (5 mL), dried over anhydrous sodium sulfate, and concentrated under reduced pressure. The residue was purified by HPLC to obtain compound 19 (7.0 mg) with a yield of 14%. LC-MS (ESI) $[M+H]^+$: 705.3.

$^1H$ NMR (400 MHz, MeOD-d4) δ 8.17 (d, *J* = 12.6 Hz, 1H), 8.09 (s, 1H), 7.85 (d, *J* = 7.1 Hz, 1H), 7.36 (d, *J* = 2.8 Hz, 2H), 6.87 (s, 1H), 5.43 (s, 2H), 4.77 - 4.66 (m, 1H), 4.42 - 4.33 (m, 2H), 3.99 (t, *J* = 5.4 Hz, 2H), 3.95 - 3.83 (m, 2H), 3.76 - 3.60 (m, 1H), 3.38 (d, *J* = 22.3 Hz, 1H), 3.18 - 3.09 (m, 3H), 2.96 - 2.84 (m, 3H), 2.79 - 2.68 (m, 1H), 1.32 (t, *J* = 7.4 Hz, 3H).

### Example 20: Synthesis of compound 20

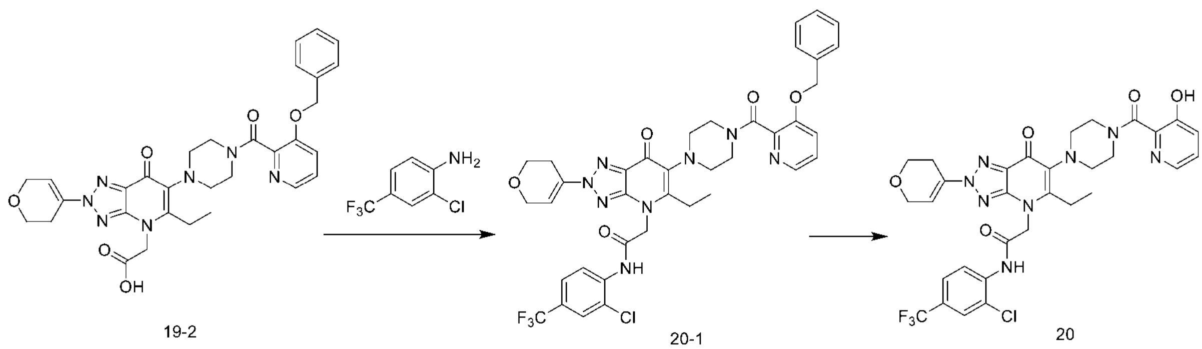

19-2 20-1 20

### Step 1: Synthesis of compound 20-1

Under a nitrogen atmosphere, compound 19-2 (100 mg, 0.17 mmol), 2-chloro-4-trifluoromethylaniline (48.92 mg, 0.25 mmol), and phosphorus oxychloride (76.71 mg, 500.3 μmol) were dissolved in DCM (6 mL), followed by the addition of pyridine (79.15 mg, 1.0 mmol). The resulting mixture was stirred and reacted at 25°C for 0.5 h until the reaction was complete. The reaction mixture was diluted with water (6 mL) and extracted with dichloromethane (5 mL × 3). The organic phases were combined, washed with saturated brine (5 mL), dried over anhydrous sodium sulfate, and concentrated under reduced pressure. The residue was purified by silica gel column chromatography (methanol/dichloromethane = 10/1) to obtain compound 20-1 (122 mg) with a yield of 94%. LC-MS (ESI) $[M+H]^+$: 777.2.

### Step 2: Synthesis of compound 20

Under a nitrogen atmosphere, compound 20-1 (122 mg, 0.16 mmol) was dissolved in trifluoroacetic acid (1 mL), heated to 90°C, and stirred for 0.5 h until the reaction was complete. The reaction mixture was concentrated to dryness under reduced pressure. The residue was purified by HPLC to obtain compound 20 (7.2 mg) with a yield of 6.7%. LC-MS (ESI) $[M+H]^+$: 687.2.

$^1H$ NMR (400 MHz, MeOD-d4) δ 8.15 (s, 1H), 8.07 (s, 1H), 7.81 (s, 1H), 7.60 (d, *J* = 10.1 Hz, 1H), 7.34 (s, 2H), 6.86 (s, 1H), 5.38 (s, 2H), 4.36 (s, 2H), 3.94 (d, *J* = 36.8 Hz, 4H), 3.64 (s, 1H), 3.45 (s, 2H), 3.15 (s, 3H), 2.90 (s, 3H), 2.69 (s, 1H), 1.33 (s, 3H).

### Example 21: Synthesis of compound 21

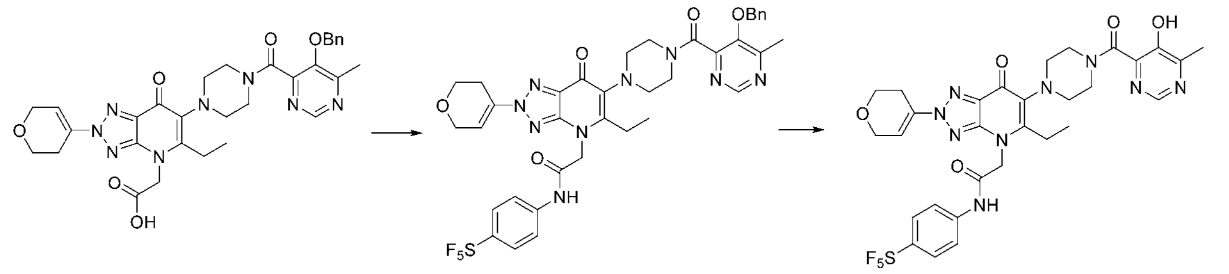

4-11 21-1 21

**Step 1: Synthesis of compound 21-1**

Under a nitrogen atmosphere, compound 4-11 (33.6 mg, 54.7 μmol), 4-(pentafluorothio)aniline (11.98 mg, 54.7 μmol), and pyridine (21.6 mg, 273.3 μmol) were dissolved in dichloromethane (5 mL). A solution of phosphorus oxychloride (25.2 mg, 164.0 μmol) in dichloromethane (0.5 mL) was added. The resulting mixture was stirred and reacted at 25°C for 1 h until the reaction was complete. The reaction mixture was poured into saturated aqueous sodium bicarbonate solution (3 mL) to quench, and extracted with dichloromethane (2 × 5 mL). The organic phase was dried over anhydrous sodium sulfate and subjected to rotary evaporation under reduced pressure. The resulting crude product was separated by silica gel column chromatography (dichloromethane:methanol = 9:1) to obtain compound 21-1 (42 mg) with a yield of 94%. LC-MS (ESI) $[M+H]^+$: 816.2.

**Step 2: Synthesis of compound 21**

Under a nitrogen atmosphere, compound 21-1 (42 mg, 51.5 μmol) was dissolved in a dichloromethane (5 mL) solution, followed by the addition of boron trichloride (25.13 mg, 214.51 μmol, 0.2 mL). The reaction was stirred at 25°C for 1 h until the reaction was complete. Methanol (1 mL) was added to quench the reaction. After concentration under reduced pressure, the resulting crude product was purified by preparative HPLC to obtain compound 21 (12 mg) with a yield of 39%. LC-MS(ESI) $[M+H]^+$=726.2.

$^1H$ NMR (400 MHz, DMSO-$d_6$) δ 11.04 (s, 1H), 10.10 (s, 1H), 8.56 (s, 1H), 7.88 (d, *J* = 9.3 Hz, 2H), 7.77 (d, *J* = 8.9 Hz, 2H), 6.70 - 6.52 (m, 1H), 5.36 (s, 2H), 4.50 (d, *J* = 12.4 Hz, 1H), 4.33 (q, *J*= 2.8 Hz, 2H), 3.93 (t, *J* = 5.4 Hz, 2H), 3.70 (t, *J* = 12.0 Hz, 2H), 3.47 (d, *J* = 12.7 Hz, 1H), 3.20 (d, *J*= 11.1 Hz, 2H), 3.09 - 2.91 (m, 3H), 2.76 (d, *J* = 24.2 Hz, 3H), 2.57 (d, *J* = 11.4 Hz, 1H), 2.44 (s, 3H), 1.17 (t, *J* = 7.4 Hz, 3H).

**Example 22: Synthesis of compound 22**

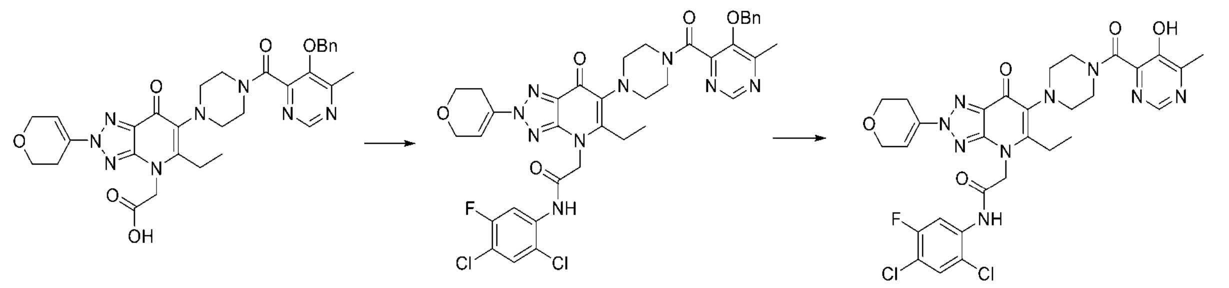

4-11 22-1 22

**Step 1: Synthesis of compound 22-1**

Under a nitrogen atmosphere, compound 4-11 (120 mg, 195.2 μmol), 2,4-dichloro-5-fluoroaniline (52.7 mg, 292.9 μmol), and pyridine (77.2 mg, 976.2 μmol) were dissolved in dichloromethane (3 mL), followed by the addition of phosphorus oxychloride (44.9 mg, 292.9 μmol). The resulting mixture was stirred and reacted at 25°C for 1 h until the reaction was complete. The reaction mixture was quenched with water (3 mL) and extracted with dichloromethane (2 × 5 mL). The organic phase was dried over anhydrous sodium sulfate and subjected to rotary evaporation under reduced pressure. The resulting crude product was separated by silica gel column chromatography (dichloromethane:methanol = 19:1) to obtain compound 22-1 (117 mg) with a yield of 87%. LC-MS (ESI) $[M+H]^+$: 776.4.

**Step 2: Synthesis of compound 22**

Under a nitrogen atmosphere, compound 22-1 (117 mg, 150.7 μmol) was dissolved in trifluoroacetic acid (2.5 mL). The reaction was stirred at 90°C for 0.5 h until the reaction was complete. After concentration under reduced pressure, the resulting residue was dissolved in dichloromethane (20 mL), washed with water (5 mL), and extracted. The organic phase was dried over anhydrous sodium sulfate and concentrated under reduced pressure. The resulting crude product was purified by slurrying with petroleum ether/ethyl acetate (4:1) to obtain compound 22 (51.7 mg) with a yield of 50%. LC-MS(ESI) $[M+H]^+$: 686.2.

$^1H$ NMR (400 MHz, DMSO-$d_6$) δ 10.28 (s, 1H), 8.58 (s, 1H), 8.00-7.90 (m, 2H), 6.82-6.70 (m, 1H), 5.28 (s, 2H), 4.51 (d, *J* = 12.0 Hz, 1H), 4.39-4.29 (m, 2H), 3.95-3.86 (m, 2H), 3.80-3.64 (m, 2H), 3.54-3.45 (m, 1H), 3.28-3.16 (m, 1H), 3.09-2.88 (m, 3H), 2.86-2.73 (m, 3H), 2.66-2.55(m,1H), 2.44 (s, 3H), 1.19 (t, *J* = 8.0 Hz, 3H).

**Example 23: Synthesis of compound 23**

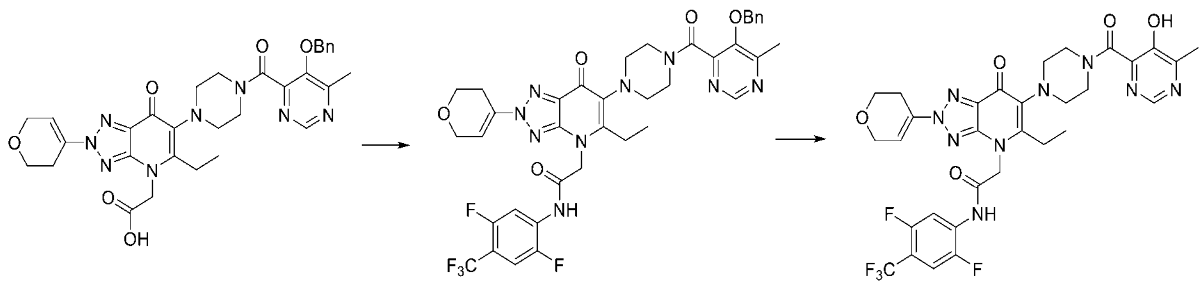

4-11 23-1 23

**Step 1: Synthesis of compound 23-1**

Under a nitrogen atmosphere, compound 4-11 (100 mg, 161.1 μmol), 4-amino-2,5-difluorobenzotrifluoride (34.9 mg, 177.2 μmol), and pyridine (63.7 mg, 805.3 μmol) were dissolved in dichloromethane (6 mL), followed by the addition of a solution of phosphorus oxychloride (27.2 mg, 177.2 μmol) in dichloromethane (0.2 mL). The resulting mixture was stirred and reacted at 25°C for 0.5 h until the reaction was complete. The reaction mixture was quenched with water (3 mL) and extracted with dichloromethane (2 × 5 mL). The organic phase was dried over anhydrous sodium sulfate and subjected to rotary evaporation under reduced pressure. The resulting residue was separated by silica gel column chromatography (dichloromethane:methanol = 93:7) to obtain compound 23-1 (120 mg) with a yield of 89%. LC-MS (ESI) $[M+H]^+$: 794.4.

**Step 2: Synthesis of compound 23**

Under a nitrogen atmosphere, compound 23-1 (110 mg, 119.7 μmol) was dissolved in trifluoroacetic acid (5 mL). The reaction was stirred at 90°C for 0.5 h until the reaction was complete. After concentration under reduced pressure, water (10 mL) was added, and the mixture was extracted with dichloromethane (20 mL × 2). The organic phase was dried over anhydrous sodium sulfate and concentrated under reduced pressure. The resulting crude product was purified by silica gel column chromatography (dichloromethane:methanol = 90:10) to obtain compound 23 (63.8 mg) with a yield of 75%. LC-MS(ESI) $[M+H]^+$ : 704.3.

$^1H$ NMR (400 MHz, DMSO-$d_6$) δ 10.92 (s, 1H), 10.23 (s, 1H), 8.57 (s, 1H), 8.20 (dd, *J* = 12.6, 6.1 Hz, 1H), 7.88 (dd, *J* = 10.6, 6.6 Hz, 1H), 6.73 (p, *J* = 1.7 Hz, 1H), 5.30 (s, 2H), 4.51 (d, *J* = 12.4 Hz, 1H), 4.31 (q, *J*= 2.9 Hz, 2H), 3.89 (t, *J* = 5.5 Hz, 2H), 3.76 - 3.64 (m, 2H), 3.49 (d, *J* = 12.6 Hz, 1H), 3.22 (t, *J* = 11.8 Hz, 1H), 2.97 (d, *J* = 9.6 Hz, 3H), 2.76 (d, *J* = 19.9 Hz, 3H), 2.58 (d, *J* = 11.2 Hz, 1H), 2.44 (s, 3H), 1.17 (t, *J* = 7.5 Hz, 3H).

**Comparative Example**

Example 42 of WO2022249060A1 was used as the reference compound HRO761, and the compound was prepared according to the method described in this patent.

**Experimental Example 1: WRN Helicase Activity Assay**

1. Experimental instruments

The information regarding the instruments used in this experimental example is shown in Table 1.

Table 1

| Instrument name | Instrument manufacturer | Model |
|---|---|---|
| Centrifuge | Eppendorf | 5810R |
| Microplate reader | PerkinElmer | EnVision-2015 |

2. Experimental materials

The WRN enzyme used in the experiment has a His-TEV tag at the N-terminus and is expressed in eukaryotic cells with a purity of 90%. The two single-stranded DNAs used for assay are labeled with BHQ2 and Cy5, respectively. When two fluorophores are in close proximity (when the DNA is in a double-stranded state), no fluorescence signal is detected due to the quenching effect. WRN has helicase activity, which enables the unwinding of double-stranded DNA to form single strands, thereby generating a fluorescence signal. The information regarding other required reagents and consumables for the experiment is shown in Table 2.

Table 2

| Reagent | Brand | Cat. No. |
|---|---|---|
| Bicine | Sigma | B8660 |
| KCl | Sigma | P9541 |
| $MgCl_2$ | Sigma | M1028 |
| F-127 | Sigma | P2443 |
| TCEP | Sigma | 646547 |
| BSG | Sigma | G9391 |
| Tris | BBI Life Sciences | A600194-0500 |
| DTT | Sigma | V900830-5G |
| NaCl | BBI Life Sciences | A610476-001 |
| DNA | GenScript | NA |
| ATP | Sigma | A7699 |
| 96-well V-bottom plate | Beyotime | FPTO 19 |
| 384-well plate | Greiner | 781209 |

3. Experimental methods

Two labeled single-stranded DNA strands were annealed to form double-stranded DNA. Annealing buffer: 12 mM Tris (pH 8.0), 300 mM NaCl, 12 mM $MgCl_2$, 2 mM DTT. Annealing program: 95°C, 5 min.

2× WRN enzyme (2 nM) and 2× substrate (100 nM double-stranded DNA, 1000 nM capture DNA, 100 μM ATP) were prepared using buffer (20 mM Bicine, 10 mM KCl, 1 mM $MgCl_2$, 0.005% BSG, 1 mM TCEP, 0.1% F-127, pH 7.5). The test compound was dissolved in DMSO to 10 mM and was gradient-diluted using a 96-well V-bottom plate. 0.5 μL of compound was added to 25 μL of 2× WRN enzyme and incubated at room temperature for 30 min. 25 μL of 2× substrate was added, and the reaction was carried out at room temperature for 30 min. Detection was performed using a microplate reader with an excitation wavelength of 620 nm and an emission wavelength of 685 nm.

4. Data analysis

The concentration-effect curve was fitted using GraphPad Prism 8 software, and the compound concentration for 50% inhibition ($IC_{50}$) was calculated. The percentage inhibition for each compound concentration was first calculated, and then the concentration-effect curve was fitted using the "log(inhibitor) vs. normalized response -- Variable slope" equation

in GraphPad Prism 8 software to obtain the $IC_{50}$.

Inhibition rate (%) = (average fluorescence intensity of positive control wells - fluorescence intensity of compound wells) / (average fluorescence intensity of positive control wells - average fluorescence intensity of negative control wells) × 100

Positive control: 25 μL 2× WRN enzyme + 0.5 μL DMSO + 25 μL 2× substrate
Negative control: 25 μL 2× buffer + 0.5 μL DMSO + 25 μL 2× substrate

The experimental results are shown in Table 3.

Table 3

| Example | WRN helicase activity ($IC_{50}$ nM) |
|---|---|
| Example 4 | 60.92 |
| Example 15 | 70.15 |
| Example 22 | 72.12 |

The above assay indicates that the compounds of the present disclosure exhibit favorable WRN helicase activity and possess the potential to be further developed as WRN helicase inhibitors.

**Experimental Example 2 WRN Hydrolase Activity Assay**

1. Experimental instruments

The information regarding the instruments used in this experimental example is shown in Table 4.

Table 4

| Instrument name | Instrument manufacturer | Model |
|---|---|---|
| Centrifuge | Eppendorf | 5810R |
| Microplate reader | PerkinElmer | EnVision-2015 |

2. Experimental materials

The WRN enzyme used in the experiment has a His-TEV tag at the N-terminus and is expressed in eukaryotic cells with a purity of 90%. The assay kit (ADP-Glo™ Kinase Assay) was purchased from Promega (Cat. No. V9101), aliquoted, and then stored in the refrigerator at -40°C. This kit could quantitatively detect the amount of ADP generated in the reaction. WRN hydrolyzed ATP to produce ADP. The ADP-Glo reagent was added to deplete excess ATP in the reaction system, followed by the addition of Kinase Detection Reagent to convert the ADP produced by the reaction into ATP and produce chemiluminescence. The enzyme activity of WRN can be reflected by detecting the chemiluminescence signal using a microplate reader. The information regarding other required reagents and consumables for the experiment is shown in Table 5.

Table 5

| Reagent | Brand | Cat. No. |
|---|---|---|
| Bicine | Sigma | B8660 |
| KCl | Sigma | P9541 |
| $MgCl_2$ | Sigma | M1028 |
| Tween 20 | Thermo Fisher | 28320 |
| Tris | BBI Life Sciences | A600194-0500 |

(continued)

| Reagent | Brand | Cat. No. |
|---|---|---|
| NaCl | BBI Life Sciences | A610476-001 |
| TCEP | Sigma | 646547 |
| BSG | Sigma | G9391 |
| DNA | GenScript | NA |
| ATP | Sigma | A7699 |
| 96-well V-bottom plate | Beyotime | FPT019 |
| 384 Well Plate | PE | 6008280 |

3. Experimental methods

Single-stranded DNA was annealed to form double-stranded DNA. 5× Annealing Buffer: 50 mM Tris pH 8.0, 100 mM NaCl. Annealing program: 95°C, 5 min.

2× WRN enzyme (1 nM) and 2× substrate (1 nM double-stranded DNA, 100 μM ATP) were prepared using a buffer (20 mM Bicine (pH 7.5), 10 mM KCl, 10 mM $MgCl_2$, 0.005% BSG, 0.002% Tween 20, 1 mM TCEP). The test compound was dissolved in DMSO to 10 mM and was gradient-diluted using a 96-well V-bottom plate. First, 25 μL of 2× WRN enzyme and 0.5 μL of compound were added to a 96-well plate and incubated at room temperature for 30 min. Then, 25 μL of 2× substrate was added, and the reaction was carried out at room temperature for 60 min. 5 μL was taken from the reaction plate and transferred to a 384-well plate for detection. 5 μL of ADP-Glo reagent was added and incubated at room temperature for 60 min, followed by the addition of 10 μL of Kinase Detection Reagent and incubation at room temperature for 40 min. The chemiluminescence signal was detected using a microplate reader.

4. Data analysis

The concentration-effect curve was fitted using GraphPad Prism 8 software, and the compound concentration for 50% inhibition ($IC_{50}$) was calculated. The percentage inhibition for each compound concentration was first calculated, and then the concentration-effect curve was fitted using the "log(inhibitor) vs. normalized response -- Variable slope" equation in GraphPad Prism 8 software to obtain the $IC_{50}$.

Inhibition rate (%) = (average luminescence intensity of positive control wells - luminescence intensity of compound wells) / (average luminescence intensity of positive control wells - average luminescence intensity of negative control wells) × 100

Positive control: 25 μL 2× WRN enzyme + 0.5 μL DMSO + 25 μL 2× substrate
Negative control: 25 μL 2× buffer + 0.5 μL DMSO + 25 μL 2× substrate

The experimental results are shown in Table 6.

Table 6

| Example | WRN helicase activity ($IC_{50}$ nM) |
|---|---|
| Example 4 | 88.53 |
| Example 5 | 79.07 |
| Example 6 | 134.5 |
| Example 7 | 86.94 |
| Example 8 | 118 |
| Example 9 | 94.9 |
| Example 10 | 237.8 |
| Example 11 | 65.27 |

(continued)

| Example | WRN helicase activity ($IC_{50}$ nM) |
|---|---|
| Example 12 | 85.98 |
| Example 13 | 104 |
| Example 14 | 30.8 |
| Example 15 | 34.29 |
| Example 16 | 42.59 |
| Example 17 | 34.66 |
| Example 18 | 64.26 |
| Example 19 | 44.29 |
| Example 20 | 35.5 |
| Example 21 | 24.39 |
| Example 22 | 46.77 |
| Example 23 | 41.94 |

The above test results indicate that the compounds of the present disclosure exhibit excellent WRN hydrolase activity.

**Experimental Example 3 Cell Proliferation Assay**

1. Experimental instruments

The information regarding the instruments used in this experimental example is shown in Table 7.

Table 7

| Instrument name | Instrument manufacturer | Model |
|---|---|---|
| Biological safety cabinet | Thermo | 1300 SERIES A2 |
| $CO_2$ Incubator | Thermo | 371 |
| Cell counter | Beckman CoμLter | Vi-CELL XR |
| Centrifuge | Eppendorf | 5810R |
| Microplate reader | MolecμLar Devices | SpectraMax i3x |

2. Experimental materials

The information regarding other required reagents and consumables for the experiment is shown in Table 8.

Table 8

| Reagent | Brand | Cat. No. |
|---|---|---|
| Celltiter Glo assay kit | Promega | G7572 |
| Leibovitz's L-15 MediuM | Gibco | 11415-064 |
| F12K MediuM | Gibco | 21127-022 |
| McCoy5A MediuM | Gibco | 16600-082 |
| DMSO | Sangon Biotech | A503039-0250 |
| PBS | Gibco | 10010-023 |
| FBS | Gibco | 10091-148 |

(continued)

| Reagent | Brand | Cat. No. |
|---|---|---|
| 0.25% Trypsin-EDTA | Gibco | 25200-056 |
| PenStrep | Gibco | 15140-122 |
| 96-well plate | Beyotime | FCP965 |

3. Experimental methods

SW48 cells were plated at a density of 1,500 cells/well, and LoVo, HCT116, and SW620 cells were plated at a density of 800 cells/well in a 96-well cell culture plate, and cultured in a 37°C incubator overnight. The test compounds, which were gradient-diluted with DMSO, were added, and after incubation for 5 days, cell viability was detected using a CellTiter-Glo kit. The CellTiter-Glo reagent was equilibrated to room temperature, and an appropriate amount was added to the cell plate, followed by shaking at room temperature for 12 min. The luminescence signal was detected using a microplate reader.

4. Data analysis

The luminescence signal values of the DMSO-treated wells and cell-free wells were used as negative and positive controls, respectively, to calculate the inhibition rate of compound on cell proliferation. The concentration-inhibition curve was fitted using the "log(inhibitor) vs. normalized response -- Variable slope" equation in GraphPad Prism 8 software to obtain the $IC_{50}$.

Inhibition rate (%) = [1 - (luminescence intensity of compound wells - average luminescence intensity of positive control wells) / (average luminescence intensity of negative control wells - average luminescence intensity of positive control wells)] $\times$ 100

The experimental results are shown in Table 9.

Table 9

| Example | Cell proliferation | | | |
|---|---|---|---|---|
| | HCT-116 ($IC_{50}$ nM) | SW-48 ($IC_{50}$ nM) | LoVo ($IC_{50}$ nM) | SW-620 ($IC_{50}$ nM) |
| Example 4 | 48.01 | 37.21 | 105.2 | >25000 |
| Example 5 | 1585 | 942.7 | 2709 | >25000 |
| Example 6 | ND | 324.5 | ND | >25000 |
| Example 7 | 289.4 | 211.5 | 735.5 | >25000 |
| Example 8 | ND | 133 | ND | >25000 |
| Example 10 | 552 | 330.9 | 871.1 | >25000 |
| Example 12 | ND | 316.6 | ND | >25000 |
| Example 13 | 434.7 | ND | ND | >25000 |
| Example 14 | ND | 42.44 | ND | >25000 |
| Example 15 | ND | 35.36 | ND | >25000 |
| Example 16 | ND | 148.5 | ND | >25000 |
| Example 17 | ND | 126.1 | ND | >25000 |
| Example 18 | ND | 51.1 | ND | >25000 |
| Example 19 | ND | 60.91 | ND | 20700 |
| Example 20 | ND | 33.82 | ND | 23679 |
| Example 22 | ND | 70.72 | ND | >25000 |

(continued)

| Example | Cell proliferation | | | |
|---|---|---|---|---|
| | HCT-116 ($IC_{50}$ nM) | SW-48 ($IC_{50}$ nM) | LoVo ($IC_{50}$ nM) | SW-620 ($IC_{50}$ nM) |
| Example 23 | ND | 102.5 | ND | >25000 |
| HRO761 | 142.9 | 144.2 | 220.5 | >25000 |

ND indicates not detected.

The above test results indicate that the compounds of the present disclosure have an inhibitory effect on the proliferation of MSI-H colorectal cancer cell lines SW48 and HCT116 cells, but no inhibitory effect on MSS SW620 cells.

**Experimental Example 4 γH2AX Induction Assay**

1. Experimental instruments

The information regarding the instruments used in this experimental example is shown in Table 10.

Table 10

| Instrument name | Instrument manufacturer | Model |
|---|---|---|
| Biological safety cabinet | Thermo | 1300 SERIES A2 |
| $CO_2$ Incubator | Thermo | 371 |
| Cell counter | Beckman CoμLter | Vi-CELL XR |
| Centrifuge | Eppendorf | 5810R |
| Shaker | Thermo | 13687716 |
| Microplate reader | PerkinElmer | EnVision-2015 |

2. Experimental materials

The information regarding other required reagents and consumables for the experiment is shown in Table 11.

Table 11

| Reagent | Brand | Cat. No. |
|---|---|---|
| Celltiter Glo assay kit | Promega | G7572 |
| HTRF Phospho-H2AX (SER139) Detection Kits | PerkinElmer | 64H2XPEH |
| Leibovitz's L-15 MediuM | Gibco | 11415-064 |
| McCoy5A MediuM | Gibco | 16600-082 |
| DMSO | Sangon Biotech | A503039-0250 |
| PBS | Gibco | 10010-023 |
| FBS | Gibco | 10091-148 |
| 0.25% Trypsin-EDTA | Gibco | 25200-056 |
| PenStrep | Gibco | 15140-122 |
| 96-well plate | Beyotime | FCP965 |
| 384-well plate | PerkinElmer | 6007299 |

3. Experimental methods:

SW48, HCT116, and SW620 cells were plated into 96-well cell culture plates at a density of 15,000-25,000

cells/well and cultured overnight in a 37°C incubator. The test compounds, which were gradient-diluted with DMSO, were added and incubated for 2-3 days. The γ-H2AX level was detected using HTRF Phospho-H2AX (SER139) Detection Kits. The 4× Lysis buffer and Detection buffer were equilibrated to room temperature for later use. The 4× Lysis buffer was diluted to 1× Lysis buffer using $ddH_2O$. After the medium was aspirated, 50 μL of 1× Lysis buffer was added to each well and shaken at room temperature for 30 minutes for lysis. 16 μL of lysate was transferred to a 384-well plate, followed by the addition of 4 μL of diluted antibody mixture (Phospho-H2AX d2 antibody and Phospho-H2AX Eu Cryptate antibody were each diluted 20-fold using Detection buffer, and the two antibodies were then mixed uniformly at a 1:1 volume ratio). The mixture was incubated at room temperature for 2-24 h, and fluorescence signals at 655 nm and 615 nm were detected using a microplate reader. Parallel treatment plates were simultaneously set up in the experiment, and cell viability was detected using the CellTiter-Glo kit. The CellTiter-Glo reagent was equilibrated to room temperature, and an appropriate amount was added to the cell plate, followed by shaking at room temperature for 12 min. The luminescence signal was detected using a microplate reader.

The fluorescence signal values of DMSO-treated wells and positive drug-treated wells were used as negative and positive controls, respectively, to calculate the level of γ-H2AX induced by the compounds. The concentration-induction rate curve was fitted using the "log(agonist) vs. response -- Variable slope (four parameters)" equation in GraphPad Prism 8 software to obtain the $EC_{50}$.

Cell viability (%) = luminescence intensity of compound wells / average luminescence intensity of negative control wells × 100

HTRF Ratio = fluorescence signal value at 665 nm / fluorescence signal value at 615 nm × $10^4$

The experimental results are shown in Table 12.

Table 12

| Example | γ-H2AX induction | | |
|---|---|---|---|
| | HCT-116 ($EC_{50}$ nM) | SW-48 ($EC_{50}$ nM) | SW-620 ($EC_{50}$ nM) |
| Example 4 | 127.6 | 53.78 | >10000 |
| Example 15 | 97.1 | 62.88 | >10000 |
| Example 22 | 168.2 | 127.8 | >10000 |
| HRO761 | 640.5 | 324.1 | >10000 |

The above test results indicate that treatment with the compounds of the present disclosure can significantly induce DNA damage accumulation (increased γ-H2AX levels) in MSI-H colorectal cancer cell lines SW48 and HCT116, but has no induction effect on MSS SW620 cells.

**Experimental Example 5 Pharmacokinetic Test in Mice**

1. Experimental purpose

CD-1 mice were taken as test animals. Example 4, Example 22, and Comparative Example HRO761 were given by gavage. The drug concentrations in mouse plasma at different time points were determined by LC-MS/MS to investigate the pharmacokinetic characteristics of the compounds of the present disclosure in mice.

2. Experimental scheme

2.1 Experimental drugs and animals

Experimental drugs: Example 4, Example 22, and Comparative Example HRO761;
Animals: CD-1 mice, male, 24-25 g, purchased from Shanghai Jihui Laboratory Animal Care Co.,Ltd.

2.2 Drug preparation

An appropriate amount of Example 4, Example 22, and Comparative Example HRO761 was weighed and sequentially added to an appropriate amount of dimethyl sulfoxide, solutol, and saline (final solvent: 5% DMSO + 10% solutol + 85% saline). The mixture was vortexed and sonicated to prepare a 1.0 mg/mL dosing solution.

2.3 Administration

Three mice in gavage groups of Example 4, Example 22, and Comparative Example HRO761 were administered by oral gavage after overnight fasting (dose: 10 mg/kg, administration volume: 10 mL/kg), and the mice were fed 4 h after administration.

3. Experimental operation

0.04 mL of blood was collected from the animals before administration and at 0.083 h, 0.25 h, 0.5 h, 1 h, 2 h, 4 h, 6 h, 8 h, and 24 h after administration, and anticoagulated with sodium heparin. Blood samples were placed on ice after collection, and plasma was separated by centrifugation (centrifugation conditions: 8000 rpm, 5 min). The collected plasma was stored at -80°C before analysis.

LC-MS/MS was used to determine the content of the test compound in mouse plasma after intragastric administration.

4. Pharmacokinetic parameter results

Based on the calculated concentrations of the compounds in plasma at different time points, the pharmacokinetic parameters of Example 4, Example 22, and Comparative Example HRO761 in mice were calculated using winnonlin software. The pharmacokinetic parameters of Example 4, Example 22, and Comparative Example HRO761 of the present disclosure are shown in Table 13.

Table 13

| Compound No. | $C_{max}$, ng/mL | $AUC_{0-i}$, ng.h/mL |
|---|---|---|
| Example 4 | 24773 | 51542 |
| Example 22 | 24612 | 83414 |
| Comparative example HRO761 | 11206 | 28343 |

The above test results indicate that the compounds of the present disclosure exhibit favorable pharmacokinetic characteristics in mice.

**Experimental Example 6 Pharmacokinetic Test in Rats**

1. Experimental purpose

SD rats were taken as test animals. Example 4, Example 15, and Comparative Example HRO761 were given by gavage. The drug concentrations in rat plasma at different time points were determined by LC-MS/MS to investigate the pharmacokinetic characteristics of Example 4, Example 15, and Comparative Example HRO761 in rats.

2. Experimental scheme

2.1 Experimental drugs and animals

Experimental drugs: Example 4, Example 15, and Comparative Example HRO761;
Animals: SD rats, male, 240-260 g, purchased from Shanghai Jihui Laboratory Animal Care Co.,Ltd.

2.2 Drug preparation

An appropriate amount of Example 4, Example 15, and Comparative Example HRO761 was weighed and added to an appropriate amount of dimethyl sulfoxide, solutol, and saline (final solvent: 5% DMSO + 10% solutol + 85% saline). The mixture was vortexed and sonicated to prepare a 1.0 mg/mL dosing solution.

2.3 Administration

The rats in gavage groups (3 rats per group) of Example 4, Example 15, and Comparative Example HRO761 were administered by oral gavage after overnight fasting (dose: 10 mg/kg, administration volume: 10 mL/kg), and the rats were fed 4 h after administration.

3. Experimental operation

0.2 mL of blood was collected from the animals before administration and at 0.083 h, 0.25 h, 0.5 h, 1 h, 2 h, 4 h, 6 h, 8 h, and 24 h after administration, and anticoagulated with sodium heparin. Blood samples were placed on ice after collection, and plasma was separated by centrifugation (centrifugation conditions: 8000 rpm, 5 min). The collected plasma was stored at -80°C before analysis.

The contents of Example 4, Example 15, and Comparative Example HRO761 in rat plasma after oral administration were determined by LC-MS/MS.

4. Pharmacokinetic parameter results

Based on the calculated concentrations of the compounds in plasma at different time points, the pharmacokinetic parameters of Example 4, Example 15, and Comparative Example HRO761 in rats were calculated using winnonlin software. The pharmacokinetic parameters of Example 4, Example 15, and Comparative Example HRO761 of the present disclosure are shown in Table 14.

Table 14

| Compound No. | $C_{max}$, ng/mL | $AUC_{0-i}$, ng.h/mL |
|---|---|---|
| Example 4 | 19180 | 170483 |
| Example 15 | 9016 | 46423 |
| Comparative example HRO761 | 6965 | 63602 |

The above test results indicate that the compounds of the present disclosure exhibit favorable pharmacokinetic characteristics in rats.

The exemplary embodiments of the present disclosure have been described above. It should be understood that the scope of protection of the present disclosure is not limited to the exemplary embodiments described above. Any modifications, equivalent replacements, improvements, etc., made by those skilled in the art within the spirit and principles of the present disclosure shall be included within the scope of protection of the present disclosure.

## Claims

**1.** A compound of formula (I), an optical isomer thereof, or a pharmaceutically acceptable salt thereof,

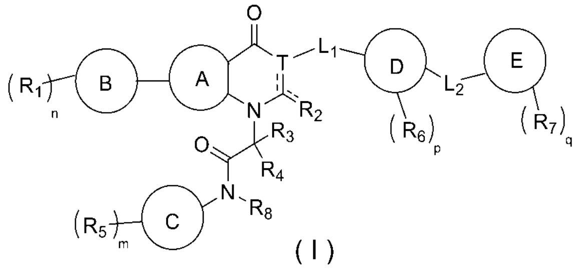

( I )

wherein

ring A is selected from the group consisting of phenyl and 5- to 10-membered heteroaryl, wherein the phenyl and 5- to 10-membered heteroaryl are optionally substituted with 1, 2, or 3 $R_a$;
ring B is selected from the group consisting of $C_{3-20}$ cycloalkyl, 3- to 20-membered heterocycloalkyl, $C_{4-20}$ cycloalkenyl, 4- to 20-membered heterocycloalkenyl, $C_{6-20}$ aryl, and 5-to 20-membered heteroaryl;
ring C is selected from the group consisting of $C_{6-20}$ aryl and 5- to 20-membered heteroaryl; ring D is selected from the group consisting of $C_{3-20}$ cycloalkyl, 3- to 20-membered heterocycloalkyl, 4- to 20-membered heterocycloalkenyl, $C_{6-20}$ aryl, and 5- to 20-membered heteroaryl;
ring E is selected from the group consisting of $C_{3-20}$ cycloalkyl, 5- to 20-membered heterocycloalkyl, $C_{6-20}$ aryl, and 5- to 20-membered heteroaryl;
$L_1$ is selected from the group consisting of a single bond, -N($R_{b1}$)-, -N($R_{b1}$)C(=O)-, -O-, - S-, -($CR_{b2}R_{b3}$)$_t$-, -C(=O)-, -C(=S)-, -S(=O)-, -S(=O)$_2$-, and

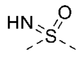;

$L_2$ is selected from the group consisting of a single bond, -N($R_{b1}$)-, -N($R_{b1}$)C(=O)-, -O-, - S-, -($CR_{b2}R_{b3}$)$_t$-, -C(=O)-, -C(=S)-, -S(=O)-, -S(=O)$_2$-, and

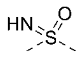;

each $R_1$ is independently selected from the group consisting of H, F, Cl, Br, OH, N($R_{b4}$)$_2$, CN, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;
alternatively, two $R_1$ are connected together to form a $C_{3-20}$ cycloalkyl, 3- to 20-membered heterocycloalkyl, $C_{6-20}$ aryl, or 5- to 20-membered heteroaryl group;

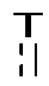 is 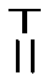, and 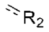 is 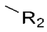;

alternatively,  is 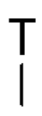, and 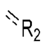 is 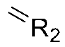;

when  is 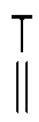, T is C;

when  is 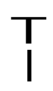, T is selected from the group consisting of N and CH;

when 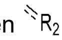 is 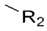, $R_2$ is selected from the group consisting of H, F, Cl, Br, OH, N($R_{b4}$)$_2$, CN, $SF_5$, CHO, COOH, $C_{1-20}$ alkyl, $C_{1-20}$ heteroalkyl, $C_{3-20}$ cycloalkyl, $C_{2-20}$ alkenyl, $C_{2-20}$ alkynyl, 3- to 20-membered heterocycloalkyl, $C_{4-20}$ cycloalkenyl, 4- to 20-membered heterocycloalkenyl, $C_{6-20}$ aryl, and 5- to 20-membered heteroaryl, wherein the $C_{1-20}$ alkyl, $C_{1-20}$ heteroalkyl, $C_{3-20}$ cycloalkyl, $C_{2-20}$ alkenyl, $C_{2-20}$ alkynyl, 3- to 20-membered heterocycloalkyl, $C_{4-20}$ cycloalkenyl, 4- to 20-membered heterocycloalkenyl, $C_{6-20}$ aryl, and 5-to 20-membered heteroaryl are optionally substituted with 1, 2, or 3 R;

when 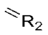 is 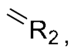, $R_2$ is selected from the group consisting of O and S;
$R_3$ and $R_4$ are each independently selected from the group consisting of H, F, Cl, Br, OH, N($R_{b4}$)$_2$, CN, $C_{1-20}$ alkyl, $C_{1-20}$ heteroalkyl, $C_{3-20}$ cycloalkyl, and 3- to 20-membered heterocycloalkyl, wherein the $C_{1-20}$ alkyl, $C_{1-20}$ heteroalkyl, $C_{3-20}$ cycloalkyl, and 3- to 20-membered heterocycloalkyl are optionally substituted with 1, 2, or 3 R;
alternatively, $R_3$ and $R_4$ are connected together to form a $C_{3-20}$ cycloalkyl or 3- to 20-membered heterocycloalkyl group, wherein the $C_{3-20}$ cycloalkyl or 3- to 20-membered heterocycloalkyl is optionally substituted with 1, 2, or 3 R;
each $R_5$ is independently selected from the group consisting of H, F, Cl, Br, OH, N($R_{b4}$)$_2$, $SF_5$, CN, CHO, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;

alternatively, two $R_5$ are connected together to form a $C_{3-20}$ cycloalkyl, 3- to 20-membered heterocycloalkyl, $C_{4-20}$ cycloalkenyl, 4- to 20-membered heterocycloalkenyl, $C_{6-20}$ aryl, or 5-to 20-membered heteroaryl group, wherein the $C_{3-20}$ cycloalkyl, 3- to 20-membered heterocycloalkyl, $C_{4-20}$ cycloalkenyl, 4- to 20-membered heterocycloalkenyl, $C_{6-20}$ aryl, and 5-to 20-membered heteroaryl are optionally substituted with 1, 2, or 3 R;
each $R_6$ is independently selected from the group consisting of H, F, Cl, Br, OH, $N(R_{b4})_2$, CN, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;
alternatively, two $R_6$ are connected together to form a $C_{3-20}$ cycloalkyl, 3- to 20-membered heterocycloalkyl, $C_{6-20}$ aryl, or 5- to 20-membered heteroaryl group, wherein the $C_{3-20}$ cycloalkyl, 3- to 20-membered heterocycloalkyl, $C_{6-20}$ aryl, and 5- to 20-membered heteroaryl are optionally substituted with 1, 2, or 3 R;
each $R_7$ is independently selected from the group consisting of H, F, Cl, Br, OH, $N(R_{b4})_2$, CN, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;
$R_8$ is selected from the group consisting of H, $C_{1-20}$ alkyl, $C_{1-20}$ heteroalkyl, $C_{3-20}$ cycloalkyl, and 3- to 20-membered heterocycloalkyl, wherein the $C_{1-20}$ alkyl, $C_{1-20}$ heteroalkyl, $C_{3-20}$ cycloalkyl, and 3- to 20-membered heterocycloalkyl are optionally substituted with 1, 2, or 3 R;
each $R_a$ is independently selected from the group consisting of H, F, Cl, Br, OH, $NH_2$, CN, $C_{1-20}$ alkyl, $C_{1-20}$ heteroalkyl, $C_{3-20}$ cycloalkyl, and 3- to 20-membered heterocycloalkyl, wherein the $C_{1-20}$ alkyl, $C_{1-20}$ heteroalkyl, $C_{3-20}$ cycloalkyl, and 3- to 20-membered heterocycloalkyl are optionally substituted with 1, 2, or 3 R;
$R_{b1}$ is selected from the group consisting of H, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;
$R_{b2}$ and $R_{b3}$ are each independently selected from the group consisting of H, F, Cl, Br, OH, $NH_2$, CN, $C_{1-20}$ alkyl, $C_{1-20}$ heteroalkyl, $C_{3-20}$ cycloalkyl, and 3- to 20-membered heterocycloalkyl, wherein the $C_{1-20}$ alkyl, $C_{1-20}$ heteroalkyl, $C_{3-20}$ cycloalkyl, and 3- to 20-membered heterocycloalkyl are optionally substituted with 1, 2, or 3 R;
alternatively, $R_{b2}$ and $R_{b3}$ are connected together to form a $C_{3-20}$ cycloalkyl or 3- to 20-membered heterocycloalkyl group;
each $R_{b4}$ is independently selected from the group consisting of H, $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R;
m, n, p, q, and t are each independently selected from the group consisting of 0, 1, 2, and 3;
each R is independently selected from the group consisting of H, F, Cl, Br, I, OH, $NH_2$, CN, $SF_5$, CHO, COOH,

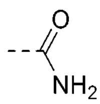,

$C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl, wherein the $C_{1-20}$ alkyl, $C_{1-20}$ heteroalkyl, $C_{3-20}$ cycloalkyl, 3- to 20-membered heterocycloalkyl, $C_{6-20}$ aryl, and 5- to 20-membered heteroaryl are optionally substituted with 1, 2, or 3 R';
R' is selected from the group consisting of H, F, Cl, Br, I, OH, $NH_2$, $CH_3$, $CF_3$, $C_2H_5$, CN, $SF_5$, CHO, COOH, and

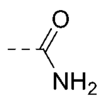;

the $C_{1-6}$ heteroalkyl, $C_{1-20}$ heteroalkyl, 3- to 20-membered heterocycloalkyl, 4- to 20-membered heterocycloalkenyl, 5- to 20-membered heteroaryl, or 5- to 10-membered heteroaryl contains 1, 2, or 3 heteroatoms or heteroatom groups independently selected from the group consisting of -O-, -NH-, -S-, -C(=O)-, -C(=O)O-, -S(=O)-, $-S(=O)_2-$, and N.

2. The compound, the optical isomer thereof, or the pharmaceutically acceptable salt thereof according to claim 1,

wherein each R is independently selected from the group consisting of H, F, Cl, Br, I, OH, $NH_2$, CN, $SF_5$, CHO, COOH,

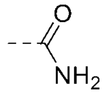,

$C_{1-6}$ alkyl, $C_{1-6}$ alkoxy, $C_{1-6}$ alkylthio, $C_{1-6}$ alkylamino, -$C_{1-6}$ alkyl-$C_{1-6}$ alkoxy, -$C_{1-6}$ alkyl-$C_{1-6}$ alkylthio, -$C_{1-6}$ alkyl-$C_{1-6}$ alkylamino, $C_{1-6}$ alkyl-OH, $C_{1-6}$ alkyl-$NH_2$, -C(=O)-$C_{1-6}$ alkyl, $C_{1-6}$ alkyl-C(=O)-$C_{1-6}$ alkyl, -NH-C(=O)-$C_{1-6}$ alkyl, $C_{1-6}$ alkyl-NH-C(=O)-$C_{1-6}$ alkyl, -NH-S(=O)$_2$-$C_{1-6}$ alkyl, $C_{1-6}$ alkyl-NH-S(=O)$_2$-$C_{1-6}$ alkyl, $C_{3-6}$ cycloalkyl, phenyl, naphthyl, pyridyl, pyrimidinyl, pyridazinyl, pyrazinyl, thienyl, pyrrolyl, pyrazolyl, imidazolyl, triazolyl, piperidinyl, piperazinyl, pyranyl, furanyl, thiazolyl, oxazolyl, and thiopyranyl,

wherein the $C_{1-6}$ alkyl, $C_{1-6}$ alkoxy, $C_{1-6}$ alkylthio, $C_{1-6}$ alkylamino, -$C_{1-6}$ alkyl-$C_{1-6}$ alkoxy, -$C_{1-6}$ alkyl-$C_{1-6}$ alkylthio, -$C_{1-6}$ alkyl-$C_{1-6}$ alkylamino, $C_{1-6}$ alkyl-OH, $C_{1-6}$ alkyl-$NH_2$, -C(=O)-$C_{1-6}$ alkyl, $C_{1-6}$ alkyl-C(=O)-$C_{1-6}$ alkyl, -NH-C(=O)-$C_{1-6}$ alkyl, $C_{1-6}$ alkyl-NH-C(=O)-$C_{1-6}$ alkyl, -NH-S(=O)$_2$-$C_{1-6}$ alkyl, $C_{1-6}$ alkyl-NH-S(=O)$_2$-$C_{1-6}$ alkyl, $C_{3-6}$ cycloalkyl, phenyl, naphthyl, pyridyl, pyrimidinyl, pyridazinyl, pyrazinyl, thienyl, pyrrolyl, pyrazolyl, imidazolyl, triazolyl, piperidinyl, piperazinyl, pyranyl, furanyl, thiazolyl, oxazolyl, and thiopyranyl are optionally substituted with 1, 2, or 3 R'.

3. The compound, the optical isomer thereof, or the pharmaceutically acceptable salt thereof according to claim 2, wherein each R is independently selected from the group consisting of H, F, Cl, Br, I, OH, $NH_2$, CN, $SF_5$, CHO, COOH,

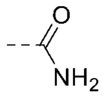,

$CH_3$, $CF_3$, $CHF_2$, $CH_2F$, $CF_2Cl$, $CF_2Br$, $CF_2I$,

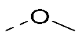, 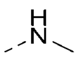, and 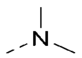.

4. The compound, the optical isomer thereof, or the pharmaceutically acceptable salt thereof according to any one of claims 1 to 3, wherein ring A is selected from the group consisting of phenyl, pyridyl, pyridazinyl, pyrimidinyl, thienyl, thiazolyl, oxazolyl, isoxazolyl, 1H-imidazolyl, 1H-pyrazolyl, and 1H-pyrrolyl, wherein the phenyl, pyridyl, pyridazinyl, pyrimidinyl, thienyl, thiazolyl, oxazolyl, isoxazolyl, 1H-imidazolyl, 1H-pyrazolyl, and 1H-pyrrolyl are optionally substituted with 1, 2, or 3 $R_a$.

5. The compound, the optical isomer thereof, or the pharmaceutically acceptable salt thereof according to claim 4, wherein ring A is selected from the group consisting of

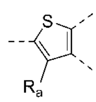,

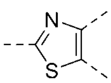, 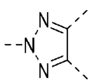, 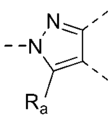, 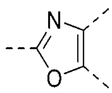, 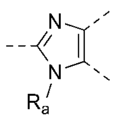, 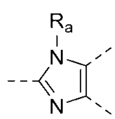, 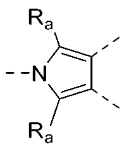, 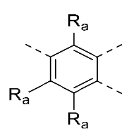, 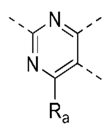,

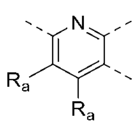, 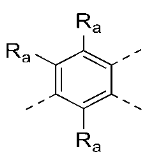, and 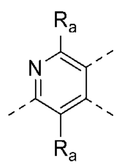.

6. The compound, the optical isomer thereof, or the pharmaceutically acceptable salt thereof according to any one of claims 1 to 3, wherein each $R_a$ is independently selected from the group consisting of H, F, Cl, Br, OH, $NH_2$, CN, Me,

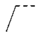, 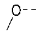, 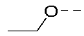, 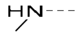, 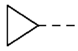,

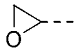, 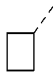, and 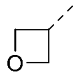,

wherein the Me,

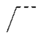, 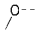, 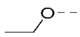, 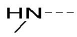, 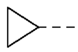, 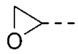, 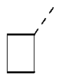,

and

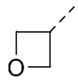

are optionally substituted with 1, 2, or 3 R.

7. The compound, the optical isomer thereof, or the pharmaceutically acceptable salt thereof according to claim 6, wherein each $R_a$ is independently selected from the group consisting of H, F, Cl, Br, OH, $NH_2$, CN, Me, $CF_3$,

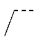, 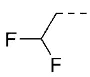, 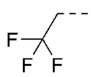, 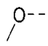, 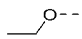, 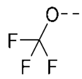,

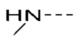, 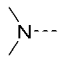, 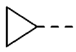, 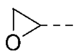, 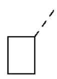, and 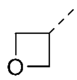.

8. The compound, the optical isomer thereof, or the pharmaceutically acceptable salt thereof according to claim 5 or 7, wherein ring A is selected from the group consisting of

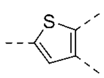, 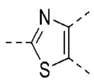, 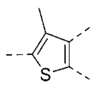, 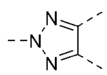, 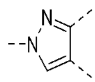, 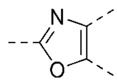, 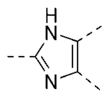, 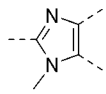, 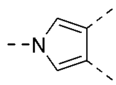,

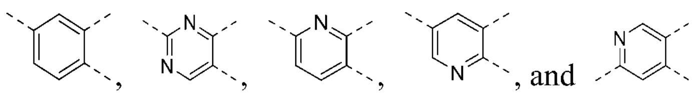

, , , , and .

9. The compound, the optical isomer thereof, or the pharmaceutically acceptable salt thereof according to any one of claims 1 to 3, wherein when 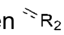 is 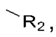, $R_2$ is selected from the group consisting of H, F, Cl, Br, OH, $NH_2$, CN, $SF_5$, CHO, COOH, $C_{1-6}$ alkyl, $C_{1-6}$ alkoxy, $C_{1-6}$ alkylthio, $C_{1-6}$ alkylamino, $C_{2-6}$ alkenyl, $C_{2-6}$ alkynyl, $C_{3-6}$ cycloalkyl, 3- to 6-membered heterocycloalkyl, $C_{4-6}$ cycloalkenyl, 4- to 6-membered heterocycloalkenyl, $C_{6-10}$ aryl, and 5- to 10-membered heteroaryl, wherein the $C_{1-6}$ alkyl, $C_{1-6}$ alkoxy, $C_{1-6}$ alkylthio, $C_{1-6}$ alkylamino, $C_{2-6}$ alkenyl, $C_{2-6}$ alkynyl, $C_{3-6}$ cycloalkyl, 3- to 6-membered heterocycloalkyl, $C_{4-6}$ cycloalkenyl, 4- to 6-membered heterocycloalkenyl, $C_{6-10}$ aryl, and 5- to 10-membered heteroaryl are optionally substituted with 1, 2, or 3 R.

10. The compound, the optical isomer thereof, or the pharmaceutically acceptable salt thereof according to claim 9,

wherein when 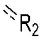 is 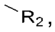, $R_2$ is selected from the group consisting of H, F, Cl, Br, OH, $NH_2$, CN, Me,

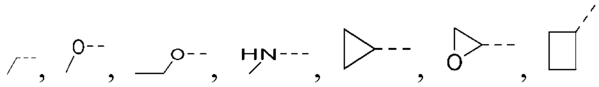, , , , , , ,

and

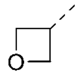,

wherein the Me,

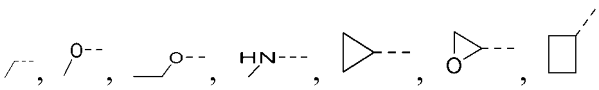, , , , , , , and 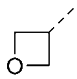

are optionally substituted with 1, 2, or 3 R.

**11.** The compound, the optical isomer thereof, or the pharmaceutically acceptable salt thereof according to claim 10, wherein $R_2$ is selected from the group consisting of H, F, Cl, Br, OH, $NH_2$, CN, Me, $CF_3$,

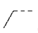, 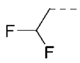, 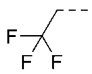, 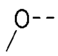, 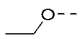, 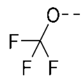, 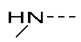, 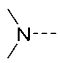, 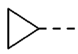,

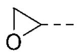, 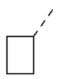, and 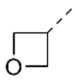.

**12.** The compound, the optical isomer thereof, or the pharmaceutically acceptable salt thereof according to claim 1 or 5, wherein the structural moiety

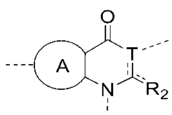

is selected from the group consisting of

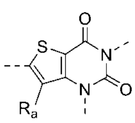, 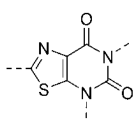, 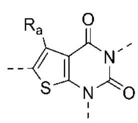, 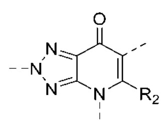, 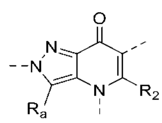,

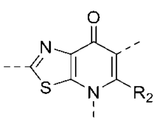, 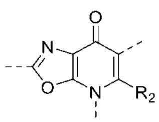, 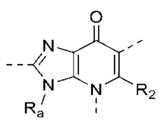, 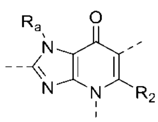, 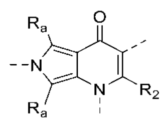, 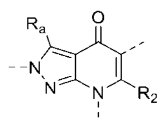,

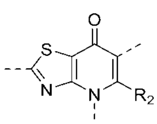, 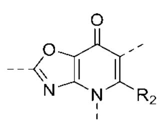, 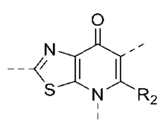, 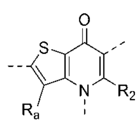, 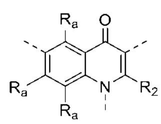, 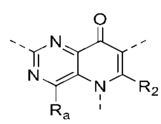,

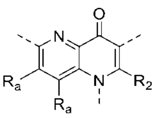, 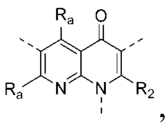, 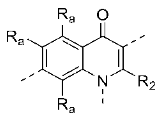, 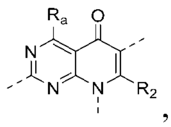, 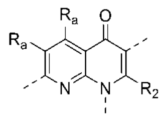, and 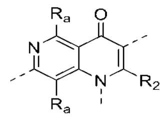.

13. The compound, the optical isomer thereof, or the pharmaceutically acceptable salt thereof according to claim 12, wherein the structural moiety

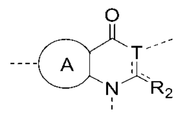

is selected from the group consisting of

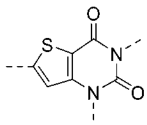, 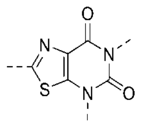, 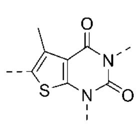, 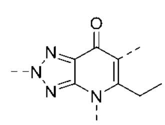, 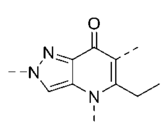,

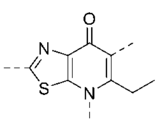, 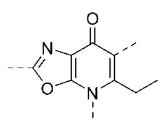, 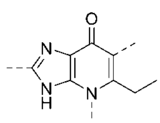, 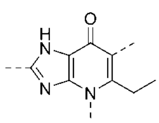, 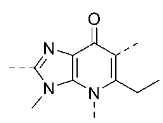, 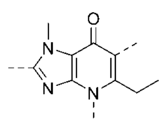,

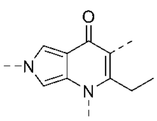, 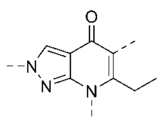, 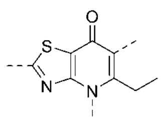, 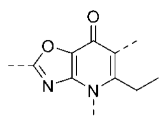, 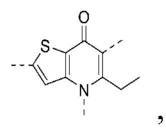, 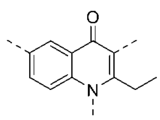,

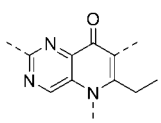, 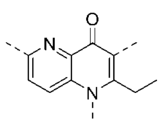, 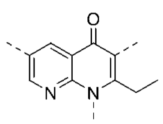, 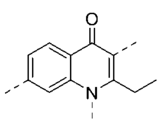, 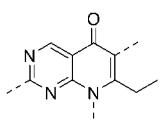, 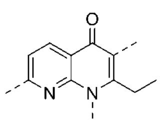,

and

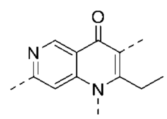.

14. The compound, the optical isomer thereof, or the pharmaceutically acceptable salt thereof according to any one of claims 1 to 3, wherein each $R_1$ is independently selected from the group consisting of H, F, Cl, Br, OH, $NH_2$, CN, $C_{1-6}$ alkyl, $C_{1-6}$ alkoxy, $C_{1-6}$ alkylthio, $C_{1-6}$ alkylamino, -$C_{1-6}$ alkyl-$C_{1-6}$ alkoxy, -$C_{1-6}$ alkyl-$C_{1-6}$ alkylthio, -$C_{1-6}$ alkyl-$C_{1-6}$ alkylamino, $C_{1-6}$ alkyl-OH, $C_{1-6}$ alkyl-$NH_2$, $C_{1-6}$ alkyl-C(=O)-, $C_{1-6}$ alkyl-C(=O)-$C_{1-6}$ alkyl, $C_{1-6}$ alkyl-O-C(=O)-, $C_{1-6}$ alkyl-O-C(=O)-$C_{1-6}$ alkyl, -NH-C(=O)-$C_{1-6}$ alkyl, $C_{1-6}$ alkyl-NH-C(=O)-$C_{1-6}$ alkyl, $C_{1-6}$ alkyl-S(=O)$_2$-, $C_{1-6}$ alkyl-S(=O)$_2$-$C_{1-6}$ alkyl, -NH-S(=O)$_2$-$C_{1-6}$ alkyl, $C_{1-6}$ alkyl-NH-S(=O)$_2$-$C_{1-6}$ alkyl, $C_{3-6}$ cycloalkyl, $C_{3-6}$ cycloalkyl-S(=O)$_2$-, and 3- to 6-membered heterocycloalkyl,
wherein the $C_{1-6}$ alkyl, $C_{1-6}$ alkoxy, $C_{1-6}$ alkylthio, $C_{1-6}$ alkylamino, -$C_{1-6}$ alkyl-$C_{1-6}$ alkoxy, -$C_{1-6}$ alkyl-$C_{1-6}$ alkylthio, -$C_{1-6}$ alkyl-$C_{1-6}$ alkylamino, $C_{1-6}$ alkyl-OH, $C_{1-6}$ alkyl-$NH_2$, $C_{1-6}$ alkyl-C(=O)-, $C_{1-6}$ alkyl-C(=O)-$C_{1-6}$ alkyl, $C_{1-6}$ alkyl-O-C(=O)-, $C_{1-6}$ alkyl-O-C(=O)-$C_{1-6}$ alkyl, -NH-C(=O)-$C_{1-6}$ alkyl, $C_{1-6}$ alkyl-NH-C(=O)-$C_{1-6}$ alkyl, $C_{1-6}$ alkyl-S(=O)$_2$-, $C_{1-6}$ alkyl-S(=O)$_2$-$C_{1-6}$ alkyl, -NH-S(=O)$_2$-$C_{1-6}$ alkyl, $C_{1-6}$ alkyl-NH-S(=O)$_2$-$C_{1-6}$ alkyl, $C_{3-6}$ cycloalkyl, $C_{3-6}$ cycloalkyl-S(=O)$_2$-, and 3- to 6-membered heterocycloalkyl are optionally substituted with 1, 2, or 3 R.

15. The compound, the optical isomer thereof, or the pharmaceutically acceptable salt thereof according to claim 14,

wherein each $R_1$ is independently selected from the group consisting of H, F, Cl, Br, OH, $NH_2$, CN, Me,

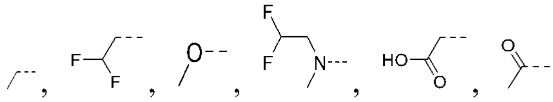, , , , , ,

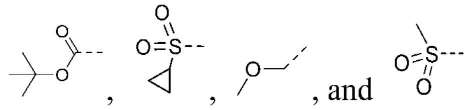, , , and .

**16.** The compound, the optical isomer thereof, or the pharmaceutically acceptable salt thereof according to claim 1, wherein ring B is selected from the group consisting of cyclohexyl, 3,6-dihydro-2H-pyranyl, 3,4-dihydro-2H-pyranyl, morpholinyl, cyclohexenyl, piperidinyl, 2,3-dihydro-1,4-dioxinyl, 6-oxa-3-azabicyclo[3.1.1]heptanyl, 1,2,3,4-tetrahydropyridinyl, 1,2,3,6-tetrahydropyridinyl, tetrahydro-2H-pyranyl, 5,6-dihydro-2H-pyran-2-keto, phenyl, pyridyl, pyrrolidinyl, 2-oxa-6-azaspiro[3.3]heptanyl, 1,1-dioxo-3,6-dihydro-2H-thiopyranyl, oxepinyl, azetidinyl, 2-oxa-7-azaspiro[4.4]nonanyl, and hexahydro-1H-furo[3,4-c]pyrrolyl.

**17.** The compound, the optical isomer thereof, or the pharmaceutically acceptable salt thereof according to claim 15 or 16, wherein the structural moiety

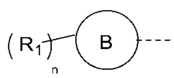

is selected from the group consisting of

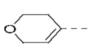, 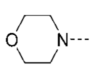, 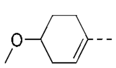, 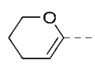, 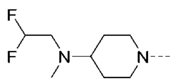, 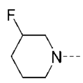,

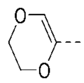, 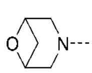, 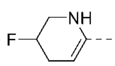, 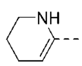, 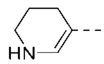, 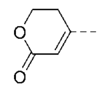, 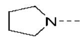, 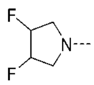, 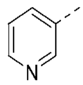, 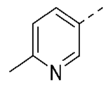,

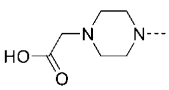, 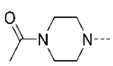, 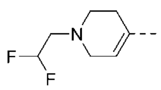, 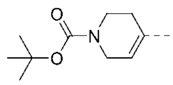, 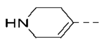, 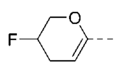, 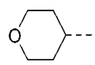, 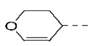,

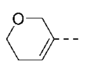, 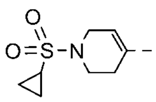, 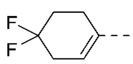, 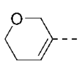, 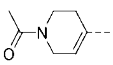, 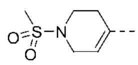, 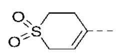, 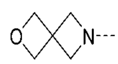,

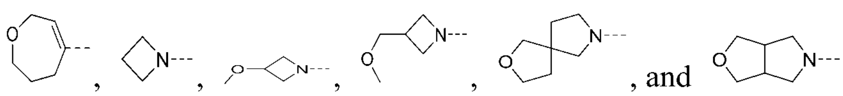, , , , , and .

**18.** The compound, the optical isomer thereof, or the pharmaceutically acceptable salt thereof according to any one of claims 1 to 3, wherein each $R_5$ is independently selected from the group consisting of H, F, Cl, Br, OH, $NH_2$, CN, $SF_5$, CHO, $C_{1-6}$ alkyl, $C_{1-6}$ alkoxy, $C_{1-6}$ alkylthio, $C_{1-6}$ alkylamino, $C_{3-6}$ cycloalkyl, and $C_{3-6}$ heterocycloalkyl, wherein the $C_{1-6}$ alkyl, $C_{1-6}$ alkoxy, $C_{1-6}$ alkylthio, $C_{1-6}$ alkylamino, $C_{3-6}$ cycloalkyl, and $C_{3-6}$ heterocycloalkyl are optionally substituted with 1, 2, or 3 R;

alternatively, two $R_5$ are connected together to form a $C_{3-6}$ cycloalkyl, 3- to 6-membered heterocycloalkyl, $C_{4-10}$ cycloalkenyl, 4- to 10-membered heterocycloalkenyl, $C_{6-10}$ aryl, or 5-to 10-membered heteroaryl group, wherein the $C_{3-6}$ cycloalkyl, 3- to 6-membered heterocycloalkyl, $C_{4-10}$ cycloalkenyl, 4- to 10-membered heterocycloalkenyl, $C_{6-10}$ aryl, and 5-to 10-membered heteroaryl are optionally substituted with 1, 2, or 3 R.

**19.** The compound, the optical isomer thereof, or the pharmaceutically acceptable salt thereof according to claim 18, wherein each $R_5$ is independently selected from the group consisting of H, F, Cl, Br, OH, $NH_2$, CN, $SF_5$, Me, CHO,

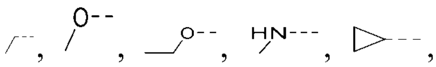

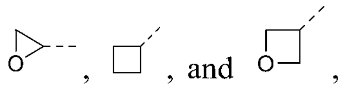

wherein the Me,

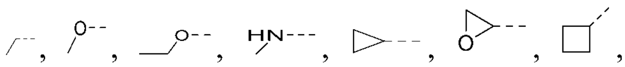

and

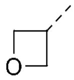

are optionally substituted with 1, 2, or 3 R;
alternatively, two $R_5$ are connected together to form

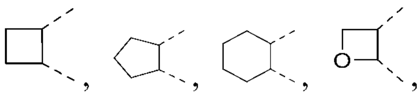

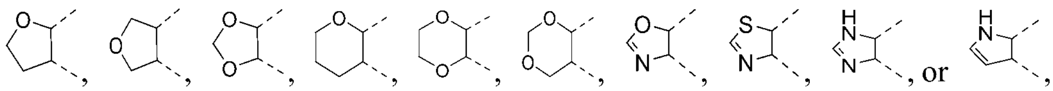

wherein the

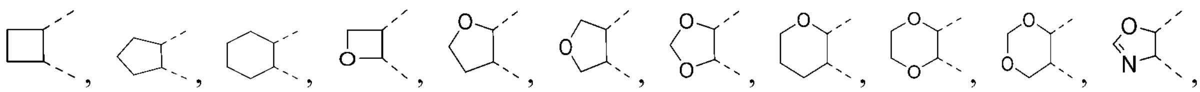

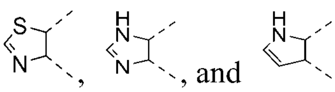

are optionally substituted with 1, 2, or 3 R.

**20.** The compound, the optical isomer thereof, or the pharmaceutically acceptable salt thereof according to claim 19, wherein each $R_5$ is independently selected from the group consisting of H, F, Cl, Br, OH, $NH_2$, $SF_5$, CN, Me, $CF_3$, CHO,

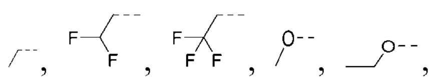

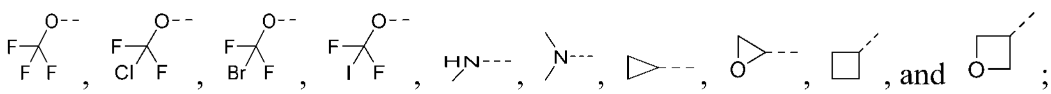

alternatively, two $R_5$ are connected together to form

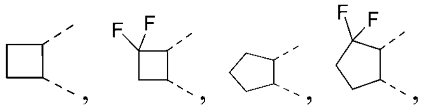

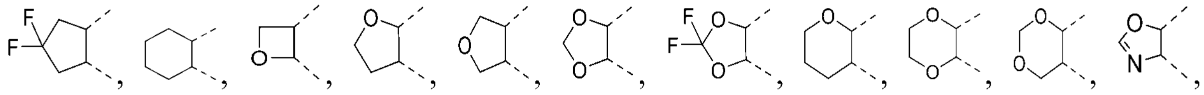

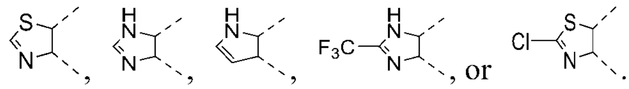

21. The compound, the optical isomer thereof, or the pharmaceutically acceptable salt thereof according to claim 1, wherein ring C is selected from the group consisting of phenyl, pyridyl, pyrimidinyl, pyridazinyl, pyrazinyl, and thienyl.

22. The compound, the optical isomer thereof, or the pharmaceutically acceptable salt thereof according to claim 20 or 21, wherein the structural moiety

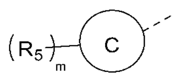

is selected from the group consisting of

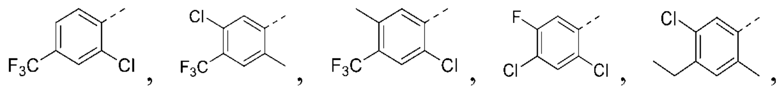

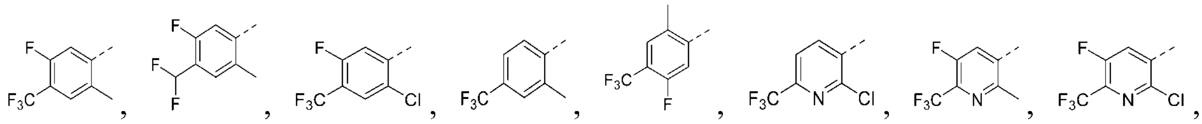

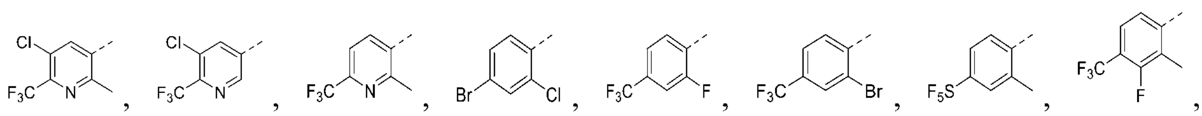

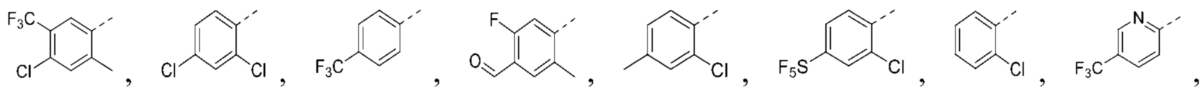

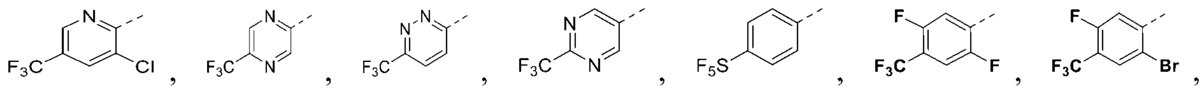

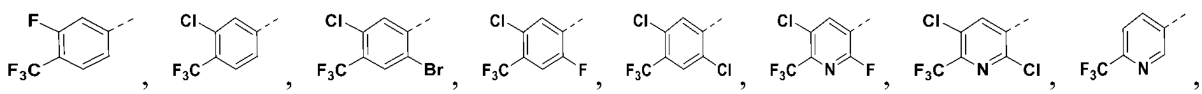

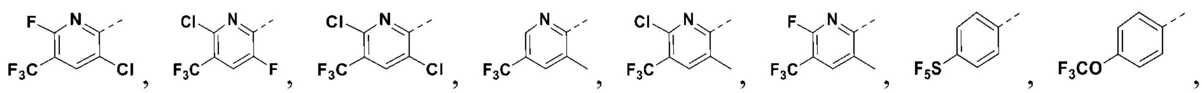

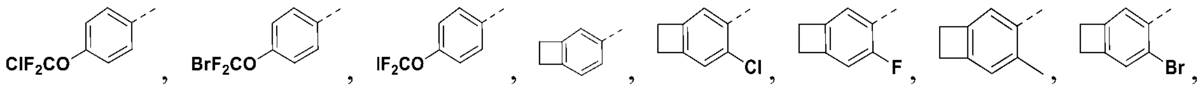,

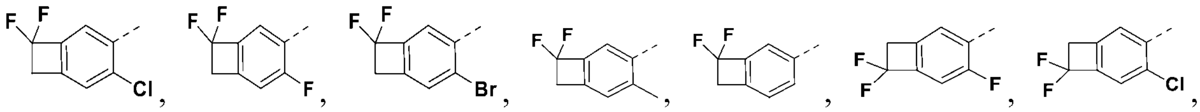,

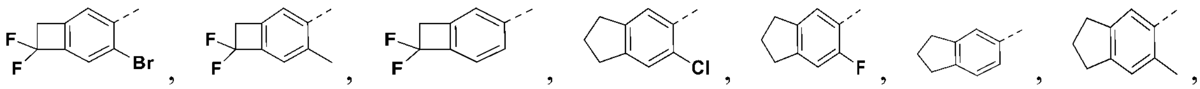,

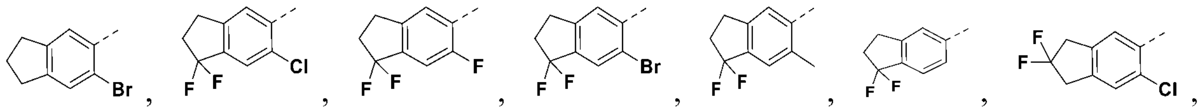,

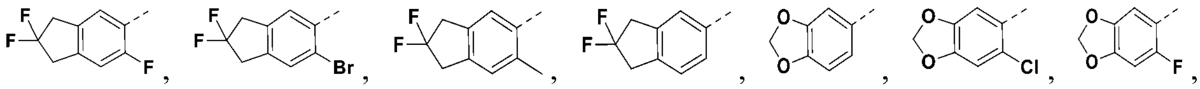,

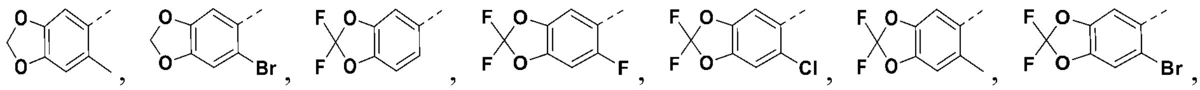,

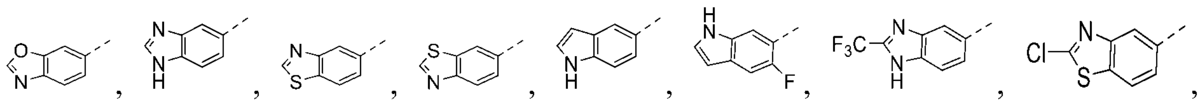,

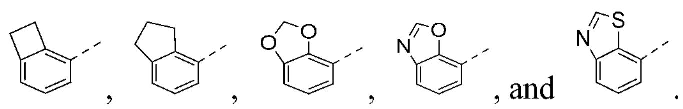, and .

**23.** The compound, the optical isomer thereof, or the pharmaceutically acceptable salt thereof according to any one of claims 1 to 3, wherein each $R_6$ is independently selected from the group consisting of H, F, Cl, Br, OH, $NH_2$, CN, Me,

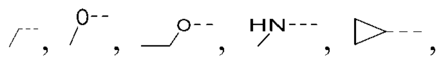,

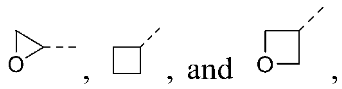, and ,

wherein the Me,

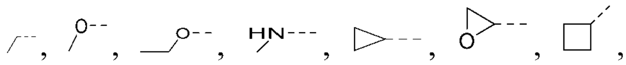,

and

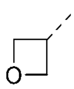

are optionally substituted with 1, 2, or 3 R.

**24.** The compound, the optical isomer thereof, or the pharmaceutically acceptable salt thereof according to claim 23, wherein each $R_6$ is independently selected from the group consisting of H, F, Cl, Br, OH, $NH_2$, CN, Me, $CF_3$,

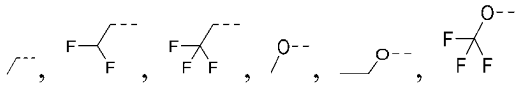 ,, , , , ,

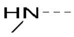, 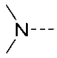, 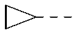, 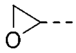, 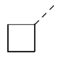, and 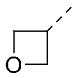.

**25.** The compound, the optical isomer thereof, or the pharmaceutically acceptable salt thereof according to claim 1, wherein ring D is selected from the group consisting of

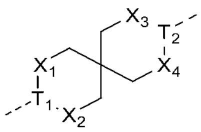, 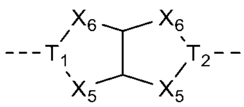, 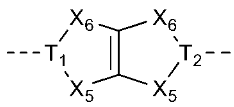, 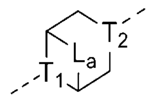, 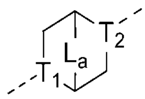,

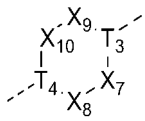, and 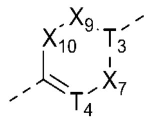;

$T_1$, $T_2$, $T_3$, and $T_4$ are each independently selected from the group consisting of N and CH;
$X_1$, $X_2$, $X_3$, and $X_4$ are each independently selected from the group consisting of a single bond and $CH_2$;
$X_5$ and $X_6$ are each independently selected from the group consisting of a single bond, $CH_2$, and $CH_2CH_2$, and $X_3$ and $X_6$ are not simultaneously a single bond;
$X_7$, $X_8$, $X_9$, and $X_{10}$ are each independently selected from the group consisting of a single bond, NH, O, S, $CH_2$, and

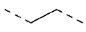,

and no more than three of $X_7$, $X_8$, $X_9$, and $X_{10}$ are single bonds simultaneously;
$L_a$ is selected from the group consisting of $C_{1-6}$ alkyl, $C_{2-6}$ alkenyl, and $C_{1-6}$ heteroalkyl, wherein the $C_{1-6}$ alkyl and $C_{1-6}$ heteroalkyl are optionally substituted with 1, 2, or 3 R.

**26.** The compound, the optical isomer thereof, or the pharmaceutically acceptable salt thereof according to claim 1, wherein ring D is selected from the group consisting of

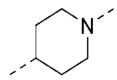,

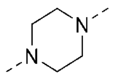, 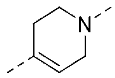, 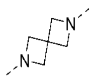, 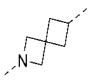, 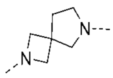, 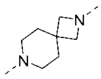, 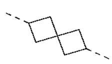, 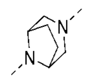, 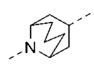,

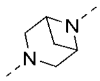, 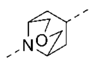, 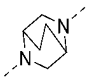, 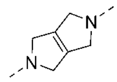, 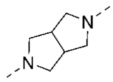, 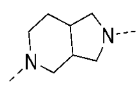, 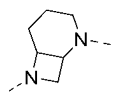, 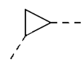, 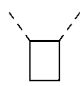,

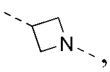, 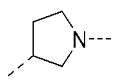, and 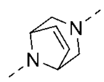.

**27.** The compound, the optical isomer thereof, or the pharmaceutically acceptable salt thereof according to claim 25 or 26, wherein the structural moiety

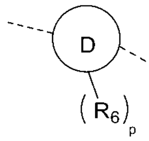

is selected from the group consisting of

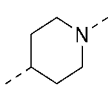, 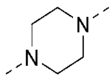, 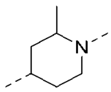, 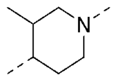, 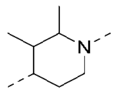, 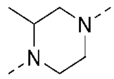,

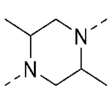, 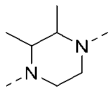, 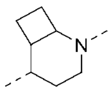, 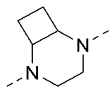, 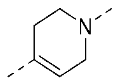, 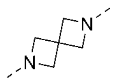, 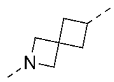, 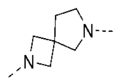,

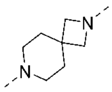, 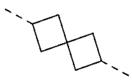, 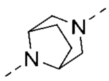, 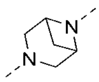, 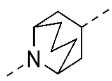, 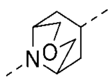, 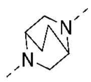, 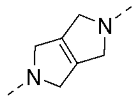,

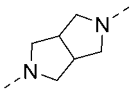, 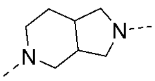, 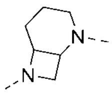, 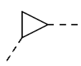, 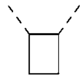, 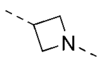, 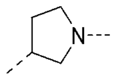, and 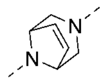.

**28.** The compound, the optical isomer thereof, or the pharmaceutically acceptable salt thereof according to any one of claims 1 to 3, wherein each $R_7$ is independently selected from the group consisting of H, F, Cl, Br, OH, $NH_2$, CN, Me,

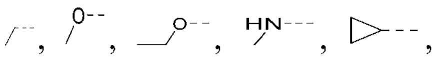 , , , , ,

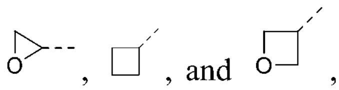 , , and ,

wherein the Me,

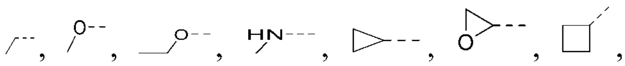 , , , , , , ,

and

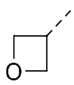

are optionally substituted with 1, 2, or 3 R.

**29.** The compound, the optical isomer thereof, or the pharmaceutically acceptable salt thereof according to claim 28, wherein each $R_7$ is independently selected from the group consisting of H, F, Cl, Br, OH, $NH_2$, CN, Me, $CF_3$,

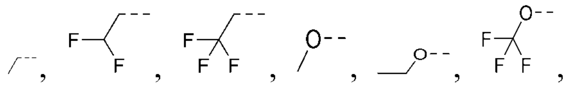

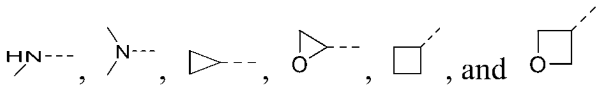

30. The compound, the optical isomer thereof, or the pharmaceutically acceptable salt thereof according to claim 1, wherein ring E is selected from the group consisting of phenyl, pyridyl, pyrimidinyl, pyridazinyl, pyrazinyl, and thienyl.

31. The compound, the optical isomer thereof, or the pharmaceutically acceptable salt thereof according to claim 29 or 30, wherein the structural moiety

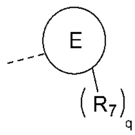

is selected from the group consisting of

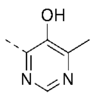, 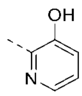, 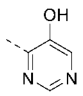, 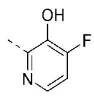, 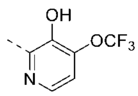, 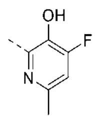, 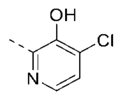,

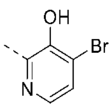, 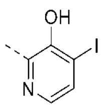, 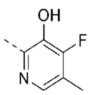, 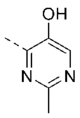, 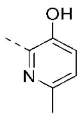, 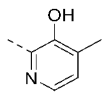, 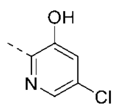, 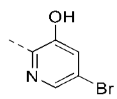, 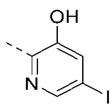,

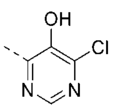, 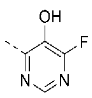, 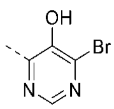, 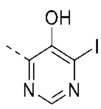, 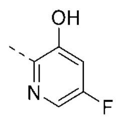, 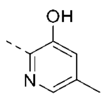, 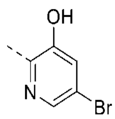, 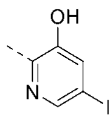, 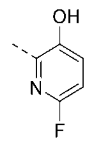,

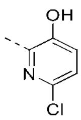, 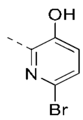, and 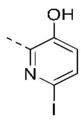.

32. The compound, the optical isomer thereof, or the pharmaceutically acceptable salt thereof according to any one of claims 1 to 3, wherein $R_3$ and $R_4$ are each independently selected from the group consisting of H, F, Cl, Br, OH, $NH_2$, CN, Me,

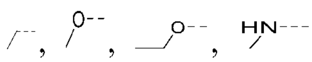

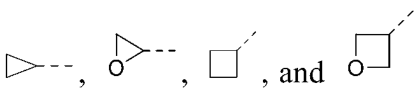

wherein the Me,

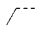, 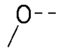, 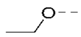, 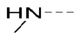, 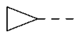, 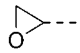,

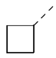, and 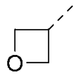

are optionally substituted with 1, 2, or 3 R.

**33.** The compound, the optical isomer thereof, or the pharmaceutically acceptable salt thereof according to claim 32, wherein $R_3$ and $R_4$ are each independently selected from the group consisting of H, F, Cl, Br, OH, $NH_2$, CN, Me, $CF_3$,

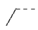, 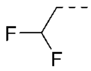, 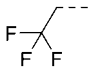, 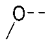, 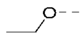,

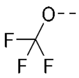, 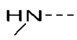, 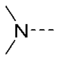, 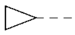, 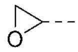, 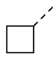, and 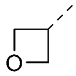.

**34.** The compound, the optical isomer thereof, or the pharmaceutically acceptable salt thereof according to any one of claims 1 to 3, wherein $R_8$ is selected from the group consisting of H, Me,

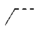, 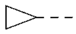, 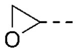, 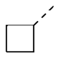, and 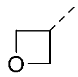,

wherein the Me,

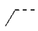, 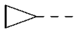, 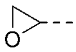, 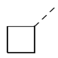,

and

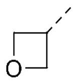

are optionally substituted with 1, 2, or 3 R.

**35.** The compound, the optical isomer thereof, or the pharmaceutically acceptable salt thereof according to claim 34, wherein $R_8$ is selected from the group consisting of H, Me, $CF_3$,

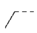, 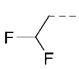, 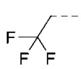, 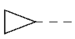, 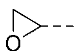, 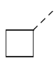, and 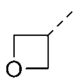.

**36.** A compound of the following formula, an optical isomer thereof, or a pharmaceutically acceptable salt thereof, selected from the group consisting of:

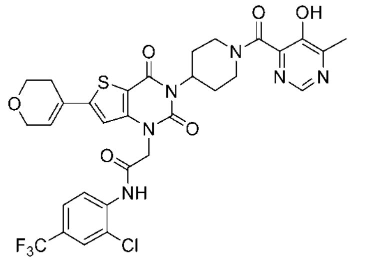
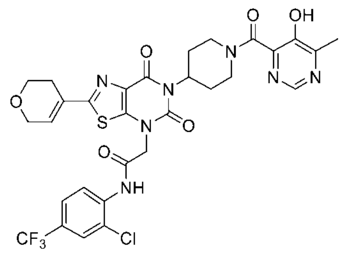
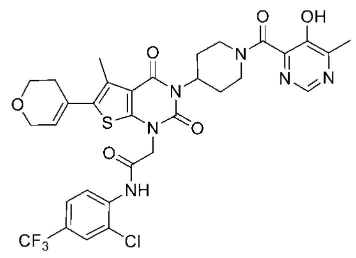

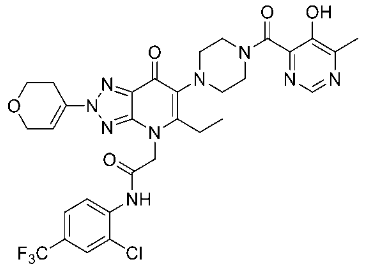
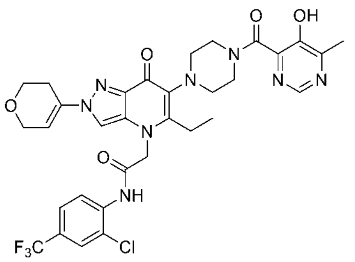
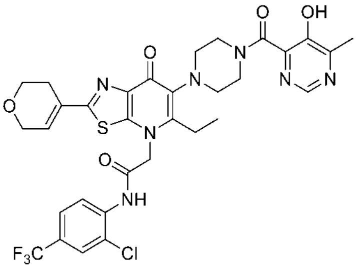

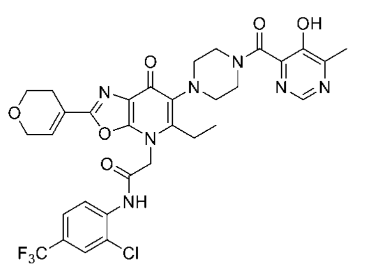
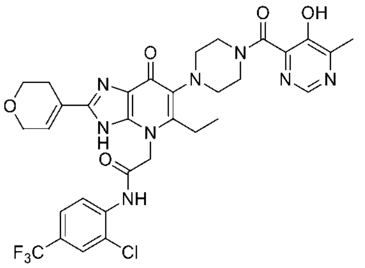
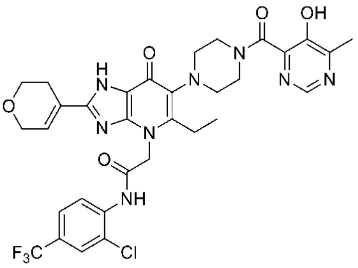

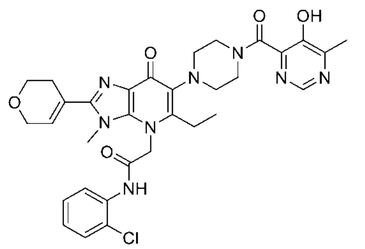
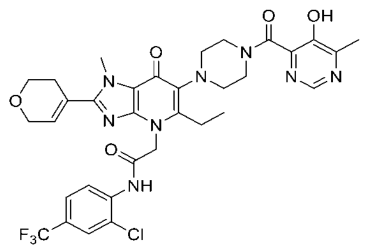
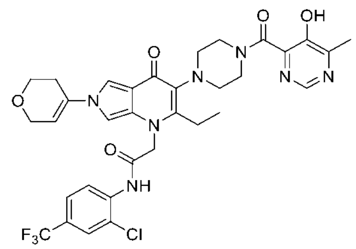

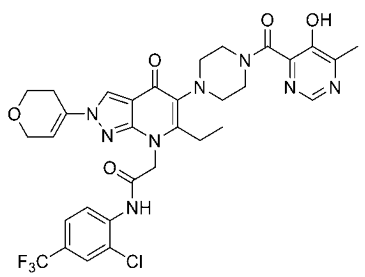
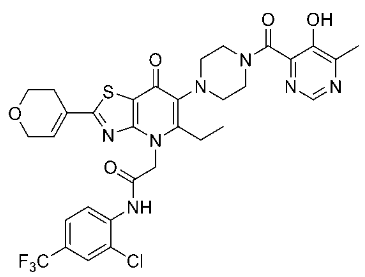
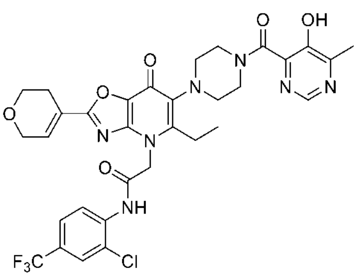

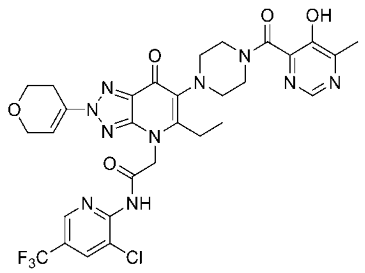
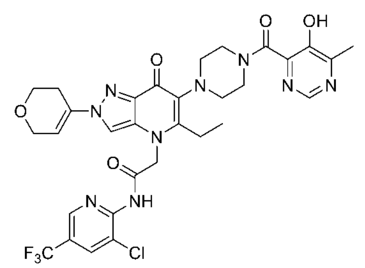
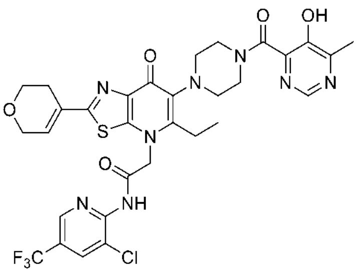

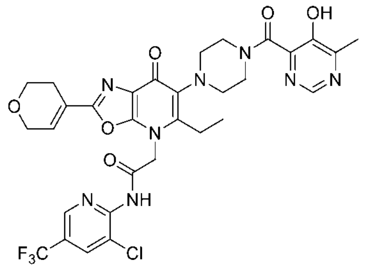
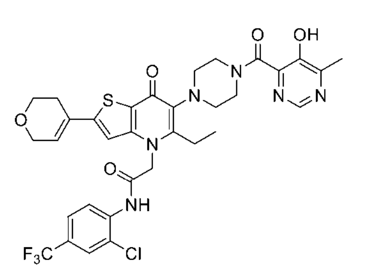
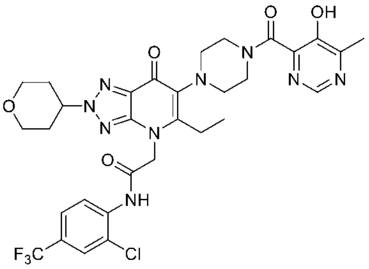

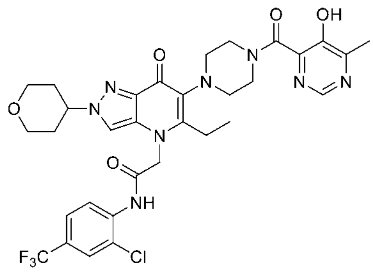
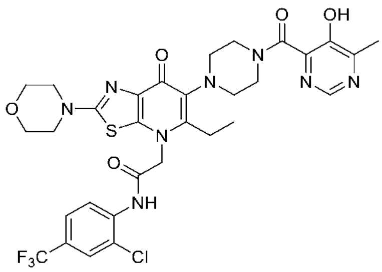
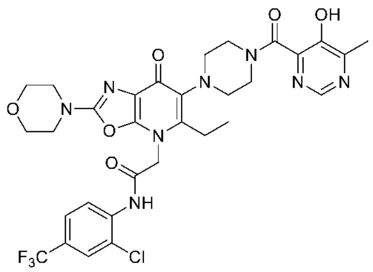

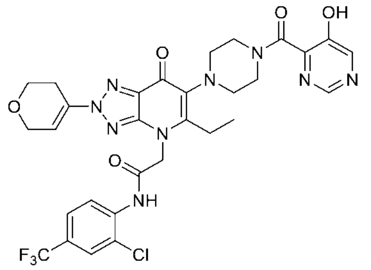
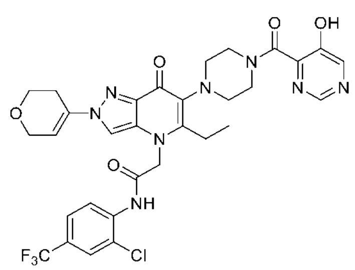
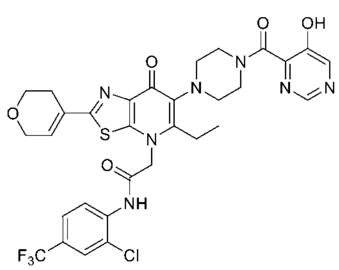

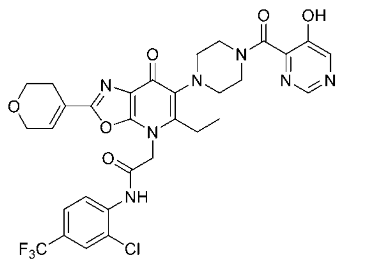
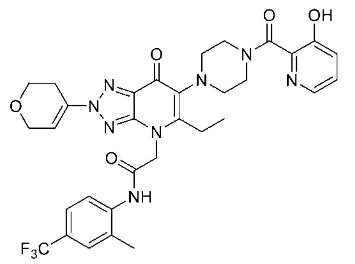
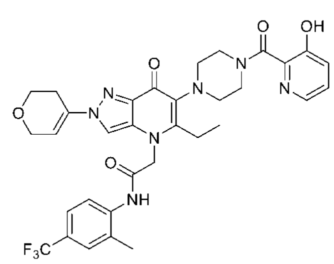

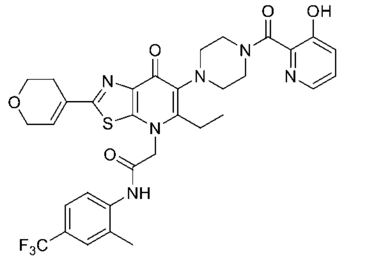
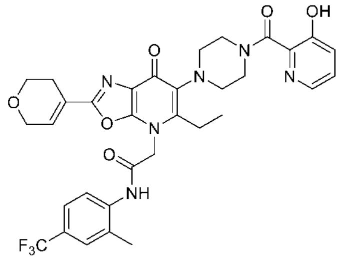
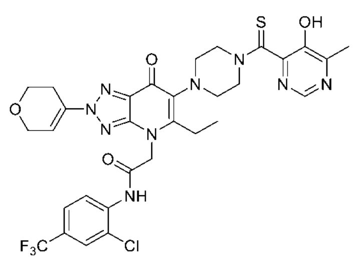

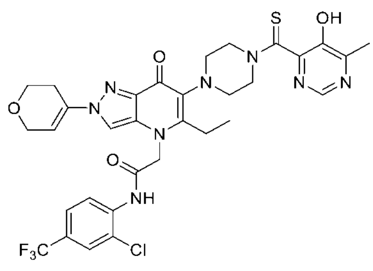
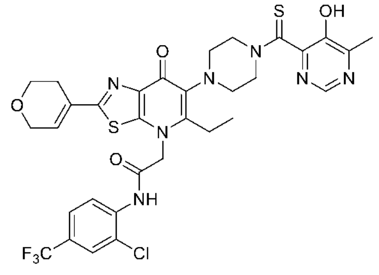
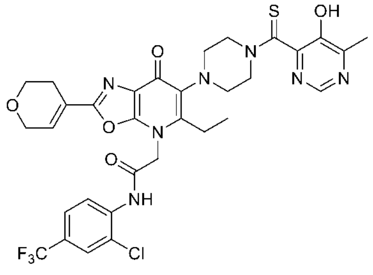

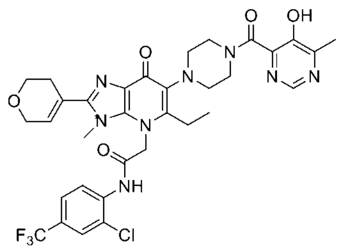
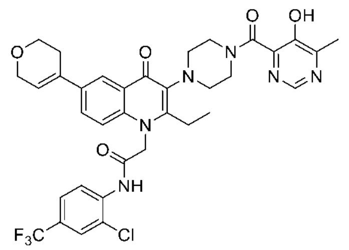
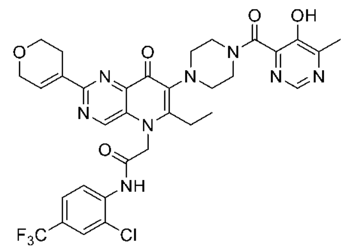
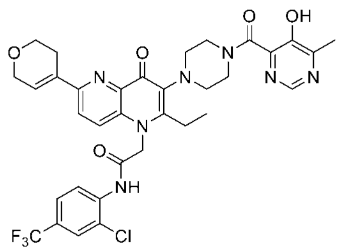
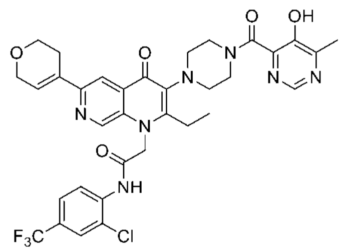
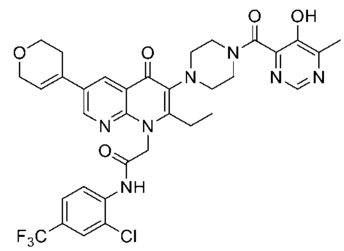
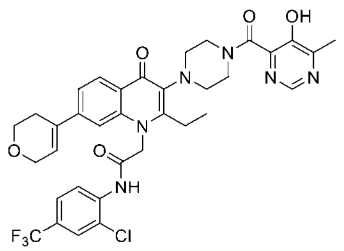
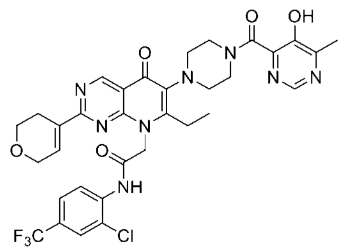
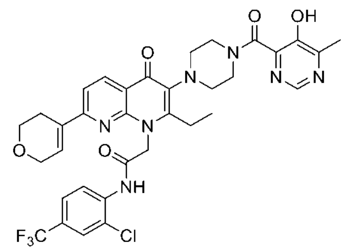
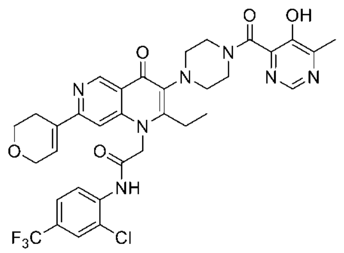
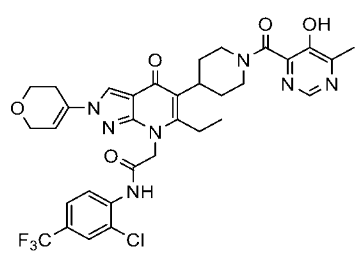
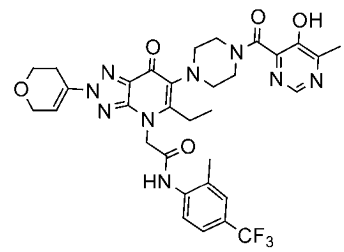
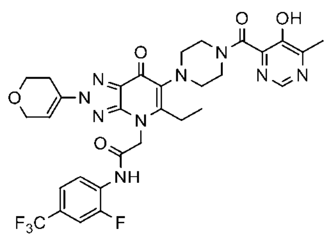
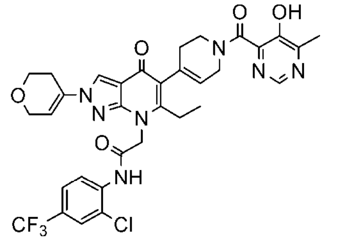
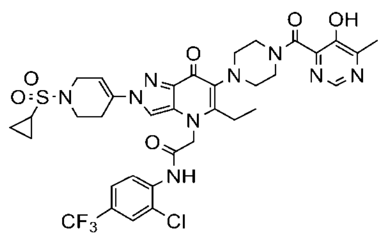
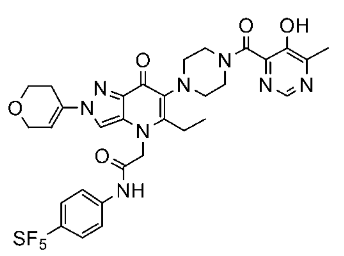
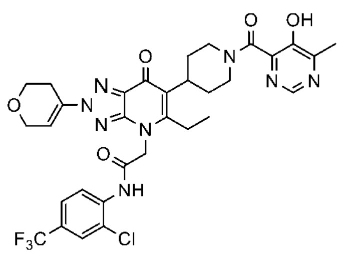
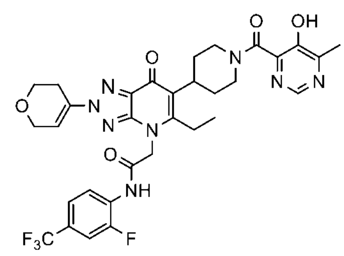

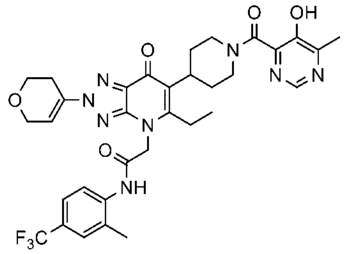
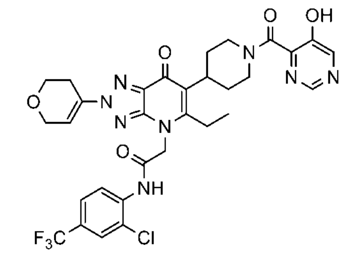
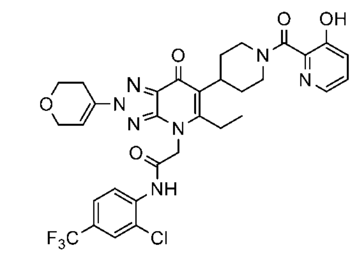

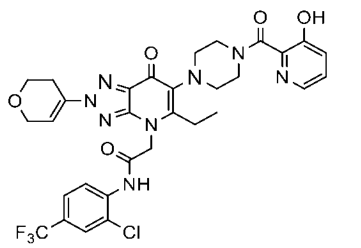
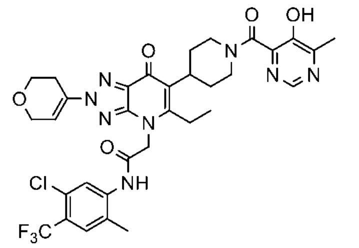
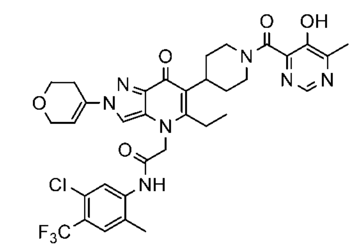

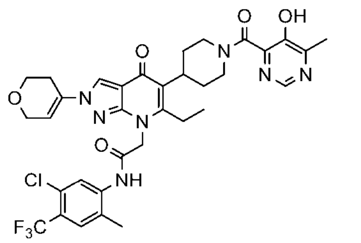
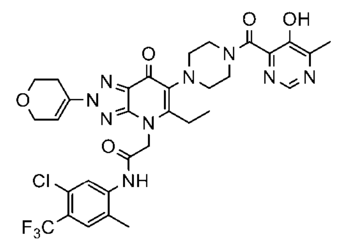
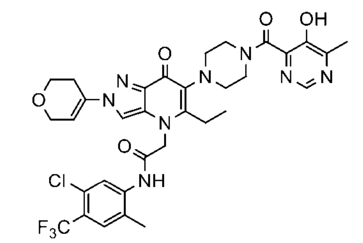

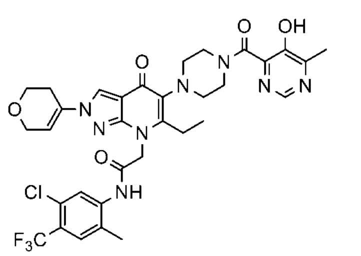
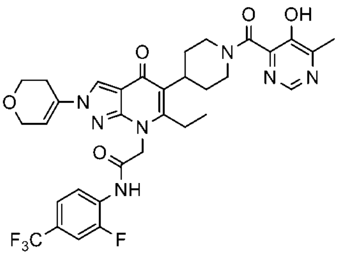
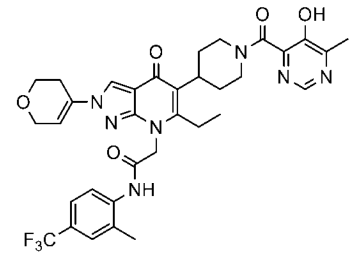

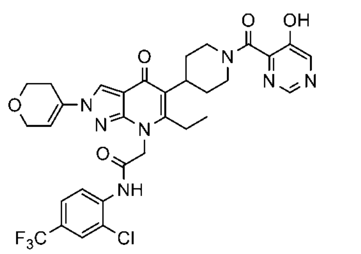
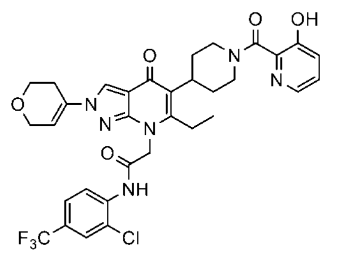
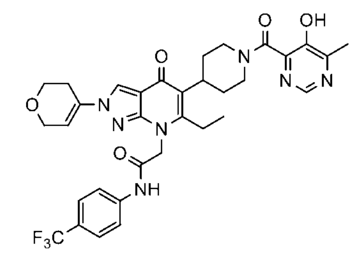

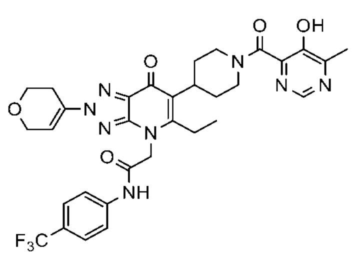
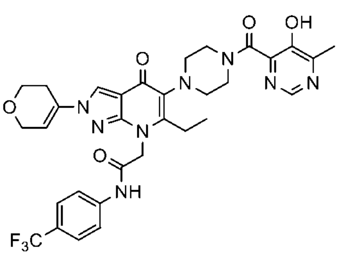
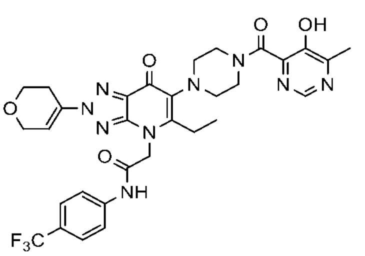

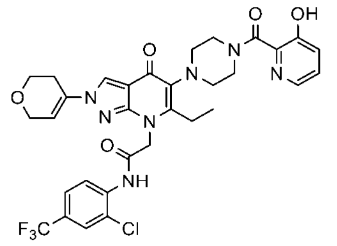
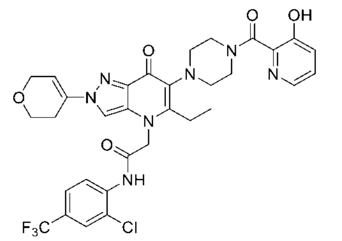
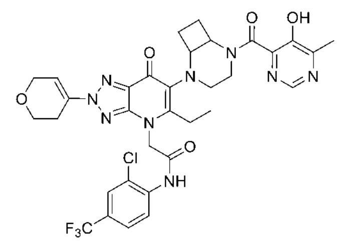

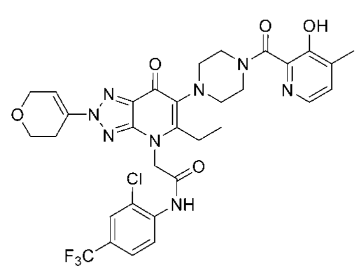
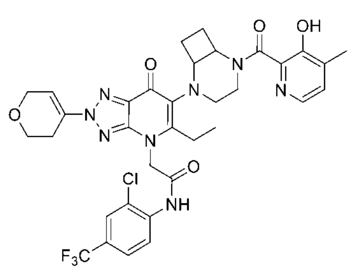
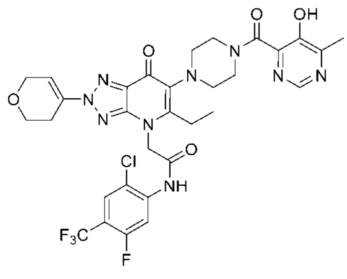

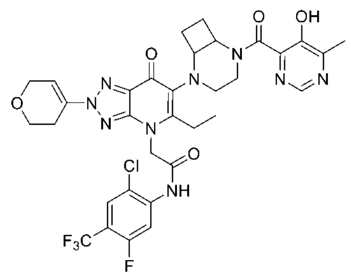
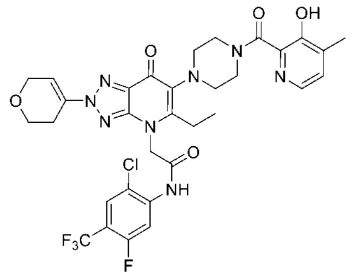
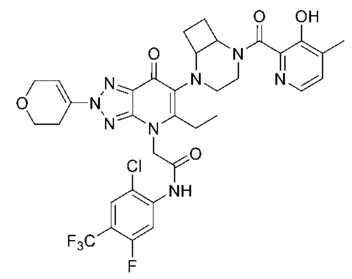

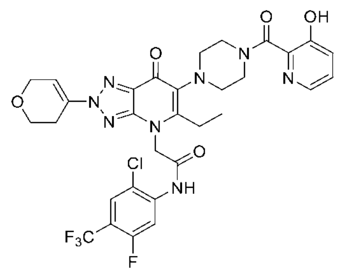
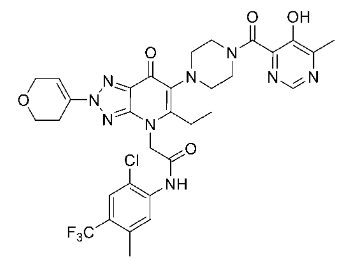
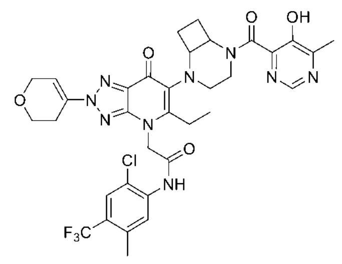

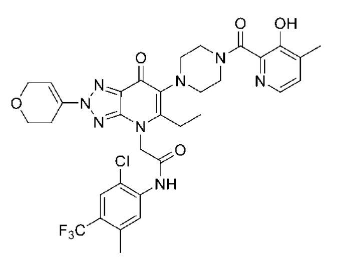
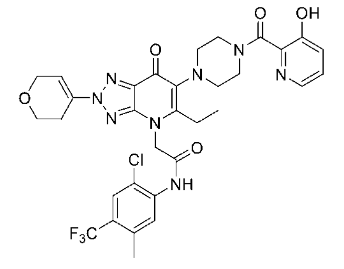
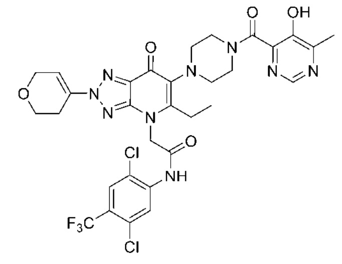

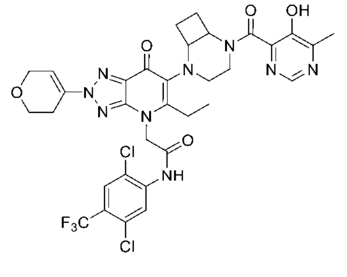
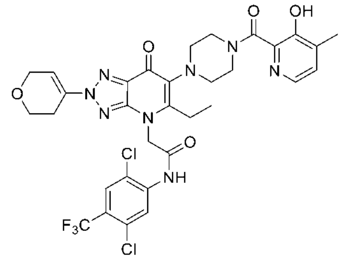
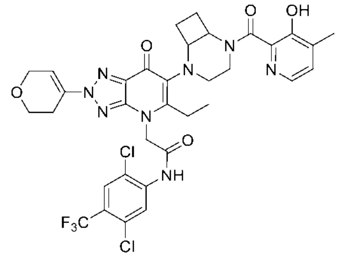

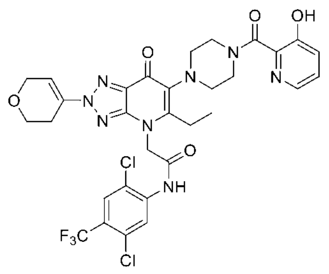
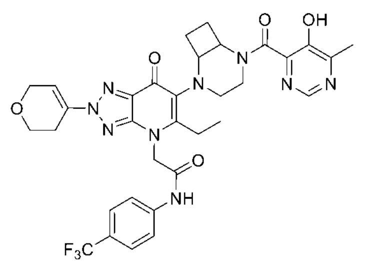
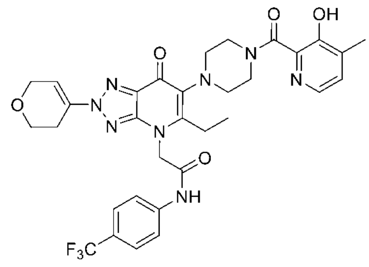

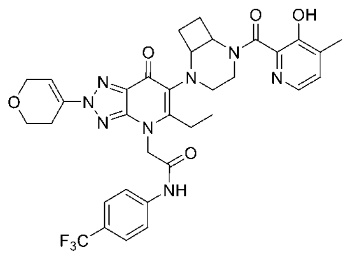
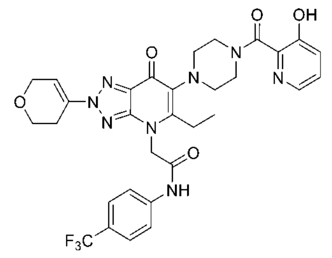
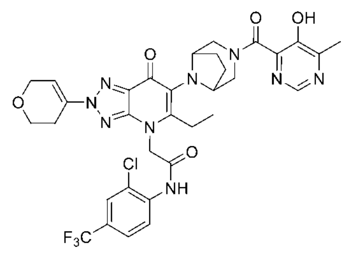

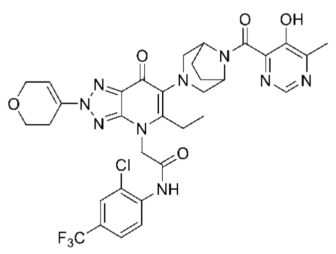
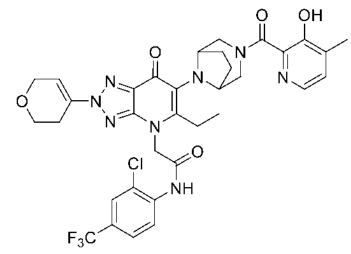
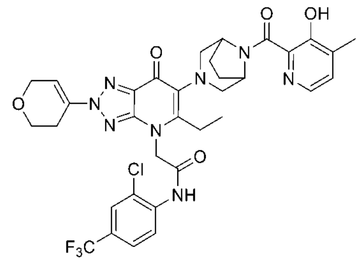

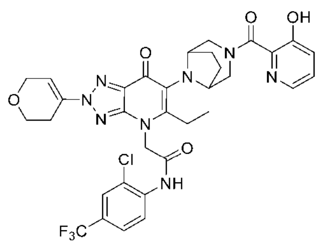
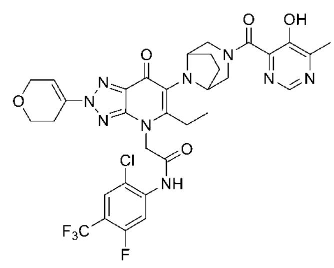
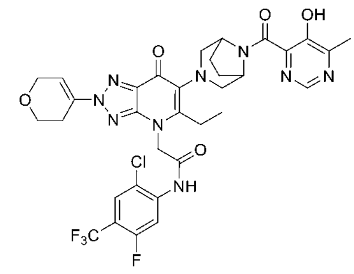

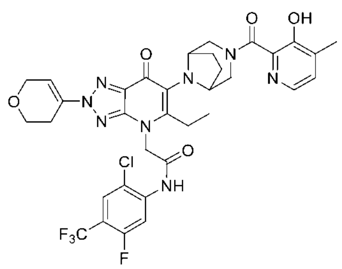
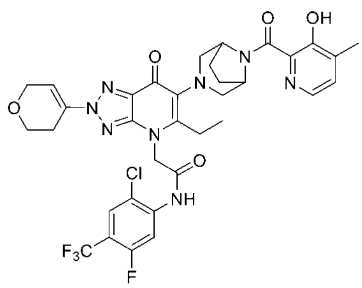
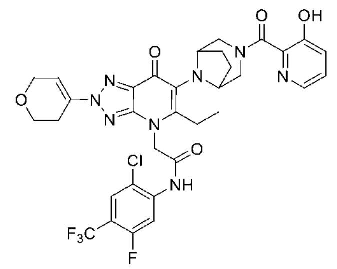

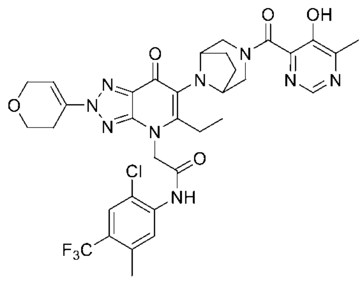
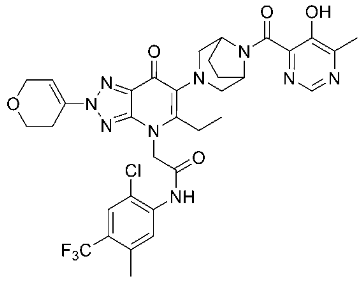
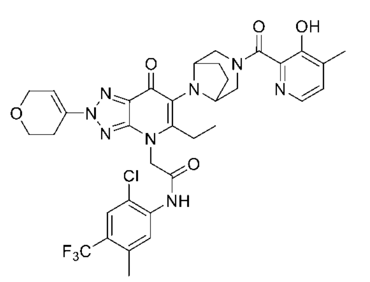

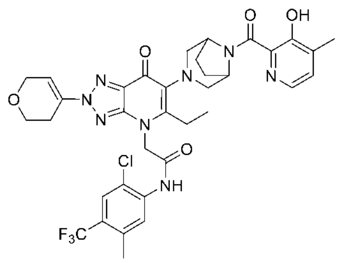
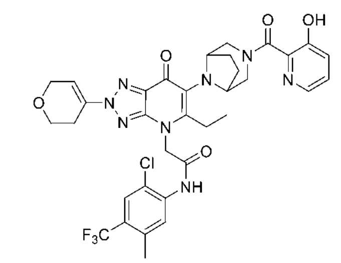
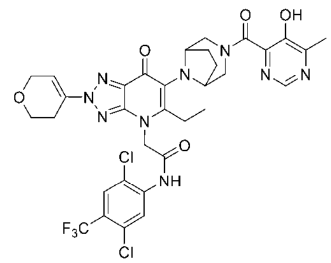

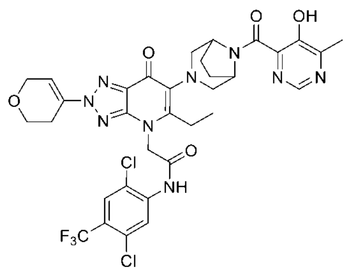
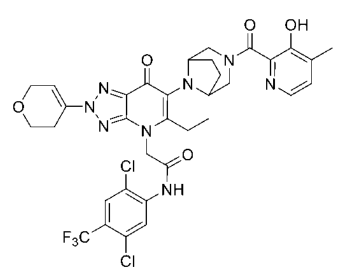
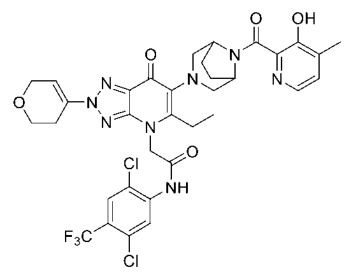

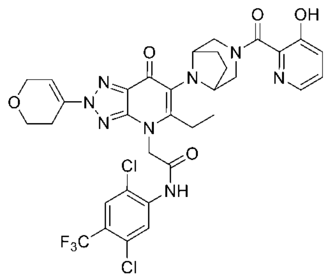
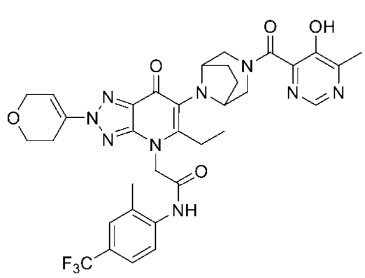
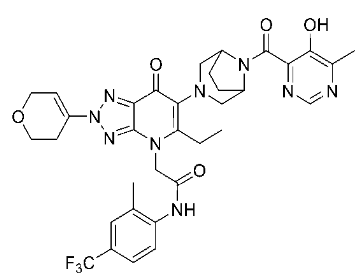

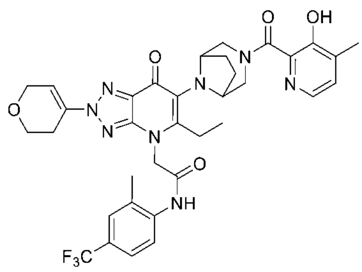
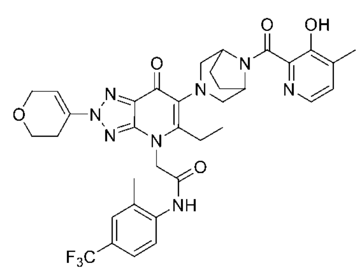
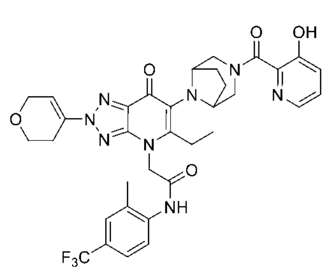

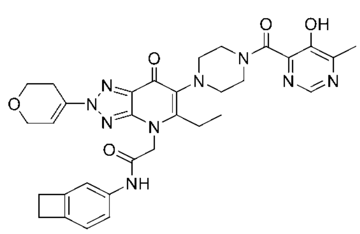
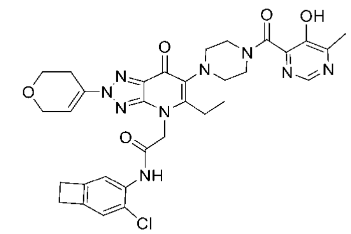
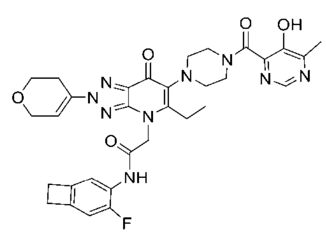

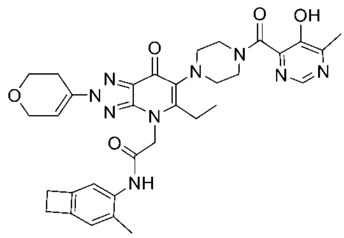
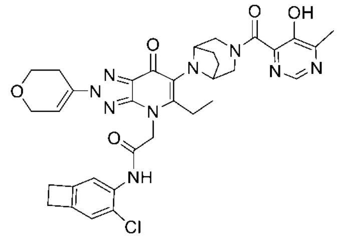
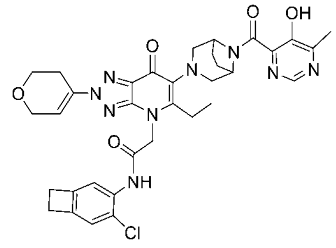

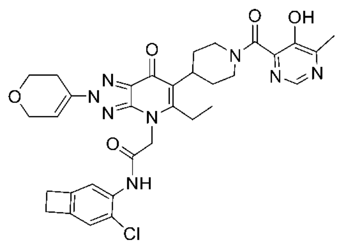
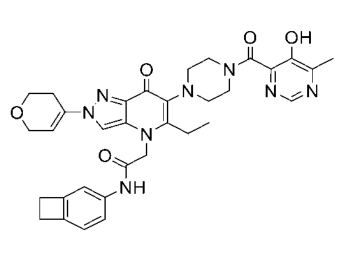
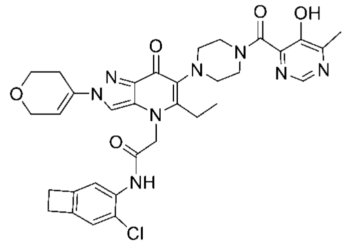

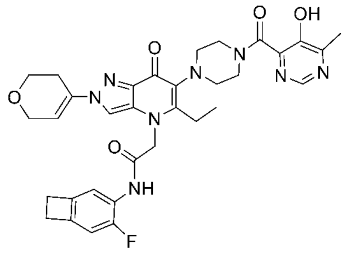
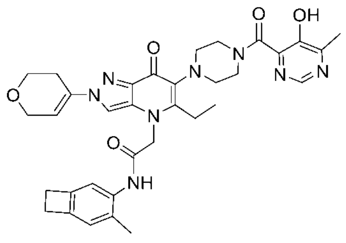
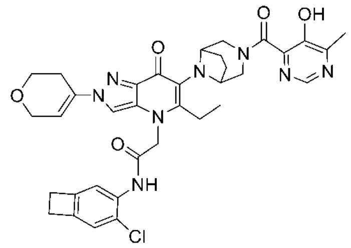

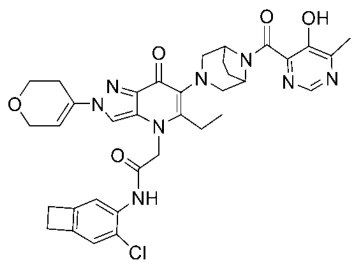
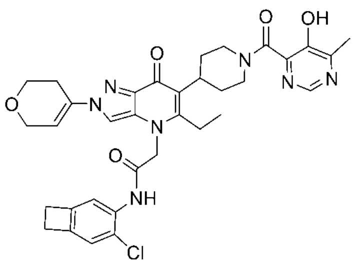
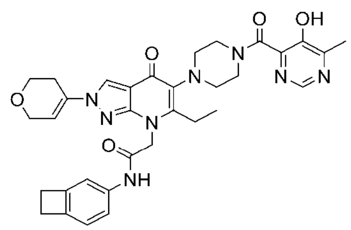

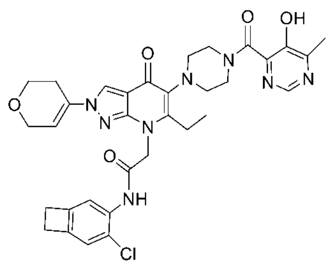
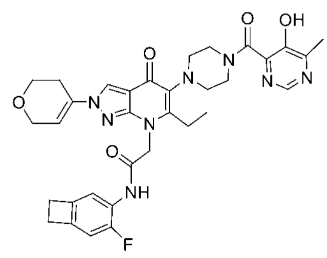
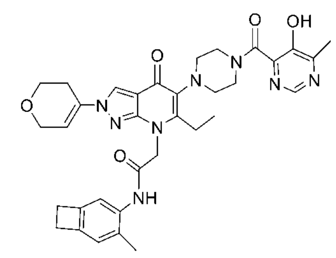

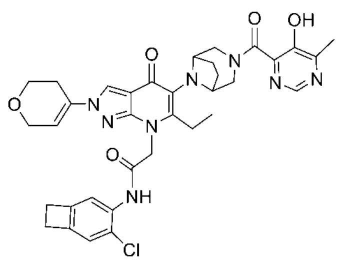
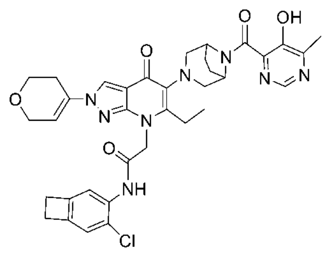
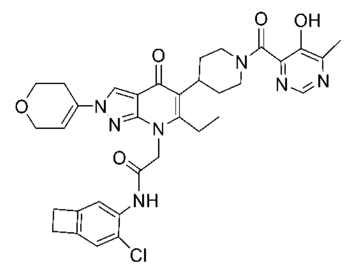

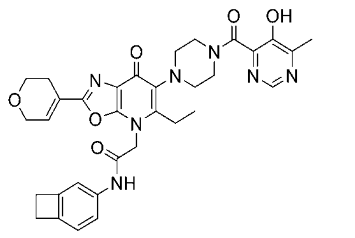
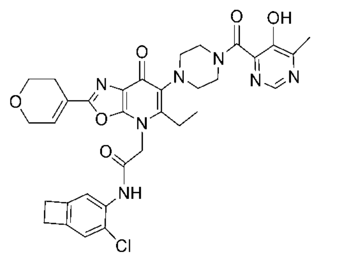
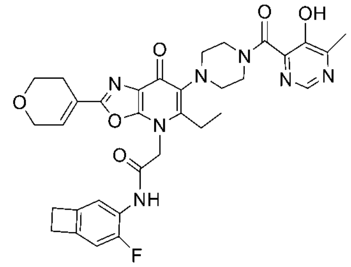

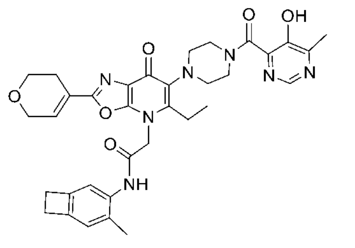
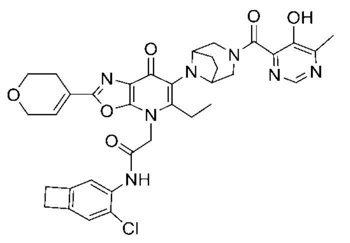
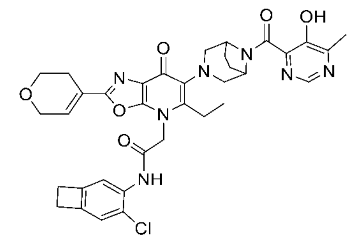

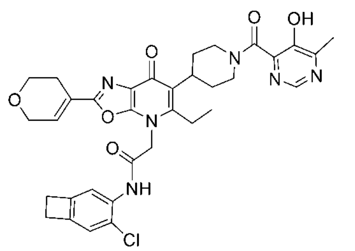
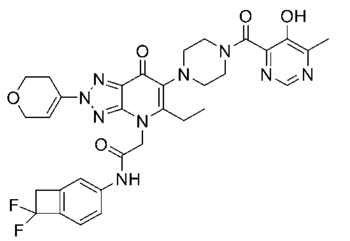
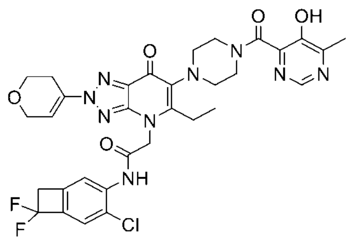

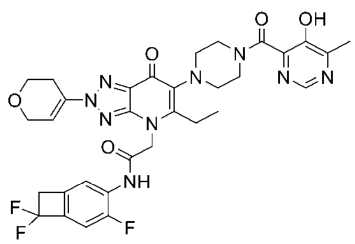
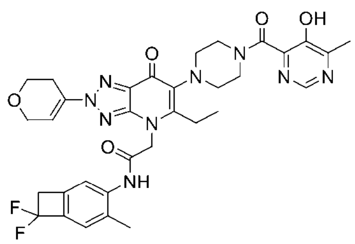
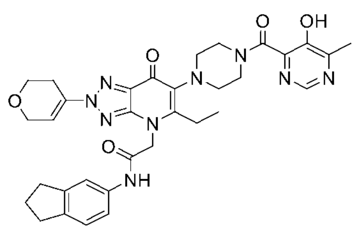

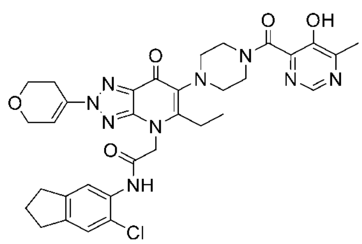
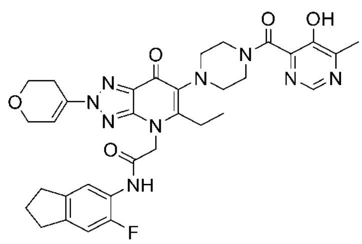
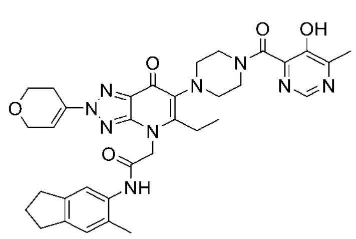

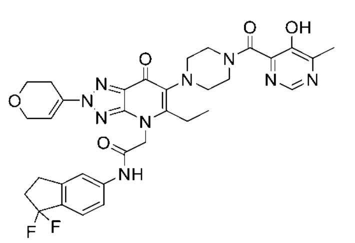
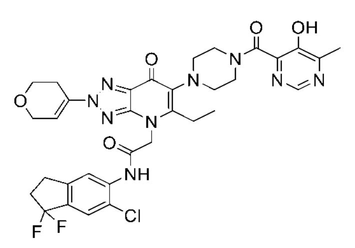
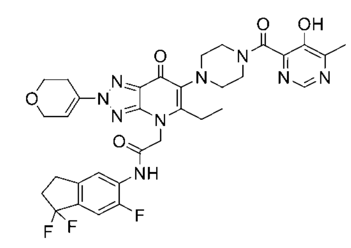

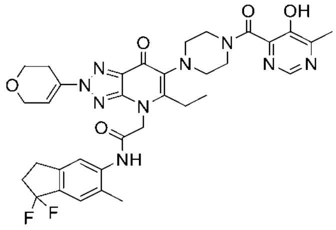

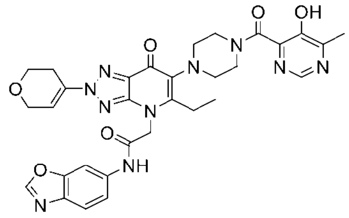

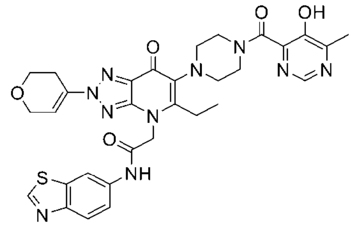

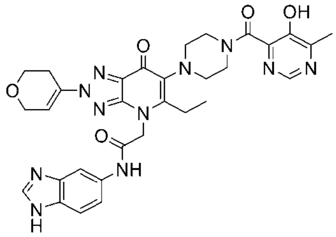

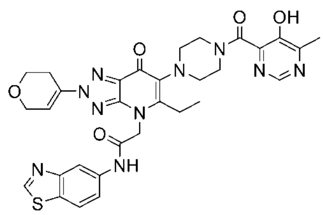

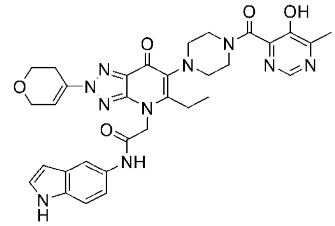

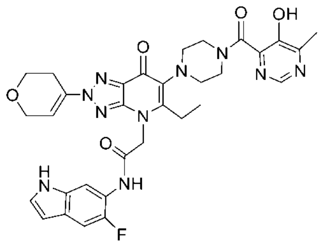

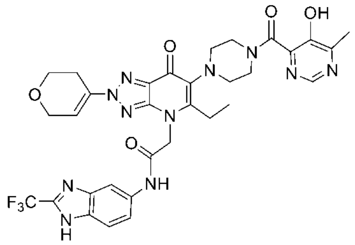

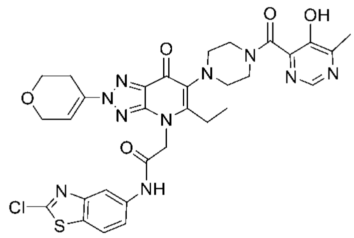

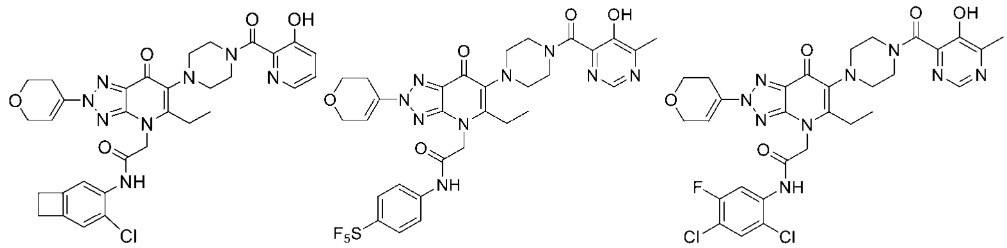

and

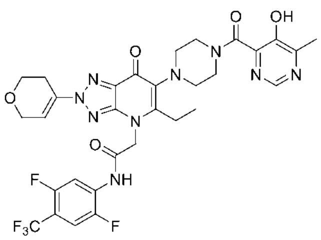

.

**37.** A pharmaceutical composition comprising the compound, the optical isomer thereof, or the pharmaceutically acceptable salt thereof according to any one of claims 1 to 36.

**38.** Use of the compound, the optical isomer thereof, or the pharmaceutically acceptable salt thereof according to any one of claims 1 to 36, or the pharmaceutical composition according to claim 37 in the manufacture of a medicament for treating a disease associated with a tumor.

**39.** The use according to claim 38, wherein the tumor is a microsatellite instability-high malignant tumor, a mismatch repair-deficient malignant tumor, or a malignant tumor detected with a large number of $(TA)_n$ repeat sequences.

**40.** The use according to claim 38, wherein the disease associated with the tumor is one or more of diseases associated

with solid tumors.

**41.** The use according to claim 38, wherein the disease associated with the tumor comprises one or more of colorectal cancer, gastric cancer, endometrial cancer, and ovarian cancer.

**INTERNATIONAL SEARCH REPORT**

International application No. **PCT/CN2024/083215**

**A. CLASSIFICATION OF SUBJECT MATTER**

C07D 487/04(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, VEN, ENTXTC, CNKI, 万方, WANFANG, 百度学术, BAIDU SCHOLAR, ISI web of Science, STN: 济煜医药, 济民可信, WRN, 解旋酶, 水解酶, werner, 抑制剂, helicase, Hydrolase, HRO761; 结构检索.

**C. DOCUMENTS CONSIDERED TO BE RELEVANT**

| Category* | Citation of document, with indication, where appropriate, of the relevant passages | Relevant to claim No. |
|---|---|---|
| A | WO 2022249060 A1 (NOVARTIS AG) 01 December 2022 (2022-12-01)<br>entire document, in particular abstract, embodiment 42, and claims 1-64 | 1-41 |
| A | CN 101528754 A (TRUSTEES OF BOSTON UNIVERSITY et al.) 09 September 2009 (2009-09-09)<br>entire document | 1-41 |
| A | Joshua A. Sommers et al. "A high-throughput screen to identify novel smallmolecule inhibitors of the Werner Syndrome Helicase-Nuclease (WRN)"<br>*Plos One*, Vol. 14, No. 1, 09 January 2019 (2019-01-09), e0210525<br>ISSN: 1932-6203,<br>figures 1-4 and 8 | 1-41 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 June 2024** | **28 June 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/083215**

| Patent document cited in search report | Publication date (day/month/year) | Patent family member(s) | Publication date (day/month/year) |
|---|---|---|---|
| WO 2022249060 A1 | 01 December 2022 | CA 3219799 A1 | 01 December 2022 |
| | | UY 39786 A | 31 January 2023 |
| | | TW 202313616 A | 01 April 2023 |
| | | PE 20240587 A1 | 21 March 2024 |
| | | DOP 2023000256 A | 31 January 2024 |
| | | BR 112023024551 A2 | 15 February 2024 |
| | | IL 308229 A | 01 January 2024 |
| | | CO 2023018119 A2 | 29 December 2023 |
| | | AU 2022279728 A1 | 16 November 2023 |
| | | ECSP 23095674 A | 31 January 2024 |
| | | AR 125966 A1 | 30 August 2023 |
| | | KR 20240013168 A | 30 January 2024 |
| | | EP 4347596 A1 | 10 April 2024 |
| | | US 2023046859 A1 | 16 February 2023 |
| | | US 11878973 B2 | 23 January 2024 |
| CN 101528754 A | 09 September 2009 | JP 2010502644 A | 28 January 2010 |
| | | CA 2664433 A1 | 06 March 2008 |
| | | US 2010093716 A1 | 15 April 2010 |
| | | WO 2008027990 A1 | 06 March 2008 |
| | | AU 2007289232 A1 | 06 March 2008 |
| | | IL 196951 A0 | 18 November 2009 |
| | | EP 2061797 A1 | 27 May 2009 |
| | | EP 2061797 A4 | 28 July 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023103018345
- CN 202311004828 X
- CN 2023110500915
- CN 2024102950619
- WO 2023284837 A
- WO 2022249060 A1